(19) Europäisches Patentamt
European Patent Office
Office européen des brevets

(11) Publication number: **0 668 352 A1**

(12) # EUROPEAN PATENT APPLICATION

(21) Application number: **95200385.3**

(22) Date of filing: **14.02.95**

(51) Int. Cl.[6]: **C12N 15/19**, C07K 14/52, C07K 16/24, A61K 38/19

(30) Priority: **14.02.94 JP 39090/94**
**25.03.94 JP 79842/94**
**01.06.94 JP 155126/94**
**15.06.94 JP 167328/94**
**17.08.94 JP 227159/94**
**01.11.94 JP 304167/94**
**28.12.94 JP 341200/94**
**17.08.94 JP 193169/94**
**01.12.94 JP 298669/94**
**18.08.94 JP 193916/94**
**14.03.94 US 212164**
**01.04.94 US 221020**
**20.07.94 US 278083**
**11.10.94 US 320300**
**22.12.94 US 361811**
**31.01.95 US 318478**

(43) Date of publication of application:
**23.08.95 Bulletin 95/34**

(84) Designated Contracting States:
**AT BE CH DE DK ES FR GB GR IE IT LI LU MC NL PT SE**

(71) Applicant: **Kirin Brewery Company, Ltd.**
**10-1, 2-chome, Shinkawa,**
**Chuo-ku**
**Tokyo (JP)**

(72) Inventor: **Miyazaki, Hiroshi**
**817-17 Shimonojo-machi**
**Takasaki-shi**
**Gunma-ken (JP)**
Inventor: **Kato, Takashi**
**819-8 Shimonojo-machi**
**Takasaki-shi**
**Gunma-ken (JP)**
Inventor: **Ohgami, Kinya**
**B-102 Shikishima-Ryo**
**2-37-16 Kami-Koide-machi**
**Maebashi-shi**
**Gunma-ken (JP)**
Inventor: **Iwamatsu, Akihiro**
**Belvedere A201**
**6-20-42 Tomiokahigashi**
**Kanazawa-ku**
**Yokohama-shi**
**Kanagawa-ken (JP)**
Inventor: **Akahori, Hiromichi**
**3439-78-2 Ishihara-machi**
**Takasaki-shi**
**Gunma-ken (JP)**
Inventor: **Kuroki, Ryota**
**13-20-2, Noukendai 5-chome**
**Kanazawa-ku**
**Yokohama.shi (JP)**
Inventor: **Shimizu, Toshiyuki**
**35-6-101, Noukendai 6-chome**
**Kanazawa-ku**
**Yokohama-shi**
**Kanagawa-ken (JP)**
Inventor: **Muto, Takanori**
**Kanazawahakkei-Ryo 102**
**2-8-4 Teramae**
**Kanazawa-ku**
**Yokohama-shi**
**Kanagawa-ken (JP)**

(74) Representative: **UEXKÜLL & STOLBERG**
**Patentanwälte**
**Beselerstrasse 4**
**D-22607 Hamburg (DE)**

(54) **Protein having TPO activity.**

(57) The present invention relates to thrombopoietin (TPO) polypeptides having the biological activity of specifically stimulating or increasing platelet production comprising the amino acid sequence 1-332 of SEQ ID NO: 6 or a derivative thereof, DNA molecules encoding TPO polypeptides, processes for production of the polypeptides, antibodies specifically immunoreactive with the polypeptides, pharmaceutical compositions com-

EP 0 668 352 A1

prising the polypeptides, and methods for using the polypeptides in treatment of platelet disorders such as thrombocytopenia.

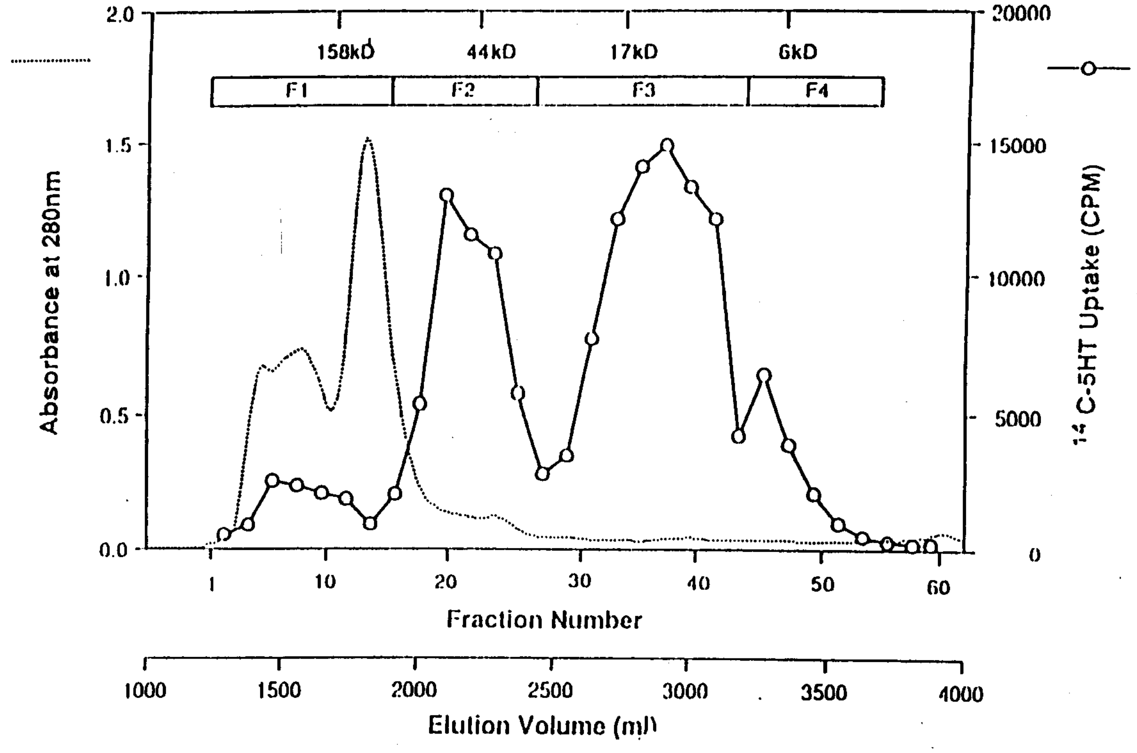


Figure 1

This application is a continuation-in-part of U.S. Patent Application Serial No. 08/361,811 filed December 22, 1994, now pending, which is a continuation-in-part of U.S. Patent Application Serial No. 08/320,300 filed October 11, 1994, now pending, which is a continuation-in-part of U.S. Patent Application Serial No. 08/278,083 filed July 20, 1994, now pending, which is a continuation-in-part of U.S. Patent Application Serial No. 08/221,020 filed April 1, 1994, now abandoned, which is a continuation-in-part of U.S. Patent Application Serial No. 08/212,164 filed March 14, 1994 now abandoned.

**FIELD OF THE INVENTION**

This invention relates to a novel protein which has an activity to stimulate or increase platelet production *in vivo* in a specific manner or to enhance proliferation and differentiation of megakaryocyte progenitor cells, to a DNA sequence coding for said protein, and to a process for the production of the same.

**BACKGROUND OF THE INVENTION**

Megakaryocytes are large cytoplasm-rich multinucleate cells which produce platelets and can be found mainly in bone marrow. Megakaryocytes originate from a pluripotent hematopoietic stem cell in bone marrow. A primitive pluripotent stem cell differentiates to some degree into megakaryocyte progenitor cells, which are committed to the megakaryocytic lineage. Megakaryocyte progenitor cells proliferate and differentiate into megakaryocytes. Megakaryocytes further undergo polyploidization and cytoplasmic maturation and finally release their anuclear cytoplasmic fragments, namely platelets, into the circulation. Two to four thousands of platelets are on an average formed from one mature megakaryocyte. Although the platelet formation mechanism is still unclear in many points, it is considered that megakaryocytes are typically localized on the albuminal surface of the sinus endothelium in the bone marrow and produce cytoplasmic processes that extend into the sinusoid where they undergo fragmentation of platelets.

There are many ambiguous points regarding the mechanism for generating the platelets though it is likely that the megakaryocytes are locally present in the dermal layer of the skin of the venous sinus of the bone marrow and that the cytoplasm comes through the dermal skin to result in a cord-like projection on the internal wall of the venous sinus whereby the platelets are discharged.

It has been suggested that there is a specific function for the production and the control of the megakaryocyte hematopoiesis platelet production. In healthy human beings and animals, the number of the effective platelets is maintained though it has been known that, upon administration of an antiplatelet antibody to the healthy animals for example, the platelet number decrease promptly, then they begin to increase temporarily to larger than usual and, finally, they return to the normal level. Also in the clinical field, it has been known that a decrease in the number of the platelets thrombocytopenia and thrombocytosis even when the numbers of erythrocytes and leucocytes are at a normal level.

Incidentally, the most important function of a platelet is the production of a thrombus in a hemostatic mechanism. If the hemostatic mechanism does not function properly due to a decrease in the platelet number, a tendency towards hemostasis results.

The presence of a specific regulatory mechanism has been suggested with regard to megakaryocytopoiesis and thrombopoiesis. Platelets are maintained in effective numbers in healthy people and normal animals. However, it is known that, when an anti-platelet antibody is administered to a normal animal, only the number of platelets decreases sharply within a short period of time, starts to increase thereafter and temporarily exceeds the normal level, but finally returns to the normal level. It is known also in the clinical field that a decrease in the number of platelets (thrombocytopenia) or an increase in their number (thrombocytosis) occurs even under normal numbers of erythrocytes and leucocytes. To date, however, success in the isolation and identification of a specific regulatory factor involved in platelet production (for example, like the case of erythropoietin in the erythrocyte formation) has not been reported.

Most important function of platelets is the formation of blood clot in the hemostatic mechanism. Bleeding tendency occurs when normal function of the hemostatic mechanism is spoiled by thrombocytopenia. In the field of radiotherapy and chemotherapy of cancers, thrombocytopenia caused by bone marrow suppression is a mortal complication, and platelet transfusion is applied to such patients in order to prevent bleeding tendency. Platelet transfusion is also applied to patients after bone marrow transplantation or of aplastic anemia.

Platelets for use in such platelet transfusion are prepared by plateletpheresis from blood of healthy blood donors, but such platelets for transfusion use have a short shelf life and a possibility of causing contamination by bacterial infection. The platelet transfusion also has a possible danger of exposing patients to dangerous viruses such as human immunodeficiency virus (HIV) or various hepatitis viruses, of inducing antibodies specific for a major histocompatibility antigen (HLA) of the transfused platelets or of causing graft versus host disease (GVHD) due to contaminated lymphocytes in the platelets for transfusion use.

In consequence, it will be of great benefit if intrinsic platelet formation can be stimulated in thrombocytopenic patients and, at the same time, their platelet transfusion dependency can be reduced. In addition, if thrombocytopenia in cancer patients undergoing radiotherapy or chemotherapy can be corrected or prevented, such treatments can be made more safer, the intensity of the treatment can possibly be increased, and further improvement of the anti-cancer effects can be expected.

For these reasons, a number of intensive studies have been made on the isolation and identification of specific regulatory factors involved in the regulation of megakaryocyte and platelet production. According to *in vitro* studies, regulatory factors governing megakaryocytopoiesis are roughly divided into the following two factors (cf. Williams *et al.*, *J. Cell. Physiol.*, vol.110, pp.101 - 104, 1982). Megakaryocyte colony stimulating factor (Meg-CSF) is a regulatory factor which stimulates proliferation and differentiation of CFU-MK to form megakaryocyte colonies in a semi-solid culture medium. The other regulatory factor, called megakaryocyte potentiating factor (Meg-Pot), megakaryocyte stimulating factor, thrombopoiesis stimulating factor or the like, acts mainly upon immature or mature megakaryocytes thereby enhancing their differentiation and maturation. The Meg-Pot is detectable in combination with Meg-CSF activity in some cases. In addition, since platelet counts increased when serum or plasma collected from experimentally induced thrombocytopenic animals was administered to other normal animals, it has been suggested that there is a humoral factor, called thrombopoietin (TPO), capable of promoting platelet production *in vivo*.

In recent years, some cytokines whose genes have been cloned were examined for their abilities to stimulate megakaryocytopoiesis and thrombopoiesis. Human IL-3 stimulated formation of human megakaryocyte colonies (Bruno *et al.*, *Exp. Hematol.*, vol.16, pp.371 - 377, 1988) and, at least in monkey, increased platelet counts (Donahue *et al.*, *Science,* vol.241, p.1820, 1988). However, since IL-3 is a factor which exerts its effects upon proliferation and differentiation of all hematopoietic cells, it can be distinguished from specific regulatory factors controlling megakaryocytopoiesis and platelet production. Human IL-6 did not show Meg-CSF activity, but acted upon immature megakaryocytes and then enhanced their differentiation into mature megakaryocytes (Williams *et al.*, *Exp. Hematol.*, vol.18, p.69, 1990). *In vivo* administration of IL-6 induced platelet production and promoted both maturation and shifted to higher ploidy of bone marrow megakaryocytes in primates, but also caused side effects such as reduction of body weight, induction of acute phase protein (Asano *et al.*, *Blood*, vol.75, pp.1602 - 1605, 1990; *Stahl et al.*, *Blood*, vol.78, pp.1467 - 1475, 1991). Human IL-11 showed no Meg-CSF activity, but exerted Meg-Pot activity and promoted platelet production in mice (Neben *et al.*, *Blood*, vol.81, pp.901 - 908, 1993). In addition, human LIF significantly increased platelet counts in primates (Mayer *et al.*, *Blood*, vol.81, pp.3226 - 3233, 1993), but its *in vitro* action upon megakaryocytes was weak (Burstein *et al.*, *J. Cell. Physiol.*, vol.153, pp.305 - 312, 1992).

Although clinical application of these cytokines as platelet increasing factors is expected, their functions are not specific for the megakaryocyte lineage and they cause side effects. Therefore, development of a platelet increasing factor which is specific for the megakaryocyte-platelet system and causes less side effects has been called for in the clinical field.

Meg-CSF, Meg-pot or TPO activity has been found in serum, plasma or urine of thrombocytopenic patients or animals, or in culture supernatant of certain human cultured cell lines. However, whether these activities are due to the presence of a single factor or a combination of several factors or whether they are different from known cytokines is presently unknown.

Hoffman *et al.* have found that sera of patients with aplastic anemia and amegakaryocytic thrombocytopenic purpura contained a Meg-CSF activity that significantly augmented human megakaryocyte colony formation (Hoffman *et al.*, *N. Eng. J. Med.*, vol.305, pp.533 - 538, 1981). Thereafter, Mazur *et al.* have reported that the Meg-CSF activity present in the serum of aplastic anemia patients was distinct from both IL-3 and GM-CSF (Mazur *et al.*, *Blood*, vol.76, pp.290 - 297, 1990). Similar Meg-CSF activity has been found in sera of cancer patients receiving intensive cytotoxic chemotherapy and bone marrow-transplant patients (Mazur *et al.*, *Exp. Hematol.*, vol.12, pp.624 - 628, 1984; de Alarcon and Schmieder, *Prog. Clin. Bio. Res.*, vol.215, pp.335 - 340, 1986). Hoffman *et al.* have reported that Meg-CSF with an apparent molecular weight of 46,000 was purified from plasma of patients with hypomegakaryocytic thrombocytopenia (Hoffman *et al.*, *J. Clin. Invest.*, vol.75, pp.1174 - 1182, 1985), but it was found by further studies that the material was not at a level of purity to allow accurate amino acid sequencing (Hoffman, *Blood*, vol.74, pp.1196 - 1212, 1989). A substance having TPO-like activity which enhanced incorporation of $^{75}$Se-selenomethionine into newly forming platelets in mice has been partially purified from plasma of thrombocytopenic patients or urine of patients with idiopathic thrombocytopenic purpura (ITP), and apparent molecular weight of the plasma-derived factor was determined to be 40,000 (Grossi *et al.*, *Hematologica*, vol.72, pp.291 - 295, 1987; Vannucchi *et al.*, *Leukemia,* vol.2, pp.236 - 240, 1988).

Meg-CSF and TPO-like activities have also been detected in urine samples of patients with aplastic anemia and severe ITP (Kawakita *et al.*, *Br. J. Haematol.*, vol.48, pp.609 - 615, 1981; Kawakita *et al.*, *Blood*, vol.556 - 560, 1983). Kawakita *et al.* have further reported that the Meg-CSF activity found in the urine extract of aplastic anemia patients showed an apparent molecular weight of 45,000 by gel filtration under a dissociation condition (Kawakita *et al.*, *Br. J. Haematol.*, vol.62, pp.715 - 722, 1986). Erikson-Miller *et al.* have also reported on the purification of Meg-CSF from similar urinary samples, but with no information on its structure (Erikson-Miller *et al.*, "Blood Cell Growth Factors: their present and future use in hematology and oncology" ed. by Murphy, AlphaMed Press, Dayton, Ohio, pp.204 - 220, 1992). Turner *et al.* have purified a megakaryocyte stimulating factor (MSF) having Meg-CSF activity from the urine of bone marrow transplant patients and cloned its gene (Turner *et al.*, *Blood*, vol.78, p.1106 279a, 1991, (abstr.,supple. 1)). This MSF has a molecular weight of 28,000 to 35,000. Identity of this factor with the Meg-CSF so far detected in serum and plasma samples of thrombocytopenic patients and its platelet increasing activity remain to be elucidated.

A substance having a TPO-like activity with a molecular weight of 32,000 has been purified from the culture supernatant of a human embryonal kidney-derived cell line (HEK cells) and its biological and biochemical properties have extensively been examined, but its structure is still unknown (McDonald *et al.*, *J. Lab. Clin. Med.*, vol.106, pp.162 - 174, 1985; McDonald, *Int. J. Cell Cloning*, vol.7, pp.139 - 155, 1989). On the contrary, other researchers have reported that the main activity in the conditioned medium of the HEK cells, which enhances megakaryocyte maturation *in vitro*, is due to known cytokines, namely IL-6 and EPO (Withy *et al.*, *J. Cell. Physiol.*, vol.15, pp.362 - 372, 1992).

With regard to animal-derived factors, Evatt *et al.* have reported that a TPO-like activity which enhanced incorporation of $^{75}$Se-selenomethionine into newly forming platelets in rabbits and mice was found in plasma of thrombocytopenic rabbits induced by antiplatelet serum injection (Evatt *et al.*, *J. Lab. Clin. Med.*, vol.83, pp.364 - 371, 1974). In addition to this, a number of similar studies have been reported from the 1960's to 1970's (for example, Odell *et al.*, *Proc. Soc. Biol. Med.*, vol.108, pp.428 - 431, 1961; Evatt and Levin, *J. Clin. Invest.*, vol.48, pp.1615 - 1626, 1969; Harker, *Am. J. Physiol.*, vol.218, pp.1376 - 1380, 1970; Shreiner and Levin, *J. Clin. Invest.*, vol.49, pp.1709 - 1713, 1970; and Penington, *Br. Med. J.*, vol.1, pp.606 - 608, 1070). Evatt *et al.*, and Hill and Levin have conducted partial purification of a TPO-like activity from plasma of thrombocytopenic rabbits (Evatt *et al.*, *Blood*, vol.54, pp.377 - 388, 1979; Hill and Levin, *Exp. Hematol.*, vol.14, pp.752 - 759, 1986). Thereafter, purification of this factor was continued, monitoring an activity to enhance differentiation and maturation of megakaryocytes *in vitro*, namely Meg-Pot activity, to reveal that this activity has an apparent molecular weight of 40,000 to 46,000 when determined by gel filtration (Keller *et al.*, *Exp. Hematol.*, vol.16, pp.262 - 267, 1988; Hill *et al.*, *Exp. Hematol.*, vol.20, pp.354 - 360, 1992). Since IL-6 activity was not detectable in plasma of rabbits with severe acute thrombocytopenia induced by antiplatelet serum administration, it was suggested that this TPO-like activity would be due to a factor other than IL-6 (Hill *et al.*, *Blood*, vol.80, pp.346 - 351, 1992).

Tayrien and Rosenberg have also purified a factor having an apparent molecular weight of 15,000 from thrombocytopenic rabbit plasma and culture supernatant of HEK cells, which stimulates production of platelet factor 4 in a rat megakaryocytic cell line, but with no information on its structure (Tayrien and Rosenberg, *J. Biol. Chem.*, vol.262, pp.3262 - 3268, 1987).

In addition, Nakeff has found a Meg-CSF activity in serum of thrombocytopenic mice induced by anti-platelet administration (Nakeff, "Experimental Hematology Today" ed. by Baum and Ledney, Springer-Verlag, NY. pp. 111 - 123, 1977). On the other hand, sera from thrombocytopenic rabbits enhanced maturation of megakaryocytes (Keller *et al.*, *Exp. Hematol.*, vol.16, pp.262 - 267, 1988; Hill *et al.*, *Exp. Hematol.*, vol. 17, pp.903 - 907, 1989) and stimulated morphological change of megakaryocytes into platelets (Leven and Yee, *Blood*, vol.69, pp.1046 - 1052, 1989), but had no detectable Meg-CSF activity.

Miura *et al.* have detected a Meg-CSF activity in plasma of rats rendered thrombocytopenic by sublethal whole body irradiation (Miura *et al.*, *Blood*, vol.63, pp.1060 - 1066, 1984), and suggested that induction of the Meg-CSF activity *in vivo* is related to decreased megakaryocytes, but not decreased platelets, because this activity did not change by platelet transfusion (Miura *et al., Exp. Hematol.*, vol.16, pp.139 - 144, 1988). Mazur and South have detected a Meg-CSF activity in serum of sublethally irradiated dogs and reported that this factor has an apparent molecular weight of 175,000 when measured by gel filtration (Mazur and South, *Exp. Hematol.*, vol.13, pp.1164 - 1172, 1985). In addition to the above, serum-, plasma- and urine-derived factors have been reported by other investigators for example by Straneva *et al.* (Straneva *et al.*, *Exp. Hematol.*, vol.15, pp.657 - 663, 1987).

Thus, as described above, various activities that stimulate megakaryocytopoiesis and thrombopoiesis have been found in biological samples derived from thrombocytopenic patients and animals, but isolation of these factors, their biochemical and biological identification and determination of their characteristics have not been achieved because of their extremely small contents in natural sources such as blood and urine.

## SUMMARY OF THE INVENTION

Objects of the present invention are to isolate a TPO protein from natural sources and identify it, the TPO protein having an activity to stimulate or increase platelet production in vivo and/or to enhance proliferation and differentiation of megakaryocyte progenitor cells (hereinafter, referred to as "TPO activity"); and to isolate a gene coding for the TPO protein and to provide a process for the production of said protein in a homogeneous quality and in a large quantity with recombinant DNA techniques. Success in accomplishing such objects will lead to the substitution or frequency-reduction of the currently used platelet transfusion, and such a novel protein will also be used for treatment and diagnosis of platelet disorders.

The present invention, therefore, relates to (i) a purified and isolated DNA sequence encoding a protein having TPO activity, which is selected from the group consisting of:

(a) the DNA sequences shown in SEQ ID NOs: 194, 195 and 196, or complementary strands thereof; and

(b) DNA sequences which hybridize under stringent conditions to the DNA sequences as defined in (a) or fragments thereof; and

(c) DNA sequences which would hybridize to the DNA sequences as defined in (a) and (b), but for the degeneracy of the genetic code,

(ii) to a process for the production of a protein having TPO activity, which comprises the steps of:

growing, under suitable nutrient conditions, procaryotic or eucaryotic host cells transformed or transfected with said DNA sequence in a manner enabling expression of the protein; and

isolating the desired protein product obtained by expression of said DNA sequence, and

(iii) to a protein product obtained by expression in a procaryotic or eucaryotic host cell of said DNA sequence.

The present invention further relates to a pharmaceutical composition comprising an effective amount of said protein having TPO activity, and to a method for treating platelet disorders, especially thrombocytopenia, comprising administering said protein to patients having the disorders.

## BRIEF DESCRIPTION OF THE DRAWINGS

Figure 1 shows Sephacryl S-200HR gel filtration chromatography of Phenyl Sepharose 6 FF/LS F2 derived from XRP.

Figure 2 shows Capcell Pak C1 reverse phase chromatography of YMC-pack CN-AP TPO-active fraction derived from low molecular TPO sample (Sephacryl S-200HR F3) of XRP.

Figure 3 shows a SDS-PAGE analysis of Capcell Pak C1 TPO-active fraction (FA) from low molecular TPO sample of XRP.

Figure 4 shows peptide maps on C18 reverse phase HPLC of rat TPO isolated by SDS-PAGE. The peptide fragments were obtained by systematic hydrolysis with three proteases.

Figure 5 shows TPO activity derived from XRP in the rat CFU-MK assay system.

Figure 6 shows a construction of expression vector pEF18S.

Figure 7 shows TPO activity in the culture supernatant of COS1 cells into which pEF18S-A2α was introduced in the rat CFU-MK assay system.

Figure 8 shows TPO activity in the culture supernatant of COS1 cells into which pEF18S-HL34 was introduced in the rat CFU-MK assay system.

Figure 9 shows TPO activity in the culture supernatant of COS1 cells into which pHT1-231 was introduced in the rat CFU-MK assay system.

Figure 10a shows TPO activity in the culture supernatant of COS1 cells into which pHTF1 was introduced in the rat CFU-MK assay system.

Figure 10b shows TPO activity in the culture supernatant of COS1 cells into which pHTF1 was introduced in the M-07e assay system.

Figure 11 shows a restriction map of the λHGT1 clone and construction of pHGT1 and pEFHGTE.

(E: EcoRI, H: HindIII, S: SalI)

Figure 12a shows TPO activity in the culture supernatant of COS1 cells into which pEFHGTE was introduced in the rat CFU-MK assay system.

Figure 12b shows TPO activity in the culture supernatant of COS1 cells into which pEFHGTE was introduced in the M-07e assay system.

Figure 13a shows TPO activity in the culture supernatant of COS1 cells into which pHT1-211#1, pHT1-191#1, or pHT1-171#2 was introduced in the rat CFU-MK assay system.

Figure 13b shows TPO activity in the culture supernatant of COS1 cells into which pHT1-163#2 was introduced in the rat CFU-MK assay system.

Figure 14 shows TPO activity in the culture supernatant of COS1 cells into which pHT1-211#1, pHT1-191#1, pHT1-171#2, or pHT1-163#2 was introduced in the M-07e assay system.

Figure 15 is a chromatogram of a reverse phase chromatography (Vydac C4 column) for purification of human TPO from the culture supernatant of CHO cells into which pDEF202-hTPO-P1 has been introduced and allowed the TPO to be expressed.

Figure 16 is a photograph showing the SDS-PAGE separation of the human TPO purified from the culture supernatant of CHO cells into which pDEF202-hTPO-P1 has been introduced and allowed the TPO to be expressed.

Figure 17 is a chromatogram of a reverse phase chromatography for purification of human TPO from E. coli into which pCFM536/h6T(1-163) has been introduced and allowed the TPO to be expressed.

Figure 18 is a photograph showing the SDS-PAGE separation of the variant human TPO, h6T(1-163) isolated and purified from E. coli into which pCFM536/h6T(1-163) has been introduced and allowed the TPO to be expressed.

Figure 19 shows an elution pattern of hTPO163 on Superdex 75 pg column with regard to the purification of the hTPO 163 from the culture supernatant, as a starting material, prepared by transfection of the human TPO expression plasmid pDEF202-hTPO163 into CHO cells. The protein amount was determined at 220 nm.

Figure 20 shows an SDS-PAGE analysis of the standard hTPO163 eluted from Superdex 75 pg column upon the purification of the hTPO163 from the culture supernatant, as a stating material, prepared by transfection of the human TPO expression plasmid pDEF202-hTPO163 into CHO cells. The hTPO163 was silver-stained on the gel.

Figure 21 shows a structure of the expression vector pSMT201.

Figure 22 shows a graph showing the TPO activity as determined by M-07e assay, in the culture supernatant of the COS7 that $\beta$GL-TPO, N3/TPO or 09/TPO has been introduced into and then expressed.

Figure 23 is a graph showing a TPO activity, as determined by M-07e assay, in the supernatants of COS7 cell cultures that an insertion or deletion derivative of human TPO has been introduced into and expressed.

Figure 24 shows increased platelet count in mice administered TPO via intravenous and subcutaneus injection.

Figure 25 shows dose dependent increased platelet count in mice following subcutaneous injection of TPO.

Figure 26 shows TPO induced increase in platelet count following treatment of mice with 5-FU to induce thrombocytopenia.

Figure 27 shows TPO induced increase in platelet count following treatment of mice with nimustine hydrochloride to induce thrombocytopenia.

Figure 28 shows TPO induced increase in platelet count thrombocytopenic mice following bone marrow transplant.

Figure 29 shorn TPO induced platelet count following x-ray irradiation of mice to induce thrombocytopenia.

Figure 30 shows dose-dependent increase in platelet count following administration of truncated TPO (amino acids 1-163 in SEQ ID NO: 6).

Figure 31 shows increase in platelet count following administration of truncated TPO (amino acids 1-163 in SEQ ID NO: 6) following administration of nimustine hydrochloride to induce thrombocytopenia.

Figure 32 shows that increasing concentrations of Mpl-X added to the human megakaryocyte culture system increasingly block megakaryocyte development.

Figure 33 describes TPO activity, as determined by M-07e assay, of TPO derivatives [$Met^{-2}$, $Lys^{-1}$, $Ala^{1}$, $Val^{3}$, $Arg^{133}$]TPO(1-163), [$Met^{-2}$, $Lys^{-1}$, $Ala^{1}$, $Val^{3}$, $Pro^{148}$]TPO(1-163), and [$Met^{-2}$, $Lys^{-1}$, $Ala^{1}$, $Val^{3}$, $Arg^{115}$]TPO(1-163) expressed in E.coli.

Figure 34 describes TPO activity, as determined by M-07e assay, of TPO derivatives [$Met^{-2}$, $Lys^{-1}$, $Ala^{1}$, $Val^{3}$, $Arg^{129}$]TPO(1-163), [$Met^{-2}$, $Lys^{-1}$, $Ala^{1}$, $Val^{3}$, $Arg^{143}$]TPO(1-163), [$Met^{-2}$, $Lys^{-1}$, $Ala^{1}$, $Val^{3}$, $Leu^{82}$]-TPO(1-163), [$Met^{-2}$, $Lys^{-1}$, $Ala^{1}$, $Val^{3}$, $Leu^{146}$]TPO(1-163) and [$Met^{-2}$, $Lys^{-1}$, $Ala^{1}$, $Val^{3}$, $Arg^{59}$]TPO(1-

163) expressed in E.coli.

DETAILED DESCRIPTION OF THE INVENTION

Specifically provided according to the present invention are thrombopoietin (TPO) polypeptides having the biological activity of specifically stimulating or increasing platelet production and comprising the amino acid sequence 1 to 332 of SEQ ID NO: 6 or derivatives thereof. Illustrative polypeptides include those which consist of the amino acid sequences 1 to 163 of SEQ ID NO: 6; 1 to 232 of SEQ ID NO: 6; 1 to 151 of SEQ ID NO: 6; the mature sequence of amino acids of SEQ ID NOs: 2, 4 and 6; including those having from 1 to 6 $NH_2$ terminal amino acids deleted. Additional illustrative polypeptides of the invention include [-$THr^{33}$, $Thr^{333}$, $Ser^{334}$, $Ile^{335}$, $Gly^{336}$, $Tyr^{337}$, $Pro^{338}$, $Tyr^{339}$, $Asp^{340}$, $Val^{341}$, $Pro^{342}$, $Asp^{343}$, $Tyr^{344}$, $Ala^{345}$, $Gly^{346}$, $Val^{347}$, $His^{348}$, $His^{349}$, $His^{350}$, $His^{351}$, $His^{352}$, $His^{353}$]TPO,[$Asn^{25}$, $Lys^{231}$, $Thr^{333}$, $Ser^{334}$, $Ile^{334}$, $Ile^{335}$, $Gly^{336}$, $Tyr^{337}$, $Pro^{339}$, $Asp^{340}$, $Val^{341}$, $Pro^{342}$, $Asp^{343}$, $Tyr^{344}$, $Ala^{345}$, $Gly^{346}$, $Val^{346}$, $Val^{347}$, $His^{348}$, $His^{349}$, $His^{350}$, $His^{351}$, $His^{352}$, $His^{353}$]TPO, and [$Thr^{33}$]TPO.

Other polypeptides of the invention include derivatives of a TPO poiypeptide [$\Delta Arg^{117}$]TPO(1-163), [$\Delta Gly^{116}$]TPO(1-163), [$His^{33}$, $Thr^{35}$, $Pro^{34}$,]TPO(1-163),[$His^{33}$, $Ala^{33}$, $Pro^{34}$]TPO(1-163),[$His^{33}$, $Gly^{33}$, $Pro^{34}$, $Ser^{35}$]TPO(1-163),[$Gly^{116}$, $Asn^{116}$, $Arg^{117}$]TPO(1-163), [$Gly^{118}$,$Ala^{118}$, $Arg^{117}$]TPO(1-163), [$Gly^{116}$, $Arg^{117}$]TPO-(1-163), [$Ala^{1}$, $Val^{3}$, $Arg^{125}$]TPO(1-163),[$Ala^{1}$, $Val^{3}$, $Arg^{133}$]TPO(1-163), ($Ala^{1}$, $Val^{3}$, $Arg^{145}$]TPO(1-163),[$Ala^{1}$, $Val^{3}$,$Leu^{52}$]TPO(1-163),[$Ala^{1}$, $Val^{3}$, $Leu^{146}$]TPO(1-163),[$Ala^{1}$, $Val^{3}$, $Pro^{148}$]TPO(1-163),[$Ala^{1}$, $Val^{3}$, $Arg^{59}$]TPO-(1-163), [$Ala^{1}$, $Val^{3}$, $Arg^{115}$]TPO(1-163).

The TPO polypeptides of the invention also include those which are covalently bonded to a polymer, preferably polyethylene glycol. The TPO polypeptides of the invention may further comprise the amino acids [$Met^{-2}$-$Lys^{-1}$], [$Met^{-1}$] or [$Gly^{-1}$]. DNAs provided by the invention include those which encode the TPO polypeptide and derivatives described above and are suitably provided as cDNA, genomic DNA and manufactured DNAs.

Also provided by the invention are processes for producing a TPO polypeptide as described above comprising the steps of expressing a polypeptide encoded by a DNA of the invention in a suitable host, and isolating said TPO polypeptide. Where the TPO polypeptide expressed is a $Met^{-2}$-$Lys^{-1}$ polypeptide, such processes can further include the step of cleaving $Met^{-2}$-$Lys^{-1}$ from said isolated TPO polypeptide.

Also provided by the invention are processes for producing TPO polypeptides such as glutathione-S-transferase (GST) fusion polypeptides. DNA encoding an amino terminal GST polypeptide, a thrombin recognition peptide and a TPO polypeptide is introduced into a suitable host, the fusion polypeptide is isolated and the GST moiety removed by treatment with thrombin. Resulting TPO polypeptides are of the [$Gly^{-1}$] structure.

Additionally provided are procaryotic or eucaryotic host cells transformed or transfected with a DNA sequence according to the invention in a manner enabling said host cell to express a polypeptide having the biological activity of specifically stimulating or increasing platelet production.

Pharmaceutical compositions of the invention comprise an effective amount of a TPO polypeptide or derivative in combination with a pharmaceutically acceptable carrier and are susceptible to use in treatment of platelet disorders, particularly the treatment of thrombocytopenia, such as is induced by chemotherapy, radiotherapy or bone marrow transplantation. Corresponding treatment methods are provided by the invention.

Finally, the present invention provides antibodies specifically immunoreactive with TPO polypeptides and derivatives thereof as described above. Such antibodies are useful in methods for isolation and quantification of TPO polypeptides of the invention.

According to the present invention, there is provided a novel DNA sequence which encodes a protein having a TPO activity (referred to as "DNA sequence of the present invention" hereinafter). The DNA sequence of the present invention includes a DNA sequence which encodes the amino acid sequence shown in SEQ ID NO: 2, 4 or 6 of the Sequence Listing attached hereto.

Also included in the DNA sequence of the present invention is a DNA sequence which encodes a partly modified (substitution, deletion, insertion or addition) version of the aforementioned amino acid sequence shown in SEQ ID NO: 2, 4 or 6, provided that such modifications do not spoil the TPO activity. That is, DNA sequences coding for TPO derivatives are also included in the present invention.

In other words, the DNA sequence of the present invention includes DNA sequences which encode protein molecules whose amino acid sequences are substantially the amino acid sequences shown in SEQ ID NO: 2, 4 or 6. The wording "amino acid sequences are substantially the amino acid sequences shown in SEQ ID NO: 2, 4 or 6" as used herein means that said amino acid sequences include those represented by SEQ ID NO: 2, 4 or 6 as well as those represented SEQ ID NO: 2, 4 or 6 which have partial modification therein such as substitution, deletion, insertion, addition or the like, provided that such modifications do not spoil the TPO activity.

Further, the DNA sequence of the present invention consists essentially of a DNA sequence encoding a protein having a TPO activity.

The wording "a DNA sequence which encodes the amino acid sequence" includes all the DNA sequences which may have degeneracy in nucleotide sequences.

The DNA sequence of the present invention also comprises the following sequences:

(a) DNA sequences represented by SEQ ID No: 7, 194, 195 and 196, or complementary strands thereof,

(b) DNA sequences which hybridize under stringent conditions to the DNA sequences as defined in (a) or fragments thereof, or

(c) DNA sequences which would hybridize to the DNA sequences as defined in (a) and (b), but for the degeneracy of the genetic code.

In other words, the DNA sequence of the present invention also comprises the following sequences:

(a) DNA sequence which is integrated in the Vector pEF18S-A2α(deposit No. FERM BP-4565) carried by an *E. coli* strain DH5, a Vector pHT1-231 (deposit No. FERM BP-4564) carried by an *E. coli* strain DH5, a Vector pHTF1 (deposit No. FERM BP-4617) carried by an *E. coli* strain DH5, a Vector pHGT1 (deposit No. FERM BP-4616) carried by an *E. coli* strain DH5, and encodes the amino acid sequence of a protein having the TPO activity; or

(b) DNA sequences which hybridize (under stringent conditions) to the DNA sequences defined in (a) or fragments thereof, and encode the amino acid sequence of a protein having the TPO activity.

As used herein, representative "stringent" hybridization conditions are those employed in the examples addressing PCR amplification of DNAs of the invention using degenerate and/or unique sequence oligonucleotide primers (probes). See also for example, Chapters 11 and 14 of *Molecular Cloning* - (Sambrook et al., Cold Spring Harbor Laboratory Press, 1989) and Unit 2.10 in Current Protocols in Molecular Biology, Ausubel et al., Eds., Current Protocols, USA, 1993).

Also included in the DNA sequence of the present invention is a DNA sequence which encodes a protein having TPO activity and comprises the nucleotide sequence encoding positions 1-163 of the amino acid sequence represented by SEQ ID NO: 6.

Such DNA sequences may be supplemented with a restriction enzyme cleavage site and/or an additional DNA sequence at the initiation, termination or intermediate site which facilitate construction of readily expressed vectors. When a non-mammalian host is used, a preferred codon for the gene expression in the host may be incorporated.

An example of the DNA sequence of the present invention is a cDNA molecule which is obtained by preparing mRNA from cells of mammals, including human, and then screening the cDNA in the usual way from a cDNA library prepared by a known method. Sources of the mRNA of this case include cells of a rat hepatocyte-derived cell line McA-RH8994, HTC cells, H4-II-E cells, rat liver, kidney, brain and small intestine human liver and the like.

Another example of the DNA sequence of the present invention is a genomic DNA molecule which is obtained by screening it in the usual way from a genomic library prepared by a known method from cells of mammals including human. Sources of the genomic DNA of this case include chromosomal DNA preparations obtained from human, rat, mouse and the like.

A DNA sequence which encodes a TPO derivative may be obtained from the thus obtained cDNA sequence that encodes a protein having TPO activity, by modifying the cDNA sequence making use of the known site-directed mutagenesis thereby effecting partial modification of the corresponding amino acid sequence.

Having elucidated the amino acid sequence or DNA sequence of a protein having TPO activity in the present invention, a DNA sequence which encodes a partially modified amino acid sequence can be obtained easily by chemical synthesis.

The DNA sequence of the present invention is a useful material for the large scale production of a protein having TPO activity making use of various recombinant DNA techniques.

Also, the DNA sequence of the present invention is useful as a labeled probe for the isolation of a gene which encodes a TPO-related protein, as well as cDNA and genomic DNA coding for TPO of other mammalian species. It is also useful in the gene therapy of human and other mammalian species. In addition, the DNA sequence of the present invention is useful for the development of a transgenic mammalian species which can be used as a eucaryotic host for large scale production of TPO (Palmiter *et al.*, *Science*, vol.222, pp.809 - 814, 1983).

Also provided by the present invention are a vector in which the aforementioned DNA sequence coding for a protein having TPO activity is integrated, host cells transformed with said vector and a process for the production of a protein having TPO activity which comprises culturing said host cells and separating and purifying the expressed protein having TPO activity.

Examples of host cells useful in this case include cells of prokaryotes, such as, *E. coli* and the like, and of eukaryotes, such as yeasts, insects, mammals and the like. Illustrative examples of mammalian cells include COS cells, Chinese hamster ovary (CHO) cells, C-127 cells, baby hamster kidney (BHK) cells and the like. Illustrative examples of yeasts include, a baker's yeast (*Saccharomyces cerevisiae*), a methanol assimilating yeast (*Pichia pastoris*) and the like. Illustrative examples of insect cells include, silkworm cultured cells and the like.

With regard to vectors for use in the transformation of these host cells, pKC30 (Shimatike H. and M. Rosenberg, *Nature*, 292, 128 - 132, 1981), pTrc99A (Amann E. *et al.*, *Gene*, 69, 301 - 315, 1988) or the like may be used for the transformation of *E. coli* cells. For transforming mammalian cells, pSV2-neo (Southern and Berg, *J. Mol. Appl. Genet.*, 1, 327 - 341, 1982), pCAGGS (Niwa *et al.*, *Gene*, 108, 193 - 200, 1991), pcDL-SRα296 (Takebe *et al.*, *Mol. Cell. Biol.*, 8, 466 - 472, 1988) or the like may be used. As for yeast cells, pG-1 (Schena M. and Yamamoto K.R., *Science*, 241, 965 - 967, 1988) or the like may be used. A transfer vector for recombinant virus construction, for example, pAc373 (Luckow *et al.*, *Bio/Technology*, 6, 47 - 55, 1988) may be used for the transformation of silkworm cells.

As occasion demands, each of these vectors may contain a replication origin, selection marker(s), a promoter and the like, as well as an RNA splice site, a polyadenylation signal and the like in the case of vectors for use in eukaryotic cells.

With regard to the replication origin, a sequence derived from, for example, SV40, adenovirus, bovine papilloma virus or the like may be used in vectors for mammallan cells. A sequence derived from ColE1, R factor, F factor or the like may be used in vectors for *E. coli* cells. A sequence derived from 2 μm DNA, ARS1 or the like may be used in vectors for yeast cells.

As to promoters for gene expression, those which are derived from, for example, retrovirus, polyoma virus, adenovirus, SV40 and the like may be used in vectors for mammalian cells. A promoter derived from bacteriophage λ, for example, *trp*, *lpp*, *lac* or *tac* promoter, may be used in vectors for *E. coli* cells. ADH, PHO5, GPD, PGK or MAFα promoter may be used in vectors for baker's yeast cells, and AOX1 promoter or the like in vectors for methanol assimilating yeast cells. A promoter derived from a nuclear polyhedrosis virus may be used in vectors for silkworm cells.

Typical examples of selection markers useful in vectors for mammalian cells include a neomycin (*neo*) resistance gene, a thymidine kinase (TK) gene, a dihydrofolate reductase (DHFR) gene, an *E. coli* xanthine-guanine phosphoribosyltransferase (*Ecogpt*) gene and the like. Illustrative examples of selection markers useful in vectors for *E. coli* cells include a kanamycin resistance gene, an ampicillin resistance gene, a tetracycline resistance gene and the like, and those for yeast cells include Leu2, Trp1, Ura3 and the like genes.

Production of a protein having TPO activity making use of appropriate combinations of these host-vector systems may be performed by transforming appropriate host cells with a recombinant DNA obtained by inserting the gene of the present invention in an appropriate site of the aforementioned vector, culturing the resulting transformant and then separating and purifying the polypeptide of interest from the resulting cells, or culture medium or filtrate. Commonly used means and procedures may be applied to this process in combination.

When expressing the gene of interest, the original signal sequence may be modified or replaced by a signal sequence derived from another protein in order to obtain homogeneous N-terminal of the expressed product. Homogenization of the N-terminal may be carried out by modification (substitution or addition) of the amino acid residues in the N-terminal or its vicinity. In the case of expression by using *E. coli,* as a host cell, for example, lysine residue may be further supplemented in addition to methionine residue.

The novel protein of the present invention having TPO activity (to be referred to as "protein of the present invention" hereinafter) includes proteins each of which comprising the amino acid sequence shown in SEQ ID NO: 2, 4 or 6. TPO derivatives whose amino acid sequences are partially modified (substitution, deletion, insertion or addition) are also included in the present invention, provided that the TPO activity is not spoiled by the modification.

In other words, the protein of the present invention includes protein molecules whose amino acid sequences are substantially the amino acid sequences shown in SEQ ID NO: 2, 4 or 6.

The wording "amino acid sequences are substantially the amino acid sequences shown in (or represented by) SEQ ID NO: 2, 4 or 6" as used herein means that said amino acid sequences include those shown in SEQ ID NO: 2, 4 or 6 as well as those shown in SEQ ID NO: 2, 4 or 6 which have partial modification therein such as substitution, deletion, insertion, addition and the like, provided that such modifications do not spoil the TPO activity.

The protein of this invention includes a protein containing the positions 7-151 of the amino acid sequence shown in SEQ ID NO: 6 and having a TPO activity. Also included in the protein of the present invention is a protein which has TPO activity and comprises positions 1-163 of the amino acid sequence represented by SEQ ID NO: 6.

Examples of other TPO derivatives of the present invention include a derivative whose stability and durability *in vivo* were improved by amino acid modification (substitution, deletion, insertion or addition), a derivative in which at least one potential glycosylation was changed by deletion or addition, a derivative in which at least one cysteine residue was deleted or substituted by other amino acid residue (alanine or serine residue for example).

Preferably, the protein of the present invention is characterized in that it is separated and purified from host cells transformed with a recombinant vector containing a cDNA molecule, a genomic DNA molecule or a DNA fragment obtained by chemical synthesis.

When intracellular expression is effected using a bacterium such as *E. coli* as the host, a protein in which an initiation methionine residue is added to the N-terminal side of a protein molecule having TPO activity is obtained, which is also included in the present invention. Depending on the host used, the produced protein having TPO activity may or may not be glycosylated, and each of such cases is included in the protein of the present invention.

The protein of the present invention also includes naturally occurring TPO-active proteins purified and isolated from natural sources such as cell culture medium having TPO activity or human urine, serum and plasma.

A process for the purification of TPO from such natural sources is also included in the present invention. Such a purification process may performed by employing one of or a combination of usually used protein purification steps such as an ion exchange chromatography, a lectin affinity chromatography, a triazine dye affinity chromatography, a hydrophobic interaction chromatography, a gel filtration chromatography, a reverse phase chromatography, a heparin affinity chromatography, a sulfated gel chromatography, a hydroxylapatite chromatography, isoelectric focussing chromatography, a metal chelating chromatography, a preparative electrophoresis, an isoelectric focussing gel electrophoresis and the like. A purification process in which certain techniques are used in combination making use of physicochemical properties of TPO which can be deduced from the Examples of this specification is also included in the present invention. In addition, an antibody affinity chromatography to which an antibody capable of recognizing TPO is applied may also be used. Furthermore, it has been found that TPO is the ligand for Mpl (de Sauvage *et al.*, *Nature 369*: 533-538 (1994); Bartley *et al.*, *Cell 77*: 1117-1124 (1994); Kaushansky *et al.*, *Nature 369*: 565-568 (1994)), by which the TPO may be purified using an affinity gel column that the Mpl has been coupled to a resin. More particularly, an example of the column is an Mpl-X column which is prepared by coupling a resin with the extracellular region of Mpl (Mpl-X) produced by recombinant DNA techniques using CHO cell as a host (Bartley *et al.* (1994), supra).

As disclosed herein, the TPO polypeptides of this invention are further characterized by the ability to bind to the Mpl receptor and specifically to the extracellular (soluble) domain thereof.

Also included in the present invention is a protein encoded by a DNA moiety which is complementary to the protein-coding strand of a human cDNA or genomic DNA sequence of TPO gene, namely a "complementary inverted protein" disclosed by Tramontano *et al.* (*Nucl. Acids Res.*, vol.12, pp.5049 - 5059, 1984).

Also included in the present invention is a protein of the present invention which is labeled with a detectable marker, for example, $^{125}I$ labeling or biotinylation, thus rendering possible provision of a reagent which is useful for the detection and quantification of TPO or TPO receptor-expressing cells in solid samples such as tissue and liquid samples such as blood, urine and the like.

The biotinylated protein of the present invention is useful in the case of its binding to immobilized streptavidin in order to remove megakaryoblasts from bone marrow at the time of autogenous bone marrow transplantation. It is also useful in the case of its binding to immobilized streptavidin in order to concentrate autogenous or allogenic megakaryocytic cells at the time of autogenous or allogenic bone marrow transplantation. A conjugate of TPO with a toxin such as ricin, diphtheria toxin or the like or with a radioactive isotope is useful in antitumor therapy and in conditioning for bone marrow transplantation.

The present invention also provides a nucleic acid material which is useful when labeled with a detectable marker including a radioactive marker or a non-radioactive marker such as biotin or when it is used in a hybridization procedure for the detection of the position of human TPO gene and/or its related gene family on a chromosomal map. Such a material is also useful for the confirmation of human TPO gene disorders at the DNA level and can be used as a genetic marker for the confirmation of adjoining genes and their disorders.

Also included in the present invention is a pharmaceutical composition which contains a therapeutically effective amount of the protein of the present invention together with a useful and effective diluent, antiseptic agent, solubilizing agent, emulsifying agent, adjuvant and/or carrier. The term "therapeutically effective amount" as used herein means an amount which provides a therapeutic effect for specified conditions and routes of administering conditioning. Such a composition is used in the form of liquid, freeze-dried or dried preparation and comprises a diluent selected from various buffers (Tris-HCl, acetate and phosphate for example) having various pH values and ionic strengths, a surface adsorption preventing additive such as albumin or gelatin, a surface active agent such as Tween 20, Tween 80, Pluronic F68 or bile acid salt, a solubilizing agent such as glycerol or polyethylene glycol, an antioxidant such as ascorbic acid or sodium metabisulfite, an antiseptic agent such as thimerosal, benzyl alcohol or paraben and a vehicle or tonicity agent such as lactose or mannitol. Also used is a composition comprising covalent bonding of the protein of the present invention to a polymer such as polyethylene glycol, chelation of the protein with metal ions, incorporation of the protein into a granular preparation, or on the surface, consisting of a polymer compound such as polylatic acid, polyglycolic acid or hydrogel, or incorporations of the protein into liposomes, microemulsion, micelles, single or multilayer vesicles, red cell ghosts or spheroplasts. Such a composition will exert influences upon physical conditions, solubility, stability, *in vivo* releasing rate and *in vivo* clearance of TPO. Selection of the composition may be decided depending on the physical and chemical properties of the used TPO-active protein. Also included in the present invention is a granular composition in which granules are coated with a polymer such as poloxamer or poloxamine and TPO which binds to antibodies for tissue specific receptors, ligands or antigens or to tissue specific receptor ligands. Other examples of the composition of the present invention are those which are in the form of granules, have a protective coating and contain a protease inhibitor or a penetration enhancer, for use in various routes of administration such as parenteral, pulmonary, transnasal and oral administration.

The pharmaceutical composition comprising the protein of the present invention may be administrated several times per day, usually in an amount of 0.05μg to 1mg/kg (as TPO protein) of body weight depending on conditions and sex of a patient, administration route and the like.

The pharmaceutical composition containing the protein of the present invention can be administered at a dose of 25,000-500,000,000 of the active ingredient (in terms of the relative activity by the M-07e assay which will be given later) per kg of body weight for one to several times a day and for one to seven days a week depending upon the symptoms, sex and route of administration.

The present inventors have confirmed that, with respect to the C-terminal site of the human TPO, the activity is maintained even when the amino acid residues up to the 152nd amino acid sequence shown in SEQ ID NO: 6 are deleted and that, with respect to the N-terminal site, the activity is maintained even when the amino acid residues up to the 6th amino acid sequence are deleted.

Accordingly, protein having TPO activity, containing the 7th to 151th amino acid sequence of SEQ ID NO: 6 and being modified (substitution, deletion, insertion or addition) in other parts may be preferably used as the effective ingredient of the present invention as well. More preferred TPO derivative is that having the 1st to 163rd amino acid sequence of SEQ ID NO: 6.

A composition which contains at least one of additional hematopoietic factors such as EPO, G-CSF, GM-CSF, M-CSF, IL-1, IL-2, IL-3, IL-4, IL-5, IL-6, IL-7, IL-8, IL-9, IL-10, IL-11, IL-12, IL-13, LIF and SCF, in addition to the protein of the present invention, is also included in the present invention.

The protein of the present invention is useful for the treatment of various thrombocytopenic diseases, either alone or in combination with other additional hematopoietic factors. Examples of other additional hematopoietic factors include EPO, G-CSF, GM-CSF, M-CSF, IL-1, IL-2, IL-3, IL-4, IL-5, IL-6, IL-7, IL-8, IL-9, IL-10, IL-11, IL-12, IL-13, LIF and SCF.

There are many diseases characterized by platelet disorders such as thrombocytopenia due to damages in the production of platelets or reduction in life of platelets (caused by destruction or consumption of platelets), which can be treated with the protein of the present invention. For example, it can be used for the enhancement of the restoration of platelets in a thrombocytopenic patient due to congenital Fanconi anemia or aplastic anemia caused by chemotherapy or irradiation, myelodysplastic syndrome, acute myelocytic leukemia, aplastic crisis after bone marrow transplantation. It is also useful in the treatment of thrombocytopenia due to diminished production of TPO. Thrombocytopenia due to reduction in life of platelets or megakaryocytes includes idiopathic thrombocytopenic purpura, hypoplastic anemia, acquired immunodeficiency syndrome (AIDS), disseminated intravascular coagulation syndrome and thrombotic thrombocytopenia. In addition, it is also useful in the case of autotransfusion of platelets, in which TPO is administered to a patient prior to a surgical operation in order to increase the number of the patient's own platelets and the thus increased platelets are used as transfusion platelets at the time of the operation.

Other use of the protein of the present invention is treatment of diseases resulting from temporal deletion or damage of platelets caused by other chemical or pharmaceutical drugs or curative treatments. TPO can be used for the purpose of enhancing release of new "intact" platelets in such patients.

The present invention further relates to an antibody specific for TPO. The protein of the present invention can be used as antigens, and the corresponding antibodies include both monoclonal and polyclonal antibodies and chimeric antibodies, namely "recombinant" antibodies, prepared by conventional methods. To prepare such an anti-TPO antibody, human TPO itself can be used as the antigen or, alternatively, a partial peptide of human TPO can be utilized as the antigen. Where an antibody to the antigen of such a peptide is utilized, the epitope can be defined by specifying the antigen region. On the other hand, where an antibody to the antigen protein (TPO) itself is utilized, the antigen region can be clarified by analyzing the epitope of the antibody. In such cases, these antibodies each having the thus-clarified epitope can be utilized to fractionate, detect, quantitate and purify various TPOs which differ in their properties such as the kind of the sugar chains added, the length of the peptide chains, etc.

A TPO peptide containing the thus-selected amino acid sequence is synthesized, and bonded to a suitable carrier protein, for example, albumin, KLH (key hole limpethemocyanine) or the like, by covalent bonding to prepare an immunoreactive antigen. Alternatively, a peptide of a multiple antigen peptide (MAP) type is produced to be an antigen, according to the Tam's method (*Proc.Natl.Acad.Sci. (USA) 85*:5409-5413, 1988). Then, with the thus-prepared antigen along with an adjuvant or the like, immunized are mammals, birds, etc. which are generally utilized to make them produce antibodies, such as rabbits, mice, rats, goats, sheep, chickens, hamsters, horses, guinea pigs, etc. From the thus-immunized animal, recovered was its anti-serum and cells, from which a polyclonal antibody is obtained. Where a peptide is used as the antigen, the epitope can be defined by specifying the antigen region. In this case, the regions of various TPO molecules having different molecular weights are screened and identified by immunological engineering. As one example, a peptide-deleted region can be screened and identified. In addition, an antibody gene or a part of the gene is cloned from the cells expressing the intended antibody to obtain an antibody molecule that has been expressed by genetic engineering.

In comparison with a polyclonal antibody which generally contains various antibodies specific for various antigenic determinants (epitopes), a monoclonal antibody is specific for a single antigenic determinant on the antigen. A TPO-specific antibody is useful in improving selectivity and specificity of an antigen-antibody reaction-aided diagnosis and an analytical assay method and in performing separation and purification of TPO. In addition, such antibodies can be applied to the neutralization or removal of TPO from serum. Monoclonal antibodies are also useful for detection and quantification of TPO in, for example, serum of whole blood.

The anti-human TPO antibody of the present invention can be used as the ligand in affinity chromatography for purification and isolation of human TPO. To fix the antibody so as to make it useful in such affinity chromatography, any conventional methods for fixing various enzymes can be employed. For instance, employable is a method of using a carrier of CNBr-activated Sepharose 4B (produced by Pharmacia Fine Chemicals Co.) or the like. To actually purify human TPO by the use of the fixed anti-human TPO antibody, the feed anti-human TPO antibody is filled into a column and a human TPO-containing liquid is passed through the column. By this operation, a large amount of human TPO is adsorbed onto the carrier in the column. As the solvent for elution, for example, usable are glycine-HCl buffer (pH 2.5), sodium chloride solution, propionic acid, dioxane, ethylene glycol, chaotropic salt, guanidine hydrochloride, urea, etc. By the elution with such a solvent, human TPO having a high purity is eluted.

The antibody of the present invention can be used for determining human TPO by immunochemical quantitative assay, especially by enzyme-immunoassay to be conducted by a solid phase sandwich process.

An advantage of monoclonal antibodies is that they can be produced by hybridoma cells in a medium which does not contain any other immunoglobulin molecules. Monoclonal antibodies can be prepared from culture supernatants of hybridoma cells or from mouse ascites induced by intraperitoneal injection of hybridoma cells. The hybridoma technique originally disclosed by Kohler and Milstein (*Eur. J. Immunol. 6*:511-519, 1976) can be used broadly for the formation of hybrid cell lines which are possessed of high level monoclonal antibodies for a number of specific antigens.

To isolate the intended antibody from the thus-obtained antibody-containing material, such as anti-serum, etc., by purifying it, one or more steps (affinity chromatooraphy such as protein A affinity chromatography, protein G affinity chromatography, Avid gel chromatography, anti-immunoglobulin-fixed gel chromatography, etc.; as well as cation-exchange chromatography, anion-exchange chromatography, lectin affinity chromatography, dye adsorption chromatography, hydrophobic interaction chromatography, gel permeation chromatography, reversed phase chromatography, hydroxyl-apatite chromatography, fluoro- apatite chromatography, metal chelating chromatography, isoelectric point chromatography, partitioning electrophoresis, isoelectric point electrophoresis, etc.) which are generally employed for purification of protein can be combined. Apart from these, a method of antigen affinity purification can also be employed, in which a gel carrier or membrane, to which has been chemically bonded a human TPO protein itself or a peptide that contains the region of the antigen or a part of the region or, that is, a molecule capable of recognizing the intended antibody, is prepared, an antibody-containing material is added thereto so that the intended antibody is made to be adsorbed onto the carrier or membrane, and the thus-adsorbed antibody is then eluted and recovered under suitable conditions.

The present invention also allows use of endogenous DNA sequences encoding a TPO polypeptide for the production of large quantities of polypeptide products. For example, *in vitro* and *ex vivo* homologous recombination procedures may be employed to transform host cells to provide for expression or enhanced expression of polypeptides. Preferred host cells include human cells (*e.g.*, liver, bone marrow and the like) wherein a promoter or enhancer sequence is inserted employing flanking sequences that are homologous to a target region in a cellular genome with the result that TPO polypeptide expression is accomplished or enhanced. See, *e.g.*, U.S. Letters Patent 5,272,071, published PCT WO 90/14092, WO 91/06666 and WO 91/09955.

Also provided by the invention are processes for producing a TPO polypeptide as described above comprising the steps of expressing a polypeptide encoded by a DNA of the invention in a suitable host, and isolating said TPO polypeptide. Where the TPO polypeptide expressed is a $Met^{-2}$-$Lys^{-1}$ polypeptide, such processes can further include the step of cleaving $Met^{-2}$-$Lys^{-1}$ from said isolated TPO polypeptide.

Also provided are methods for producing TPO polypeptides having [$Gly^{-1}$] structure comprising the steps of introducing into a suitable host cell a DNA encoding a glutathione-S-transferase (GST) polypeptide 5′ to TPO polypeptide encoding sequences, the GST and TPO polypeptide encoding sequences separated by DNA encoding a thrombin recognition polypeptide, isolating the GST-TPO expression product and treating the expressed polypeptide with thrombin to remove GST amino acids. The resulting TPO polypeptides have a [$Gly^{-1}$] structure.

The following describes the present invention in detail.

(A) Purification of rat TPO, analysis of partial amino acid sequences of purified rat TPO and analysis of biological characteristics of purified rat TPO

The inventors of the present invention have firstly attempted to purify a protein (rat TPO) which has an activity to enhance proliferation and differentiation of rat CFU-MK. In this purification study, many trial and error attempts were made on the purification procedures, such as selection of various natural supply sources, selection of gels for chromatographies and separation modes. As the result, the present inventors have succeeded in purifying a protein having a TPO activity from blood plasma of thrombocytopenic rats induced by X-ray or $\gamma$-ray irradiation, making use of the TPO activity as a marker based on a rat CFU-MK assay which will be described later in ⟨Reference Example⟩, and in determining partial amino acid sequences of the purified protein ⟨Examples 1 and 2⟩.

Biological characteristics of the plasma-derived rat TPO were also examined ⟨Example 3⟩.

An outline of the procedures from the purification of rat TPO to the determination of partial amino acid sequences of the purified protein is shown in the following.

(i) A blood plasma sample was prepared from about 1,100 thrombocytopenic rats induced by $\gamma$-ray or g-ray irradiation and subjected to a Sephadex G-25 chromatography, an anion exchange chromatography (Q-Sepharose FF) and a lectin chromatography (WGA-Agarose) in that order, thereby obtaining a WGA-Agarose-adsorbed TPO-active fraction.

(ii) Next, the thus obtained WGA-Agarose-adsorbed TPO-active fraction was subjected to a triazine dye affinity chromatography (TSK AF-BLUE 650MH), a hydrophobic interaction chromatography (Phenyl Sepharose 6 FF/LS) and a gel filtration chromatography (Sephacryl S-200 HR) in that order. Since the TPO activity was divided into 4 peaks (F1 as the highest molecular weight fraction, followed by F2, F3 and F4) by the Sephacryl S-200 HR gel filtration, each of the TPO-active fractions F2 and F3 was concentrated to obtain a high molecular weight TPO sample F2 and a low molecular weight TPO sample F3 which were used separately in the subsequent purification steps.

(iii) The low molecular weight TPO sample F3 was subjected to a preparative reverse phase chromatography (YMC-Pack PROTEIN-RP), a reverse phase chromatography (YMC-Pack CN-AP) and another reverse phase chromatography (Capcell Pack C1) in that order. When the thus obtained TPO-active fraction was applied to Sodium dodecylsulfate (SDS) polyacrylamide gel electrophoresis (SDS-PAGE) and the TPO-active substance was extracted from the gel, the presence of the TPO activity was confirmed in a band corresponding to apparent molecular weights of about 17,000 to 19,000 under a non-reducing condition.

(iv) In consequence, all TPO-active fractions were applied to SDS-PAGE under a non-reducing condition and transferred on a PVDF membrane. By carrying out systematic limited enzymatic hydrolysis of the protein on the PVDF membrane into peptide fragments, partial amino acid sequences of the rat TPO protein were determined. Based on the amino acid sequence information of two peptide fragments, cloning of rat TPO gene was performed.

(v) Separately from this, the high molecular weight TPO sample F2 obtained by the Sephacryl S-200 HR was subjected to purification in the same manner as in the case of the low molecular weight TPO sample F3. When a TPO-active fraction obtained by the final step reverse phase chromatography (Capcell Pack C1) was applied to SDS-PAGE and the TPO-active substance was extracted from the gel, the presence of the TPO activity was confirmed in a band corresponding to apparent molecular weights of about 17,000 to 22,000 under a non-reducing condition.

(B) Specialization of rat TPO producing cells, preparation of mRNA and construction of rat cDNA library

Since the thus purified rat TPO has been derived from blood plasma, it was necessary to screen organs or cells as the source of mRNA for use in the cDNA cloning. In consequence, TPO activities in various organs and cell culture supernatants were screened based on the biochemical and biological properties of the rat plasma-derived TPO. As the result, TPO activities almost equal to the rat plasma-derived TPO were found in the culture supernatant of rat hepatocyte-derived cell lines McA-RH8994, HTC and H4-II-E, as well as in the culture supernatant of rat primary hepatocytes ⟨Example 4⟩.

On the other hand, the expression vector pEF18S was constructed from 2 expression vectors pME18S and pEFBOS ⟨Example 5⟩. Construction of this vector has rendered possible easy cloning of cDNA using a high efficiency expression vector having multiple cloning sites which can be used for the integration of inserts.

In addition to the above expression vector, pUC and pBR based plasmid vectors and λ based phage vectors are mainly used for the construction of cDNA libraries.

The McA-RH8994 cells were cultured, homogenized by adding guanidine thiocyanate solution and then subjected to CsCl density gradient centrifugation to obtain total RNA ⟨Example 6⟩.

Preparation of RNA can also be made by a hot phenol method, an acid guanidium phenol chloroform method and the like.

After purifying poly $(A)^+$ RNA from the total RNA using oligo dT-immobilized latex particles, first strand cDNA was synthesized by the action of reverse transcriptase using oligo dT as a primer to which a restriction enzyme *Not*I recognition sequence has been added, followed by the synthesis of 2nd strand cDNA using RNase H and *E.coli* DNA polymerase I. An *Eco*RI adaptor was added to the thus obtained double-strand cDNA, the resulting cDNA was ligated with a mammalian cell expression vector pEF18S constructed in Example 5, which has been digested with *Not*I and *Eco*RI and then the thus ligated cDNA was transformed into competent cells of an *E. coli* strain DH5 to construct a cDNA library ⟨Example 7⟩.

(C) Preparation (cloning) of rat TPO cDNA fragment by PCR

A DNA sequence was deduced from the partial amino acid sequence of the rat TPO which has been purified from rat blood plasma, in order to synthesize degenerate primers to be used in the polymerase chain reaction (PCR). Primers to be used may also be obtained based on an amino acid sequence other than the position of the primers used herein. Highly degenerate primers may also be used without employing inosine. In addition, primers with reduced degeneracy can be designed making use of a codon having high usage in rat (Wada et al., *Nucleic Acids Res.*, vol.18, p.2367-2411, 1990).

When plasmid DNA was extracted from entire portion of the cDNA library prepared above and PCR was carried out using the extracted DNA as a template, a band of about 330 bp was detected which was subsequently determined by nucleotide sequence analysis as a DNA fragment (A1 fragment) that encodes a portion of the rat TPO ⟨Example 8⟩.

(D) Screening of rat TPO cDNA by PCR, sequence of rat TPO cDNA and confirmation of TPO activity

The cDNA library prepared above was divided into pools, each containing about 10,000 clones, and plasmid DNA was extracted from each of 100 pools. When PCR was carried out using each pool of plasmid DNA as a template and primers newly synthesized based on the nucleotide sequence of the A1 fragment, bands considered to be specific were detected in 3 pools. One of these 3 pools was divided into sub-pools, each containing about 900 clones, and plasmid DNA was purified from 100 sub-pools to carry out PCR in the same manner. As the result, a specific band was detected in 3 sub-pools. One of these pools was divided into sub-pools, each containing 40 clones, and finally each clone was screened by PCR in the same manner. As the result, a clone pEF18S-A2α which seemed to encode the rat TPO cDNA was isolated ⟨Examples 9 and 10⟩.

When nucleotide sequence of this clone was analyzed, it was revealed that it encodes the partial amino acid sequence of the protein purified from rat blood plasma, thus confirming a strong possibility that this clone contains the rat TPO cDNA ⟨Example 10⟩.

When plasmid DNA was purified from the clone pEF18S-A2α obtained above and transfected into COS 1 cells, TPO activity was found in the culture supernatant of the transfected cells. As the result, it was confirmed that the clone pEF18S-A2α contains a cDNA which encodes the rat TPO ⟨Example 11⟩.

(E) Detection of TPO mRNA in various rat tissues

TPO mRNA expression in rat tissues was analyzed by PCR, and specific expression was detected in the brain, the liver, the small intestines and the kidney ⟨Example 12⟩.

(F) Construction of human cDNA library

Based on the results of Examples 4 and 12, the liver was selected as the starting tissue for the cloning of human TPO cDNA. In consequence, a cDNA library was constructed using a commercial mRNA derived from normal human liver. Using pEF-18S as a vector like the case of the rat library, cDNA was synthesized by the same procedure as the case of rat to obtain a directionally cloned cDNA library by making use of restriction enzymes *Not*I and *Eco*RI. The library prepared by introducing vector-ligated cDNA into *E. coli* DH5 contained about 1,200,000 clones ⟨Example 13⟩.

(G) Preparation (cloning) of human TPO cDNA fragment by PCR

Several primers for PCR were synthesized based on the nucleotide sequence of the clone pEF18S-A2α which encodes the rat TPO cDNA. When cDNA was synthesized using a commercial mRNA derived from normal human liver and PCR was carried out using these primers and cDNA as a template, a band of around 620 bp was observed. When the nucleotide sequence was analyzed, it was revealed that this clone contains a DNA fragment which has a homology of about 86% with the rat TPO cDNA, thus confirming a strong possibility that this is a part of a gene which encodes human TPO ⟨Example 14⟩.

(H) Screening of human TPO cDNA by PCR, sequence of human TPO cDNA and confirmation of TPO activity

The human cDNA library prepared above was amplified, divided into pools, each containing about 100,000 clones, and plasmid DNA was extracted from each of 90 pools. When PCR was carried out using each pool of plasmid DNA molecules as a template and primers newly synthesized based on the nucleotide sequence of the human TPO fragment obtained in Example 14, possible bands were detected in 3 pools. One of these pools was divided into sub-pools, each containing 5,000 clones, and plasmid DNA was purified from each of 90 sub-pools. When PCR was carried out using each pool of plasmid DNA as a template in the same manner, possible bands were detected in 5 sub-pools. When one of these pools was divided into sub-pools, each containing 250 clones, and plasmid DNA was purified from each of 90 sub-pools and subjected to PCR, possible bands were detected in 3 sub-pools. When one of these pools was divided into sub-pools, each containing 30 clones, and plasmid DNA was purified from each of 90 sub-pools and subjected to PCR, possible bands were detected in 3 sub-pools. Thereafter, 90 colonies were isolated from one of these sub-pools and plasmid DNA purified from each of the colonies was subjected to PCR to finally obtain a clone named HL34 ⟨Example 15⟩.

When nucleotide sequence of plasmid DNA in this clone was analyzed, it was revealed that this clone contains a cDNA which has about 84% homology with the rat TPO cDNA nucleotide sequence ⟨Example 16⟩.

When the cloned plasmid DNA was purified and transfected into COS 1 cells, TPO activity was found in the culture supernatant of the transfected cells. As the result, it was confirmed that this plasmid clone contains the cDNA which encodes the human TPO ⟨Example 17⟩.

However, this cDNA seemed to be an artificial product of the cloning, because no termination codon was found in this clone and a poly A tail-like sequence was found on its 3'-end. In consequence, an expression vector was constructed which encodes the amino acid sequence excluding a corresponding portion to the poly A tail-like sequence. When the thus constructed vector was expressed in COS 1 cells, TPO activity was found in the resulting culture supernatant ⟨Example 18⟩.

DNA fragment in the 3'-end region of the human TPO was obtained by PCR so as to analyze the structure of a full-length cDNA.

The determination of the nucleotide sequence of this fragment revealed that it partly overlaps with cDNA carried by the clone HL34 obtained in Example 15. It was also expected that a full length human TPO cDNA may contain an open reading frame thereof and code for a protein consisting of 353 amino acids. Thus, it was suggested that the human TPO consisted of 353 amino acids including a signal sequence of 21 amino acids ⟨Example 19⟩.

In addition to the above cloning method, cloning of human TPO cDNA can be attained by colony hybridization or plaque hybridization using a rat TPO cDNA fragment as a probe, making use of a library constructed using a pUC or pBR based plasmid vector, a λ phage based vector or the like. In designing a degenerate probe, inosine may be used to decrease the degree of degeneracy. When an assay method which can detect TPO activity in a specific manner or with a high sensitivity is available, it is possible to carry out expression cloning making use of an expression library as used in the present invention.

However, since the content of human TPO-encoding RNA seems to be extremely low in the normal human liver as will be described later in Example 15 (the content calculated from the result of this Example was in a ratio of one per three million), the hybridization screening in which a synthesized oligonucleotide or a rat or a human TPO cDNA fragment is used as a probe will be extremely difficult to effect, because the number of clones or plaques to be treated becomes bulky and sensitivity and specificity of the hybridization method are inferior to those of the PCR method. As a matter of fact, the inventors of the present invention have carried out colony hybridization of two million clones in a normal human liver cDNA library prepared in Example 13 using a rat TPO cDNA fragment as a probe, but a human TPO cDNA clone was not able to be obtained.

(I) Reconstruction of human normal liver-derived cDNA

Since the clone HL34 obtained in Example 15 seemed to contain an incomplete cDNA, a cDNA library was reconstructed using a commercial normal human liver-derived poly $(A)^+$ RNA preparation to obtain a complete human TPO cDNA. The library (hTPO-F1) prepared by introducing vector-ligated cDNA into E. coli DH5 contained 1.0 x $10^6$ transformants ⟨Example 20⟩.

(J) Screening of TPO cDNA clone, sequence determination and expression of human TPO cDNA, and confirmation of TPO activity

Primers for PCR here synthesized based on the nucleotide sequence (SEQ ID NO: 3) of a partial cDNA obtained in Example 14 and the nucleotide sequence (SEQ ID NO: 196) of a complete cDNA of human TPO predicted in Example 19.

The human liver cDNA library (hTPO-F1) constructed in Example 20 was divided into 3 pools (pool # 1 - 3). PCR was carried out using the plasmid DNA prepared from each pool as a template and the synthesized primers. As the result, a DNA fragment having the expected size was amplified when the plasmid DNA prepared from the pool # 3 was used. The # 3 pool was then divided into subpools, each containing 15,000 transformants, and screening using PCR was carried out as above. As the result, amplification of DNA having the expected size was found in 6 of the 90 pools. When one of these positive pools was divided into subpools each containing 1,000 clones, and plasmid DNA was extracted and PCR was carried out in the same manner, DNA amplification was not observed. It was considered to be caused by a low recovery of the plasmid DNA due to poorer growth of the clone of interest than the other clones. Therefore, the original # 3 pool was spread on 100 LB plates in such an inoculum size that 4,100 colonies grew on each LB plate, and a replica plate was prepared from each of the thus inoculated plates. When PCR using these DNAs extracted from colonies grown on the plate was carried out in the same manner as described above, amplification of a band expected was observed in one of 100 subpools.

Two replica filters were prepared from the plate of this subpool, and colony hybridization was carried out using the labeled probe(the EcoRI/BamHI fragment of plasmid pEF18S-HL34). As the result, a single signal considered to be positive was observed. The colonies were collected from the original plate and inoculated again onto an LB plate. DNA samples were prepared from a total of 50 colonies grown on the plate and were subjected to PCR to finally obtain a clone named pHTF1 ⟨Example 21⟩. In screening of cDNA clone, methods such as hybridization, PCR, and expression cloning were mainly used and combination of these methods increased efficiency and sensitivity of the screening, or decreased labor for it. When the nucleotide sequence of the thus obtained clone pHTF1 was determined, it was revealed that the clone pHTF1 had an open reading frame and the amino acid sequence of a protein considered to be encoded by the open reading frame completely coincided with the deduced amino acid sequence (SEQ ID NO: 6) of human TPO. The nucleotide sequence was different from the deduced one (SEQ ID NO: 196) at 3 positions, but these differences did not cause amino acid exchange. It is confirmed that human TPO protein comprises 353 amino acid residues having a 21 amino acid signal sequence. When the plasmid DNA was prepared from the thus obtained clone pHTF1 and transfected into COS1 cells, TPO activity gas found in the culture supernatant ⟨Example 23⟩.

(K) Screening of human TPO chromosomal DNA by means of plaque hybridization, sequence determination and expression of human TPO chromosomal DNA and confirmation of TPO activity

A genomic library given from Prof. T. Yamamoto at the Gene Research Center, Tohoku University was inoculated onto 18 NZYM plates in such an inoculum size that one plate contained 30,000 phage particles, and two replica filters were prepared from each of the thus prepared 18 plates.A human TPO cDNA fragment (base position numbers 178 to 1,025 in SEQ ID NO: 7) was amplified by PCR and purified. Using the purified fragment labeled with $^{32}P$ as a probe plaque hybridization was carried out. As the result, 13 positive signals were obtained. Plaques were collected from the original plates and inoculated again onto NZYM plates in such an inoculum size that 1,000 plaques were formed on each plate. Two replica filters were prepared from each of the resulting plates to carry out plaque hybridization under the same conditions described above. As the result, positive signals were detected on all filters of the 13 groups. A single plaque was recovered from each of the resulting plates to prepare phage DNA. Phage DNA samples thus prepared from the 13 clones were checked for the presence of the cDNA coding region by PCR. Five of the 13 clones seemed to contain entire amino acid coding region predicted from the cDNA.In consequence, one of these clones (clone λHGT1) was selected and analyzed by Southern blotting (using the aforementioned probe). Since a single band of about 10 kb was observed in the case of the HindIII digestion. DNA of the clone λHGT1 was digested with HindIII and subjected to agarose gel electrophoresis. The 10 kb band was excised from the gel, purified, and subcloned into a cloning vector pUC13 to finally obtain a clone named pHGT1 ⟨Example 24⟩.

When the nucleotide sequence of the thus obtained clone pHGT1 was determined, chromosomal DNA carried by the clone was found to contain an entire coding region of the protein predicted in Example 19, and nucleotide sequence of the coding region coincided completely with that of the predicted nucleotide sequence (SEQ ID NO: 196).

In addition, a region corresponding to the amino acid-encoding exon contained 4 introns and the nucleotide sequence was different from that of the complete cDNA carried by clone pHTF1 obtained in Example 21 (SEQ ID NO: 7).

When nucleotide sequences of 4 remaining clones among the 5 chromosomal DNA clones independently selected by the screening were determined, it was found that 2 of the 4 clones were identical to the clone pHGT1 and other 2 clones were the same as the clone pHGT1 except that they had a different nucleotide within 3' noncoding region as observed with the clone pHTF1 ⟨Example 25⟩.

An EcoRI fragment in the thus obtained plasmid clone pHGT1 was ligated with an expression vector pEF18S to obtain human TPO expression plasmid pEFHGTE. When the plasmid DNA (pEFHGTE) was prepared and transfected into COS1 cells, TPO activity was found in the culture supernatant ⟨Example 26⟩.

(L) Preparation of human TPO deletion derivatives, their expression in COS1 cells and confirmation of TPO activity

The results of Example 18 and 22 revealed that human TPO protein could exhibit its biological activity even after the removal of its carboxyl-terminal third. Thus, in order to further analyze the biologically active portions, deletion derivative experiments were performed. A series of expression plasmids were prepared by PCR using the DNA of the plasmid clone pHT1-231 obtained in Example 18 as a template and synthesized oligonucleotides as primers.

Expression plasmids which contained DNA coding for human TPO deletion derivatives lacking the carboxyl-terminal region of the TPO protein, that is, deletion derivatives coding for positions 1-211, 1-191, 1-171 and 1-163 amino acid sequence, were obtained. When the plasmid DNA was prepared from each of clones and transfected into COS1 cells, TPO activity was detected in each of the culture supernatant ⟨Example 27⟩.

When derivatives having a series of deletion of C-terminal amino acid residues up to the 151 position and other derivatives having respective deletion of N-terminal side amino acid residues up to the 6, 7 and 12 position were designed and the activity after their expression in COS 1 cells was measured in order to analyze the TPO activity-bearing region further in detail, the activity became undetectable when N-terminal side amino acid residues were deleted up to the 7 position or C-terminal side amino acid residues were deleted up to the 151 position. ⟨Examples 28 and 29⟩

Derivatives of a protein having its TPO biological activity can be obtained by modifying (deletion, substitution, insertion or addition) cDNA coding the protein. Methods such as PCR, site directed mutagenesis and chemical synthesis can be used for the modification.

(M) Expression of human TPO cDNA in CHO cell and purification of TPO.

An expression vector, pHTP1, was constructed which can express cDNA coding for a deduced amino acid sequence of human TPO as shown in SEQ ID NO: 6, in animal cells. ⟨Example 30⟩

The TPO cDNA region in pHTP1 was used to construct a vector pDEF202-hTPO-P1 for its expression in CHO cell. ⟨Example 31⟩

As a result of the transfection of the vector into CHO cells and subsequent selection of transfected cells, the transfected cells in which the expression vector carrying human TPO cDNA has been integrated into chromosomes of CHO cells were obtained. ⟨Example 32⟩

In Example 32, large scale cultivation was carried out for a human TPO producing CHO cell line (CHO 28-30 cell, resistant to 25 nM MTX) which has been prepared by transfecting the human TPO expression plasmid pDEF202-hTPO-P1 into CHO cells. ⟨Example 55⟩

From the culture supernatant 100 L, human TPO was purified. ⟨Example 56⟩

Alternatively, human TPO was purified from the culture supernatant in Example 55 in a different manner. ⟨Example 57⟩

(N) Expression of human TPO cDNA in X63.6.5.3. cells and confirmation of the activity

Using the TPO cDNA region encoded in the plasmid pBLTEN prepared in Example 30, an expression vector BMCGSneo-hTPO-P1 was constructed for use in X63.6.5.3. cells. ⟨Example 33⟩

When X63.6.5.3. cells were transfected with this vector, a transformant cell was obtained in which the human cDNA-encoding expression vector was integrated into its chromosome. When this cell was cultured, TPO activity was detected in the culture supernatant. ⟨Example 34⟩

(O) Large quantity expression of human TPO in COS 1 cells, and purification, molecular weight measurement and biological characteristics thereof

The expression vector pHTP1 prepared in Example 30 was transfected into COS 1 cells to obtain a large quantity (a total of about 40 liters) of culture supernatant containing the expressed product. ⟨Example 35⟩.

Purification of TPO was carried out from about 7 liters of the COS 1 cell serum-free culture supernatant prepared in Example 35 containing the expression vector pHTP1-derived TPO. It was able to obtain high activity TPO through the steps of a hydrophobic interaction chromatography, a cation exchange column chromatography, a WGA column chromatography and a reverse phase column chromatography. ⟨Example 36⟩

The TPO thus partially purified from the COS 1 cell culture supernatant was subjected to molecular weight measurement and biological characteristics analysis. ⟨Examples 37 and 38⟩

(P) Expression of human TPO in *E. coli*

A vector pGEX-2T/hT(1-174) was constructed for use in the expression of a fusion protein (to be referred to as "GST-TPO(1-174)") of glutathione-S-transferase and human TPO (amino acid residues of positions 1 to 174) in *E. coli*. In this case, a portion of the human TPO cDNA nucleotide sequence (about half the area of the 5'-side) was exchanged with *E. coli* preference codons. ⟨Example 39⟩

GST-TPO(1-174) was expressed in *E. coli*, the resulting cells were disintegrated and then the GST-TPO(1-174) contained in the precipitation fraction was solubilized. Next, as a result of the combination of various steps including examination of the TPO refolding conditions, examination of purification conditions (glutathione affinity column, cation exchange column and the like) and digestion of the GST protein region with thrombin, it was able to perform partial purification of a protein containing a TPO amino acid sequence as designed. It was confirmed that this protein shows TPO activity in the rat CFU-MK assay system. 〈Examples 40 and 41〉

Also, a vector pCFM536/h6T(1-163) was constructed for use in the expression in *E. coli* of a mutation type human TPO protein (to be referred to as "h6T(1-163)") in which the 1-position Ser residue and the 3-position Ala residue of human TPO (amino acid residues of positions 1 to 163) were respectively replaced by an Ala residue and a Val residue and, at the same time, a Lys residue and a Met residue were respectively added to the -1 and -2 positions. Entire portion of the human TPO cDNA nucleotide sequence (corresponding to 1 to 163 amino acid residues) carried by this vector was exchanged with *E. coli* preference codons. 〈Example 42〉

The h6T(1-163) was expressed in *E. coli*, the resulting cells were lysed and then solubilization of h6T(1-163) contained in the precipitated fraction and conditions for the refolding of the protein were examined, thereby succeeding in partially purifying a protein containing a TPO amino acid sequence as designed. It was confirmed that the protein shows TPO activity in the rat CFU-MK assay system. 〈Examples 43 and 44〉

In addition, a vector pCFM536/hMKT(1-163) was constructed for use in the expression in *E. coli* of a mutation type human TPO protein (to be referred to as "hMKT(1-163)") in which a Lys residue and a Met residue were respectively added to the -1 and -2 positions. The hMKT(1-163) was expressed in *E. coli* in the same manner as described in Example 43, and the expression protein was subjected to SDS-PAGE, transferred on a PVDF membrane and then subjected to N-terminal amino acid sequence analysis, thereby confirming that this protein contains the designed amino acid sequence. 〈Example 52〉

In addition, the vector pCFM536/hMKT(1-332) for expression in *E. coli* of a mutation type human TPO protein in which a Lys residue is added at the position-1 and Met residue at the position-2 of human TPO (amino acid positions 1 to 332) (referred to as "hMKT (1-332)") respectively was constructed. The hMKT (1-332) was expressed within *E. coli* in the same manner as in Example 42, and its expression was confirmed by Western blotting using the anti-human TPO peptide antibody prepared in Example 45 set forth below. 〈Example 66〉

(Q) <u>Preparation of anti-TPO peptide antibody and anti-TPO peptide antibody column</u>

Rabbit polyclonal anti-TPO peptide antibodies were prepared by synthesized peptides corresponding to three partial regions of the rat TPO amino acid sequence determined in Example 10. It was confirmed that these antibodies can recognize rat and human TPO molecules. Furthermore, peptides corresponding to 6 partial regions in the amino acid sequence of human TPO shown in SEQ ID NO: 6 (or SEQ ID NO: 7) were synthesized and then employed to prepare rabbit polyclonal anti TPO peptide antibodies. The resultant antibodies were confirmed to recognize human TPO. 〈Example 45〉

It is possible to purify TPO by an affinity column chromatography using a column packed with certain molecules having binding affinity for TPO, such as anti-TPO antibodies, TPO receptors and the like, are bound. In consequence, an anti-TPO antibody column was firstly prepared by binding the anti-TPO peptide antibody obtained in Example 45. 〈Example 46〉

(R) <u>Purification of human TPO expressed in COS 1 cells making use of anti-TPO peptide antibody column, and molecular weight and biological characteristics thereof</u>

Using a culture supernatant from the transfection of COS 1 cells with the expression vector pHTP1 as a starting material, partially purified TPO was obtained and applied to the anti-TPO antibody column. Since the TPO activity was found in the adsorbed fraction, this fraction was further subjected to a reverse phase column chromatography to purify the protein and examine its molecular weight and biological characteristics. 〈Example 47〉

(S) <u>Confirmation of the activity of partially purified sample of human TPO expressed in COS 1 cells</u>

The TPO-active fraction purified in Example 36, namely the TPO sample purified up to the step of Capcell Pak Cl 300A column from a culture supernatant obtained by transfecting COS 1 cells with the expression vector pHTP1, was checked for its biological activities to determine if it exerts a platelet number increasing action in the living body. 〈Example 48〉

Also, a crude TPO fraction obtained by a cation exchange column from 33 liters of a culture supernatant prepared by transfecting COS 1 cells with the expression vector pHTP1 was checked for its biological activities to find that it increases platelet number in the body. 〈Example 49〉

(T) Expression of human TPO chromosomal DNA in CHO cells and confirmation of the activity

A vector pDEF202-ghTPO was constructed for use in the expression of human TPO chromosome in CHO cells. 〈Example 50〉

When this vector was introduced into CHO cells, a transformant cell was obtained in which the human TPO chromosomal DNA-encoding expression vector was integrated into its chromosome. When this cell clone was cultured, TPO activity was detected in the culture supernatant. 〈Example 51〉

(U) Partial purification and activity confirmation of mutation type human TPO expressed in *E. coli*

The mutation type human TPO derived from the human TPO nucleotide sequence-encoding clone, pCFM536/h6T(1-163), and expressed in *E. coli* was subjected to refolding using guanidine hydrochloride and glutathione to find that the thus obtained h6T(1-163) exerts a platelet number increasing action in the living body. 〈Example 53〉

The mutation type human TPO derived from the human TPO nucleotide sequence-encoding clone, pCFM536/h6T(1-163), and expressed in *E. coli* was subjected to refolding using sodium N-lauroyl sarcosinate and copper sulfate and then to a cation exchange chromatography to find that the thus purified h6T(1-163) increases platelet number in the living body. 〈Example 54〉

In addition, the refolding and purification of the mutation type human TPO, h6T (1-163) was conducted using another procedures. 〈Examples 60 and 61〉

(V) Expression of human TPO cDNA in insect cells and identification of TPO activity

A recombinant virus for the expression of human TPO in insect cells was prepared 〈Example 58〉 and expressed in the insect cell Sf 21, and subsequently the TPO activity was identified in the culture supernatant. 〈Example 59〉

(W) Expression of human TPO (amino acid positions 1 to 163) in CHO cells and purification thereof

The vector pDEF202-hTPO163 for expression in CHO cells of the human TPO protein (referred to as "hTPO163") having amino acids 1 to 163 in the human TPO amino acid sequence shown in SEQ ID NO: 7 was constructed. The expression vector pDEF202-hTPO163 was transfected into CHO cells and thus-obtained hTPO163-producing CHO cell line was cultured in a large scale. From the supernatant of the culture hTPO163 was purified. 〈Examples 62 to 65〉

(X) Preparation of substitution derivatives of human TPO

A derivative (referred to as "N3/TPO") wherein Arg-25 and Glu-231 of human TPO have been substituted by Asn and Lys, respectively, was prepared as well as a derivative (referred to as "09/TPO") wherein His-33 has been replaced by Thr.

A plasmid coding for each derivative was transfected into COS7 cells which were then cultured. TPO activity was detected in the supernatant of the culture. 〈Example 67〉

Derivatives wherein one amino acid of h6T(1-163) has been substituted by another amino acid were prepared by using E. coli expression systems. TPO activity was found in each derivate. 〈Example 94〉

(Y) Preparation of insertion or deletion derivatives of human TPO

An amino acid is inserted into or deleted from hTPO163 to prepare its insertion or deletion derivatives, and thereafter COS7 cells are transfected with plasmids encoding the resultant derivatives. In the resultant derivatives. In the supernatant of the COS7 cell cultures, the TPO activity was detected. 〈Example 68〉

A method for the measurement of TPO activity (an *in vitro* assay system) used in the present invention is described in the following as "Reference Example".

(Z) Preparation and purification of anti-human TPO antibodies

Polyclonal antibodies are prepared using peptide fragments of human TPO 〈Example 69〉 and subsequently utilized in Western blot analysis 〈Example 70〉 and in the construction of anti-TPO antibody affinity columns 〈Example 71〉.

(AA) *In vivo* activity of human TPO

Purified human TPO is administered to mice with induced thrombocytopenia and changes in platelet counts are monitored versus a control group. 〈Example 72〉, through 〈Example 79〉. Pharmaceutical compositions are described as potential useful in treatment of thrombocytopenia. 〈Example 80〉 through 〈Example 89〉.

(BB) TPO activity as a function of MLP receptor binding

An assay is provided wherein TPO activity is defined in terms of specific binding interaction with the Mpl receptor. 〈Example 90〉 through 〈Example 93〉.

Reference Example

A. Rat megakaryocyte progenitor cell assay (rat CFU-MK assay) system (liquid culture system)

Megakaryocytes incorporate and store serotonin in cytoplasmic dense granules through an active, energy-dependent process (Fedorko, *Lab. Invest.,* vol.36. pp.310 - 320, 1977). This phenomenon can be observed already in small and mononuclear acetylcholinesterase-positive cells which are considered to be positioned between CFU-MK and recognizable megakaryocytes (Bricker and Zuckerman, *Exp. Hematol.*, vol. 12, p.672, 1984). The amount of serotonin incorporated increases in response to the increment of the size of megakaryocytes (Schick and Weinstein, *J. Lab. Clin. Med.*, vol.98, pp.607 - 615, 1981). In addition, it is known that such phenomenon is specific for only megakaryocytic cells among bone marrow cells (Schick and Weinstein, *J. Lab. Clin. Med.*, vol.98, pp.607 - 615, 1981). In the present assay system, highly enriched rat CFU-MK cells (GpIIb/IIIa$^+$ CFU-MK fraction which will be described later) are cultured in the presence of samples to be assayed, and incorporation of $^{14}$C-serotonin ($^{14}$C-hydroxy tryptamine creatine sulphate; $^{14}$C-5HT) into megakaryocytes grown from CFU-MK is measured.

Advantages of this assay system are that indirect influences of contaminated cells (for example, formation of Meg-CSF activity by contaminated cells stimulated by a certain substance other than the factor of interest, or formation of a certain factor by contaminated cells, which undergoes a combined action with the factor of interest) can be reduced, because the percentage of CFU-MK in cells of GpIIb/IIIa$^+$ CFU-MK fraction is markedly high (see the following [Assay method]) while the number of contaminating cells is small. In addition appropriate culture conditions can be maintained for a relatively long period, because the total number of cells seeded per one well is small. Another advantage is that a number of large-size mature megakaryocytes grown from CFU-MK in the presence of an active sample during a culture period can visually detected under a phase contrast microscope, thus rendering possible qualitative judgement of the presence and degree of the activity. Results of the qualitative judgement correspond well to the results of quantitative determination based on the incorporation of $^{14}$C-serotonin. Therefore, reliability of the quantitative determination can be further improved by joint use of the qualitative judgement.

Assay method:

Highly enriched rat CFU-MK cells (GpIIb/IIIa$^+$ CFU-MK fraction) used in the assay were prepared by slightly modifying the method of Miyazaki *et al.* (*Exp. Hematol.*, vol.20, pp.855 - 861, 1992).

Briefly, femur and tibia are removed from Wister rats (male, 8 to 12 weeks of age) to prepare bone marrow cell suspension as described. A suspending medium (a solution consisting of 13.6 mM trisodium citrate, 11.1 mM glucose, 1 mM adenosine, 1 mM theophylline, 10 mM HEPES (pH 7.25), 0.5% bovine serum albumin (to be referred to as "BSA" hereinafter) (Path-O-Cyte 4; Seikagaku Kogyo Co., Ltd.) and $Ca^{2+}$ and $Mg^{2+}$-free Hanks' balanced salt solution : to be referred to as "HATCH solution" hereinafter) which is a slightly modified version of the megakaryocyte separation medium reported by Levine and Fedoroko (Levine and Fedoroko, *Blood*, vol.50, pp.713 - 725, 1977) is used for the preparation of the bone marrow cell suspension. The thus prepared bone marrow cell suspension is layered over a Percoll discontinuous density gradient solution (density; 1.050 g/ml/1.063 g/ml/1.082 g/ml) which is prepared by diluting Percoll solution (manufactured by Pharmacia) with HATCH solution, and centrifuged at 20 °C at 400 x g for 20 minutes. After the centrifugation, cells between the 1.063 g/ml and 1.082 g/ml density layers are recovered. After washing, the recovered cells are suspended in Iscove's modified Dulbecco culture medium (to be referred to as "IMDM culture medium" hereinafter) containing 10% fetal calf serum (to be referred to as "FCS" hereinafter), put in 100-mm tissue culture plastic dishes and cultured at 37 °C for 1 hour in a 5% $CO_2$ incubator. After incubation, nonadherent cells are recovered and cultured again in plastic dishes at 37 °C for 1 hour. Non adherent cells are suspended in HATCH solution and incubated for 1 hour on 100-mm bacteriological petri dishes to which a mouse monoclonal anti-rat platelet GpIIb/IIIa antibody, P55 antibody (Miyazaki et al., Thromb. Res., vol.59, pp.941 - 953, 1990) has previously been adsorbed. After incubation, nonadherent cells are removed by thorough washing with HATCH solution, and the remaining cells adsorbed to the immobilized P55 antibody are detached by pipetting and collected. In general, 3-4 x $10^5$ cells are obtained from one rat. The thus obtained cell fraction contains highly enriched rat CFU-MK (to be referred to as "GpIIb/IIIa$^+$ CFU-MK fraction" hereinafter), and it is known that this cell fraction contains about 5 to 10% of CFU-MK based on the measurement by a colony assay system in the presence of a saturated concentration of rat IL-3. A hybridoma capable of producing the aforementioned P55 antibody has been deposited under the deposit No. FERM BP-4563 in National Institute of Bioscience and Human Technology, Agency of Industrial Science and Technology, Ministry of International Trade and Industry on February 14, 1994.

Next, cells of the thus obtained GpIIb/IIIa$^+$ CFU-MK fraction are suspended in IMDM culture medium containing 10% FCS and dispensed in portions of $10^4$ cells per well of a 96 well tissue culture plate. Each well is further supplied with a standard sample (will be described later in detail), or a sample to be assayed, thereby adjusting the final medium volume to 200 µl/well. The thus prepared plate is put in a $CO_2$ incubator and incubated for 4 days at 37 °C. On 4th days of incubation, 0.1 µCi (3.7 KBq) of $^{14}C$-serotonin is added to each well 3 hours before the completion of culturing, and the final incubation is continued at 37 °C. After 3-hr incubation, the plate is centrifuged at 1,000 rpm for 3 minutes, and the resulting supernatant fluid is removed by suction. A 200 µl portion of PBS containing 0.05% EDTA is added to each well, and the plate is centrifuged to wash the plate by removing the resulting supernatant fluid. This washing step is repeated once again. A 200 ml portion of 2% Triton X-100 is added to the thus obtained pellet of cells in each well, and the plate is shaken on a plate mixer for approximately 5 to 10 minutes to lyse the cells thoroughly. A 150 µl portion of the thus obtained cell lysate is transferred into a commercial cap-shaped solid scintillator (Ready Cap; manufactured by Beckman) which is subsequently left overnight at 50 °C in an oven to dry the lysate. On the next day, the Ready Cap is put into a glass vial to measure radioactivity of $^{14}C$ by a liquid scintillation counter.

In this instance, almost the same results as those of the $^{14}C$-serotonin incorporation assay can be obtained when acetylcholinesterase activity in the aforementioned cell lysate is measured according to the procedure of Ishibashi and Burstein (Blood, vol.67, pp.1512 - 1514, 1986).

Standard samples:

Firstly, blood plasma of thrombocytopenic rats was prepared in the following manner, for use in the preparation of standard samples.

Normal male Wistar rats (7 to 8 weeks old) were rendered thrombocytopenic by intravenous administration of the aforementioned P55 antibody with a dose of 0.5 mg per animal, twice with an interval of about 24 hours. About 24 hours after the second administration, blood was collected from the abdominal aorta under ether anesthesia, using a 10 ml capacity syringe in which 1 ml of 3.8% (v/v) trisodium citrate solution has been included as an anti-coagulation agent. The thus collected blood sample was dispensed into plastic centrifugation tubes and centrifuged at 1,200 x g for 10 minutes to recover blood plasma fraction. The thus recovered blood plasma fraction was again centrifuged at 1,200 x g for 10 minutes, and the resulting blood plasma fraction was recovered, taking care to avoid the pellet consisting of cells, platelets and the like, and pooled. (The blood plasma obtained in such a manner is referred to as "TRP" hereinafter.) Ca-treated TRP (standard sample C), WGA-Agarose column active fraction (standard sample W) or Phenyl Sepharose 6 FF/LS column active fraction (standard sample P) obtained from TRP in accordance with the procedure of Example 1 were dialyzed extensively against a sufficient volume of IMDM culture medium and used as assay standards.

In the rat TPO purification process described in Example 1, the standard sample C was used in the early stage, but was changed to the standard sample W halfway thereafter and then to the standard sample P. Specific activity of the standard sample C was defined tentatively as 1, and relative activities of the standard samples W and P were calculated based on the definition. Relative activity of each sample to be assayed was determined by comparing the dose-response curve of the standard sample to those of the samples to be assayed, and relative activity of the sample to be assayed was defined as n where its activity is n times higher than that of the standard sample C.

B. Colony assay system

In this assay system, bone marrow cells are cultured in a semi-solid culture medium in the presence of a sample to be assayed, and the Meg-CSF activity is measured by counting the number of megakaryocyte colonies formed by the proliferation and differentiation of CFU-MK.

Assay method:

(a) In the case of the use of unseparated rat bone marrow cells and the like

A 1 ml final volume of IMDM culture medium containing un-separated rat bone marrow cells, cells obtained in each separation step of GpIIb/IIIa$^+$ CFU-MK of the aforementioned assay system A, or cells of GpIIb/IIIa$^+$ CFU-MK fraction, and 10% FCS, 2 mM glutamine, 1 mM sodium pyruvate, 50 $\mu$M 2-mercaptoethanol and 0.3% agar (AGAR NOBLE, manufactured by DIFCO) was put in a 35-mm tissue culture plastic dish and, after solidification at room temperature, cultured at 37°C in a $CO_2$ incubator. In general, the number of cells per one dish is adjusted to 2-4 x $10^5$ in the case of unseparated bone marrow cells, 2-5 x $10^4$ in the case of cells at the step of the Percoll density gradient or of the adherence depletion, or 0.5-2 x 103 in the case of cells of GpIIb/IIIa$^+$ CFU-MK fraction. After 6 to 7 days of the culture, the agar disks are detached from the dishes and placed on glass slides (76 mm x 52 mm). To dry each agar disk, a piece of 50-$\mu$m nylon mesh and filter paper are placed, in that order, over the gel. The thus dried agar slides are heat-fixed for 5 minutes at 50°C on a hot plate and soaked for 2 to 4 hours in an acetylcholinesterase staining solution prepared according the procedure of Jackson (*Blood*, vol.42, pp.413 - 421, 1973). When sufficient staining of megakaryocytes is confirmed, the agar slides are washed with water, dried, subjected to post-staining with Harris' hematoxylin solution for 30 seconds, washed with water and then air-dried. Megakaryocyte colonies are defined as 3 or more tightly grouped acetylcholinesterase-positive cells.

(b) In the case of the use of unseparated mouse bone marrow cells

The colony assay can be performed in the same manner as the above procedure (a) using 2-4 x $10^5$ cells per one dish.

(c) In the case of the use of human bone marrow cells or human cord blood cells

Human bone marrow cells or human cord blood cells may be used as such, or as CFU-MK fractions enriched by the following manner.

Firstly, bone marrow fluid or cord blood is layered over Lymphoprep (manufactured by Daiichi Kagaku Co., Ltd.) and centrifuged, and the resulting interface leucocyte fraction is recovered. From the thus recovered cell fraction, cells to which biotinylated monoclonal antibodies specific for human surface antigens (CD2, CD11c and CD19) bind are removed with avidin-linked magnetic beads. The cells removed by the magnetic beads method are mainly B cells, T cells, macrophages and a part of granulocytes. The remaining cells are stained with FITC-labeled anti-CD34 antibody and PE-labeled anti-HLA-DR antibody, and then a CD34- and HLA-DR-positive fraction is recovered using a cell sorter (for example, ELITE manufactured by COULTER). CFU-MK cells are concentrated in this fraction (to be referred to as "CD34$^+$DR$^+$ CFU-MK fraction" hereinafter). Colony assay of human cells can be carried out in the same manner as the aforementioned case of the use of rat bone marrow cells, except that 3-5 x $10^3$ cells of the CD34$^+$DR$^+$ CFU-MK fraction are used in one dish and a mixture of 12.5% human AB plasma and 12.5% FCS is used instead of 10% FCS. The culturing period for the formation of megakaryocyte colonies is 12 to 14 days. For the detection of human megakaryocytes, immunological staining of megakaryocytes is carried out by an alkaline phosphatase-anti-alkaline phosphatase antibody method with a mouse monoclonal antibody specific for a megakaryocyte surface antigen GpIIb/IIIa (for example, Teramura *et al.*, *Exp. Hematol.*, vol. 16, pp. 843 - 848, 1988), and colonies each consisting of 3 or more megakaryocytes are counted.

C. Assay system with a human megakaryoblast cell line (M-07e assay)

M-07e cells, a human megakaryoblast cell line, proliferate in response to GM-CSF, IL-3, SCF, IL-2 and the like (Avanzi *et al.*, *J. Cell. Physiol.,* vol.145, pp.458 - 464, 1990). Since these cells also respond to TPO, they are applicable to a substitutive assay system for the rat CFU-MK assay system.

Assay method:

M-07e cells maintained in the presence of GM-CSF are recovered, washed thoroughly and then suspended in IMDM culture medium containing 10% FCS. The resulting M-07e cell suspension is dispensed in portions of $10^4$ cells into wells of a 96 well tissue culture plate, and each well is further supplied with a standard sample or a sample to be assayed, thereby adjusting the final volume to 200 µl/well. The thus prepared plate is put in a 5% $CO_2$ incubator and incubated for 3 days at 37° C. After 3 days of culture, 1 µCi (37 KBq) of $^3$H-thymidine is added to each well 4 hours before the completion of culturing. After completion of the culturing, the cells are collected on a glass fiber filter using a cell harvester to measure $^3$H radioactivity with a liquid scintillation counter (for example, Beta Plate manufactured by Pharmacia).

D. An assay system (Ba/F3 assay) in which a mouse pro-B cell line is used

Mouse pro-B cell line Ba/F3 is known as a cell line which proliferates in response to IL3, IL4 and the like (Palacios *et al.*, *Cell*, vol.41, pp.727 - 734). Since its substrain BF-TE22 is capable of cell proliferation in response to not only IL3 and IL4 but also TPO, it can be applied to an assay system as a substitute for the rat CFU-MK assay or the human megakaryoblastic cell line-aided assay (M-07e assay) is as follows. Briefly, BF-TE22 cells growing in Iscove's modified DME medium(IMDM:GIBCO) in the presence of 1 ng/ml of mouse IL-3 are recovered, washed three times with IMDM and suspended in IMDM medium containing 10% FCS. Next, the cell suspension is dispensed into wells of a 96 well tissue culture plate of a density of 1 x $10^4$ cells per well, each well is further supplemented with a standard TPO solution or a sample to be tested and adjusted to a final volume of 200 µl/well. The resulting plate is incubated for 2 to 3 days in a 5% $CO_2$ incubator. On the 2nd or 3rd day, 1 µCi (37 KBq) of $^3$H-thymidine is added to each well and the culturing is continued for additional 4 hours. Finally, the cells are collected on a glass fiber filter using a cell harvester to measure $^3$H radioactivity with a liquid scintillation counter. In this assay system, almost all of cells cultured in media lacking TPO activity die and therefore do not incorporate $^3$H-thymidine. However, cells cultured in media containing TPO activity show active proliferation in a TPO concentration-dependent manner and incorporate $^3$H-thymidine. In addition, results of this assay are parallel to those of the M-07e and CFU-MK assays.

The following examples are provided to further illustrate the present invention.

**EXAMPLE 1-1**

Purification of rat TPO from blood plasma of thrombocytopenic rats induced by anti-platelet antibody administration Preparation of blood plasma of thrombocytopenic rats induced by anti-platelet antibody administration

TRP was prepared as the purification source from about 1,000 rats in accordance with the procedure described in the aforementioned ⟨Reference Example⟩ A, rat megakaryocyte precursor cell (CFU-MK) assay system.

Purification of rat TPO from TRP

Initially, purification was carried out using TRP of about 1,000 rats on an assumption that TPO content in TRP would be one per three million at the most, and a little less than 1 pmole of partially purified rat TPO was obtained. Though it is difficult in general, an attempt was made to analyze its partial amino acid sequences and three partial amino acid sequences were obtained but with a low accuracy. These sequences coincided with or are close to the amino acid sequence of a serine protease inhibitor (SPI) produced in the liver. It was unclear from these results whether TPO has a structure similar to that of the serine protease inhibitor or the sequences are derived from those of contaminated proteins other than TPO. Using these uncertain sequences, cloning of TPO gene from a rat cDNA library was carried, but potential TPO-encoding genes could not be obtained. Thereafter, intensive studies were carried out based on an assumption that such a failure was due to low purity and insufficient amount of the sample analyzed. In order to obtain a purified final sample necessary for the amino acid analysis, purification was carried out in accordance with a process described in the following ⟨Example 1-2⟩. Surprisingly, it was estimated from results of ⟨Example 1-2⟩ that the TPO content in TRP or XRP (will be described below) was such a extremely small amount as one per one hundred million to one billion of total plasma proteins, so that its complete purification was extremely difficult.

## EXAMPLE 1-2

Purification of rat TPO from blood plasma of thrombocytopenic rats induced by total body X-ray or γ-ray irradiation [Preparation of blood plasma of thrombocytopenic rats induced by total body X-ray or g-ray irradiation]

Normal male Wister rats (7 to 8 weeks old) were rendered thrombocytopenic by whole body irradiation with X-rays or γ-rays at a sublethal dose (6 to 7 Gy). Blood was collected from the rats on the 14th day after irradiation and a blood plasma fraction (to be referred to as "XRP" hereinafter) was prepared in the same manner as the aforementioned procedure for the preparation of TRP.

In this case, XRP (ca. 8 L) prepared from a total of about 1,100 rats was used as the purification source.

Purification of rat TPO from XRP

The blood plasma sample of about 1,100 rats was too large to process at one time because its total protein content reached 493,000 mg. In consequence, the blood plasma sample was divided into 11 lots, each corresponding to about 100 rats, in the following purification steps (1) to (4). In the subsequent purification steps (5) to (7), the sample was divided into 6 batches. In and after the purification step (8), a sample crudely purified from the total blood plasma of about 1,100 rats was used. In the following, typical example of each purification step is described in the case of a lot (XW9) and a batch (XB6).

In all of the purification steps, TPO activity was measured using the rat CFU-MK assay system described in ⟨Reference Example⟩. Unless otherwise noted, all purification steps were carried out at 4°C, excluding the reverse phase chromatographies, and Superdex 75pg gel filtration in the presence of surfactant, which were carried out at room temperature. Protein assay was carried out by a Coomassie dye binding assay (a reagent system manufactured by PIERCE, catalog No. 23236X) or a method using bicinchoninic acid (a reagent system manufactured by PIERCE, catalog No. 23225).

An outline of the purification is shown in Table 1.

Table 1 Purification of rat plasma TPO

| | Step | Total protein (mg) | Relative activity | Total activity | Activity recovery (%) |
|---|---|---|---|---|---|
| | Total plasma | 493000 | — | — | — |
| | Ca-Treated plasma/G25 | 480300 | 2 | 864600 | 100 |
| <Ion exchange> | Q-Sepharose FF | 314400 | 9 | 704000 | 313 |
| <Lectin> | WGA-Agarose | 15030 | 132 | 1987000 | 230 |
| <Triazine dye> | TSK-gel AF-Blue 650MH | 4236 | 905 | 3834000 | 443 |
| <Hydrophobic> | Phenyl-Sepharose 6 FF/LS | 2762 | 847 | 2339000 | 271 |
| <Gel filtration> | S-200HR F3 (low mol. TPO) | 51 | 20000 | 1020000 | 118 |
| | S-200HR F2 (high mol. TPO) | 262 | 7838 | 2055000 | 238 |

Low molecular TPO (from S-200HR F3)

| | Step | Total protein (mg) | Relative activity | Total activity | Activity recovery (%) |
|---|---|---|---|---|---|
| <Gel filtration> | S-200HR F3 (low mol. TPO) | 50.3300 | 20000 | 1007000 | 116 |
| <Reverse phase> | YMC Protein-RP prep | 2.8540 | 130000 | 371000 | 43 |
| <Reverse phase> | YMC CN-AP | 0.3730 | 800000 | 298400 | 35 |
| (Reverse phase> | Capcell Pak C1 | 0.0396 | 4890000 | 193600 | 22 |
| <Electrophoresis> (under non-reduction cond.) | 15% SDS-PAGE | 0.0017 | 149000000 | 250300 | 29 |

High molecular TPO (from S-200HR F2)

| | Step | Total protein (mg) | Relative activity | Total activity | Activity recovery (%) |
|---|---|---|---|---|---|
| <Gel filtration> | S-200HR F2 (high mol. TPO) | 257.0000 | 7840 | 2015000 | 233 |
| <Reverse phase> | YMC Protein-RP prep | 11.4000 | 227000 | 2588000 | 300 |
| <Gel filtration> | Superdex 75pg/CHAPS | 1.1660 | 1750000 | 2041000 | 236 |
| <Reverse phase> | YMC CN-AP | 0.6200 | 700000 | 434000 | 50 |
| <Reverse phase> | Capcell Pak C1 | 0.0630 | 20000000 | 1260000 | 146 |
| <Electrophoresis> (under non-reduction cond.) | 15% SDS-PAGE | 0.0030 | 330000000 | 990000 | 117 |

(1) Calcium chloride treatment, centrifugation and protease inhibitor treatment of rat blood plasma

In the case of lot No. XW9

XRP (742 ml; protein concentration, 54.8 mg/ml; total protein, 40,686 mg) corresponding to about 100 rats, which had been stored at -80 °C, was thawed and dispensed into polypropylene centrifugation tubes (manufactured by Nalgene). Calcium chloride powder was added to each tube to a final concentration of 100 mM. After overnight incubation at 4 °C, the resulting mixture was centrifuged at 8,000 rpm for 60 minutes. To the resulting TPO-active supernatant fluid (742 ml; protein concentration, 54.9 mg/ml; total protein, 40,740 mg) was added a protease inhibitor p-APMSF ((p-aminodiphenyl)methanesulfonyl fluoride hydrochloride, manufactured by Wako Pure Chemical Industries, Ltd., catalog No. 010-10393) to a final concentration of 1 mM. The thus obtained sample was used in the following Sephadex G-25 column buffer exchange step.

In this manner, calcium chloride/p-APMSF treatment of the whole XRP derived from about 1,100 rats (total volume, 8,184 ml; total protein, 493,000 mg) was effected by dividing it into 11 lots, each lot being equivalent to about 100 rats, and carrying out treatment of these lots one by one. The resulting 11 samples were processed separately in the subsequent Sephadex G-25 column step.

(2) Sephadex G-25 ⟨buffer exchange⟩

In the case of lot No. XW9

The supernatant fluid obtained after the calcium treatment of the step (1) (742 ml; protein concentration; 54.9 mg/ml; total protein, 40,740 mg) was applied at a flow rate of 40 to 70 ml/min to a Sephadex G-25 column (manufactured by Pharmacia Biotech, catalog No. 17-0033-03; diameter, 11.3 cm: bed height, 47 cm) which had been equilibrated in advance with 20 mM Tris-HCl, pH 8. Elution was carried out with the same buffer. A 1.300 ml portion of the eluate before the elution of protein was discarded. When UV absorption was detected in the eluates, fractions were pooled until electric conductivity reached 500 μS/cm, thereby recovering a TPO-active protein fraction exchanged by 20 mM Tris-HCl, pH 8 (1,377 ml; protein concentration, 27.56 mg/ml; total protein, 37,882 mg; protein yield, 93%). Relative activity of TPO in the fraction was found to be 2.3.

As the result of the Sephadex G-25 column treatment of the whole lots of samples, a total of 21,117 ml of the Sephadex G-25 column TPO-active fraction was obtained (total protein, 480,300 mg; average relative activity, 2; total activity, 864,600).

(3) Q-Sepharose FF ⟨strong anion exchange chromatography⟩

In the case of lot No. XW9

The TPO-active fraction obtained in the above step (2) by the Sepharose G-25 treatment (1,377 ml; protein concentration, 27.5 mg/ml; total protein, 37,841 mg; relative activity, 2.3) was applied to a Q-Sepharose FF (manufactured by Pharmacia Biotech, catalog No. 17-0510-01; diameter, 5 cm; bed height, 27 cm) at a flow rate of 40 ml/min, eluted with 20 mM Tris-HCl (pH 8) and a fraction F1 which passed through the column was pooled (3,949 ml; protein concentration, 0.98 mg/ml; total protein, 3,870 mg; relative activity, 0).

Next, the buffer was changed to 20 mM Tris-HCl (pH 8) containing 175 mM NaCl to elute a TPO-active fraction F2 (4,375 ml; protein concentration, 5.36 mg/ml).

Finally, a fraction F3 (1,221 ml; protein concentration, 3.9 mg/ml; total protein, 4,783 mg; relative activity, 3.8) was eluted with 20 mM Tris-HCl (pH 8) containing 1,000 mM NaCl. Total protein in the TPO-active fraction F2 was found to be 23,440 mg, and protein yield of F2 by this step was 61.9%. In addition, relative activity of TPO was increased to 6.8.

As the result of the application of the whole lots of the Sephadex G-25 TPO-active fractions to the Q-Sepharose FF, a total of 35,842 ml of the Q-Sepharose FF TPO-active fraction F2 was obtained (total protein, 314,384 mg; average relative activity, 9; total activity, 2,704,000).

(4) Wheat germ agglutinin (WGA)-Agarose ⟨lectin affinity chromatography⟩

In the case of lot No. XW9

The TPO-active fraction F2 obtained in the above step (3) by the Q-Sepharose FF treatment was divided into three portions and applied to a WGA-Agarose (manufactured by Honen Corp., catalog No. 800273; diameter, 5 cm; bed height, 22.5 cm) at a flow rate of 5 ml/min, and eluted with Dulbecco's isotonic phosphate buffer (DPBS). A fraction F1 which passed through the column was pooled (9,336 ml; protein concentration, 2.30 mg/ml; total protein, 21,407 mg; relative activity, 6.9).

Next, the buffer was changed to 20 mM sodium phosphate buffer (pH 7.2) containing 0.2 M N-acetyl-D-glucosamine (GlcNAc, manufactured by Nacalai tesque, catalog No. 005-20), 150 mM NaCl and 0.02% sodium azide, and the resulting eluates were pooled and concentrated using an ultrafiltration unit (Omega Ultrasette, nominal molecular weight cutoff of 8,000; manufactured by Filtron), thereby obtaining a WGA-Agarose adsorbing TPO-active fraction F2 (2,993 ml; protein concentration, 0.376 mg/ml).

Total protein in the TPO-active fraction F2 was found to be 1,125 mg, and protein yield of F2 by this step was 4.8%. In addition, relative activity of TPO was increased to 101. The thus obtained F2 fraction was stored at -80°C.

As the result of the application of the whole lots of the Q-Sepharose FF TPO-active F2 fractions to the WGA-Agarose, a total of 33,094 ml of the WGA-Agarose TPO-active fraction F2 was obtained (total protein, 15,030 mg; average relative activity, 132; total activity, 1,987,000).

(5) TSK-gel AF-BLUE 650 MH ⟨triazine dye affinity chromatography⟩

In the case of batch No. XB6

The WGA-Agarose adsorbing TPO-active fraction of lot No. XW8 and the WGA-Agarose adsorbing TPO-active fraction F2 of lot No. XW9 obtained in the above step (4) starting from 215 rats-equivalent XRP were combined as a batch No. XB6 (5,947 ml, protein concentration, 0.388 mg/ml; total protein, 2,319 mg; relative activity, 150).

A 5,974 ml portion of the combined sample was mixed with 0.85 moles of NaCl for 1,000 ml (296.76 g in total) to make a 6,132 ml of solution having a final NaCl concentration of 0.822 M, and the resulting solution was applied at a flow rate of 7 ml/min to a TSK-gel AF-BLUE 650 MH column (TOSOH CORP., catalog No. 08705; diameter, 5 cm; bed height, 23 cm) which had been equilibrated in advance with 20 mM sodium phosphate buffer (pH 7.2) containing 1 M NaCl.

After completion of the application, protein was eluted using 20 mM sodium phosphate buffer (pH 7.2) containing 1 M NaCl at a flow rate of 10 ml/min. The resulting eluates were pooled and concentrated using an ultrafiltration unit (Omega Ultrasette, nominal molecular weight cutoff of 8,000), thereby obtaining a passed-through fraction F1 (543 ml; protein concentration, 2.05 mg/ml; total protein, 1,112 mg; relative activity, 31).

Next, the elution buffer was changed to 2 M NaSCN to obtain an eluted TSK-gel AF-BLUE 650 MH adsorbing TPO-active fraction F2 (1,427 ml; protein concentration, 0.447 mg/ml).

Total protein in the TPO-active fraction F2 was found to be 638 mg, and protein yield of F2 by this step was 27.5%. In addition, relative activity of TPO was increased to 1,500.

As the result of the application of all of the batches of the WGA-Agarose adsorbing TPO-active F2 fractions to the TSK-gel AF-BLUE 650 MH, a total of 10,655 ml of the TSK-gel AF-BLUE 650 MH TPO-active fraction F2 was obtained (total protein, 4,236 mg; average relative activity, 905; total activity, 3,834,000).

(6) Phenyl Sepharose 6 FF/LS ⟨hydrophobic interaction chromatography⟩

In the case of batch No. XB6

A 1,424 ml portion of the TSK-gel AF-BLUE 650 MH TPO-active fraction F2 (1,424 ml; protein concentration, 0.447 mg/ml; total protein, 638 mg; relative activity, 1,500) was mixed with 1.5 moles of ammonium sulfate powder per 1,000 ml (282.2 g in total) to make a 1,581 ml of solution having a final ammonium sulfate concentration of 1.35 M.

The resulting solution was applied at a flow rate of 7 ml/min to a Phenyl Sepharose 6 FF (Low Sub) column (Pharmacia Biotech, catalog No. 17-0965-05; diameter, 5 cm; bed height, 10 cm) which has been equilibrated in advance with 50 mM sodium phosphate buffer (pH 7.2) containing 1.5 M ammonium sulfate. After completion of the application, elution was carried out using 36 mM sodium phosphate buffer containing 0.8 M ammonium sulfate at a flow rate of 10 ml/min The resulting eluates (about 3,160 ml) were pooled and concentrated using an ultrafiltration unit (Omega Ultrasette, nominal molecular weight cutoff of 8,000), thereby obtaining a fraction F1 (485 ml; protein concentration, 0.194 mg/ml; total protein, 94.2 mg; relative activity, 0).

Next, the elution buffer was changed to 20 mM sodium phosphate buffer (pH 7.2) to obtain an eluted TPO-active fraction F2 (about 3,500 ml). The eluted fraction was concentrated using an ultrafiltration unit (Omega Ultrasette, nominal molecular weight cutoff of 8,000) and a sample for assay was taken out. At this stage, protein concentration and total protein of the TPO-active fraction F2 (220 ml) were found to be 1.45 mg/ml and 319 mg, respectively, and protein yield of F2 by this step was 50.0%. Relative activity of TPO was found to be 1,230.

As the result of the application of all of the batches of the TSK-gel AF-BLUE 650 MH TPO-active F2 fractions to the Phenyl Sepharose 6 FF/LS, a total of 1,966 ml of the Phenyl Sepharose 6 FF/LS TPO-active fraction F2 was obtained (total protein, 2,762 mg; average relative activity, 847; total activity, 2,339,000).

(7) Sephacryl S-200 HR ⟨gel filtration chromatography⟩

In the case of batch No. XB6 (cf. Fig. 1)

The Phenyl Sepharose 6 FF/LS TPO-active fraction F2 (217 ml; protein concentration, 1.45 mg/ml; total protein, 315 mg; relative activity, 1,230) was mixed with 144.8 ml of 5 M NaCl solution to make a 362 ml solution having a final NaCl concentration of 2 M, and the resulting solution was concentrated to about 50 ml using an ultrafiltration unit with YM 3 membrane (76 mm in diameter, manufactured by Amicon Corp.).

To this was added the same volume (50 ml) of 8 M urea, thereby obtaining about 100 ml of a solution containing 1 M NaCl and 4 M urea as final concentrations. This was concentrated to about 80 ml, made into a sample of 88.78 ml and then applied to a Sephacryl S-200 HR column (manufactured by Pharmacia Biotech, catalog No. 17-0584-01; 7.5 cm in diameter and 100 cm in bed height).

Thereafter, elution was effected with DPBS at a flow rate of 3 ml/min, and the eluates after a void volume of 1,200 ml were collected in 45 ml portions in 60 polypropylene tubes. Assay was carried out every two tubes and the rest were stored at -85 °C until the Sephacryl S-200 HR treatment of all samples derived from 1,100 rats was completed. Based on the assay results, fractions of the batch No. XB6 were grouped as follows.

(F1) tube numbers 1 to 15 (fractions around void volume; molecular weight, 94,000 or more)
(F2) tube numbers 16 to 26 (molecular weight, 94,000 to 33,000)
(F3) tube numbers 27 to 44 (molecular weight, 33,000 to 3,000)
(F4) tube numbers 45 to 55 (molecular weight, 3,000 or less)

In this manner, all batches of the TPO-active F2 fractions obtained by Phenyl Sepharose 6 FF/LS were separately subjected to Sephacryl S-200 HR, assayed and stored at -85 °C. After completion of the S-200 HR treatment of all batches and just before the subsequent operation of reverse phase chromatography (YMC-Pack PROTEIN-RP), these stored samples were thawed and concentrated using an ultrafiltration unit with YM 3 membrane (76 mm in diameter, manufactured by Amicon Corp.) to obtain the following two samples. The concentrated sample of the TPO-active fraction F2 obtained by the Sephacryl S-200 HR is referred to as "high molecular TPO sample F2" hereinafter, and that of F3 as " low molecular TPO sample F3".

The high molecular TPO sample F2 and the low molecular TPO sample F3 are pooled fractions of different elution areas in the gel filtration chromatography as a matter of convenience, and the term "high molecular" and "low molecular" therefore may not mean their true molecular weights.

| | High molecular TPO sample F2 | Low molecular TPO sample F3 |
|---|---|---|
| Molecular weight | 94,000 - 33,000 | 33,000 - 3,000 |
| Total volume | 3,420 ml | 6,480 ml |
| Total volume (after concentration) | 293 ml | 280 ml |
| Total protein | 262 mg | 51.3 mg |
| Average relative activity | 7,838 | 20,000 |
| Total activity | 2,055,000 | 1,020,000 |

The low molecular TPO sample F3 and the high molecular TPO sample F2 are separately subjected to the subsequent purification steps.

Purification of the low molecular TPO sample F3 is described in the following steps (8) to (11).

(8) YMC-Pack PROTEIN-RP ⟨reverse phase chromatography⟩

The low molecular TPO sample F3 (total protein, 50.3 mg; protein concentration, 0.184 mg/ml; relative activity, 20,000; total activity, 1,007,000; total volume, 274 ml) obtained in the above step (7) was mixed with a solvent A (0.025% trifiuoroacetic acid (TFA)) and a solvent B (1-propanol containing 0.025% TFA) to obtain a solution having a total volume of 508.63 ml and final propanol, TFA and protein concentrations of about 20%, 0.012% and 0.0989 mg/ml, respectively. Insoluble materials formed during the preparation of this solution were separated by centrifugation and the resulting supernatant was divided into two 254.3 ml (25.2 mg protein) portions and applied at a flow rate of 2 ml/min to a column packed with YMC-Pack PROTEIN-RP (manufactured by YMC, catalog No. A-PRRP-33-03-15; 3 cm in diameter and 7.5 cm in bed height) which has been equilibrated in advance with 30% B. The precipitate resulting from the centrifugation was dissolved in 20 ml of 20 mM sodium acetate (pH 5.5) containing 5 mM CHAPS (3-[3-cholamidopropyl)-dimethylammonio]-1-propane sulfonate: manufactured by Dojindo Laboratories,. catalog No. 75612-03-3) and also applied to the column.

After the sample loading, about 50 ml of a solvent (solvents A:B = 3:1) was passed through the column to obtain a passed-through fraction. Next. a developing program (120 minutes of linear gradient from 30% B to 45% B) was started, and the eluates were collected in 36 polypropylene tubes in 10 ml portions. This process was repeated again for the remaining sample using the same collection tubes, thus obtaining a total of 36 fraction tubes each containing 20 ml eluate. The passed-through fraction was directly concentrated to 20 ml using an ultrafiltration unit with YM 3 membrane (76 mm in diameter, manufactured by Amicon Corp.).

A 0.1 ml portion of each of the passed-through fraction and the 20 ml fractions of tube numbers 1 to 36 was mixed with 20 µl of 5% BSA, dried by evaporation, dissolved in 0.25 ml of IMDM assay culture medium and then assayed to specify TPO-active fractions. As the result, the TPO activity was found in the fractions of tube numbers 17 to 27 (36.0 to 43.0% propanol concentration range) which were combined and used as a low molecular TPO sample F3-derived YMC-Pack PROTEIN-RP TPO-active fraction F2. This fraction was stored at -85°C until its use in the subsequent YMC-Pack CN-AP step.

| Low molecular TPO sample F3-derived YMC-Pack PROTEIN-RP TPO-active fraction F2 | |
|---|---|
| Total volume | 220 ml |
| Protein concentration | 0.0130 mg/ml |
| Total protein | 2.85 mg |
| Relative Activity | 130,000 |
| Total activity | 371,000 |

(9) YMC-Pack CN-AP ⟨reverse phase chromatography⟩

A 214.9 ml portion of the low molecular TPO sample F3-derived YMC-Pack PROTEIN-RP TPO-active fraction F2 (total protein, 2.79 mg; protein concentration, 0.0130 mg/ml; relative activity, 130,000; total activity, 36,300) obtained in the above step (8) was mixed with 0.6 ml of 50% glycerol and concentrated to 1.8 ml. The concentrate was finally adjusted to a volume of 5 ml containing 20% or less of propanol and about 6% of glycerol.

The concentrate was divided into 5 portions for use in the following column operation (0.555 mg protein and 1 ml volume for each operation). Each of the thus divided samples was applied at a flow rate of 0.6 ml/min to a column packed with "YMC-Pack CN-AP (manufactured by YMC, catalog No. AP-513; 6 mm in diameter and 250 mm in bed height) which has been equilibrated in advance with 15% B, using 0.1% TFA as the solvent A and 0.05 % TFA-containing 1-propanol as the solvent B. After the application, propanol concentration was increased from 15% B to 25% B, and 65 minutes of linear gradient from 25% B to 50% B was carried out. After completion of the final (5th) operation, 1 ml of a solution having the same composition excluding protein was applied to the column and developed in the same manner to recover TPO activity retained in the column. Since the same polypropylene tubes were used to pool the eluates of the 6 operations, a total of 44 tubes each containing 7.2 ml eluate were obtained.

A 30 µl portion of each of the thus obtained fractions (1/240 portion of each fraction) was mixed with 20 µl of 5% BSA, dried by evaporation dissolved in 0.24 ml of IMDM assay culture medium and then assayed to specify TPO-active fractions. As the result, strong TPO activity was found in the fractions of tube numbers 28 to 33 (37.0 to 42.0% propanol concentration range) which were combined and used as a low molecular TPO sample F3-derived YMC-Pack CN-AP main TPO-active fraction FA.

| Low molecular TPO sample F3-derived YMC-Pack CN-AP TPO-active fraction FA | |
|---|---|
| Total volume | 43.20 ml |
| Protein concentration | 0.00863 mg/ml |
| Total protein | 0.373 mg |
| Relative Activity | 800,000 |
| Total activity | 298,400 |

(10) Capcell Pak C1 300 ⟨final reverse phase chromatography⟩

A 43.12 ml portion of the 43.20 ml low molecular TPO sample F3-derived TPO-active fraction FA (total protein, 0.372 mg; protein concentration, 0.00863 mg/ml; relative activity, 800,000; total activity, 297,500) obtained in the above step (9) was mixed with 0.2 ml of 50% glycerol and concentrated to obtain 0.1 ml of glycerol solution.

This solution was mixed with 2 ml of a solution of solvent A (0.1% TFA): solvent B (1-propanol containing 0.05% TFA) = 85:15 (15% B) to prepare 2.1 ml of sample containing about 14% propanol, about 4.8% glycerol and 0.177 mg/ml of protein. The thus prepared sample was applied to a column packed with Capcell Pak C1 300A (manufactured by Shiseido Co., Ltd., catalog No. C1 TYPE:SG300A; 4.6 mm in diameter and 250 mm in bed height) which has been equilibrated in advance with 15% B and developed by 65 minutes of linear gradient from 27% B to 38% B at a flow rate of 0.4 ml/min. The eluates were collected in 72 polypropylene tubes in 0.6 ml portions.

A 3 µl portion of each of the thus obtained fractions (1/200 portion of each fraction) was mixed with 20 µl of 5% BSA, the medium was exchanged to 225 µl of IMDM assay culture medium and the thus 75 times diluted fraction was assayed.

A 1 µl portion of each fraction (1/600 fraction) was aliquotted for use in electrophoresis, dried by evaporation and treated at 95°C for 5 minutes with 10 µl of a nonreducing sample buffer for SDS-PAGE. The thus treated sample was subjected to SDS-PAGE using a 15-25% SDS-polyacrylamide precast gel (manufactured by Daiichi Pure Chemicals Co., Ltd.) and then stained with a silver staining kit of 2D-Silver Stain II "DAIICHI" (manufactured by Daiichi Pure Chemicals Co., Ltd., catalog No. 167997; to be referred to as "silver staining kit" hereinafter). As to molecular weight markers, [DAIICHI]-III Low Molecular Weight Markers (manufactured by Daiichi Pure Chemicals Co., Ltd., catalog No. 181061; to be referred to as "DPCIII" hereinafter) were used.

As the result of the above assay, significant TPO activity was found in the fractions of tube numbers 35 to 43 (30.0 to 32.5% propanol concentration range). Of these, fractions from tube numbers 36 to 42 (30.5 to 32.0% propanol concentration range) were combined and used as the main TPO-active fraction FA. The results are shown in Fig. 2.

When the protein content deduced from the chromatogram was compared with the assay results, the thus obtained fraction was found to have a total protein of 39.6 µg, a protein concentration of 9.4 µg/ml, a relative activity of 4,890,000 and a total activity of 193,600. Examination of the SDS-PAGE patterns of the TPO-active fraction numbers 36 to 42 revealed the presence of a band whose staining density correlates with the activity strength. In addition, apparent molecular weight of this non-reduced band was found to be 17,000 to 19,000 when compared with those of the standard molecular weight proteins on the same gel, which were reduced, thus revealing that this band is a strong candidate of TPO.

(11) Extraction of TPO-active protein from electrophoresis gel ⟨15% SDS-PAGE⟩

Example of the analysis of TPO-active fraction FA

Of the 4,200 µl of the low molecular TPO sample F3-derived TPO-active fraction FA (total protein, 39.6 µg; protein concentration, 9.4 mg/ml; relative activity, 4,890,000; total activity, 193,600) obtained in the above step (10), 5.5 µl (1/764 fraction) for use in active protein extraction and 2.5 µl (1/1680 fraction) for use in silver staining were transferred into respective sample tubes, dried by evaporation, reacted with 10 µl of a nonreducing sample buffer for SDS-PAGE at 37° C for 1 hour and then allowed to stand still for 18 hours at room temperature, thereby effecting the SDS reaction.

Pre-stained Low Range Marker (manufactured by Bio-Rad Laboratories, Inc., 161-0305) and the aforementioned DPCIII were used as molecular weight markers. Making use of a microslab gel, 15% SDS-PAGE of these samples was carried out at 4°C in accordance with a usually used procedure (Laemmli, *Nature*, vol.227,pp.680 - 685, 1970). After completion of the electrophoresis, gel portions to be subjected to silver staining were immediately cut out with a knife, put into a fixing solution and then stained using the aforementioned silver staining kit.

Separately from this, all molecular weight range of the gel to be used for the detection of the activity was cut with a knife into 34 slices, and each gel slice having a width of 1.5 to 2.5 mm was smashed by a slightly modified method of Kobayashi (Kobayashi, M., *Seikagaku (*Biochemistry), vol.59, no.9, 1987, published by the Japanese Biochemical Society). Each gel slice thus smashed into minute fragments was mixed with 0.3 ml of an extraction buffer (20 mM Tris-HCl, pH 8, 500 mM NaCl, 0.05% BSA) and shaken for 6 hours at 4°C to effect extraction.

To this was added 500 mM potassium phosphate (pH 6.8) to a final concentration of 20 mM. After 1 hour of shaking at 4°C, the resulting mixture was transferred into an Ultra Free C3GV 0.22 µm filtration unit (manufactured by Millipore Corp., UFC3 OGV 0S) and centrifuged at 1,000 x g (4,000 rpm) for 15 minutes to remove precipitated SDS and recover the resulting supernatant fluid. The filtrate was transferred into an Ultra Free C3-LGC ultrafiltration unit (nominal molecular weight cutoff of 10,000, manufactured by Millipore Corp., UFC3 LGC 00) and centrifuged at 3,000 x g (7,000 rpm). When volume of the concentrated solution reached about 50 µl. 300 µl of 20 mM sodium phosphate buffer (pH 7.2) was added and again subjected to the ultrafiltration.

The ultrafiltration using 300 µl of 20 mM sodium phosphate buffer was repeated twice to remove remaining SDS. Thereafter, a similar step was repeated to exchange to the assay culture medium for preparation of a sample (300 µl) which was subsequently sterilized and subjected to TPO activity measurement.

Three protein bands were detected clearly by the silver staining which showed apparent molecular weights of about 17,000 to 19,000, 14,000 and 11,000 based on the DPCIII molecular weight markers.

In the previous step (10), a band having a correlation between activity strength and staining density and having an apparent molecular weight of about 17,000 to 19,000 was observed in the electrophoresis of the Capcell Pak C1 column TPO-active fraction. In the experiment of this step (11), TPO activity was found also in the band having an apparent molecular weight of about 17,000 to 19,000 (cf. Fig. 3).

On the basis of the above results, it was confirmed that the TPO-active protein was purified in the active fraction of Capcell Pak C1 300A column to a detectable level on the electrophoresis gel. Based on the silver-stained density of the band obtained by 15% SDS-PAGE, the amount of the candidate TPO protein (an apparent molecular weight of about 17,000 to 19,000) in the total TPO-active fraction was determined to be about 1.7 µg.

Purification of the high molecular TPO sample F2 is described in the following steps (12) to (15).

(12) YMC-Pack PROTEIN-RP ⟨reverse phase chromatography⟩

The high molecular TPO sample F2 (total protein, 257 mg; protein concentration, 0.894 mg/ml; relative activity, 7,840; total activity, 2,015,000; total volume, 287 ml) obtained in the above step (7) was mixed with 95.8 ml (1/3 volume of the sample) of a solvent B (1-propanol containing 0.025% TFA) to obtain a solution having a total volume of 383 ml and final propanol, TFA and protein concentrations of about 25%, 0.006% and 0.671 mg/ml, respectively. A solution of 0.025% TFA was used for a solvent A. Insoluble materials formed during the preparation of input sample were separated by centrifugation, and the resulting supernatant fluid was divided into six 62.3 ml (42.8 mg protein) portions and applied at a flow rate of 2 ml/min to a column packed with YMC-Pack PROTEIN-RP (manufactured by YMC, catalog No. A-PRRP-33-03-15; 3 cm in diameter and 7.5 cm in bed height) which has been equilibrated in advance with 30% B. The precipitate resulting from the centrifugation was dissolved in 10 ml of 20 mM sodium acetate (pH 5.5) containing 5 mM CHAPS and also applied to the column.

After the sample loading, about 50 ml of a solvent (solvents A:B = 3:1) was passed through the column to obtain a passed-through fraction. Next, a developing program (120 minutes of linear gradient from 30% B to 45% B) was started, and the eluates were collected in 24 polypropylene tubes in 15 ml portions. This process was repeated for the 6 divided samples using the same collection tubes, thus obtaining a total of 24 fraction tubes each containing 90 ml eluate. The passed-through fraction and the tube number 1 fraction were combined and directly concentrated to 90 ml using an ultrafiltration unit with YM3 membrane (76 mm in diameter, manufactured by Amicon Corp.).

A 0.3 ml portion of each of the passed-through fraction plus tube number 1 fraction and the fractions of tube numbers 2 to 24 was mixed with 10 µl of 5% BSA, dried by evaporation, dissolved in 0.30 ml of IMDM assay culture medium and then assayed to specify TPO-active fractions. As the result, the TPO activity was found in the fractions of tube numbers 10 to 15 (34.0 to 39.5% propanol concentration range) which were combined and used as a high molecular TPO sample F2-derived YMC-Pack PROTEIN-RP TPO-active fraction F2. This fraction was stored at -85°C until its use in the subsequent YMC-Pack CN-AP step.

| High molecular TPO sample F2-derived YMC-Pack PROTEIN-RP TPO-active fraction F2 | |
|---|---|
| Total volume | 540 ml |
| Protein concentration | 0.021 mg/ml |
| Total protein | 11.4 mg |
| Relative Activity | 227,000 |
| Total activity | 2,588,000 |

(13) Superdex 75 pg 〈gel filtration chromatography in the presence of CHAPS〉

A 538.2 ml portion of the high molecular TPO sample F2-derived YMC-Pack PROTEIN-RP TPO-active fraction F2 (total protein, 11.3 mg; protein concentration, 0.021 mg/ml; relative activity, 227,000; total activity, 2,565,000) obtained in the above step (12) was mixed with 0.6 ml of 50% glycerol and concentrated by evaporation. To this was added 18 ml of 20 mM CHAPS, followed by stirring and a subsequent 41 hour incubation at 4° C. Thereafter, the first sample was applied to a column packed with HiLoad 26/60 Superdex 75 pg (manufactured by Pharmacia Biotech, catalog No. 17-1070-01; 2.6 cm in diameter and 60 cm in bed height) and eluted with DPBS containing 5 mM CHAPS at a flow rate of 1 ml/min. A 4 ml portion of each sample (protein concentration, 0.466 mg/ml; protein, 1.86 mg) was applied to the column.

In this case, the Superdex 75 column operation was repeated 6 times by dividing the entire YMC-Pack PROTEIN-RP TPO-active fraction into 6 portions. The eluates of each operation were collected in 5 ml portions in 45 tubes, thus finally obtaining 45 fractions each containing 30 ml eluate after completion of the 6 column operations.

A 0.1 ml portion of each of the thus obtained fractions was mixed with 10 µl of 5% BSA, dried by evaporation, dissolved in 0.25 ml of IMDM assay culture medium and then assayed to specify TPO-active fractions. As the result, the TPO activity was found in the fractions of tube numbers 13 to 31 (molecular weight range of from 78,000 to 3,000) which were combined and used as a high molecular TPO sample F2-derived Superdex 75 pg TPO-active fraction F2.

| High molecular TPO sample F2-derived Superdex 75 pg TPO-active fraction F2 | |
|---|---|
| Total volume | 540 ml |
| Protein concentration | 0.00216 mg/ml |
| Total protein | 1.17 mg |
| Relative Activity | 1,750,000 |
| Total activity | 2,041,000 |

(14) YMC-Pack CN-AP 〈reverse phase chromatography〉

A 513.2 ml portion of the high molecular TPO sample F2-derived Superdex 75 pg TPO-active fraction F2 540 ml (molecular weight, 78,000 - 3,000) (total protein, 1.11 mg; protein concentration, 0.00216 mg/ml; relative activity, 1,750,000; total activity, 1,943,000) obtained in the above step (13) was mixed with 1/10 volume of a solvent B (1-propanol containing 0.05 % TFA) and applied at a flow rate of 0.6 ml/min to a column packed with YMC-Pack CN-AP (manufactured by YMC, catalog No. AP-513; 6 mm in diameter and 250 mm in bed height) which has been equilibrated in advance with 15% B, using a solvent A (0.1% TFA) and the solvent B. After the application, propanol concentration was increased from 15% B to 25% B, and 65 minutes of linear gradient from 25% B to 50% B was carried out.

Prior to the commencement of the YMC-Pack CN-AP column chromatography, a 1/20 portion of the total input sample was subjected to a trial operation in order to confirm proper recovery of the activity. Thereafter, the remaining 19/20 portion was divided into 2 samples to carry out the operation twice. In other words, the column operation was repeated three times. Since the same 44 polypropylene tubes were used to pool the eluates of the 3 operations, a total of 44 tubes each containing 3.6 ml eluate were obtained.

A 5 µl portion of each of the thus obtained fractions (1/720 portion of each fraction) was mixed with 0.25 ml of IMDM assay culture medium and then assayed to specily TPO-active fractions. As the result, markedly strong TPO activity was found in the fractions of tube numbers 24 to 30 (36.0 to 42.0% propanol concentration range) which were combined and used as a high molecular TPO sample F2-derived YMC-Pack CN-AP main TPO-active fraction FA.

| High molecular TPO sample F2-derived YMC-Pack CN-AP TPO-active fraction FA | |
|---|---|
| Total volume | 25.20 ml |
| Protein concentration | 0.0246 mg/ml |
| Total protein | 0.620 mg |
| Relative Activity | 700,000 |
| Total activity | 434,000 |

(15) Capcell Pak C1 300A 〈final reverse phase chromatography〉

A 24.66 ml portion of the 25.20 ml high molecular TPO sample F2-derived YMC-Pack CN-AP TPO-active fraction FA (total protein, 0.606 mg; protein concentration, 0.0246 mg/ml; relative activity, 700,000; total activity, 424,000) obtained in the above step (14) was mixed with 0.4 ml of 50% glycerol and concentrated by evaporation.

In this way, 2 ml of a concentrated sample was obtained with a propanol concentration of a few percent, a glycerol concentration of 10% and a protein concentration of 0.303 mg/ml. This was applied to a column packed with Capcell Pak C1 300A (manufactured by Shiseido Co., Ltd., catalog No. C1 TYPE:SG300A; 4.6 mm in diameter and 250 mm in bed height) which has been equilibrated in advance with 15% B, using a solvent A (0.1% TFA) and a solvent B (1-propanol containing 0.05% TFA), and developed by 65 minutes of linear gradient from 27% B to 38% B at a flow rate of 0.4 ml/min. The eluates were collected in 72 polypropylene tubes in 0.6 ml portions.

A 0.75 μl portion of each of the thus obtained fractions (1/800 portion of each fraction) was mixed with 20 μl of 5% BSA, the medium was exchanged to 225 μl of IMDM assay culture medium and the thus 300 times diluted fraction was assayed.

A 2 μl portion of each fraction (1/300 fraction) was sampled for use in electrophoresis, dried by evaporation and treated at 95°C for 5 minutes with 10 μl of a nonreducing sample buffer for SDS-PAGE. The thus treated sample was subjected to SDS-PAGE using a 15-25% SDS-polyacrylamide precast gel (manufactured by Daiichi Pure Chemicals Co., Ltd.) and then stained using the aforementioned silver staining kit. DPCIII described in the foregoing was used as molecular weight markers.

As the result of the above assay, significant TPO activity was found in the fractions of tube numbers 33 to 39 (29.5 to 31.5% propanol concentration range). Of these, fractions of tube numbers 34 to 39 (30.0 to 31.5% propanol concentration range) were combined and used as the main TPO-active fraction FA. Examination of the SDS-PAGE pattern of the main TPO-active fraction revealed the presence of a band whose staining density correlates with the activity strength, within an apparent molecular weight range of 17,000 to 19,000 under nonreducing condition, which was similar to the case of the low molecular TPO sample F3-derived TPO-active fraction described in the aforementioned step (10).

〈Example 2〉

Analysis of partial amino acid sequences of purified rat TPO

Amino acid sequence analysis of the rat TPO candidate protein in the Capcell Pak C1 300A column TPO-active fraction FA obtained in the purification step (10) of Example 1 was carried out in accordance with the procedure of Iwamatsu (Iwamatsu *et al.*, "Shin Kiso Seikagaku Jikken-ho (New Basic Biochemical Experiments)", vol.4, pp.33 - 84, pub. Maruzen; Iwamatsu, A, *Seikagaku* (Biochemistry), vol.63, no.2, pp.139 - 143, 1991; Iwamatsu, A., *Electrophoresis*, vol.13, pp.142 - 147, 1992). That is, the sample was subjected to SDS-PAGE and transferred electrically onto a polyvinylidene difluoride (PVDF) membrane. After reduction and S-alkylation of the protein on the PVDF membrane, the thus treated protein was digested into peptide fragments by systematic and stepwise restrictive enzymatic hydrolysis *in situ* with three proteases, and the resulting peptide fragments on each digestion step were separated and purified by reverse phase chromatography and analyzed for their amino acid sequences by a high sensitivity amino acid sequence determination method. The following describes this process in detail.

Example of the analysis of the TPO candidate protein in the low molecular TPO sample F3-derived Capcell Pak C1 300A TPO fraction FA

(1) Concentration of Capcell Pak C1 300A column TPO fraction FA (tube numbers 36 to 42)

Of the 4,200 µl of the low molecular TPO sample F3-derived Capcell Pak C1 300A column TPO-active fraction FA (tube numbers 36 to 42) obtained in the purification step (10) of Example 1 (total protein, 39.6 µg; protein concentration, 9.4 µg/µl; relative activity, 4,890,000; total activity, 193,600), 4,151 µl (98.8% of the total fraction) was used in the amino acid sequence analysis. The total protein deduced from the chromatogram was 39.1 µg, of which about 1.6 µg was the TPO candidate protein stained with silver after SDS-PAGE and having an apparent molecular weight of about 17,000 to 19,000.

This sample was mixed with glycerol and concentrated by evaporation to obtain 5 µl of a glycerol solution. To this were added a non-reducing buffer for SDS-PAGE and 1 M Tris-HCl (pH 8) in order to adjust its pH, thereby obtaining about 25 µl of sample containing 200 mM Tris-HCl (pH 8.0), 50 mM Tris-HCl (pH 6.8), 1.1% SDS, 2 mM EDTA, 0.02% bromophenol blue and 30% glycerol.

The thus prepared sample was maintained at room temperature for 14 hours and then treated at 60°C for 5 minutes, in order to effect proper SDS reaction without excess heating.

(2) Electrophoresis

Micro-slab gels (4.0% acrylamide stacking gel and 15% acrylamide separation gel) were prepared and SDS-PAGE was carried out at room temperature for 2 hours at a constant current of 12.5 mA and then at 17.5 mA. Pre-stained Low Range Marker (Bio-Rad, 161-0305) and DOCIII were used as molecular weight markers. Immediately after the electrophoresis, the resulting protein molecules were transferred on a PVDF membrane (see the following step).

A portion of the sample was also used in another electrophoresis using 15-25 % polyacrylamide precast gel (Multi Gel 15/25 manufactured by Daiichi Pure Chemicals Co., Ltd., catalog No. 211072) under non-reducing conditions or after reducing the sample with dithiothreitol (DTT). When the resulting gel was stained using the silver staining kit described in the foregoing, it was confirmed that molecular weight of the protein band expected to be TPO was about 19,000 under a reducing condition and purity of the TPO candidate protein of Capcell Pak C1 300A column was a few percent. In addition, since mobility of the band varied under non-reducing and reducing conditions, it was suggested that the candidate protein contains at least one disulfide bond.

(3) Transfer of protein on PVDF membrane by electroblotting and detection of band

Protein transfer on a PVDF membrane (ProBlott, manufactured by Applied Biosystems; catalog No. 400994) was carried out for 1 hour at a constant current of 160 mA (11 to 17 V) using a semi-dry transfer apparatus (Model KS-8460, manufactured by Marysol). A solution consisting of 0.3 M Tris and 20% methanol (pH 10.4) was used as the anolyte, a solution consisting of 25 mM Tris and 20% methanol (pH 10.4) as the transfer membrane solution and a solution consisting of 25 mM Tris, 40 mM aminocaproic acid and 20% methanol (pH 10.4) as the cathlyte.

When the thus transferred membrane was stained with a Ponceau S staining solution (0.1 g of Ponceau S and 1 ml of acetic acid in 100 ml water), a plurality of bands were detected and one of these bands was confirmed to be that of the candidate protein having a molecular weight of about 19,000. This band was cut out for use in the subsequent peptide fragment formation steps.

(4) Peptide fragment formation, peptide mapping and amino acid sequence analysis

In order to perform systematic fragmentation of the TPO candidate protein which has been transferred and S-alkylated after reduction on the PVDF membrane, stepwise restrictive enzymatic hydrolysis was carried out using the following three proteases.

First digestion: Lysyl endopeptidase (*Achromobacter lyticus* m497-1, manufactured by Wako Pure Chemical Industries, Ltd., catalog No. 129-02541)

Second digestion: Endoproteinase Asp-N (manufactured by Boehringer-Mannheim Corp., catalog No. 1054 589)

Third digestion: Trypsin-TPCK (manufactured by Worthington Biochemical, catalog No. 3740)

Peptide fragments obtained by each enzyme digestion were recovered, applied to a Wakosil-II 5C18 C18 reverse phase column (manufactured by Wako Pure Chemical Industries, Ltd., 2.0 mm in diameter and 150 mm in length) and eluted using a solvent A (0.05 % TFA) and a solvent B (isopropanol:acetonitrile = 7:3, 0.02% TFA), by 30 minutes of linear gradient of from 1% B to 50% B at a flow rate of 0.25 ml/min and at a column temperature of 30 °C. In this way, peptide mappings were made (see Fig. 4). The resulting peptide fragments were recovered and subjected to Edman degradation using a gas phase amino acid sequencer (PPSQ-2, manufactured by Shimadzu Corp.). Thereafter, each amino acid recovered in turn from the sequencer, whose N-terminal has been coupled with PTH, was identified using the C18 reverse phase column chromatography by isocratic elution. The results are summarized in the following.

Amino acid sequences of peptide fragments obtained by the first digestion with lysyl endopeptidase

| Fragment | Amino acid sequence |
|---|---|
| AP3 | (K)XYYESZ (X is A, S, G, M or Q and Z is E or K) (SEQ ID NO: 184) |
| AP6 | (K)XRAAZ (X is E or Q and Z is E or N) (SEQ ID NO: 185) |
| AP7 | (K)AGXCSG (cannot be totally identified) (SEQ ID NO: 186) |
| AP8 | (K)XPVPPACDPRLL (X is I, T OR S) (SEQ ID NO: 187) |
| AP12 | (K)DSFLADVK (SEQ ID NO: 188) |
| AP5 | (cannot be identified) |
| AP20 | (cannot be identified) |
| AP21 | (cannot be identified) |
| AP23 | (cannot be identified) |

Amino acid sequences of peptide fragments obtained by the second digestion with endoproteinase Asp-N

| Fragment | Amino acid sequence |
|---|---|
| ASP1 | (cannot be identified) |
| ASP2 | (cannot be identified) |
| ASP6 | (cannot be identified) |
| ASP11 | (cannot be identified) |

Amino acid sequences of peptide fragments obtained by the third digestion with trypsin-TPCK

| Fragment | Amino acid sequence |
|---|---|
| TP2 | (K or R)TLPTXAVP (SEQ ID NO: 189) |
| TP3 | (K or R)TLPTXAVP |

In the above sequences, those shown in parentheses in N-termini are amino acid residues which can be deduced from the systematic enzyme digestion.

(5) Analysis of the homology of obtained amino acid sequences ⟨homology search⟩

In order to know if the thus obtained amino acid sequences are contained in a known reported protein or there is a protein having similar sequences, they were analyzed using sequence analyzing software Mac Vector (Kodak International Biotechnologies, Inc.). As for the information on known proteins or known genes, Entrez Release 6 data base (National Center for Biotechnology Information, National Library of Medicine, National Institute of Health, USA, published on August 15, 1993) was used. Data bases included therein are as follows.

Entrez Release 6 database
NCBI-GenBank, August 15, 1993 (Release 78.0)
EMBL, July 15, 1993 (Release 35.0 plus updates)
DDBJ, July 15, 1993
SWISS-PROT, April, 1993 (Release 25.0)
PIR, June 30, 1993 (Release 37.0)
PDB, April, 1993
PRF, May, 1993
dbEST, July 15, 1993 (Release 1.10)
U.S. and European Patents

As the result, the sequence (K)DSFLADVK of AP12 completely coincided with an internal sequence KDSFLADVK of a rat corticosteroid-binding globulin (CBG) precursor [PIR data base accession No. A40066;

Smith and Hammond; "Rat corticosteroid-binding globulin: primary structure and messenger ribonucleic acid levels in the liver under different physiological conditions.", *Mol. Endocrinol.*, (1989), 3, 420 - 426].

Further detailed examination revealed that a sequence KQYYESE (SEQ ID NO: 193) having high similarity to the sequence (K)XYYESZ (X is A, S, G, M or Q and Z is E or K) of AP3 is contained in the rat CBG amino acid sequence. In the rat CBG, sequences which correspond to those of AP12 and AP3 are linked to each other thereby forming an internal amino acid sequence KDSFLADVKQYYESE (SEQ ID NO: 190).

With regard to the amino acid sequences of other fragments than AP12 and AP3, no protein or gene was found whose similarity could be taken into consideration.

〈Example 3〉

Analysis of biological characteristics of TPO derived from blood plasma of thrombocytopenic rats

(1) In the rat CFU-MK assay system (liquid culture system)

As a typical example, Fig. 5 shows a dose response curve in the case of the use of a TPO sample partially purified from thrombocytopenic rat plasma (TPO-active fraction F2 from YMC Pack Protein-RP column described in the purification step (8) of Example 1-2). When the cultured cells were observed periodically under a microscope, generation and maturation of megakaryocytes, namely increase in the cell size, was recognized, probably together with increase in the number of megakaryocytes. As an especially significant change, process formation by megakaryocytes were found on the 4th day which was the final day of the culturing (they were hardly recognizable on the 3rd day). Such a process formation is called cytoplasmic process formation (Leven and Yee, *Blood*, vol.69, pp.1046 - 1052, 1987) or proplatelet process formation (Topp *et al.*, *Blood,* vol.76, pp.912 - 924, 1990), which is considered to be a platelet precursor structure further differentiated from mature megakaryocytes and is considered to be at the final stage of megakaryocyte differentiation so far observable *in vitro*. Since such a morphological change was observed with a high frequency by using the TPO sample alone, it is possible that this factor alone can stimulate proliferation and differentiation of CFU-MK, can produce mature megakaryocytes and can finally release platelets.

(2) In the colony assay system

When a TPO sample partially purified from blood plasma of thrombocytopenic rats was examined by the colony assay system using unseparated rat bone marrow cells, cells of each of the separation/concentration steps or a GpIIb/IIIa$^+$ CFU-MK fraction, the rat plasma-derived TPO stimulated formation of megakaryocyte colonies. In comparison with megakaryocyte colonies induced by other cytokine such as rat IL-3, mouse GM-CSF or human EPO, the TPO-induced megakaryocyte colonies are characterized in that each colony consists of smaller numbers of megakaryocytes, but each megakaryocyte is larger in size, which means advanced maturity. In addition, since TPO produced no or few colonies of the other cell lineages, the Meg-CSF activity of TPO can be regarded as megakaryocyte-specific. On the basis of these facts, it is evident that TPO has different biological properties from those of other cytokines such as rat IL-3, mouse GM-CSF and human EPO and exerts unique Meg-CSF activity.

The TPO sample partially purified from blood plasma of thrombocytopenic rats also exerted its Meg-CSF activity on CD34$^+$,DR$^+$ cell fractions derived from human bone marrow cells or human cord blood cells and induced significant numbers of human megakaryocyte colonies, indicating that this factor has no species specificity.

〈Example 4〉

Specialization of rat TPO producing cells

(1) Screening of rat TPO producing organs

Screening and specialization of rat TPO-producing organs were carried out in order to ensure a mRNA source for use in the cloning of a rat TPO gene. Firstly, bone marrow, lungs, livers and spleens were taken periodically from rats rendered thrombocytopenic by P55 antibody administration, their cells (organ sections in the case of lungs and livers) were cultured and activities in the resulting culture supernatant were examined by the rat CFU-MK assay system. This initial attempt, however, did not yield distinctive results. In consequence, further attempt was made to culture hepatocytes prepared by collagenase perfusion from livers of thrombocytopenic rats induced by P55 antibody administration, talking into consideration a report which indicated a relationship between the liver and TPO production in rats (Siemensma *et al.*, *J. Lab. Clin. Med.*, vol.86, pp.817 - 833, 1975). When the resulting culture supernatant was applied to a WGA-Agarose column and then the adsorbed fraction was fractionated on a Vydac phenyl reverse phase column, exceedingly similar activity was found at the same position of the rat plasma-derived TPO activity in the rat CFU-MK assay system. Weak but the same activity was also found in culture supernatant of normal rat hepatocytes. These results strongly suggested that the liver would be one of the TPO-producing organs.

(2) Screening of rat TPO-producing cell lines

On the basis of the above results, screening of rat TPO-producing cell lines was carried out. Firstly, each of 20 rat liver-derived cell lines was cultured in respective subculture liquid medium until the cells reached almost confluent stage, the culture medium was exchanged with the same respective medium supplemented with 5% FCS, and the culturing was continued for 3 days. When each of the resulting culture supernatant was partially purified in accordance with the procedure described in the above step (1) to examine existence of TPO production, the TPO activity was distinctively found in three rat hepatic parenchymal cell-derived cell lines, namely McA-RH8994 cells (ATCC deposit No. CRL1602; Becker *et al.*, "Oncodevelopmental Gene Expression", ed. by Fishman and Sell, Academic Press, NY, pp.259 - 270, 1976; purchased from Dainippon Pharmaceutical Co., Ltd.), H4-II-E cells (ATCC deposit No. CRL1548; Pitot *et al.*, *Nat. Cancer Inst. Monogr.*, vol. 13, pp.229 - 245, 1964; purchased from Dainippon Pharmaceutical Co., Ltd.) and HTC cells (Thompson *et al.*, *Proc. Natl. Acad. Sci. USA*, vol.56, pp.296 - 303, 1966; purchased from Dainippon Pharmaceutical Co., Ltd.).

(3) Detailed analysis of TPO activities in McA-RH8994 cells, H4-II-E cells and HTC cells

Biochemical and biological properties of the TPO-active proteins secreted from these 3 rat cell lines were examined in detail and compared with those of the rat plasma-derived TPO.

McA-RH8994 cells were suspended in alpha-MEM(-) liquid culture medium containing 10% FCS and transferred into a 175 $cm^2$ plastic tissue culture flask at 1 x $10^6$ cells/flask. After 3 days of culturing at 37°C in a 5% $CO_2$ incubator, the medium was exchanged with IMDM culture medium containing 5% FCS and the culturing was continued for additional 3 days to recover the resulting culture supernatant. H4-II-E cells were suspended in Dulbecco's modified Eagle liquid culture medium (glucose 4.5 g/l, to be referred to as "DMEM" hereinafter) containing 10% FCS and transferred into a 175 $cm^2$ plastic tissue culture flask at 5 x $10^5$ cells/flask. After 3 days of culturing at 37°C in a 5% $CO_2$ incubator, the medium was exchanged with IMDM culture medium containing 5% FCS and the culturing was continued for additional 3 days to recover the resulting culture supernatant. Also, HTC cells were suspended in DMEM liquid culture medium containing 5% FCS and transferred into a 175 $cm^2$ plastic tissue culture flask at 2.5 x $10^5$ cells/flask. After 3 days of culturing at 37°C in a 5% $CO_2$ incubator, the medium was exchanged with IMDM culture medium containing 5% FCS and the culturing was continued for additional 3 days to recover the resulting culture supernatant.

Using a 2 liter portion of the thus obtained culture supernatant from each of the 3 cell lines, partial purification of the cell line-derived TPO was carried out in accordance with the procedure for the purification of TPO from XRP described in Example 1-2. The following describes an outline of the results.

Firstly, each culture supernatant was concentrated about 6 times using an ultrafiltration apparatus and the medium was exchanged with 20 mM Tris-HCl buffer (pH 8.0) using a Sephadex G-25 column. The resulting eluate was applied to a Q-Sepharose FF column, the column was washed with 20 mM Tris-HCl (pH 8.0) and then the adsorbed fraction was eluted with 20 mM Tris-HCl (pH 8.0) containing 175 mM NaCl. The thus eluted fraction was applied to a WGA-Agarose column, the column was washed with PBS and the adsorbed fraction was eluted with 20 mM sodium phosphate (pH 7.2) containing 0.2 M GlcNAc and 0.15 M NaCl. The resulting eluate was applied to an TSK-gel AF-BLUE 650MH column, the column was washed with 20 mM sodium phosphate (pH 7.2) containing 1 M NaCl and then the adsorbed fraction was eluted with 2 M NaSCN. The resulting eluate was applied to a Phenyl-Sepharose 6 FF/LS column, the column was washed with 50 mM sodium phosphate (pH 7.2) containing 1.5 M ammonium sulfate followed by 0.8 M ammonium sulfate containing 36 mM sodium phosphate, and then the adsorbed fraction was eluted with 20 mM sodium phosphate (pH 7.2). The thus obtained adsorption fraction was concentrated and then fractionated using a reverse phase Vydac Protein C4 column (manufactured by The Separations Group, catalog No. 214TP51015; 1 cm in diameter and 15 cm in bed height). That is, the sample was applied to the C4 column which has been equilibrated in advance with 20% B and then elution was effected by 90 minutes of linear gradient of from 20% B to 40% B at a flow rate of 1 ml/min, using 0.1% TFA in the developing solvent A and 1-propanol containing 0.05% TFA in the developing solvent B. As the result, each of the TPO-active proteins derived from these cell lines was eluted at a 1-propanol concentration of 30 to 43%.

When TPO activities in samples of each purification step were measured by the rat CFU-MK assay system, the results showed that TPO activities of the 3 cell lines behaved with the similar patterns to those of the XRP-derived TPO (cf. Example 1-2). Relative activity. activity yield and the like at each purification step measured by the rat CFU-MK assay system are shown in Table 2. In addition, when TPO activities in the eluates from the final reverse phase column were measured by the rat CFU-MK assay system, TPO activities of the 3 cell lines and the XRP-derived TPO were found in the same peak area. When colony assay of TPO-active fractions from the reverse phase column was carried out using nonadherent cells obtained in the adherence depletion step for the separation and concentration of rat GpIIb/IIIa$^+$ CFU-MK, elution patterns of Meg-CSF activity closely resembled those of TPO activity measured by the rat CFU-MK assay system (see Table 3). In addition, similar to the case of the XRP-derived TPO, each of TPO activities of the 3 cell lines formed no or few colonies of the other cell lineages.

Each of the pooled active fractions from the reverse phase column was subjected to SDS-PAGE in accordance with the procedure described in Example 1-2. When protein was extracted from the resulting gel and TPO activity was measured by the rat CFU-MK assay system, apparent molecular weights of the XRP-derived TPO, McA-RH8994 cell-derived TPO and H4-II-E cell-derived TPO were found to be 17,000 to 22,000, 33,000 to 39,000 and 31,000 to 38,000, respectively, and the HTC cell-derived TPO showed apparent molecular weights of 17,000 to 22,000 and 28,000 to 35,000.

Thus, these results revealed that the TPO proteins produced by McA-RH8994 cells, H4-II-E cells and HTC cells are equivalent to the XRP-derived TPO in terms of their biological properties, though their biochemical properties are slightly different from one another in terms of apparent molecular weight. A remarkable finding in the present invention is a possibility that a TPO-active protein molecule contained in blood may be a product of partial digestion at its specific or irregular site in its production cells or after its secretion from the production cells. It also suggests possible existence of TPO genes (mRNA and cDNA) having various lengths.

Table 2 Purification of TPO from rat cell lines

| Cell Line | McA-RH8994 | | | HTC | | | H4-II-E | | |
|---|---|---|---|---|---|---|---|---|---|
| Step | TP*[1] (mg) | RA*[2] | TA*[3] | TP (mg) | RA | TA | TP (mg) | RA | TA |
| Culture supernatant | 4806 | 8 | 38450 | 5760 | 5 | 28800 | 4087 | 5 | 20440 |
| Sephadex G25 | 4824 | 16 | 77180 | 6458 | 12 | 77500 | 4011 | 5 | 20050 |
| Q-Sepharose FF | 1973 | 20 | 39460 | 3112 | 15 | 46680 | 1821 | 15 | 27320 |
| WGA-Agarose | 76.0 | 1350 | 102600 | 132.0 | 250 | 33000 | 80.0 | 90 | 7200 |
| TSK-gel AF-Blue 650MH | 5.0 | 12500 | 62500 | 9.2 | 7500 | 69000 | 5.4 | 150 | 810 |
| Phenyl-Sepharose 6 FF/LS | 14.7 | 500 | 7350 | 13.1 | 2500 | 32750 | 11.4 | 170 | 1940 |
| Vydac Protein C4 | 0.23 | — | — | 0.75 | — | — | 0.11 | — | — |

*[1], total protein; *[2], relative activity; *[3], total activity

Table 3 Rat megakaryocyte colony formation by rat TPO (reverse phaseC4 column fraction)

| Elution propanol | XRP colonies | McA-RH8994 colonies | HTC colonies | H4-II-E colonies |
|---|---|---|---|---|
| 30.0 - 32.6% | 0 | 33 | 4 | 18 |
| 32.6 - 34.7% | 0 | 132 | 19 | 50 |
| 34.7 - 36.7% | 67 | 290 | 291 | 230 |
| 36.7 - 38.8% | 70 | 214 | 183 | 103 |
| 38.8 - 40.9% | 2 | 15 | 5 | 28 |
| 40.9 - 43.0% | 0 | 4 | 0 | 4 |

〈Example 5〉

Construction of expression vector (pEF18S) for cDNA library

A vector into which a cDNA fragment can be integrated easily and which can exert a high expression efficiency of the cloned cDNA was constructed for use in the cloning and expression of TPO cDNA. That is, an expression vector pEF18S was constructed from an expression vector pME18S by replacing the SRα-promoter with an elongation factor 1a (EF1α) promoter which is known as a high expression promoter (cf. Fig. 6). The promoter of elongation factor 1α was obtained by partially digesting 1 μg of an expression

vector pEF-BOS (Mizushima *et al.*, *Nucleic Acids Res.*, vol.18, p.5322, 1990) with restriction enzymes *Hin*dIII and *Eco*RI, subjecting the digest to 2% agarose gel (manufactured by FMC BioProducts) electrophoresis to isolate a DNA fragment of about 1,200 bp and purifying the DNA fragment using a Prep-A-Gene DNA purification kit (manufactured by Bio-Rad Laboratories, Inc.; a kit for use in the selective purification of DNA which is effected by porous silica matrix-aided DNA adsorption, developed on the basis of the procedure reported by Willis *et al.* in *Bio Techniques*, vol.9, pp.92-99, 1990). A 100 ng portion of the thus obtained DNA fragment was ligated with 50 ng of an expression vector pME18S (Liu *et al., Proc. Natl. Acad. Sci. USA*, vol.90, pp.8957 - 8961, 1993) which has been digested in advance with *Hin*dIII and *Eco*RI. Cells of a commercial host strain, Competent High *E. coli* DH5 (manufactured by Toyobo Co., Ltd.; a competent *E. coli* strain prepared by a modified method of Hanahan *et al.*, *J. Mol. Biol.*, vol.166, pp.557 - 580, 1983), were transformed with the thus constructed vector. After culturing the transformed cells, 12 colonies were selected at random and plasmid DNA was purified from each colony. A total of 10 clones containing a plasmid of interest (pEF18S) were obtained by analyzing restriction enzyme digestion patterns of these DNA molecules. Thereafter, a clone selected therefrom was cultured to prepare a large quantity of the plasmid DNA.

Purification of the plasmid DNA was carried out basically in accordance with the procedure described in *Molecular Cloning* (Sambrook *et al.*, Cold Spring Harbor Laboratory Press, 1989). That is, the clone pEF18S obtained above was cultured overnight at 37 °C in 50 ml of an LB medium (1% Bacto-tryptone, 0.5% Bacto-yeast extract, 0.5% NaCl) containing 50 µg/ml of ampicillin, and the resulting cells collected by centrifugation were suspended in 4 ml of TEG-lysozyme solution (25 mM Tris_Cl, pH 8, 10 mM EDTA, 50 mM glucose, 0.5% lysozyme). To this were added 8 ml of 0.2 N NaOH/1% SDS solution and then 6 ml of 3 M potassium/5 M acetate solution to suspend the cells thoroughly. After centrifugation of the suspension, the resulting supernatant fluid was treated with phenol/chloroform (1:1), mixed with the same volume of isopropanol and then centrifuged. The resulting pellet was dissolved in TE solution (10 mM Tris-HCl, pH 7.5, 1 mM EDTA) and treated with RNase and then with phenol/chloroform (1:1), followed by ethanol precipitation. The resulting pellet was again dissolved in TE solution to which were subsequently added NaCl and polyethylene glycol 3,000 to their final concentrations of 0.63 M and 7.5%, respectively. After centrifugation, the pellet was dissolved in TE solution and precipitated with ethanol. In this way, about 300 µg of the plasmid DNA was obtained. A 100 µg portion of the DNA was completely digested with restriction enzymes *Eco*RI and *Not*I and subjected to 0.8% agarose gel (manufactured by FMC BioProducts) electrophoresis, and the thus recovered vector fragments were purified using a Prep-A-Gene DNA purification kit (manufactured by Bio-Bad Laboratories, Inc.) to obtain about 55 µg of plasmid DNA which was used in the subsequent cDNA library construction.

〈Example 6〉

Purification of mRNA from McA-RH8994 cells

McA-RH8994 cells which showed relatively high activity in the colony assay of Example 4 were selected as the material for use in the rat TPO cDNA cloning and subjected to the following experiments.

Isolation of total RNA was carried out basically in accordance with the procedure described in *Molecular Cloning* (Sambrook *et al.*, Cold Spring Harbor Laboratory Press, 1989). That is, McA-RH8994 cells were grown to confluence in 15 culture dishes of 90 mm in diameter. After removing the liquid medium, cells in each dish were thoroughly suspended in 0.8 ml of a 5 M guanidine solution (5 M guanidine thiocyanate, 5 mM sodium citrate (pH 7.0), 0.1 M b-mercaptoethanol, 0.5% sodium sarcosylsulfate), and the resulting suspensions in all dishes were collected in a single tube and the total volume was adjusted to 20 ml with the guanidine solution. The resulting mixture, which became viscous due to cell disruption, was subjected to about 20 repetitions of suction/discharge with a 20 ml syringe equipped with a 18G needle and then 21G needle until the mixture became almost non-viscous. A 18 ml portion of 5.7 M CsCl-0.1 M EDTA (pH 7.5) was used as a cushion in a polyallomer centrifugation tube for use in Beckman SW28 rotor, and the aforementioned mixture of about 20 ml was gently layered over the cushion in such a manner that the layers were not disturbed and the tube was almost filled. The thus prepared tube was subjected to 20 hours of centrifugation at 25,000 rpm and at 20 °C. The resulting pellet was washed twice with a small amount of 80% ethanol. dissolved in TE solution, extracted with phenol/chloroform (1:1) and then subjected to ethanol precipitation with 1/10 volume of 3 M sodium acetate and 2.5 volumes of ethanol. In this way, about 2.5 mg of total RNA was obtained from about $10^8$ cells.

Purification of poly $(A)^+$ RNA from the total RNA was carried out using Oligotex™-dT30 (Super) (manufactured by Japan Synthetic Rubber/Nippon Roche; oligo dT molecules are immobilized on latex particles by covalent bonding, and purification of poly $(A)^+$ RNA can be attained similar to use of an oligo dT column which is generally used for such purpose). As the result, 20 μg of poly $(A)^+$ RNA was obtained from about 500 μg of the total RNA.

⟨Example 7⟩

Construction of rat cDNA library

Double-stranded cDNA having an *Eco*RI recognition site at its 5'-end and a *Not*I recognition site at the 3'-end was synthesized from 5 μg of the poly $(A)^+$ RNA obtained in Example 6, using a Time Saver™ cDNA synthesis kit (manufactured by Pharmacia; a cDNA synthesis kit based on the modified method of Okayama and Berg, *Mol. Cell. Biol.*, vol.2, pp.161 - 170, 1982) and DIRECTIONAL CLONING TOOLBOX (manufactured by Pharmacia; a set of a primer 5'-AACTGGAAGAATTCGCGGCCGCAGGAA$(T)_{18}$-3' (SEQ ID NO:15) containing a NotI recognition sequence for use in the synthesis of cDNA and adapters 5'-AATTCGGCACGAG-3' (SEQ ID NO:16) and 5-CTCGTGCCG-3' (SEQ ID NO:17) for use in the addition of an *Eco*RI recognition sequence). The thus synthesized cDNA was ligated with 1.2 μg of the expression vector pEF18S (cf. Example 5) which had been digested in advance with *Eco*RI and *Not*I and transformed into 8.4 ml of the aforementioned Competent High *E. coli* DH5 (manufactured by Toyobo Co., Ltd.) As the result, $5.3 \times 10^5$ transformants were obtained.

⟨Example 8⟩

Preparation (cloning) of rat TPO cDNA fragment by PCR

The 530,000 clones of the McA-RH8994 cDNA library constructed in Example 7 were cultured overnight in 50 ml of LB medium containing 50 μg/ml of ampicillin, and McA-RH8994 cDNA library plasmids were purified from the resulting cells collected by centrifugation, using QIAGEN-tip100 (manufactured by DIAGEN; an anion exchange silica column for DNA purification use). About 200 μg of plasmid DNA was obtained.

Two anti-sense nucleotide primers AP8-1R and AP8-2R, which correspond to the amino acid sequence of the peptide fragment AP8 described in Example 2, and two sense nucleotide primers EF1α-1 and EF1α-2, which correspond to the first intron of the human elongation factor 1α of the plasmid vector pEF-18S (cf. Fig. 6) used for the preparation of cDNA library, were synthesized. Synthesis of these primers was effected making use of a 394DNA/RNA Synthesizer (manufactured by Applied Biosystems; a synthesizer based on the b-cyanoethylamidite method) and their purification was effected by the use of an OPC column for synthetic DNA purification use (manufactured by Applied Biosystems; a reverse phase silica gel column for use in the purification of synthetic DNA having trityl groups). Each of the thus synthesized and purified DNA molecules was dissolved in TE solution to a final concentration of 50 μM and stored at -20 °C until its use. Synthetic oligonucleotides used in the following procedure were synthesized and purified in the same manner.

The primers AP8-1R and AP8-2R are mixed primers consisting of 17 continued nucleotides in which deoxyinosine is incorporated, as reported by Takahashi *et al.* (*Proc. Natl. Acad. Sci. USA*, vol.82, pp.1931 - 1935, 1985).

The primers EF1α-1 and EF1α-2 consisting of 21 and 20 nucleotides, respectively, were synthesized based on the nucleotide sequences of positions 1491 to 1512 and 1513 to 1532 of the genomic sequence reported by Uetsuki *et al.* (*J. Biol. Chem.*, vol.264, pp.5791 - 5798, 1989).

```
AP8: Ile Pro Val Pro Pro Ala Cys Asp Pro Arg Leu Leu
                                                (SEQ ID NO:18)
          Thr
                                                (SEQ ID NO:19)
          Ser
                                                (SEQ ID NO: 20)
```

```
AP8-1R:  3'-ACG CTG GGG GCI GAI GA-5'  (SEQ ID NO:21)
              A   A   A T   A   A      (SEQ ID NO:22)
                      T                (SEQ ID NO:23)
                      C                (SEQ ID NO:24)
AP8-2R:  3'-GGG GGG CGG ACG CTG GG-5'  (SEQ ID NO:25)
              A   A   A   A   A        (SEQ ID NO:26)
              T   T   T                (SEQ ID NO:27)
              C   C   C                (SEQ ID NO:28)
EF1α-1:  5'-GGA TCT TGG TTC ATT CTC AAG-3'  (SEQ ID NO:29)
EF1α-2:  5'-CCT CAG ACA GTG GTT CAA AG-3'   (SEQ ID NO:30)
```

Using 3 μg of the McA-RH8994 cDNA library plasmid as a template, AP8-1R (500 pmol) and EF1α-1 (100 pmol) as primers and GeneAmp™ PCR Reagent Kit with AmpliTaq™ DNA Polymerase (manufactured by Takara Shuzo Co., Ltd.; a set of a thermostable TaqI polymerase, a reaction buffer and dNTP for use in PCR), PCR was carried out by GeneAmp™ PCR system 9600 (manufactured by Perkin-Elmer; a thermal cycker for PCR) (100 μl volume; heating at 95°C for 2 minutes, a total of 35 cycles, each cycle consisting of denaturation at 95°C for 1 minute, annealing at 40°C for 1 minute and synthesis at 72°C for 1 minute, and final incubation at 72°C for 7 minutes). In order to improve specificity of the thus amplified DNA fragments, further PCR was carried out (100 μl volume; heating at 95°C for 2 minutes, a total of 35 cycles, each cycle consisting of denaturation at 95°C for 1 minute, annealing at 45°C for 1 minute and synthesis at 72°C for 1 minute, and final incubation at 72°C for 7 minutes), using 1 μl of the thus obtained PCR reaction solution as a template, and EF1α-2 (100 pmol) and AP8-2R (500 pmol) as primers.

The thus obtained reaction solution was subjected to 2% agarose gel (manufactured by FMC BioProducts) electrophoresis to isolate a DNA fragment of about 330 bp as the main product of the PCR which was subsequently purified making use of the aforementioned Prep-A-Gene DNA purification kit (manufactured by Bio-Rad Laboratories, Inc.). Using T4 DNA ligase (manufactured by Life Technologies), the thus purified DNA fragment was subcloned into pCR™II vector (a vector for the use of TA cloning of PCR products, manufactured by Invitrogen). Since the thermostable polymerase to be used in PCR has a terminal transferase activity, the amplified DNA fragment is directly subcloned into the pCR™II vector which has a 5'-dT cohesive end, making use of the enzyme's property to add one deoxyadenylic acid to the 3'-end of the PCR-amplified DNA. Plasmid DNA molecules were purified from 28 clones selected at random from the resulting clones using QIAGEN-tip100 (manufactured by DIAGEN) and their nucleotide sequences were determined by a 373A DNA sequencer (a fluorescence sequencer, manufactured by Applied Biosystems), making use of a Taq Dye Deoxy™ Terminater Cycle Sequening Kit (manufactured by Applied Biosystems; a kit for use in PCR-aided nucleotide sequence determination using fluorescent dyes, based on the dideoxy method of Sanger *et al.*, *Proc. Natl. Acad. Sci. USA*, vol.74, pp.5463 - 5467, 1977).

When a DNA fragment which encodes three amino acid residues, (Ile/Thr/Ser)-Val-Pro, of the amino acid sequence of AP8 shown above, adjacent to the AP8-2R primer, was selected from the thus obtained DNA fragments and its whole length was sequenced, it contained cDNA of 261 bp. Position 173 to 175 of this cDNA fragment encoded methionine, and the coding frame coincided with the amino acid frame of AP8. Since the sequence interposing the position 173-175 of the DNA fragment coincided with the sequence of Kozak (Kozak, M., *Cell*, vol.44, pp.238 - 292, 1986), it seems that this cDNA fragment contains a translation initiation region, coding for the N-terminal of rat TPO protein. This cDNA fragment was named A1. Nucleotide sequence of the A1 fragment excluding the vector sequence, and an amino acid sequence deduced therefrom are shown in the Sequence Listing (SEQ ID NO: 1) attached hereto.

〈Example 9〉

## Screening of rat TPO cDNA clone by PCR

The aforementioned cDNA library was divided into pools of about 10,000 clones, cultured overnight in 1 ml of aforementioned LB medium containing 50 μg/ml of ampicillin and then subjected to plasmid DNA extraction using an automatic plasmid isolation apparatus PI-100 (VER-3.0, manufactured by Kurabo Industries, Ltd.; an automatic plasmid DNA extraction apparatus based on a modified method of the alkali SDS method disclosed in *Molecular Cloning*, Sambrook *et al.*, Cold Spring Harbor Laboratory Press, 1989). Separately from this, the following two oligonucleotides were synthesized based on the cDNA fragment A1 and purified.

5' CGAGGGTGTACCTGGGTCCTG 3' (SEQ ID NO:31) (a sense sequence of positions 1 to 17 of the sequence of SEQ ID NO: 1; CGAG represents an adapter sequence)

5' CAGAGTTAGTCTTGCGGTGAG 3' (SEQ ID NO:32) (an anti-sense sequence of positions 212 to 232 of the sequence of SEQ ID NO: 1)

Using 1/30 volume of each plasmid DNA sample obtained above as a template, the thus synthesized oligonucleotides as primers and GeneAmp™ PCR Reagent Kit with AmpliTaq™ DNA Polymerase (manufactured by Takara Shuzo Co., Ltd.), PCR was carried out by GeneAmp™ PCR System 9600 (manufactured by PERKIN-ELMER) (a total of 30 cycles, each cycle consisting of denaturation at 94°C for 30 seconds, annealing at 66°C for 30 seconds and synthesis at 72°C for 1 minute). As the result, a specific band of 236 bp was detected in 3 of the 100 pools examined. When one of these 3 pools was divided into sub-pools of about 900 clones to extract plasmid DNA and PCR was carried out in the same manner, a specific band was detected in 3 of the 100 sub-pools tested. When one of these sub-pools was further divided into pools of 40 clones and the same screening process was carried out, the specific band was found in 3 of the 100 pools tested. One of the candidate pools was cultured on an LB plate (LB medium containing 15% agar) supplemented with 50 μg/ml of ampicillin and each of the thus formed colonies was subjected to plasmid DNA extraction and PCR in the same manner. As the result, a positive band was detected in 2 of the 100 clones examined.

⟨Example 10⟩

Sequencing of rat TPO cDNA

Purification of plasmid DNA was carried out basically in accordance with the procedure described in *Molecular Cloning* (Sambrook *et al.*, Cold Spring Harbor Laboratory Press, 1989). Each of the two clones obtained in Example 9 were cultured overnight in 50 ml of LB medium containing 50 μg/ml of ampicillin, and about 300 μg of plasmid DNA was obtained after purification in the same manner as described in Example 6.

The thus obtained plasmid DNA was subjected to sequencing in the same manner as described in Example 8 using the Taq Dye Deoxy™ Terminater Cycle Sequening Kit (manufactured by Applied Biosystems) to determine complete nucleotide sequence containing the A1 fragment. As the result, nucleotide sequences of the two clones completely coincided with each other, showing that they are the same clone. One of these clones was selected, and the plasmid carried by this clone was named pEF18S-A2α. Its nucleotide sequence and an amino acid sequence deduced therefrom are shown in the Sequence Listing (SEQ ID NO: 2).

The sequence shown in the Sequence Listing (SEQ ID NO: 2) has distinct characteristics. The sequence downstream of the 172 bp 5' non-translation region encodes an amino acid sequence rich in hydrophobic amino acids which is presumed to be a protein secretion signal sequence consisting of 21 amino acids starting with methionine. This protein contains 126 amino acid residues, and a 3' non-translation sequence of 1,025 nucleotides and a poly A tail following the termination codon (TAA). This protein contains a sequence which corresponds to the amino acid sequence AP8 analyzed in Example 2 (amino acid numbers 1 to 12 in SEQ ID NO: 2), but does not contain a consensus sequence for N-glycosylation. The 1624-1629 nucleotide sequence located closely to the terminal of the 3' non-translation sequence is different from a consensus sequence but seems to be a potential polyadenylation sequence.

The vector pEF18S-A2α carried by an *E. coli* strain DH5 has been deposited by the present inventors on February 14, 1994, under the deposit No. FERM BP-4565, in National Institute of Bioscience and Human Technology, Agency of Industrial Science and Technology, Ministry of International Trade and Industry, Japan.

⟨Example 11⟩

Expression of rat TPO cDNA in COS 1 cells and confirmation of TPO activity

Transfection of plasmid pEF18S-A2α into COS 1 cells was carried out in the following manner by slightly modifying the DEAE-dextran method which includes chloroquine treatment (Sompayrac *et al.*, *Proc. Natl. Acad. Sci. USA*, vol.78, pp.7575 - 7578, 1981; Luthman *et al.*, *Nucl. Acids Res.*, vol.11, pp.1295 - 1308, 1983). COS 1 cells (ATCC CRL1650) were suspended in the aforementioned DMEM medium containing 10% FCS, put into a 100-mm plastic tissue culture dish and cultured at 37°C in a 5% $CO_2$ incubator until the cells reached a stage of about 40% confluence. Separately, plasmid pEF18S-A2α (10 µg) dissolved in 30 µl of HBS (21 mM HEPES, 145 mM NaCl, pH 7.1) was added to 4 ml of the DMEM culture medium which has been supplemented with 500 µg/ml of DEAE-dextran (manufactured by Pharmacia), 80 µM of chloroquine (manufactured by Sigma Chemical Co.), 8% (v/v) of HBS and 9% (v/v) of Nu-Serum (manufactured by Collaborative Research, Inc.). The thus prepared mixture was added to the above cultured COS 1 cells which were washed twice with the DMEM culture medium, and the culturing was continued for 5 hours at 37°C in the 5% $CO_2$ incubator. Thereafter, culture supernatant in the dish was removed by suction, and the remaining cells in the dish were washed twice with the DMEM culture medium, supplemented with 15 ml of the DMEM culture medium containing 10% FCS and cultured at 37°C for 3 to 5 days in the 5% $CO_2$ incubator to recover the resulting culture supernatant.

The thus obtained culture supernatant was extensively dialyzed against IMDM culture medium and evaluated by the rat CFU-MK assay system. TPO activity was found in a dose-dependent fashion in the culture supernatant of COS 1 cells in which the plasmid pEF18S-A2α was expressed (Fig. 7). Similar to the case of XRP-derived TPO, many megakaryocytes formed elongated cytoplasmic processes on 4 days of culture. By contrast, TPO activity was not found in the culture supernatant of COS 1 cells to which the plasmid pEF18S alone was transfected (Fig. 7). In the M-07e assay system, M-07e cell proliferation enhancing activity was also found in a dose dependent fashion in the culture supernatant of COS 1 cells in which the plasmid pEF18S-A2α as expressed, but not in the culture supernatant of COS 1 cells in which the plasmid pEF18S alone was expressed. These results demonstrated that A2α contains a cDNA which encodes a protein having a TPO activity.

Next, a partially purified TPO sample was prepared in order to examine for the ability of this TPO activity to promote platelet production *in vivo*. Firstly, COS 1 cells transfected with the plasmid pEF18S-A2α were cultured for 3 days in a serum free culture medium which has been supplemented with 0.2 mg of BSA, thereby obtaining about 5.8 liters of serum free culture supernatant. Protease inhibitor p-APMSF was added to the serum-free culture supernatant to the final concentration of 1 mM, and the mixture was filtered using a 0.22 µm filter. To 5,793 ml of the resulting filtrate (protein concentration, 0.229 mg/ml; total protein, 1.326 mg; relative activity, 1,000; total activity, 1,326,000) was added 0.85 moles of NaCl for 1000 ml (288 g in total), thereby obtaining 5,849 ml of solution containing 0.822 M NaCl. The thus prepared solution was applied, at a flow rate of 7 ml/min, to a TSK-gel AF-BLUE 650 MH column (manufactured by Tosoh Corp., catalog No. 08705; 5 cm in diameter and 6 cm in bed height) which has been equilibrated in advance with 20 mM sodium phosphate (pH 7.2) containing 1 M NaCl. After completion of the sample application, about 7,900 ml of eluate passed through the column when eluted with 20 mM sodium phosphate (pH 7.2) containing 1 M NaCl. This eluate was pooled and concentrated using an ultrafiltration unit (Omega Ultrasette, nominal molecular weight cutoff of 8,000; manufactured by Filtron), thereby obtaining a passed-through fraction F1 (460 ml; protein concentration, 2.11 mg/ml; total protein, 973 mg; relative activity, 16.3). Next, the elution solution was changed to 2 M NaSCN, and the thus eluted TSK-gel AF-BLUE 650MH-adsorbed TPO-active fraction F2 (2840 ml) was concentrated to 6.81 ml using an ultrafiltration unit with YM 3 membrane (76 mm in diameter, manufactured by Amicon Corp.). Total protein in this TPO-active fraction F2 was 12.5 mg, and protein yield of F2 at this step was 0.62%. Relative activity of TPO was calculated to be 240. Next, the F2 was applied to a HiLoad 26/60 Superdex 200 pg (manufactured by Pharmacia Biotech, catalog No. 17-1071-01; 2.6 cm in diameter and 60 cm in bed height) and developed with 20 mM sodium acetate (pH 5.5) containing 50 mM NaCl at a flow rate of 1 ml/min. After commencement of the development, TPO activity was found in eluates in the range of from 194 to 260 ml after appliction. These eluates were pooled to obtain a Superdex 200 pg TPO-active fraction F2 (66 ml; protein concentration, 0.112 mg/ml; total protein, 7.41 mg; relative activity, 142,860; total activity, 1,058,600). Next, buffer A (20 mM sodium acetate, pH 5.5) and buffer B (20 mM sodium phosphate, pH 7.2, containing 500 mM NaCl) were prepared, and the TPO-active fraction was applied at a flow rate of 1 ml/min to a strong cation exchange column RESOURCE S (manufactured by Pharmacia Biotech, catalog No. 17-1178-01; 0.64 cm in diameter and 3 cm in bed height) which has been equilibrated in advance with 100% A. Thereafter, elution was carried out at a flow rate of 0.3 ml/min with 40 minutes of a linear gradient of from 100% A to 100% B. When assayed, the TPO activity was detected in a broad range of eluates (from 5% B to 32% B). These TPO-active eluates were pooled and concentrated using an ultrafiltration unit with YM 3 membrane (25 mm in diameter, manufactured by Amicon Corp.) to obtain a RESOURCE S TPO-active fraction F2 (1.65 ml; protein concentration, 4.74 mg/ml; total protein, 7.82 mg; relative activity, 71,400; total activity, 558,600).

The partially purified TPO sample was subcutaneously administered for consecutive 5 days (474 µg (100 µl)/mouse/day) to ICR male mice (9 weeks old) whose platelet numbers had been measured on the day before the administration. As a control, BSA (200 µg (100 µl)/mouse/day) or a buffer used as the solvent of the partially purified TPO (100 μl/mouse/day) was administered in the same manner. On the next day of the final administration, blood was collected from the heart of each mouse to measure platelet counts using a hemocytometer (F800, manufactured by Toa Iyo Denshi). In the partially purified TPO-administered mice, an increase in platelet counts by a factor of about 2.14 was observed in comparison with platelet counts before the administration. When compared with the control groups, increased platelet counts in the partially purified TPO-administered mice were about 1.74 times higher than those in the BSA-administered mice and about 1.90 higher than those in the buffer-administered mice. These results demonstrated that TPO promotes platelet production *in vivo*. In addition, since the amount of immunosuppressive acidic protein (IAP), known as a mouse acute phase protein, was not elevated in the partially purified TPO-administered mice, it was strongly suggested that TPO is different from IL-6 and IL-11 with regard to the induction of acute phase protein.

〈Example 12〉

Detection of TPO mRNA in rat tissues

In order to locate rat TPO mRNA expressing tissue in the rat body, RNA was extracted from various rat tissues. A total of 6 rats were subjected to X-ray irradiation in the same manner as described in Example 1, and various tissues (brain, thymus, lung, liver, heart, spleen, small intestine, kidney, testis and bone marrow cells) were excised from the rats on the 11th to 14th day after the X-ray irradiation and immediately frozen in liquid nitrogen. Extraction of total RNA was effected by the use of an RNA isolation reagent ISOGEN (manufactured by Wako Pure Chemical Industries, Ltd.). Amount of ISOGEN to be used was determined according the weight of each frozen tissue sample, and the reagent-added tissue sample was treated with a homogenizer (Physcotron$^R$ NS-60, manufactured by NITI-ON Medical & Physical Instruments MFG. Co., LTD) until complete disintegration of the tissue was attained (approximately 45 to 60 seconds at 10,000 rmp). The tissue homogenate was then subjected to total RNA extraction making use of a procedure based on the acid guanidium phenol chloroform method of Chomczynski *et al.* (*Anal. Biochem.*, vol.162, pp.156 - 159, 1987). As the result, 1.1 to 5.6 mg of total RNA was obtained from respective tissues.

Making use of Oligotex™-dT30 (Super) (manufactured by Japan Synthetic Rubber/Nippon Roche), 20 μg of poly $(A)^+$ RNA was purified from about 500 μg of total RNA.

Using random primer, first strand of cDNA was synthesized from 1 μg of the poly $(A)^+$ RNA obtained from each tissue. That is, 1 μg of the poly $(A)^+$ RNA was dissolved in 10 μl of sterile water, incubated at 70°C for 15 minutes and then rapidly cooled down. To this were added 75 pmoles of random primer (Takara Shuzo Co., Ltd.), 10 U of RNase inhibitor (Boehringer-Mannheim Corp.), 50 mM of Tris-HCl (pH 8.3), 75 mM of KCl, 3 mM of $MgCl_2$ and 200 U of Super Script™ II (a reverse transcriptase manufactured by Life Technologies). The thus prepared solution (total volume, 20 μl) was incubated at 37°C for 1 hour, and the resulting reaction solution was incubated at 70°C for 10 minutes to inactivate the enzyme and stored at -20°C until its use.

New primers for PCR were synthesized based on the rat TPO cDNA sequence obtained in Example 10. Sequences of the thus synthesized primers are as follows.

rTPO-I: 5'-CCT GTC CTG CTG CCT GCT GTG-3' (SEQ ID NO:33) (positions 347 to 367 in SEQ ID NO: 2)

rTPO-N: 5'-TGA AGT TCG TCT CCA ACA ATC-3' (SEQ ID NO:34) (anti-sense primer corresponding to positions 1005 to 1025 in SEQ ID NO: 2)

Using 1/10 volume of each of the synthesized cDNA solution as a template, 1 μM each of the thus synthesized rTPO-I and rTPO-N as primers and GeneAmp™ PCR Reagent Kit with AmpliTaq™ DNA Polymerase (manufactured by Takara Shuzo Co., Ltd.), PCR was carried out in a volume of 100 μl, making use of GeneAmp™ PCR System 9600 (manufactured by PERKIN-ELMER) and heating at 95°C for 2 minutes, repeating a total of 30 cycles, each cycle consisting of denaturation at 95°C for 1 minute, annealing at 57°C for 1 minute and synthesis at 72°C for 1 minute, followed by the final incubation at 72°C for 7 minutes. When each of the thus obtained reaction solutions was subjected to electrophoresis using 2% agarose gel (manufactured by FMC BioProducts) and the amplified bands were examined, bands that seemed to be specific for TPO mRNA were found on gels resulting from the brain, the liver, the small intestines and the kidney. These results indicate that expression of TPO mRNA in rat occurs in tissues of these organs, though its expression quantity cannot be judged. When possible existence of similar expression mode in the human body and the results of Example 4 are taken together into consideration, it seems that the liver is an appropriate starting material for the acquisition of human TPO cDNA.

⟨Example 13⟩

Construction of human normal liver-derived cDNA library

Based on the results of Example 12, the liver was selected as the starting material for use in the cloning of human TPO cDNA. In the same manner as described in Example 7, double-stranded cDNA having an *Eco*RI recognition site on its 5'-end and a *Not*I recognition site on its 3'-end was synthesized from 5 µg of commercial normal human liver-derived poly $(A)^+$ RNA (manufactured by Clontech; a product extracted based on the acid guanidium phenol chloroform method of Chomczynski *et al.*), using a Time Saver™ cDNA synthesis kit (manufactured by Pharmacia) and DIRECTIONAL CLONING TOOLBOX (manufactured by Pharmacia). The thus synthesized cDNA was ligated with 1.2 µg of the aforementioned expression vector pEF18S which had been digested in advance with *Eco*RI and *Not*I and transformed into 8.4 ml of the aforementioned Competent High *E. coli* DH5 (manufactured by Toyobo Co., Ltd.) As the result, 1.2 x $10^6$ transformants were obtained.

⟨Example 14⟩

Preparation (cloning) of human TPO cDNA fragment by PCR

Using random primers, first strand of cDNA was synthesized from 1 µg of the commercial normal human liver-derived poly $(A)^+$ RNA (manufactured by Clontech). That is, 1 µg of the poly $(A)^+$ RNA was dissolved in 10 µl of sterile water, incubated at 70 °C for 15 minutes and then rapidly cooled down. To this were added 75 pmoles of random primers (Takara Shuzo Co., Ltd.), 10 U of RNase inhibitor (Boehringer-Mannheim Corp.), 50 mM of Tris-HCl (pH 8.3), 75 mM of KCl, 3 mM of $MgCl_2$ and 200 U of a reverse transcriptase Super Script™ II (manufactured by Life Technologies). The thus prepared solution (total volume, 20 µl) was incubated at 37 °C for 1 hour, and the resulting reaction solution was incubated at 70 °C for 10 minutes to inactivate the enzyme and stored at -20 °C until its use.

Primers for PCR use were synthesized based on the rat TPO cDNA sequence (SEQ ID NO: 2). Sequences of the thus synthesized primers are as follows.

rTPO-AIN: 5'-ATG GAG CTG ACT GAT TTG CTC-3' (SEQ ID NO:35) (positions 173 to 193 in SEQ ID NO: 2)

rTPO-N: 5'-TGA AGT TCG TCT CCA ACA ATC-3' (SEQ ID NO:36) (anti-sense primer corresponding to positions 1005 to 1025 in SEQ ID NO: 2)

Using 1/10 volume of the synthesized cDNA solution as a template, 1 µM each of the thus synthesized rTPO-AIN and rTPO-N as primers and GeneAmp™ PCR Reagent Kit with AmpliTaq™ DNA Polymerase (manufactured by Takara Shuzo Co., Ltd.), PCR was carried out with a volume of 100 µl, making use of GeneAmp™ PCR System 9600 (manufactured by PERKIN-ELMER) and heating at 95 °C for 2 minutes, repeating a total of 35 cycles, each cycle consisting of denaturation at 95 °C for 1 minute, annealing at 40 °C for 1 minute and synthesis at 72 °C for 1 minute, followed by the final incubation at 72 °C for 7 minutes.

The thus obtained reaction solution was subjected to 2% agarose gel (manufactured by FMC BioProducts) electrophoresis to isolate a DNA fragment of about 620 bp as the main product of the PCR which was subsequently purified making use of the aforementioned Prep-A-Gene DNA purification kit (manufactured by Bio-Rad Laboratories, Inc.). Nucleotide sequence of the thus purified DNA fragment was directly determined by a 373A DNA sequencer (manufactured by Applied Biosystems) making use of the aforementioned Taq Dye Deoxy™ Terminater Cycle Sequening Kit (manufactured by Applied Biosystems). The thus determined nucleotide sequence excluding the primer moiety and an amino acid sequence deduced therefrom are shown in the Sequence Listing (SEQ ID NO: 3).

This DNA fragment, excluding the primer sequences, has a length of 580 bp. When compared, the human cDNA showed a homology of 86% with the rat cDNA nucleotide sequence, indicating that this DNA fragment encodes a portion of human TPO cDNA.

⟨Example 15⟩

Screening of human TPO cDNA clone by PCR

Human TPO-corresponding primers for PCR were synthesized based on SEQ ID NO: 3. Sequences of the thus synthesized primers are as follows.

hTPO-I: 5'-TTG TGA CCT CCG AGT CCT CAG-3' (SEQ ID NO:37) (positions 60 to 80 in SEQ ID NO: 3)
hTPO-J: 5'-TGA CGC AGA GGG TGG ACC CTC-3' (SEQ ID NO:38) (anti-sense primer corresponding to positions 479 to 499 in SEQ ID NO: 3)

The human cDNA library constructed in Example 13 was amplified and divided into pools (each pool containing about 100,000 clones), cultured overnight in 1 ml of the aforementioned LB medium containing 50 µg/ml of ampicillin and then subjected to plasmid DNA extraction using an automatic plasmid isolation apparatus PI-100 (VER-3.0, manufactured by Kurabo Industries, Ltd.). The thus extracted DNA was dissolved in TE solution.

Using 5% of the extracted DNA sample as a template, 1 µM each of the synthesized oligonucleotides (hTPO-I and hTPO-J) as primers and GeneAmp™ PCR Reagent Kit with AmpliTaq™ DNA Polymerase (manufactured by Takara Shuzo Co., Ltd.), PCR was carried out in 20 µl volume by GeneAmp™ PCR System 9600 (manufactured by PERKIN-ELMER) (a total of 35 cycles, each cycle consisting of denaturation at 95°C for 1 minute, annealing at 59°C for 1 minute and synthesis at 72°C for 1 minute, followed by the final incubation at 72°C for 7 minutes). As the result, a specific band was detected in 3 of the 90 pools used. One of these 3 pools was divided into sub-pools, each containing about 5,000 clones, and plasmid DNA was purified from 90 sub-pools to carry out PCR in the same manner. As the result, specific band was detected in 5 sub-pools. One of these 5 pools was divided into sub-pools, each containing 250 clones, and plasmid DNA was extracted from 90 sub-pools. When these extracted samples were subjected to PCR in the same manner, a specific band was detected in 3 sub-pools. One of these 3 pools was further divided into sub-pools, each containing 30 clones, and plasmid DNA was purified from 90 sub-pools to cab out PCR in the same manner. As the result, a specific band was detected in 3 sub-pools. One of the candidate pools was cultured on the aforementioned LB plate containing 50 µg/ml of ampicillin and each of the thus formed 90 colonies was subjected to plasmid DNA extraction and PCR in the same manner. As the result, a clone HL34 was finally obtained.

⟨Example 16⟩

Sequencing of human TPO cDNA

Purification of plasmid DNA was carried out basically in accordance with the procedure described in *Molecular Cloning* (Sambrook *et al.*, Cold Spring Harbor Laboratory Press, 1989). The clone HL34 was cultured overnight in 50 ml of the LB medium containing 50 µg/ml of ampicillin, and the resulting cells collected by centrifugation were suspended in 4 ml of the aforementioned TEG-lysozyme solution. To this were added 8 ml of 0.2 N NaOH/1% SDS solution and then 6 ml of 3 M potassium/5 M acetate solution to suspend the cells thoroughly. After centrifugation of the suspension, the resulting supernatant fluid was treated with phenol/chloroform (1:1), mixed with the same volume of isopropanol and then centrifuged. The resulting pellet was dissolved in the TE solution and treated with RNase and then with phenol/chloroform (1:1), followed by ethanol precipitation. The resulting pellet was again dissolved in the TE solution to which were subsequently added NaCl and polyethylene glycol 3,000 to their final concentrations of 0.63 M and 7.5%, respectively. After centrifugation, the pellet was dissolved in the TE solution and precipitated with ethanol. In this way, about 300 µg of the pEF18S-HL34 plasmid DNA was obtained.

The thus purified plasmid DNA was applied to the aforementioned 373A DNA sequencer (manufactured by Applied Biosystems) to determine its complete nucleotide sequence using the aforementioned Taq Dye Deoxy™ Terminater Cycle Sequening Kit (manufactured by Applied Biosystems). The thus determined nucleotide sequence and an amino acid sequence deduced therefrom are shown in the Sequence Listing (SEQ ID NO: 4). In this case, oligonucleotides synthesized based on the nucleotide sequence of SEQ ID NO: 3 and synthetic oligonucleotides designed based on the internal sequence obtained by the sequence analysis were used in the nucleotide sequence determination as primers.

As the result, it was confirmed that the plasmid clone pEF18S-HL34 comprises a cDNA fragment of 861 bp and contains highly homologous sequences with the amino acid sequences AP8 (amino acid numbers 1 to 12 in SEQ ID NO: 4) and TP2/TP3 (amino acid numbers 157 to 162 in SEQ ID NO: 4) analyzed in Example 2. This DNA fragment seems to encode an open reading frame starting at its position 25, but has no termination codon and contains a poly A tail-like sequence of 76 bases on its 3'-end. Its amino acid sequence, consisting of 253 amino acid residues just before the poly A tail-like sequence, showed 84% homology with the corresponding portion of the rat TPO cDNA (an amino acid sequence moiety consisting of 147 residues shown in SEQ ID NO: 2). In consequence, this clone was found to be a DNA fragment which encodes a portion of human cDNA that corresponds to rat TPO cDNA. Because of the absence of a termination codon and the presence of a poly A tail-like sequence on its 3'-end, this clone did not seem to be a complete cDNA, but an artificial product which resulted during the procedure of cDNA library construction.

⟨Example 17⟩

Expression of human TPO cDNA in COS 1 cells and confirmation of TPO activity

Transfection of the thus obtained plasmid clone pEF18S-HL34 to COS 1 cells was carried out according to the procedure of Example 11. That is, transfection was performed with 10 μg of the plasmid DNA by the DEAE-dextran method which includes chloroquine treatment. The COs-1 cells were cultured for 3 to 5 days at 37°C, and then the supernatant was collected.

The thus obtained culture supernatant was extensively dialyzed against the IMDM culture medium and evaluated by the rat CFU-MK assay system. TPO activity was detected in a dose-dependent fashion in the culture supernatant of COS 1 cells in which a plasmid pEF18S-HL34 was expressed (Fig. 8). After 4 days of culturing, many megakaryocytes formed elongated cytoplasmic processes. In contrast, TPO activity was not found in the culture supernatant of COS 1 cells in which the plasmid pEF18S alone was expressed (Fig. 8). In the M-07e assay system, M-07e cell proliferation enhancing activity was found only in the culture supernatant of COS 1 cells in which the plasmid pEF18S-HL34 was expressed through its transfection. These results demonstrated that pEF18S-HL34 contains a gene which encodes a protein having a TPO activity. In addition, it was shown that human TPO acts on rat megakaryocytic progenitor cells (no species specificity).

⟨Example 18⟩

Expression of human TPO deletion type cDNA and confirmation of TPO activity

The clone HL34 obtained in Example 15 contained the cDNA comprising a poly A tail-like continuous sequence on its 3'-side, which did not exist in the rat TPO cDNA and therefore seemed to be an artificial product of the experiment. In consequence, a cDNA molecule from which the poly A tail-like sequence was deleted was prepared in order to see if a protein expressed by the deletion cDNA had a TPO activity. The deletion cDNA was prepared making use of PCR. Sequences of primers used for PCR are as follows in which a restriction enzyme recognition site is added to the 5'-end of each primer (*Eco*RI to hTPO5 and *Not*I and two stop codons TAA and TGA to hTPO3).

hTPO5: 5'-TTT GAA TTC GGC CAG CCA GAC ACC CCG GCC-3' (SEQ ID NO:170) (positions 1 to 21 in SEQ ID NO: 4)

hTPO3: 5'-TTT GCG GCC GCT CAT TAT TCG TGT ATC CTG TTC AGG TAT CC-3' (SEQ ID NO:171) (anti-sense primer corresponding to positions 757 to 780 in SEQ ID NO: 4)

Using 1 μg of plasmid DNA of the plasmid clone pEF18S-HL34 obtained in Example 16 as a template, 10 μM each of the thus synthesized hTPO5 and hTPO3 as primers and GeneAmp™ PCR Reagent Kit with AmpliTaq™ DNA Polymerase (manufactured by Takara Shuzo Co., Ltd.), PCR was carried out with a volume of 100 μl, making use of GeneAmp™ PCR System 9600 (manufactured by PERKIN-ELMER) and repeating a total of 15 cycles, each cycle consisting of denaturation at 95°C for 1 minute, annealing at 65°C for 1 minute and synthesis at 72°C for 1 minute, followed by the final incubation at 72°C for 7 minutes. A band of about 800 bp thus obtained was digested with restriction enzymes *Eco*RI and *Not*I, purified and then sub-cloned into the expression vector pEF18S which has been treated in advance with the same restriction enzymes. From the resulting transformants, 5 clones which contained a DNA fragment of about 800 bp were selected to prepare a large quantity of plasmid DNA in accordance with the procedure described in Example 5. The whole length of the amplified region of about 800 bp of each plasmid was subjected to nucleotide sequence analysis to find its complete identity with the nucleotide sequence analyzed in Example 16 (positions 1 to 780 in SEQ ID NO: 4).

The plasmid clone thus obtained was named pHT1-231. The vector pHT1-231 carried by an *E. coli* strain DH5 has been deposited by the present inventors on February 14, 1994 under the deposit No. FERM BP-4564, in National Institute of Bioscience and Human Technology, Agency of Industrial Science and Technology, Ministry of International Trade and Industry, Japan.

Transfection of the thus obtained plasmid to COS 1 cells was carried out according to the procedure of Example 11. That is, transfection was performed with 10 μg of the plasmid DNA by the DEAE-dextran method which includes chloroquine treatment. The COS1 cells were cultured for 3 to 5 days at 37°C, and then the supernatant was collected.

The thus obtained culture supernatant was extensively dialyzed against the IMDM culture medium and evaluated by the rat CFU-MK assay system. TPO activity was found in the culture supernatant of COS 1 cells in which the plasmid pHT1-231 was expressed (Fig. 9). On 4 days of the culture, many megakaryocytes formed elongated cytoplasmic processes. By contrast, TPO activity was not found in the culture supernatant of COS 1 cells in which the plasmid pEF18S alone was expressed (Fig. 9). In the M-07e assay system, M-07e cell proliferation enhancing activity was found only in the culture supernatant of COS 1 cells in which the plasmid pHT1-231 was expressed. These results demonstrated that pHT1-231 contains a cDNA which encodes a protein having a TPO activity.

〈Example 19〉

Preparation of the 3'-end region of human TPO cDNA by PCR

The clone HL34 prepared in Example 15 contained the cDNA comprising poly (A) tail-like sequence so that it was suggested that its 3'-end region was incomplete. It was therefore tried to obtain a full-length 3'-end region by PCR. The following four kinds of 5'-side primer for PCR were synthesized based on the sequences determined in Example 16

hTPO-H: 5'-AGC AGA ACC TCT CTA GTC CTC-3' (SEQ ID NO:39) (positions 574 to 594 in SEQ ID NO: 4)
hTPO-K: 5'-ACA CTG AAC GAG CTC CCA AAC-3' (SEQ ID NO:40) (positions 595 to 615 in SEQ ID NO: 4)
hTPO-N: 5'-AAC TAC TGG CTC TGG GCT TCT-3' (SEQ ID NO:41) (positions 660 to 680 in SEQ ID NO: 4)
hTPO-O: 5'-AGG GAT TCA GAG CCA AGA TTC-3' (SEQ ID NO:42) (positions 692 to 712 in SEQ ID NO: 4)

The following 3'-side primers containing mixed nucleotides at the four bases of 3'-end were synthesized in order to amplify the cDNA from the beginning of the poly (A) portion. As anchor primer, without the mixed nucleotide, was also synthesized.

```
hTPO3mix: 5'-TAG CGG CCG C(T)17G GGG-3'    SEQ ID NO:43)
                                A AAA-3'    SEQ ID NO:44)
                                C TTT-3'    (SEQ ID NO:45)
                                  CCC-3'    (SEQ ID NO:46)
hTPO3anchor: 5'-TAG CGG CCG C(T)11-3'       (SEQ ID NO:47)
```

The first strand of cDNA was synthesized from 1 µg of the commercial normal human liver-derived poly $(A)^+$ RNA (manufactured by Clontech), such as in Example 14, but using 0.5 µg of oligo dT primer (it is included in TimeSaver™ cDNA Synthesis Kit manufactured by Pharmacia). Using 1/10 volume of the synthesized cDNA solution as a template 20 µM of the hTPO-H primer and 10 µM of the hTPO3mix primer and GeneAmp™ PCR Reagent Kit with AmpliTaq™ DNA Polymerase (manufactured by Takara Shuzo Co., Ltd.), PCR was carried out with volume of 50 µl, making use of GeneAmp™ PCR System 9600 (manufactured by PERKIN-ELMER) and heating at 96°C for 2 minutes, repeating a total of 10 cycles, each cycle consisting of heat denaturation at 96°C for 1 minute, annealing at 48°C for 1 minute and synthesis at 72°C for 1 minute, followed by the final incubation at 72°C for 7 minutes.

The second PCR was carried out by using 1/10 volume of the first PCR resulting solution as a template, 20 µM of the hTPO-K primer and 10 µM of the hTPO3mix primer with volume of 50 µl, heating at 96°C for 2 minutes, repeating a total of 10 cycles, each cycle consisting of heat denaturation at 96°C for 1 minute, annealing at 63°C for 1 minute and synthesis at 72°C for 1 minute, followed by the final incubation at 72°C for 7 minutes.

The third PCR was carried out by using 1/10 volume of the second PCR resulting solution as a template, 20 µM of the hTPO-N primer and 10 µM of the hTPO3mix primer with volume of 50 µl, heating at 96°C for 2 minutes, repeating a total of 10 cycles, each cycle consisting of heat denaturation at 96°C for 1 minute, annealing at 63°C for 1 minute and synthesis at 72°C for 1 minute, followed by the final incubation at 72°C for 7 minutes.

The fourth PCR was carried out by using 1/10 volume of the third PCR resulting solution as a template, 20 µM of the hTPO-O primer and 10 µM of the hTPO3mix primer with volume of 50 µl, heat at 96°C for 2 minutes, repeating a total of 10 cycles, each cycle consisting of heat denaturation at 96°C for 1 minute, annealing at 63°C for 1 minute and synthesis at 72°C for 1 minute, followed by a final incubation at 72°C for 7 minutes.

The fifth PCR was carried out by using 1/10 volume of the fourth PCR resulting solution as a template, 20 μM of the hTPO-O primer and 20 μM of the hTPO3anchor primer with volume of 50 μl, heating at 96°C for 2 minutes, repeating a total of 10 cycles, each cycle consisting of heating denaturation at 96°C for 1 minute, annealing at 58°C for 1 minute and synthesis at 72°C for 1 minute, followed by the final incubation at 72°C for 7 minutes.

The thus obtained reaction solution was subjected to 2% agarose gel (manufactured by FMC BioProducts) electrophoresis to isolate a DNA fragment of about 600 bp as the main product of the PCR which was subsequently purified making use of the aforementioned Prep-A-Gene DNA purification kit (manufactured by Bio-Rad Laboratories, Inc.). Nucleotide sequence of the thus purified DNA fragment was directly determined by a 373A DNA sequencer (manufactured by Applied Biosystems) making use of the aforementioned Taq Dye Deoxy™ Terminater Cycle Sequening Kit (manufactured by Applied Biosystems). The thus determined nucleotide sequence and an amino acid sequence deduced therefrom are shown in the Sequence Listing (SEQ ID NO: 5).

This DNA fragment has a nucleotide sequence coding for 130 amino acids starting with the primer hTPO-O, followed by sequence of more than 180 nucleotides at 3'-end (the nucleotides after 577 position could not determined). The 30 amino acid sequence starting with glycine coincides with the amino acid sequence of the positions 203 to 232 in SEQ ID NO: 4. The nucleotide sequence of positions 1 to 94 also coincides with the nucleotide sequence of the positions 692 to 785 in SEQ ID NO: 4.

As the result of sequence analysis of the cDNA fragment in Example 17 and PCR-amplified fragment in the present Example, it is reasonably estimated that human TPO protein consists of 353 amino acids containing 21 amino-acid signal sequence, as shown in SEQ ID NO: 6.

〈Example 20〉

Reconstruction of human normal liver-derived cDNA library

Since the clone HL34 obtained in Example 15 contained the poly A tail-like sequence directly on its 3'-end of its open reading frame without termination codons and seemed therefore to be an artificial product of the cDNA synthesis a cDNA library was reconstructed using 5 μg of a commercial normal human liver-derived poly $(A)^+$ RNA preparation (manufactured by Clontech). Synthesis of cDNA was carried out using SuperScript™ Lambda System for cDNA Synthesis and λ Cloning Kit and SuperScript™ II RNase $H^-$ (both manufactured by LIFE TECHNOLOGIES). The poly $(A)^+$ RNA was subjected to heat denaturation and then added to 20 μl of a reaction solution containing a *Not*I sequence-included oligo dT as a primer attached to the kit (50 mM Tris-HCl, pH 8.3, 75 mM KCl, 3 mM $MgCl_2$, 1 mM DTT, 1 mM dNTP mix, 200 U SuperScript™ II RNase $H^-$), followed by 60 minutes of incubation at 37°C. After synthesis of the second strand of cDNA (2 hours of incubation at 16°C in 150 μl of a reaction solution containing 25 mM Tris-HCl, pH 8.3, 100 mM KCl, 5 mM $MgCl_2$, 250 μM dNTP mix, 5 mM DTT, 40 U of *E. coli* DNA polymerase I, 2 U of *E. coli* RNase H and 10 U of *E. coli* DNA ligase), 10 U of T4 DNA polymerase was added and the resulting mixture was incubated at 16°C for 5 minutes. The reaction solution was heated at 65°C for 10 minutes and extracted with the same volume of phenol/chloroform, and cDNA molecules having a length of less than 400 bp were removed using SizeSep™ 400 spun column (a spun column for the removal of low molecular weight DNA, attached to TimeSaver™ cDNA Synthesis Kit manufactured by Pharmacia). After the addition of *Eco*RI Adaptor (attached to Directional Cloning Toolbox manufactured by Pharmacia), the thus treated sample was digested with *Not*I and again applied to SizeSep™ 400 spun column to remove low molecular weight DNA. The thus synthesized 1.3 μg of the double-stranded cDNA having an *Eco*RI recognition sequence on its 5'-end and a *Not*I recognition sequence on its 3'-end was ligated with the expression vector pEF18S which had been previously digested with *Eco*RI and *Not*I, and then transformed into 9.2 ml of Competent High *E. coli* DH5 (manufactured by Toyobo Co., Ltd.) The thus obtained human liver cDNA library (hTPO-F1) contained 1.0 x $10^6$ transformants.

〈Example 21〉

Screening of TPO cDNA clone from human liver cDNA library hTPO-F1

Human TPO cDNA-corresponding primers for PCR were synthesized based on the Sequence ID Nos. 3 and 6 shown in the SEQUENCE LISTING. Sequences of the thus synthesized primers are as follows.
hTPO-I: 5'-TTG TGA CCT CCG AGT CCT CAG-3' (SEQ ID NO:48) (positions 60 to 80 in SEQ ID NO: 3)
hTPO-KU: 5'-AGG ATG GGT TGG GGA AGG AGA-3' (SEQ ID NO:49) (anti-sense primer corresponding to the sequence of positions 901 to 921 in SEQ ID NO: 6)

The human liver cDNA library hTPO-F1 (1.0 x $10^6$ transformants) constructed in Example 20 was divided into 3 pools (pool # 1 - 3) and the pools were frozen. PCR was carried out using 2 µg of the plasmid DNA prepared from each pool as a template and 1 µM each of the synthesized oligonucleotides (hTPO-I and hTPO-KU) as primers. Using GeneAmp™ PCR Reagent Kit with AmpliTaq™ DNA Polymerase (manufactured by Takara Shuzo Co., Ltd.), PCR was performed in 100 µl volume by GeneAmp™ PCR System 9600 (manufactured by PERKIN-ELMER) (a total of 35 cycles, each cycle consisting of denaturation at 95°C for 1 minute, annealing at 59°C for 1 minute and synthesis at 72°C for 1 minute, followed by the final incubation at 72°C for 7 minutes). As the result, a DNA fragment having the expected size was amplified when the plasmid DNA prepared from the pool # 3 was used. The # 3 pool was then divided into subpools, each containing 15,000 transformants, and these subpools were cultured overnight in 1 ml of LB medium containing 50 µg/ml of ampicillin, followed by extraction of plasmid DNA using an automatic plasmid isolation apparatus PI-100. The thus extracted DNA was dissolved in TE solution, and 5% of the resulting solutions were used as templates to carry out PCR using the same primers and under the same conditions described above. As the result, amplification of DNA having the expected size was found in 6 of the 90 pools. When one of these positive pools was divided into subpools, each containing 1,000 clones, and plasmid DNA was extracted and PCR was carried out in the same manner as described above, DNA amplification was not observed. The density of bands on electrophoresis gels of DNA fragments amplified by a series of PCR became thinner as the subpool was further subdivided, which seemed to be caused by a low recovery of the plasmid DNA due to poor growth of the clone of interest. Therefore, another screening was carried out by means of colony hybridization using the original # 3 pool.

The # 3 pool was spread on 100 LB agar plates of 15 cm in diameter in such an inoculum size that 4,100 colonies grew on each LB agar plate. After preparing a replica plate from each of the thus inoculated plates, one of the duplicate plates was cultured at 37°C for 6 hours to recover colonies grown on the plate and to extract the plasmid DNA samples. When PCR of these DNA samples was carried out in the same manner as described above, amplification of a band equivalent to the expected size was observed in one of 100 subpools. Two replica filters were prepared from the plate of this subpool using BIODYNE™ A TRANSFER MEMBRANE (manufactured by PALL). Denaturation of the filters were performed by soaking them in 10% SDS for 10 minutes, in 0.5 N NaOH/1.5 M NaCl for 10 minutes and in 0.5 M Tris-HCl (pH 8.0)-/1.5 M NaCl for 10 minutes in that order, followed by 30 minutes of air-drying and subsequent 1 hour of baking at 80°C in a vacuum oven. The thus baked filters were washed with 6 x SSC (prepared by diluting 20 x SSC stock solution consisting of 175.3 g of NaCl and 88.2 g of Na citrate dissolved in 1 liter water, pH 7.0) supplemented with 1 % SDS. Prehybridization of the thus washed filters were carried out by incubating it at 42°C for 30 minutes with shaking in 30 ml of a reaction solution consisting of 50% formamide, 5 x SSC, 5 x Denhardt's solution (prepared from 50 x Denhardt's solution containing 5 g of Ficoll. 5 g of polyvinylpyrrolidone and 5 g of bovine serum albumin fraction V in 500 ml water), 1% SDS and 20 µg/ml of salmon sperm DNA. After prehybridization, the reaction solution was exchanged with 30 ml of a hybridization solution having the same composition, mixed with a probe which had been labeled with [$\alpha$-$^{32}$P] dCTP (manufactured by Amersham) and then incubated with shaking at 42°C for 20 hours. The labeled probe used in this experiment was the *Eco*RI/*Bam*HI fragment of the plasmid pEF18S-HL34, which was obtained by purifying a portion of SEQ ID NO: 4 ranging from its 5'-end to the 458 position base and by labeling the purified portion by random primer technique with Megaprime DNA Labelling System (a kit manufactured by Amersham based on the method disclosed in *Anal. Biochem.*, 132, 6 - 13, 1983). The thus treated filters were washed in 2 x SSC/0.1% SDS solution at 42°C for 30 minutes and then in 0.2 x SSC/0.1% SDS solution at 42°C for 30 minutes. The filters were then subjected to 16 hours of autoradiography at -70°C using an intensifying screen and an X-OMAT™ AR5 film (manufactured by Eastman Kodak). As the result, a single signal considered to be positive was observed. Colonies approximately corresponding to this signal were collected from the original plate and inoculated again onto a 10-cm LB agar plate. A total of 50 colonies grown on the plate were separately cultured, and their DNA samples were subjected to PCR using the aforementioned primers hTPO-I and hTPO-KU under the same conditions as described above. As the result, amplification of a band equivalent to the expected size was found in only one clone, which was designated pHTF1.

⟨Example 22⟩

Determination of nucleotide sequence of human TPO cDNA clone pHTF1

Purification of plasmid DNA was carried out basically according to the procedure described in *Molecular Cloning* (Sambrook *et al.*, Cold Spring Harbor Laboratory Press, 1989). The clone pHTF1 was cultured overnight in 50 ml of the LB medium containing 50 µg/ml of ampicillin. The resulting cells were collected by centrifugation and suspended in 4 ml of the aforementioned TEG-lysozyme solution. To this were added 8 ml of 0.2 N NaOH/1% SDS solution and then 6 ml of 3 M potassium/5 M acetate solution to suspend the cells thoroughly. After centrifugation of the suspension, the resulting supernatant was treated with phenol/chloroform (1:1), mixed with the same volume of isopropanol and then centrifuged. The resulting pellet was dissolved in TE solution and treated with RNase and then with phenol/chloroform (1:1), followed by ethanol precipitation. The resulting pellet was again dissolved in TE solution to which were subsequently added NaCl and polyethylene glycol 3,000 to their final concentrations of 0.63 M and 7.5%, respectively. After centrifugation, the pellet was dissolved in TE solution and precipitated with ethanol. In this way, about 300 µg of the plasmid DNA pHTF1 was obtained.

The thus purified plasmid DNA was applied to the aforementioned 373A DNA sequencer (manufactured by Applied Biosystems) to determine its complete nucleotide sequence using the aforementioned Taq Dye Deoxy™ Terminater Cycle Sequening Kit (manufactured by Applied Biosystems). The thus determined nucleotide sequence and the deduced amino acid sequence are shown in the Sequence Listing (SEQ ID NO: 7). Oligonucleotides synthesized based on the nucleotide sequence of SEQ ID NO: 6 and on the internal sequence obtained by their sequence reaction analysis were used in the nucleotide sequence determination as primers.

As the result, it was confirmed that the clone pHTF1 contains a cDNA fragment of 1,721 bp and has high homology with the amino acid sequences AP8 (amino acid numbers 1 to 12 in SEQ ID NO: 7) and TP2/TP3 (amino acid numbers 157 to 162 in SEQ ID NO: 7) analyzed in Example 2. This DNA fragment seems to have a 5′ noncoding region of 101 bases, an open reading frame consisting of 353 amino acid residues, starting at a methionine residue encoded at the 102 to 104 nucleotide positions and ending at a glycine residue encoded at the 1,158 to 1,160 nucleotide positions, the subsequent termination codon (TAA), a 3′ noncoding region of 531 bases and a poly A tail sequence of 30 bases. The amino acid sequence of a protein considered to be encoded by the open reading frame completely coincided with the predicted amino acid sequence of human TPO shown in SEQ ID NO: 6. The cDNA sequence of pHTF1 has a larger size than the putative cDNA sequence shown in SEQ ID NO: 6, and contains 77 more additional bases at the 5' side and 347 more additional bases at the 3' side in front of its poly A tail sequence. Also, the nucleotide sequence was different from SEQ ID NO: 6 at 3 positions. That is, A (position 84), A (position 740) and G (position 1,198) in SEQ ID NO: 7 were C, T and A in SEQ ID NO: 6, respectively. Only the mutation at position 740 was included in the protein-encoding region, but this mutation did not cause amino acid exchange because both bases A and T were the third base of threonine codons. Although the cause of these base substitutions was not clear at that time; analysis of the plasmid clone pHTF1 confirmed that human TPO protein comprised 353 amino acid residues having a 21 amino acid signal sequence. Molecular weight of the mature protein excluding the signal sequence was estimated to be 35,466.

The vector pHTF1 carried by an *E. coli* strain DH5 has been deposited by the present inventors on March 24, 1994 under the deposit No. FERM BP-4617, in National Institute of Bioscience and Human-Technology, Agency of Industrial Science and Technology, Ministry of International Trade and Industry, Japan.

⟨Example 23⟩

Expression of human TPO cDNA clone pHTF1 in COS 1 cells and confirmation of TPO activity

Transfection to COS 1 cells of the thus obtained plasmid clone pHTF1 was carried out according to the procedure of Example 11. That is, transfection was performed with 10 µg of the plasmid DNA by the DEAE-dextran method which includes chloroquine treatment. The transfected COS1 cells were cultured for 3 days at 37 °C, and the culture supernatant was collected.

The culture supernatant was extensively dialyzed against the IMDM culture medium and evaluated by the rat CFU-MK assay system. TPO activity was detected in a dose-dependent fashion in the supernatant of COS1 cells in which the plasmid pHTF1 was expressed (Fig. 10a). By contrast, TPO activity was not found in the culture supernatant of COS1 cells in which the plasmid pEF18S alone was expressed (Fig. 10a).

Similar results were obtained in the M-07e assay system. The supernatant of COS1 cells, in which the plasmid pHTF1 was expressed, significantly augmented M-07e cell proliferation in a dose-dependent manner (Fig. 10b). These results demonstrated that pHTF1 contains a gene which encodes a protein having TPO activity.

⟨Example 24⟩

Cloning of human TPO chromosomal DNA

Cloning of human TPO chromosomal DNA was carried out using human TPO cDNA as a probe. A genomic library used in the cloning was a gift from Prof. T. Yamamoto at the Gene Research Center, Tohoku University (the library reported by Sakai *et al.* in *J. Biol. Chem.*, 269, 2173 - 2182, 1994, constructed by partially digesting human chromosomal DNA with a restriction enzyme *Sau*3AI and ligating the partial digest to the *Bam*HI site of a phage vector Lambda EMBL3 manufactured by Stratagene). Screening from this library using human TPO cDNA as a probe was carried out basically according to the procedure described in *Molecular Cloning* (Sambrook *et al.*, Cold Spring Harbor Laboratory Press, 1989). Using *E. coli* LE392 as a host, the library was inoculated onto a 15-cm plate containing NZYM (10g of NZ amine, 5 g of NaCl, 5 g of Bacto Yeast Extract, 2 g of $MgSO_4 \cdot 7H_2O$ and 15 g of agar in 1 liter water, pH 7.0) in such an inoculum size that one plate contained 30,000 phage particles. Two replica filters were prepared from each of the thus prepared 18 plates using BIODYNE™ A TRANSFER MEMBRANE (manufactured by PALL). Denaturation of the filters was performed by soaking them in 0.5 N NaOH/1.5 M NaCl for 10 minutes and then in 0.5 M Tris-HCl (pH 8.0)/1.5 M NaCl for 10 minutes, followed by 30 minutes of air-drying and subsequent 1.5 hours of baking at 80°C in a vacuum oven. Prehybridization was carried out by incubating the thus treated filters at 42°C for 1 hour in 500 ml of a reaction solution consisting of 50% formamide, 5 x SSC, 5 x Denhardt's solution, 1% SDS and 20 μg/ml of salmon sperm DNA. For use as a probe, a human TPO cDNA fragment (base position numbers 178 to 1,025 in SEQ ID NO: 7) was amplified by PCR and purified, and the purified fragment was labeled with $^{32}P$ making use of Random Primer DNA Labelling Kit (a DNA labeling kit manufactured by Takara Shuzo based on the random primer method disclosed in *Anal. Biochem.*, 132, 6 - 13, 1983). Sequences of primers used in this PCR are as follows.

hTPO-I: 5'-TTG TGA CCT CCG AGT CCT CAG-3' (SEQ ID NO:50) (positions 178 to 198 in SEQ ID NO: 7) hTPO-N: 5'-AGG GAA GAG CGT ATA CTG TCC-3' (SEQ ID NO:51) (anti-sense primer corresponding to the sequence of positions 1,005 to 1,025 in SEQ ID NO: 7)

Using the isotope-labeled probe, hybridization was carried out at 42°C for 20 hours in 500 ml of a reaction solution having the same composition of the prehybridization solution. The resulting filters were washed 3 times in 2 x SSC/0.1% SDS solution at room temperature for 5 minutes, and then once in 0.1 x SSC/0.1% SDS solution at 68°C for 1 hour. The filters were then subjected to 16 hours of autoradiography at -70°C using an intensifying screen and an X-OMAT™ AR5 film (manufactured by Eastman Kodak). As the result, 13 positive signals were obtained. Plaques approximately corresponding to each of the positive signals were collected from the original plates and inoculated again onto 15-cm NZYM plates in such an inoculum size that 1,000 plaques were formed on each plate. Two replica filters were prepared from each of the resulting plates to carry out hybridization under the same conditions described above. As the result, positive signals were detected on all filters of the 13 groups. A single plaque was recovered from each of the resulting plates to prepare phage DNA by the plate lysate method described in *Molecular Cloning*. Phage DNA samples thus prepared from the 13 clones were checked for the presence of the cDNA coding region, by PCR using primers having the following sequences.

hTPO-L: 5'-GGC CAG CCA GAC ACC CCG GCC-3' (SEQ ID NO:52) (positions 1 to 21 in SEQ ID NO: 6) hTPO-F: 5'-ATG GGA GTC ACG AAG CAG TTT-3' (SEQ ID NO:53) (anti-sense primer corresponding to the sequence of positions 127 to 147 in SEQ ID NO: 6)

hTPO-P: 5'-TGC GTT TCC TGA TGC TTG TAG-3' (SEQ ID NO:54) (positions 503 to 523 in SEQ ID NO: 6) hTPO-V: 5'-AAC CTT ACC CTT CCT GAG ACA-3' (SEQ ID NO:55) (anti-sense primer corresponding to the sequence of positions 1,070 to 1,090 in SEQ ID NO: 6)

When PCR was carried out using these primer combinations, 5 of the 13 clones seemed to contain entire amino acid coding region predicted from the cDNA. Chromosomal DNA molecules contained in these 5 clones had a similar length of about 20 kb and showed almost the same pattern when a preliminary restriction enzyme analysis was performed. Therefore, one of these clones (clone λHGT1) was selected and analyzed by Southern blotting. That is, 1 μg of DNA of the clone λHGT1 was digested completely with a restriction enzyme *Eco*RI or *Hin*dIII and applyeded to 0.8% agarose gel electrophoresis and bands on the gel were transferred on BIODYNE™ A TRANSFER MEMBRANE (manufactured by PALL). The resulting filter was air-dried for 30 minutes and subjected to 2 hours of baking at 80°C in a vacuum oven. Prehybridization was performed by incubating the thus treated filter at 42°C for 1 hour in 50 ml of a reaction solution consisting of 50% formamide, 5 x SSC, 5 x Denhardt's solution, 1% SDS and 20 μg/ml of salmon sperm DNA. For use as a probe, a human TPO cDNA fragment (base position numbers 178 to 1,025 in SEQ ID NO: 7) was amplified by PCR and purified, and the purified fragment was labeled with $^{32}P$ using Random Primer DNA Labelling Kit (manufactured by Takara Shuzo). Using the isotope-labeled probe, hybridization was carried out at 42°C for 20 hours in 50 ml of a reaction solution having the same composition of the prehybridization solution. The resulting filter was washed 3 times in 2 x SSC/0.1% SDS solution at room temperature for 5 minutes, and then once in 0.1 x SSC/0.1% SDS solution at 68°C for 1 hour. The filter was then subjected to 16 hours of autoradiography at -70°C using an intensifying screen and an X-OMAT™ AR5 film (manufactured by Eastman Kodak). As the result, a single band of about 10 kb was observed in the case of the *Hin*dIII digestion. Therefore, 10 μg of DNA of the clone λHGT1 was digested with *Hin*dIII and subjected to 0.8% agarose gel electrophoresis, and the 10 kb band was excised from the gel, purified using Prep-A-Gene DNA Purification Kit (manufactured by Bio-Rad) and subcloned into a cloning vector pUC13 (manufactured by Pharmacia) which had been previously digested with *Hin*dIII. In this case, Competent-High *E. coli* DH5 manufactured by TOYOBO was used as the host strain.

Of the thus obtained clones, a clone containing the 10 kb *Hin*dIII fragment was selected and designeted pHGT1.

A restriction map of phage clone λHGT1 is shown in Fig.11.

⟨Example 25⟩

Determination of nucleotide sequence of human TPO chromosomal clone pHGT1

Culturing of the clone pHGT1 and purification of plasmid DNA were carried out basically according to the method described in *Molecular Cloning* (Sambrook *et al.*, Cold Spring Harbor Laboratory Press, 1989). The clone pHGT1 was cultured overnight in 50 ml of the LB medium containing 50 μg/ml of ampicillin. The resulting cells were collected by centrifugation and suspended in 4 ml of the aforementioned TEG-lysozyme solution. To this were added 8 ml of 0.2 N NaOH/1% SDS solution and then 6 ml of 3 M potassium/5 M acetate solution to suspend the cells thoroughly. After centrifugation of the suspension, the resulting supernatant was treated with phenol/chloroform (1:1), mixed with the same volume of isopropanol and then centrifuged. The resulting pellet was dissolved in the TE solution and treated with RNase and then with phenol/chloroform (1:1), followed by ethanol precipitation. The resulting pellet was again dissolved in the TE solution to which were subsequently added NaCl and polyethylene glycol 3,000 to their final concentrations of 0.63 M and 7.5%, respectively. After centrifugation, the pellet was dissolved in the TE solution and precipitated with ethanol. In this way, about 300 μg of the plasmid DNA pHGT1 was obtained.

Using the aforementioned Taq Dye Deoxy™ Terminater Cycle Sequening Kit (manufactured by Applied Biosystems), the thus purified plasmid DNA was applied to the aforementioned 373A DNA sequencer (manufactured by Applied Biosystems) to determine its nucleotide sequence around the protein-encoding region predicted from the cDNA nucleotide sequence. The thus determined nucleotide sequence and the deduced amino acid sequence are shown in the Sequence Listing (SEQ ID NO: 8). In this case, oligonucleotides used in Example 22 for the nucleotide sequence analysis of cDNA and synthetic oligonucleotides designed based on the internal sequence obtained by their sequence reaction analysis were used as primers in the nucleotide sequence determination.

As the result, chromosomal DNA carried by the plasmid clone pHGT1 was found to contain an entire coding region of the amino acid sequence deduced from SEQ ID NO: 6, and nucleotide sequence of the coding region coincided completely with that of SEQ ID NO: 6. In addition, a region corresponding to the amino acid-encoding exon contained 4 introns having lengths of 231 bp, 286 bp, 1,932 bp and 236 bp in that order counting from the 5' side (Fig. 11). Also, the nucleotide sequence was different from the cDNA nucleotide sequence of SEQ ID NO: 7 at 3 positions which were the same as the positions described in Example 22 (different positions between Sequence ID Nos. 6 and 7). That is, A (position 84), A (position 740) and G (position 1,198) in SEQ ID NO: 7 was respectively C, T and A in SEQ ID NO: 8. Thus, it was revealed that the nucleotide sequence of the human TPO cDNA clone pHTF1 obtained in Example 21 is different from the nucleotide sequence of the human chromosomal DNA clone pHGT1 at 3 positions. Therefore, in order to analyze if these mutations in the cDNA clone pHTF1 sequence reflect the chromosomal DNA sequence, nucleotide sequences of other 4 clones among the 5 chromosomal DNA clones independently selected by the screening were determined. The sequence analysis was carried out by a direct nucleotide sequence determination method using a phage DNA sample prepared according to the plate lysate method described in *Molecular Cloning*. Sequencing of each clone was performed by applying it to the aforementioned 373A DNA sequencer (manufactured by Applied Biosystems), using the sequence primers used in Example 22, which have been synthesized based on such sequence portions that the aforementioned 3 mutation positions in the nucleotide sequence could be analyzed, and the aforementioned Taq Dye Deoxy™ Terminater Cycle Sequening Kit (manufactured by Applied Biosystems). It was found that nucleotide sequences of all 4 clones were identical to that of SEQ ID NO: 6. The positions 84 and 740 corresponding to SEQ ID NO: 7 were substituted by C and T, respectively, in these 4 clones. But the position 1,198 was G in 2 clones and A in other 2 clones. In other words, it was revealed that there are two types of nucleotide sequences inherent to the original chromosomal DNA. At present, it is not clear if such differences in the nucleotide sequence are derived from homologous chromosome or from plural genes. In addition, it is suggested that the differences in the nucleotide sequence may be due to a racial difference, because the poly $(A)^+$ RNA purchased from Clontech is a Caucasian origin while the chromosomal DNA is a Japanese origin.

The vector pHGT1 carried by an *E. coli* strain DH5 has been deposited by the present inventors on March 24, 1994 under the deposit No. FERM BP-4616, in National Institute of Bioscience and Human-Technology, Agency of Industrial Science and Technology, Ministry of International Trade and Industry, Japan.

〈Example 26〉

Expression of human TPO chromosomal DNA in COS 1 cells and confirmation of TPO activity

The plasmid clone pHGT1 obtained by the subcloning contained a total of 4 *Eco*RI recognition sequences, 3 in the insert moiety and 1 in the vector. The nucleotide sequence analysis revealed that the entire human TPO protein-encoding region was included in a DNA fragment of about 4.3 kbp which was interposed between the *Eco*RI recognition sequence closest to the 5' side in the insert and the *Eco*RI recognition sequence in the vector. Therefore, this fragment was ligated with an *Eco*RI-treated expression vector pEF18S, and obtained 4 human TPO expression plasmid pEFHGTE#1-4 (see Fig. 11). By preparing these plasmid DNA, an expression experiment was carried out. Purification of plasmid DNA was carried out basically according to the procedure described in *Molecular Cloning* (Sambrook *et al.*, Cold Spring Harbor Laboratory Press, 1989), thereby obtaining about 250 µg of the plasmid DNA.

Transfection to COS 1 cells of the thus obtained clones pEFHGTE#1-4 was carried out according to the procedure of Example 11. That is, transfection was performed with 10 µg of the plasmid DNA by the DEAE-dextran method which includes chloroquine treatment. The transfected COS1 cells were incubated for 3 days at 37°C and then the culture supernatants were collected.

The culture supernatants were extensively dialyzed against the IMDM culture medium and evaluated by the rat CFU-MK assay system. TPO activity was detected in a dose-dependent fashion in the supernatant of COS1 cells in which each of four clones (pEFHGTE#1-4) was expressed. By contrast, TPO activiy was not found in the supernatant of COS1 cells in which the plasmid pEF18S alone was expressed. Representative data with pEFHGTE#1 is shown in Fig. 12a. Similar results were obtained in the M-07e assay system. The supernatant of COS1 cells, in which each of four clones (pEFHGTE#1-4) was expressed, significantly augmented M-07e cell growth in a dose-dependent fashion. Representative data with pEFHGTE#1 is shown in Fig. 12b.

These results demonstrated that the plasmid clones pEFHGTE#1-4 carry the functional chromosomal DNA of human TPO.

〈Example 27〉

Preparation of human TPO deletion type DNA and its expression in COS 1 cells and confirmation of TPO activity

The results in Example 18 indicated that human TPO could exhibit its biological activity even after the removal of its carboxyl third. Thus, in order to further evaluate the biologically active portions, deletion derivative experiments were performed. In this Example, TPO deletion derivatives lacking 20, 40, 60 or 68 amino acids from the carboxyl-terminal end of the TPO protein (amino acids 1-231) encoded by pHT1-231 were examined for their ability to exert TPO *in vitro* biological activity. The shortest derivative (amino acids 1-163) still included amino acid sequences corresponding to TP2/3 of rat plasma TPO described in Example 2. The deletion plasmids were prepared by PCR using the DNA of the plasmid clone pHT1-231 obtained in Example 18 as template and synthesized oligonucleotides as primers. Sequences of primers used for PCR were as follows:

hTPO-5: 5'-TTT GAA TTC GGC CAG CCA GAC ACC CCG GCC-3' (SEQ ID NO:56) (prepared by adding an *Eco*RI recognition sequence to the sequence of positions 1 to 21 in SEQ ID NO:4; this is the identical sequence described in Fig.9);

hTPO-S: 5'-TTT GCG GCC GCT CAT TAG CTG GGG ACA GCT GTG GTG GGT-3' (SEQ ID NO:57) (an antisense primer corresponding to the sequence of positions 555 to 576 in SEQ ID NO:4, prepared by adding two termination codons TAA and TGA and a *Not*I recognition sequence for use in the preparation of a deletion derivative coding for positions 1 to 163 amino acid residues);

hTPO-4: 5'-TTT GCG GCC GCT CAT TAC AGT GTG AGG ACT AGA GAG GTT CTG-3' (SEQ ID NO:58) (an antisense primer corresponding to the sequence of positions 576 to 600 in SEQ ID NO:4, prepared by adding two termination codons TAA and TGA and a *Not*I recognition sequence for use in the preparation of a deletion derivative coding for positions 1 to 171 amino acid residues);

hTPO-30: 5'-TTT GCG GCC GCT CAT TAT CTG GCT GAG GCA GTG AAG TTT GTC-3' (SEQ ID NO:59) (an antisense primer corresponding to the sequence of positions 636 to 660 in SEQ ID NO:4, prepared by adding two termination codons TAA and TGA and a *Not*I recognition sequence for use in the preparation of a deletion derivative coding for positions 1 to 191 amino acid residues); and hTPO-2: 5'-TTT GCG GCC GCT CAT TAC AGA CCA GGA ATC TTG GCT CTG AAT-3' (SEQ ID NO:60) (an antisense primer corresponding to the sequence of positions 696 to 720 in SEQ ID NO:4, prepared by adding two termination codons TAA and TGA and a *Not*I recognition sequence for use in the preparation of a deletion derivative coding for positions 1 to 211 amino acid residues).

Using 1 µg of plasmid DNA of the clone pHT1-231 obtained in Example 18 as a template, 10 µM each of the thus synthesized hTPO-5 (for 5' side) and hTPO-2, -3, -4 and -S (for 3' side) as primers and GeneAmp™ PCR Reagent Kit with AmpliTaq™ DNA Polymerase (manufactured by Takara Shuzo), PCR was carried out with a volume of 100 µl, using GeneAmp™ PCR System 9600 (manufactured by PERKIN-ELMER) and repeating a total of 20 cycles, each cycle consisting of denaturation at 95°C for 1 minute, annealing at 65°C for 1 minute and synthesis at 72°C for 1 minute, followed by the final incubation at 72°C for 7 minutes. A band of interest thus obtained by each PCR was digested with restriction enzymes *Eco*RI and *Not*I and the resulting digest was subjected to 1% agarose gel (manufactured by FMC BioProducts) electrophoresis to isolate a main DNA fragment having an expected size amplified by each PCR. The thus isolated DNA fragment was purified using Prep-A-Gene DNA Purification Kit (manufactured by Bio-Rad) and then sub-cloned into the expression vector pEF18S which had been previously treated with the same restriction enzymes. In this case, Competent-High *E. coli* DH5 manufactured by TOYOBO was used as the host strain. From the resulting transformants which originated from each PCR, 4 to 5 clones containing the insert of expected size were selected to prepare plasmid DNA samples basically according to the procedure described in *Molecular Cloning* (Sambrook *et al.*, Cold Spring Harbor Laboratory Press, 1989). In a series of such operations, plasmid DNA samples were obtained from a deletion derivative coding for positions 1 to 163 amino acid residues (pHT1-163 #1-5), a deletion derivative coding for positions 1 to 171 amino acid residues (pHT1-171 #1-4), a deletion derivative coding for positions 1 to 191 amino acid residues (pHT1-191 #1-4) and a deletion derivative coding for positions 1 to 211 amino acid residues (pHT1-211 #1-4). The whole length of the amplified regions of each plasmid DNA was subjected to nucleotide sequence analysis to find its complete identity with the nucleotide sequence shown in SEQ ID NO:4.

Transfection to COS 1 cells of the thus obtained clones was carried out according to the procedure of Example 11. That is, transfection was performed with 10 µg of each of the plasmid DNA samples by the DEAE-dextran method which includes chloroquine treatment. The transfected COS1 cells were cultured for 3 days and then the culture supernatants were recovered.

The culture supernatants were extensively dialyzed against the IMDM culture medium and evaluated by the rat CFU-MK assay system. TPO activity was detected in a dose-dependent fashion in the supernatant of COS1 cells in which pHT1-211, pHT1-191, pHT1-171 or pHT1-163 each was expressed. Representative data with pHT1-211#1, pHT1-191#1 and pHT1-171#2 is shown in Fig. 13a and with pHT1-163#2 in Fig. 13b. Similar results were obtained in the M-07e assay system. The supernatant of COS1 cells, in which pHT1-211, pHT1-191, pHT1-171 or pHT1-163 each was expressed, significantly augmented M-07e cell proliferation. Representative data with pHT1-211#1, pHT1-191#1, pHT1-171#2 or pHT1-163#2 is shown in Fig. 14.

These results showed that human TPO still retains its *in vitro* biological activity even after its carboxyl-terminal half beyond Ser (position 163) is deleted, and strongly suggested that the biologically active portions of human TPO localize within the amino-terminal half ending at Ser (position 163).

⟨Example 28⟩

Preparation of C-terminal deletion type human TPO and its expression and activity in COS 1 cells

In order to analyze the region necessary for the human TPO protein that exhibits its activity, the C-terminal amino acids were further deleted from the deletion derivatives obtained in Example 27 and the TPO activity expressed in COS 1 cells was examined. Using the plasmid clone pEF18S-HL34 obtained in Example 16, expression plasmids were prepared by deleting nucleotide sequences coding for C-terminal amino acid residues starting from the 163 position serine. Construction of these deletion plasmids was effected making use of PCR. Sequences of primers prepared for use in PCR are as follows:

hTPO-5: 5'-TTT GAA TTC GGC CAG CCA GAC ACC CCG GCC-3' (SEQ ID NO:61) (prepared by adding an *Eco*RI recognition sequence to the sequence of positions 1 to 21 in SEQ ID NO: 4; the same sequence shown in Example 18);

hTPO-150: 5'-TTT GCG GCC GCT CAT TAG AGG GTG GAC CCT CCT ACA AGC AT-3' (SEQ ID NO:62) (an antisense primer corresponding to the sequence of positions 514 to 537 in SEQ ID NO:4; prepared by adding two termination codons TAA and TGA and a *Not*I recognition sequence for use in the preparation of a deletion mutant coding for positions 1 to 150 amino acid residues);

hTPO-151: 5'-TTT GCG GCC GCT CAT TAG CAG AGG GTG GAC CCT CCT ACA A-3' (SEQ ID NO:63) (an antisense primer corresponding to the sequence of positions 518 to 540 in SEQ ID NO:4; prepared by adding two termination codons TAA and TGA and a *Not*I recognition sequence for use in the preparation of a deletion derivative coding for positions 1 to 151 amino acid residues);

hTPO-153: 5'-TTT GCG GCC GCT CAT TAC CTG ACG CAG AGG GTG GAC CC-3' (SEQ ID NO:64) (an antisense primer corresponding to the sequence of positions 526 to 546 in SEQ ID NO:4; prepared by adding two termination codons TAA and TGA and a *Not*I recognition sequence for use in the preparation of a deletion derivative coding for positions 1 to 153 amino acid residues);

hTPO-154: 5'-TTT GCG GCC GCT CAT TAC CGC CTG ACG CAG AGG GTG GA-3' (SEQ ID NO:65) (an antisense primer corresponding to the sequence of positions 529 to 549 in SEQ ID NO:4, prepared by adding two termination codons TAA and TGA and a *Not*I recognition sequence for use in the preparation of a deletion derivative coding for positions 1 to 154 amino acid residues);

hTPO-155: 5'-TTT GCG GCC GCT CAT TAG GCC CGC CTG ACG CAG AGG GT-3' (SEQ ID NO:66) (an antisense primer corresponding to the sequence of positions 532 to 552 in SEQ ID NO:4, prepared by adding two termination codons TAA and TGA and a *Not*I recognition sequence for use in the preparation of a deletion derivative coding for positions 1 to 155 amino acid residues);

hTPO-156: 5'-TTT GCG GCC GCT CAT TAT GGG GCC CGC CTG ACG CAG AG-3' (SEQ ID NO:67) (an antisense primer corresponding to the sequence of positions 535 to 555 in SEQ ID NO:4, prepared by adding two termination codons TAA and TGA and a *Not*I recognition sequence for use in the preparation of a deletion derivative coding for positions 1 to 156 amino acid residues); and

hTPO-157: 5'-TTT GCG GCC GCT CAT TAG GGT GGG GCC CGC CTG ACG CA-3' (SEQ ID NO:68) (an antisense primer corresponding to the sequence of positions 538 to 558 in SEQ ID NO:4, prepared by adding two termination codons TAA and TGA and a *Not*I recognition sequence for use in the preparation of a deletion derivative coding for positions 1 to 157 amino acid residues).

PCR was carried out using 1 μg of plasmid DNA of the clone pEF18S-HL34 obtained in Example 16 as a template and 10 μM each of the thus synthesized oligonucleotides (hTPO-5 for 5' side and hTPO-150, -151, -153, -154, -155, -156 and -157 for 3' side) as primers. Using GeneAmp™ PCR Reagent Kit with AmpliTaq™ DNA Polymerase (manufactured by Takara Shuzo), the PCR reaction was performed in 100 μl volume making use of GeneAmp™ PCR System 9600 (manufactured by PERKIN-ELMER) and repeating a total of 20 cycles, each cycle consisting of denaturation at 95°C for 1 minute, annealing at 66°C for 1 minute and synthesis at 72°C for 1 minute, followed by the final incubation at 72°C for 7 minutes. A band of interest thus obtained by each PCR was digested with restriction enzymes *Eco*RI and *Not*I and the resulting digest was subjected to 1% agarose gel (manufactured by FMC BioProducts) electrophoresis to isolate a main DNA fragment having a size expected by each PCR. The thus isolated DNA fragment was purified using Prep-A-Gene DNA Purification Kit (manufactured by Bio-Rad) and then sub-cloned into the expression vector pEF18S which has been treated in advance with the same restriction enzymes. In this case. Competent-High *E. coli* DH5 manufactured by TOYOBO was used as the host strain. From the resulting transformants which originated from each PCR experiment, 3 to 5 clones containing the insert of expected size were selected to prepare plasmid DNA samples basically in accordance with the procedure described in *Molecular Cloning* (Sambrook *et al.*, Cold Spring Harbor Laboratory Press, 1989). By a series of such operations, plasmid DNA samples were obtained from a deletion derivative coding for positions 1 to 150 amino acid residues (pHT1-150 # 21, 22 and 25), a deletion derivative coding for positions 1 to 151 amino acid residues (pHT1-151 # 16, 17 and 18), a deletion derivative coding for positions 1 to 153 amino acid residues (pHT1-153 # 1 to 5), a deletion derivative coding for positions 1 to 154 amino acid residues (pHT1-154 # 1 to 5), a deletion derivative coding for positions 1 to 155 amino acid residues (pHT1-155 # 1 to 5), a deletion derivative coding for positions 1 to 156 amino acid residues (pHT1-156 # 1 to 5) and a deletion derivative coding for positions 1 to 157 amino acid residues (pHT1-157 # 1 to 5).

Of these purified plasmid DNA samples, the sequence of pHT1-150 # 21, 22 and 25 and pHT1-151 # 16, 17 and 18 were checked by 373A DNA Sequencer manufactured by Applied Biosystems making use of Taq Dye Deoxy™ Terminater Cycle Sequening Kit (Applied Biosystems), thereby confirming expected TPO cDNA sequences with no substitutions over the entire nucleotide sequences.

Transfection of COS 1 cells with each of the thus obtained clones was carried out in accordance with the procedure of Example 11. That is, transfection was performed with 10 μg of each of the plasmid DNA samples by the DEAE-dextran method which includes chloroquine treatment, and the culture supernatant was recovered alter 3 days of the culture. The thus obtained culture supernatants were dialyzed against the IMDM culture medium described in the foregoing and evaluated by the M-07e assay system. TPO activities were detected in the culture supernatant of COS 1 cells transfected with respective clones coding for C-terminal deletion derivatives, consisting of 1 to 151 position amino acids, 1 to 153 position amino acids, 1 to 154 position amino acids, 1 to 155 position amino acids, 1 to 156 position amino acids or 1 to 157 position amino acids. All of these derivatives contain a cysteine residue at position 151. However, no TPO activities were detected in the culture supernatant of COS 1 cells transfected with the clone coding for a C-terminal side deletion derivative consisting of 1 to 150 position amino acids whose cysteine residue at the 151 position was also deleted.

⟨Example 29⟩

Preparation of N-terminal deletion type human TPO and its expression and activity in COS 1 cells

In order to analyze the region necessary for the TPO protein that exhibits its biological activity, the N-terminal side amino acids of the deletion derivatives obtained in Example 28 were further deleted and expression of TPO activity of the thus prepared deletion derivativess was examined. Using the plasmid clone pEF18S-HL34 obtained in Example 16 and the plasmid clone pHT1-163 obtained in Example 27, expression plasmids were prepared by deleting nucleotide sequences coding for N-terminal amino acid residues after the signal sequence. Construction of these deletion plasmids were effected making use of PCR. Sequences of primers prepared for use in PCR are as follows:

hTPO-5: 5'-TTT GAA TTC GGC CAG CCA GAC ACC CCG GCC-3' (SEQ ID NO:69) (prepared by adding an *Eco*RI recognition sequence to the sequence of positions 1 to 21 in SEQ ID NO:4; the same sequence shown in Example 18);

hTPO3: 5'-TTT GCG GCC GCT CAT TAT TCG TGT ATC CTG TTC AGG TAT CC-3' (SEQ ID NO:70) (an antisense primer corresponding to the sequence of positions 757 to 780 in SEQ ID NO:4; prepared by adding two termination codons TAA and TGA and a *Not*I recognition sequence; the same sequence synthesized for use in the preparation of a deletion derivative coding for positions 1 to 231 amino acid residues);

hTPO-S: 5'-TTT GCG GCC GCT CAT TAG CTG GGG ACA GCT GTG GTG GGT-3' (SEQ ID NO:71) (an antisense primer corresponding to the sequence of positions 555 to 576 in SEQ ID NO:4; prepared for use in the preparation of a deletion derivative coding for positions 1 to 163 amino acid residues);

hTPO-13: 5'-AGT AAA CTG CTT CGT GAC TCC CAT GTC CTT CAC AGC AGA CTG AGC CAG TG-3' (SEQ ID NO:72) (positions 124 to 173 in SEQ ID NO:4; prepared for use in the preparation of a derivative in which amino acid residues of positions 1 to 12 are deleted);

hTPO-13R: 5'-CAT GGG AGT CAC GAA GCA GTT TAC TGG ACA GCG TTA GCC TTG CAG TTA G-3' (SEQ ID NO:73) (an antisense primer corresponding to the sequence of positions 64 to 87 and 124 to 148 in SEQ ID NO: 4, prepared for use in the preparation of a derivative in which amino acid residues of positions 1 to 12 are deleted);

hTPO-7: 5'-TGT GAC CTC CGA GTC CTC AGT AAA CTG CTT CGT GAC TCC CAT GTC CTT C-3' (SEQ ID NO:74) (positions 106 to 154 in SEQ ID NO:4, prepared for use in the preparation of a derivative in which amino acid residues of positions 1 to 6 are deleted);

hTPO-7R: 5'-TTT ACT GAG GAC TCG GAG GTC ACA GGA CAG CGT TAG CCT TGC AGT TAG-3' (SEQ ID NO:75) (an antisense primer corresponding to the sequence of positions 64 to 87 and 106 to 129 in SEQ ID NO:4, prepared for use in the preparation of a derivative in which amino acid residues of positions 1 to 6 are deleted);

hTPO-8: 5'-GAC CTC CGA GTC CTC AGT AAA CTG CTT CGT GAC TCC CAT GTC CTT CAC A-3' (SEQ ID NO:76) (positions 109 to 157 in SEQ ID NO:4, prepared for use in the preparation of a derivative in which amino acid residues of positions 1 to 7 are deleted); and

hTPO-8R: 5'-CAG TTT ACT GAG GAC TCG GAG GTC GGA CAG CGT TAG CCT TGC AGT TAG-3' (SEQ ID NO:77) (an antisense primer corresponding to the sequence of positions 64 to 87 and 109 to 132 in SEQ ID NO:4, prepared for use in the preparation of a derivative in which amino acid residues of positions 1 to 7 are deleted).

(1) Preparation of a derivative (pHT13-231) in which amino acid residues of positions 1 to 12 are deleted

PCR was carried out using 1.4 µg of plasmid DNA of the clone pEF18S-HL34 obtained in Example 18 as a template and 5 µM each of the thus synthesized oligonucleotides (hTPO-13 and hTPO3 in one combination and hTPO-5 and hTPO-13R in another combination) as primers. Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 65°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. Thereafter, second PCR was carried out using 1 µl portion of each of the thus prepared solution as a template.

The second PCR was carried out using 5 µM each of the synthesized primers (hTPO-5 and hTPO3). Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 60°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. After digestion with restriction enzymes *Eco*RI and *Not*I, the resulting solution was subjected to extraction with the same volume of phenol/chloroform and subsequent ethanol precipitation. After centrifugation, the resulting precipitate was dissolved in 15 µl of TE buffer and then sub-cloned into the expression vector pEF18S which has been treated in advance with the same restriction enzymes. In this case, Competent-High *E. coli* DH5 manufactured by TOYOBO was used as the host strain. From the resulting transformants, 45 clones containing the insert of expected size were selected to prepare plasmid DNA samples basically in accordance with the procedure described in *Molecular Cloning* (Sambrook *et al.*, Cold Spring Harbor Laboratory Press, 1989). In this way, it was able to obtain plasmid DNA of a deletion derivative (pHT13-231) coding for a protein molecule in which amino acids residues of positions 1 to 12 have been deleted from the original amino acid residues of positions 1 to 231. When these 45 clones were subjected to PCR using primers hTPO-5 and hTPO3, inserts having approximately expected sizes were confirmed in 8 clones. Of these, the sequence of 3 clones were checked by 373A DNA Sequencer manufactured by Applied Biosystems making use of Taq Dye Deoxy™ Terminater Cycle Sequening Kit (Applied Biosystems), thereby obtaining a clone pHT13-231 #3 having a TPO cDNA sequence as designed and a deletion as expected with no substitutions and the like over the entire nucleotide sequence.

(2) Preparation of a derivative (pHT7-163) in which amino acid residues of positions 1 to 6 are deleted

In this case, the 163 position amino acid was used as the C-terminal for the preparation of deletion derivativess because, though the 231 position amino acid was designed as the C-terminal of TPO protein for the preparation of deletion derivativess in the above step (1), it was found that TPO activity could be expressed even when the C-terminal amino acids were further deleted. On the basis of the same reason, the 163 position amino acid was used as the C-terminal also in the subsequent step (3). PCR was carried out using 1.4 µg of plasmid DNA of the clone pHT1-163 obtained in Example 27 as a template and 5 µM each of the synthesized oligonucleotides (hTPO-7 and hTPO-S in one combination and hTPO-5 and hTPO-7R in another combination) as primers. Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 65°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. Thereafter, second PCR was carried out using 1 µl portion of each of the thus prepared solution as a template.

The second PCR was carried out using 5 µM each of the synthesized primers (hTPO-5 and hTPO-S). Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 60°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. After digestion with restriction enzymes *Eco*RI and *Not*I, the resulting solution was subjected to extraction with the same volume of phenol/chloroform and subsequent ethanol precipitation. After centrifugation, the resulting precipitate was dissolved in 15 µl of TE buffer and then sub-cloned into the expression vector pEF18S which has been treated in advance with the same restriction enzymes. In this case, Competent-High *E. coli* DH5 manufactured by TOYOBO was used as the host strain. From the resulting transformants, 30 clones containing the insert of expected size were selected to prepare plasmid DNA samples basically in accordance with the procedure described in *Molecular Cloning* (Sambrook *et al.*, Cold Spring Harbor Laboratory Press, 1989). In this way, it was able to obtain plasmid DNA of a deletion derivative (pHT7-163) coding for a protein molecule in which amino acids residues of positions 1 to 6 have been deleted from the original amino acid residues of positions 1 to 163 in SEQ ID NO: 4. When these clones were subjected to PCR using primers hTPO-5 and hTPO-S, inserts having approximately expected sizes were confirmed in all clones. Of these, the sequences of 3 clones were checked by 373A DNA Sequencer manufactured by Applied Biosystems making use of Taq Dye Deoxy™ Terminater Cycle Sequening Kit (Applied Biosystems), thereby obtaining 2 clones, pHT7-163 #4 and #29, each having a TPO cDNA sequence as designed and a deletion as expected with no substitutions and the like over the entire nucleotide sequence.

(3) Preparation of a derivative (pHT8-163) in which amino acid residues of positions 1 to 7 are deleted

A derivative (pHT8-163) having deletion of amino acid residues of positions 1 to 7 was prepared basically in the same manner as the above case for the preparation of a derivative (pHT7-163) having deletion of amino acid residues of positions 1 to 6. PCR was carried out using 1.4 µg of plasmid DNA of the clone pHT1-163 obtained in Example 27 as a template and 5 µM each of the synthesized oligonucleotides (hTPO-8 and hTPO-S in one combination and hTPO-5 and hTPO-8R in another combination) as primers. Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 65°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. Thereafter, second PCR was carried out using 1 µl portion of each of the thus prepared solutions as a template.

The second PCR was carried out using 5 µM each of the synthesized primers (hTPO-5 and hTPO-S). Using GeneAmp™ PCR Reagent Kit with AmpliTaq™ DNA Polymerase (manufactured by Takara Shuzo), the PCR reaction was performed in 100 µl volume making use of GeneAmp™ PCR System 9600 (manufactured by PERKIN-ELMER) and, after 5 minutes of denaturation at 95°C, repeating a total of 30 cycles, each cycle consisting of denaturation at 95°C for 1 minute, annealing at 60°C for 1 minute and synthesis at 72°C for 1 minute, followed by the final incubation at 72°C for 7 minutes. Each of the PCR products was subjected to 1.2% agarose gel (manufactured by FMC BioProducts) electrophoresis to isolate a main DNA fragment having an expected size which was subsequently purified using Prep-A-Gene DNA Purification Kit (manufactured by Bio-Rad) and dissolved in 15 µl of TE buffer. After digestion with restriction enzymes *Eco*RI and *Not*I, the resulting solution was subjected to extraction with the same volume of phenol/chloroform and subsequent ethanol precipitation. After centrifugation, the resulting precipitate was dissolved in 15 µl of TE buffer and then sub-cloned into the expression vector pEF18S which has been treated in advance with the same restriction enzymes. In this case, Competent-High *E. coli* DH5 manufactured by TOYOBO was used as the host strain. From the resulting transformants, 30 clones containing the insert of expected size were selected to prepare plasmid DNA samples basically in accordance with the procedure described in *Molecular Cloning* (Sambrook *et al.*, Cold Spring Harbor Laboratory Press, 1989). In this way, it was able to obtain plasmid DNA of a deletion derivative (pHT8-163) coding for a protein molecule in which amino acids residues of positions 1 to 7 have been deleted from the original amino acid residues of positions 1 to 163 in SEQ ID NO:4. When these clones were subjected to PCR using primers hTPO-5 and hTPO-S, inserts having approximately expected sizes were confirmed in all clones. Of these, the sequence of 3 clones were checked by 373A DNA Sequencer manufactured by Applied Biosystems making use of Taq Dye Deoxy™ Terminater Cycle Sequening Kit (Applied Biosystems), thereby obtaining 2 clones, pHT8-163 #33 and #48, each having a TPO cDNA sequence as designed and a deletion as expected with no substitutions and the like over the entire nucleotide sequence.

(4) Expression of deletion derivative in COS 1 cells and confirmation of TPO activity

Transfection of COS 1 cells with each of the thus obtained deletion clones was carried out according to the procedure of Example 11. That is, transfection was performed with 10 µg of each of the plasmid DNA samples by the DEAE-dextran method which includes chloroquine treatment, and the culture supernatants were recovered after 3 days of culture. The thus obtained culture supernatant was thoroughly dialyzed against the IMDM culture medium described in the foregoing and evaluated by the M-07e assay system.

As a result, weak, but significant, TPO activity was detected in the culture supernatant of COS 1 cells transfected with a clone coding for the deletion derivative consisting of 7 to 163 position amino acids. However, no TPO activity was detected in the culture supernatant of COS 1 cells transfected with a clone coding for the deletion derivative consisting of 8 to 163 position amino acids or of 13 to 231 position amino acids.

〈Example 30〉

Preparation of complete length of human TPO cDNA plasmid (pHTP1)

An expression vector having all amino acid coding regions of human TPO cDNA presumed in SEQ ID NO:6 was constructed for the expression in mammalian cells.

PCR was carried out in the following manner in order to prepare a DNA fragment which covers all human TPO cDNA coding regions.

Nucleotide sequences of the used primers are as follows:

hTPO-I: 5'-TTG TGA CCT CCG AGT CCT CAG-3' (SEQ ID NO:78) (positions 105 to 125 in SEQ ID NO:6);

SA: 5'-CAG GTA TCC GGG GAT TTG GTC-3' (SEQ ID NO:79) (an antisense primer corresponding to the sequence of positions 745 to 765 in SEQ ID NO:6);

hTPO-P: 5'-TGC GTT TCC TGA TGC TTG TAG-3' (SEQ ID NO:80) (positions 503 to 523 in SEQ ID NO:6); and

hTPO-KO: 5'-GAG AGA GCG GCC GCT TAC CCT TCC TGA GAC AGA TT-3' (SEQ ID NO:81) (a sequence prepared by adding a restriction enzyme *Not*I recognition sequence and a GAGAGA sequence (SEQ ID NO:82) to an antisense sequence corresponding to the sequence of positions 1066 to 1086 in SEQ ID NO:6).

A first PCR was carried out using 300 ng of the clone pEF18S-HL34 obtained in Example 16 as a template. Using 0.5 µM each of the primers hTPO-I and SA and 1 unit of Vent R™ DNA polymerase (manufactured by New England BioLabs), the PCR (repetition of a total of 30 cycles, each cycle consisting of incubations at 96°C for 1 minute, at 62°C for 1 minute and at 72°C for 1 minute, followed by the final incubation at 72°C for 7 minutes) was carried out. Composition of the reaction solution in final concentration is as follows: 10 mM KCl, 10 mM $(NH_4)_2SO_4$, 20 mM Tris-HCl (pH 8.8), 2 mM $MgSO_4$, 0.1% Triton X-100 and 200 µM dNTP mix.

One microgram portion of commercially available poly(A)$^+$ RNA preparation derived from human normal liver (manufactured by Clontech) was heated at 70°C for 10 minutes, rapidly cooled on ice and then mixed with 10 mM of DTT, 500 μM of dNTP mix, 25 ng of random primer (manufactured by Takara Shuzo), 10 units of RNase Inhibitor (manufactured by Boehringer-Mannheim) and 200 units of SuperScript™ II RNase $H^-$ (manufactured by LIFE TECHNOLOGIES), followed by 1 hour of incubation at 37°C to perform synthesis of cDNA. Thereafter, second PCR (repetition of a total of 30 cycles, each cycle consisting of incubations at 96°C for 1 minute, at 58°C for 1 minute and at 72°C for 1 minute) was carried out using 1/20 volume of the reaction solution of synthesized cDNA as a template and 2.5 μM each of the primers hTPO-P and hTPO-KO and 2.5 units of AmpliTaq™ DNA polymerase (manufactured by Takara Shuzo).

Each of the resulting solutions of the first and second PCR was subjected to 1 % agarose gel electrophoresis to isolate respective main DNA fragments of expected size which was subsequently purified using Prep-A-Gene DNA Purification Kit (manufactured by Bio-Rad). Thereafter, third PCR was carried out using 1/20 volume of each of the thus prepared solutions as a template. This reaction (heating at 96°C for 2 minutes, followed by the repetition of a total of 3 cycles, each cycle consisting of incubations at 96°C for 2 minute and at 72°C for 2 minute, and subsequent incubation at 72°C for 7 minutes) was carried out using 1 unit of Vent R™ DNA polymerase (manufactured by New England BioLabs). The resulting reaction solution was mixed with 1 μM each of hTPO-I and hTPO-KO, heated at 96°C for 2 minutes, subjected to 25 cycles of reaction, each cycle consisting of incubations at 96°C for 1 minute, at 62°C for 1 minute and at 72°C for 1 minute, and then incubated at 72°C for 7 minutes. The resulting reaction solution was extracted with the same volume of water saturated phenol-chloroform and then with the same volume of chloroform, and the extract was subjected to ethanol precipitation (2.5 volumes of ethanol in the presence of 0.3 M sodium acetate and 0.5 μl of glycogen manufactured by Boehringer-Mannheim) to recover DNA. The thus recovered DNA was digested with restriction enzymes *Bam*HI and *Not*I and subjected to 1% agarose gel electrophoresis, and a main band having an expected size thus isolated was purified using Prep-A-Gene DNA Purification Kit (manufactured by Bio-Rad), ligated with pBluescript II $SK^+$ vector (manufactured by Stratagene) which had been digested in advance with the restriction enzymes *Bam*HI and *Not*I and then transformed into Competent-High *E. coli* DH5 (manufactured by TOYOBO). From the resulting colonies, 4 clones were selected to prepare plasmid DNA samples. The sequence of purified plasmid DNA samples were checked by 373A DNA Sequencer manufactured by Applied Biosystems making use of Taq Dye Deoxy™ Terminater Cycle Sequening Kit (Applied Biosystems), thereby obtaining a clone, pBLTP, having a TPO cDNA sequence as designed with no substitution in nucleotide sequence within the region between *Bam*HI and *Not*I.

The clone pBLTP was digested with restriction enzymes *Eco*RI and *Bam*HI and subjected to 1% agarose gel electrophoresis, and a band of high molecular weight thus isolated was purified using Prep-A-Gene DNA Purification Kit (manufactured by Bio-Rad). In the same manner. pEF18S-HL34 was treated with the restriction enzymes to purify a DNA fragment of about 450 bp. Each of the thus obtained DNA samples was subjected to ligation and transformed into Competent-High *E. coli* DH5 (manufactured by TOYOBO). Plasmid DNA samples were prepared from the resulting colonies to obtain a clone, pBLTEN, containing human TPO cDNA insert. The thus obtained pBLTEN was digested with restriction enzymes *Eco*RI and *Not*I and subjected to 1% agarose gel electrophoresis, and a DNA fragment of about 1,200 bp thus isolated was purified using Prep-A-Gene DNA Purification Kit (manufactured by Bio-Rad), ligated with the expression vector pEF18S which has been digested in advance with the same restriction enzymes and then transformed into Competent-High *E. coli* DH5 (manufactured by TOYOBO). Plasmid DNA samples were prepared from the resulting colonies to obtain the clone of interest, pHTP1, which contains entire human TPO cDNA coding region. Plasmid DNA was prepared from this clone in a large quantity and used in the following experiments. Preparation of plasmid DNA was carried out basically in accordance with the procedure described in *Molecular Cloning* (Sambrook *et al.*, Cold Spring Harbor Laboratory Press, 1989).

〈Example 31〉

<u>Construction of mammalian expression plasmid, pDEF202-hTPO-P1, for the expression of human TPO in Chinese hamster ovary (CHO) cells.</u>

One microgram of a plasmid pMG1 containing mouse dihydrofolate reductase (DHFR) minigene was digested with restriction enzymes, EcoRI and BamHI, and the subjected to agarose gel electrophoresis to isolate a fragment (about 2.5 kb) containing mouse DHFR minigene. The isolated DNA fragment was dissolved in 25 ml of a reaction solution composed of 50 mM Tris-HCl (pH 7.5), 7 mM $MgCl_2$, 1 mM 2-mercaptoethanol and 0.2 mM dNTP mixture, mixed with 2 units of Klenow fragment and then incubated at room temperature for 30 miniutes to form blunt ends at both termini of the DNA fragment. After phenol/chloroform treatment and ethanol precipitation, the resulting fragment was dissolved in 10 ml of a TE solution (10 mM Tris-HCl (pH 8.0)/1 mM EDTA). The mammalian expression vector pEF18S was digested with a restriction enzyme SmaI, dephosphorylated with alkaline phosphatase (manufactured by Takara Shuzo) and then ligated with a DNA fragment containing mouse DHFR minigene by using T4 DNA ligase (manufactured by Takara Shuzo) to obtain an expression vector pDEF202.

Next, the constructed expression vector pDEF202 was digested with restriction enzymes, EcoRI and SpeI, and then subjected to agarose gele electrophoresis to isolate a larger DNA fragment. Human TPO cDNA which has been obtained by digesting the plasmid pHTP1 containing human TPO cDNA (clone p1) with restriction enzymes, EcoRI and SpeI, was ligated with this linearized pDEF202 vector by using T4 DNA ligase (manufactured by Takara Shuzo) to obtain a plasmid pDEF202-hTPO-P1 for expression of human TPO cDNA. This constructed plasmid contains SV40 replication origin, human elongation factor 1-α-promoter, and SV40 early polyadenylation signal for transcription of human TPO cDNA, mouse DHFR minigene, and pUC18-replication origin and β-lactamase gene ($Amp^r$).

⟨Example 32⟩

Expression of human TPO in CHO cells

CHO cells (a DHFR- strain; Urlaub and Chasin, Proc. Natl. Acad. Sci. USA, vol.77, p4216, 1980) were maintained in α-minimum essential medium (α-MEM(-), supplemented with hypoxanthine and thymidine) containing 10% fetal calf serum (FCS) by using a 6 cm diameter tissue culture plate (by Falcon). The transformation of CHO cells with a pDEF202-hTPO-P1 plasmid was carried out by a calcium phosphate method (CellPhect, manufactured by Pharmacia) as described below. Ten micrograms of the plasmid pDEF202-hTPO-P1 prepared in Example 31 was mixed with 120 ml of buffer A and 120 ml of H2O, and the resulting mixture was incubatedat room temperature for 10 minutes. This solution was further mixed with 120 ml of buffer B and allowed to stand at room temperature for additional 30 minutes. This DNA mixture solution was then added into the culture and incubated for 6 hours in a $CO_2$ incubator. After 6 hours, the culture was washed twice with serum-free α-MEM(-), treated with α-MEM(-) containing 10% dimethyl sulfoxide for 2 minutes, and then incubated for 2 days in non-selection medium (α-MEM(-) supplememted with hypoxanthine and thymidine) containing 10 % dialyzed FCS. After 2 days culture, the cells were selected in a selection medium (α-MEM(-)) containing 10% dialyzed FCS. For selection of transformed cells with DHFR-positive phenotype, the cells were treated with a trypsin/EDTA solution, and resuspended with a selection medium. The cells in 6 cm tissue culture plate were split into five 10 cm tissue culture plates or twelve 24-well tissue culture plates, and then cultured in the selection medium. The culture medium was exchanged at the intervals of two days. The human TPO activity in the cultured medium of CHO cells with DHFR-positive phenotype was measured by CFU-MK assay, M-O7e assay or Ba/F3 assay. The cells secreting human TPO into culture medium were further selected in the selection medium supplemented with 25 nM methotrexate in order to isolate the cell clone producing a higher amount of human TPO.

In this instance, transfection of CHO cells may also be effected by carrying out co-transfection of CHO cells with pHTP1 and pMG1 plasmids.

CHO strain (CHO-DUKXB11) transfected with the plasmid pDEF202-hTPO-P1 has been deposited by the present Inventors on January 31, 1995, under the deposit No. FERM BP-4988, in the National Institute of Bioscience and Human Technology, Agency of Industrial Science and Technology, Ministry of International Trade and Industry, Japan.

⟨Example 33⟩

Construction of a recombinant vector, BMCGSneo-hTPO-P1, for use in X 63.6.5.3. cells

One microgram of a mammalian expression vector pBMCGSneo was digested with restriction enzymes, *Xho*I and *Not*I, and the subjected to agarose gel electrophoresis to isolate the vector DNA portion. This DNA fragment and human TPO cDNA (P1 clone) which had been obtained by digesting the plasmid pBLTEN containing human TPO cDNA with the restriction enzymes *Xho*I and *Not*I were ligated using T4 DNA ligase to obtain the expression vector of interest, pBMCGSneo-hTPO-P1. This expression plasmid contains cytomegalovirus early promoter, parts of rabbit β-globin gene-derived intron and polyadenylated region for transcription of human TPO cDNA, human β-globin gene, 69% of bovine papilloma virus 1 gene, thymidine kinase promoter and polyadenyl region, phosphotransferase I gene (neomycin resistant gene) and pBR 322 replication origin and β-lactamase gene ($Amp^r$), and human TPO cDNA was ligated at a downstream site of the cytomegalovirus promoter.

⟨Example 34⟩

Expression in X 63.6.5.3.cells

X 63.6.5.3.cells were cultured in Dulbecco's minimal essential (DME) medium containing 10% fetal bovine serum. The transfection of X63.6.5.3 cells with BMCGSneo hTPO plasmid was carried out by electroporation method as described below.

20 micrograms of the plasmid BMCGSneo-hTPO-P1 prepared in Example 33 were added to 750 μl of the DME medium containing $10^7$ cells, and the mixture was put into an electroporation cuvette having a gap of 4 mm and allowed to stand for 10 minutes at 4°C. The cuvette was then set into an electroporation apparatus (BTX 600, manufactured by BTX) to carry out gene transfer under conditions of 380 V, 25 mF and 24 angstroms. After the gene transfer, the cuvette was again allowed to stand for 15 minutes at 4°C, and the cells were then washed once with DEM containing 10% fetal bovine serum. The transfected cells were suspended in 50 ml of DEM containing 10% fetal bovine serum, dispensed into wells of five 96 well tissue culture plate and then cultured for 2 days in a $CO_2$ incubator. After the 2 days culture, the culture medium was exchanged with DEM containing 10% fetal bovine serum and 1 mg/ml of G418 (manufactured by GIBSCO), and the medium exchange was carried out every 3 days thereafter. The human TPO activity in the culture medium of transformants was mesured by CFU-MK, M-07e or Ba/F3 assay. The transformants secreting human TPO into culture medium were cloned twice by a limiting dilution to establish a human TPO producing cell lines.

⟨Example 35⟩

Large scale expression of human TPO in COS 1 cells

Transfection of COS 1 cells was carried out according to the DEAE-dextran method which includes chloroquine treatment as described in Example 11 . COS 1 cells (ATCC CRL1650) were cultured in IMDM containing 10% (v/v) FCS using a collagen-coated 175 $cm^2$ culture flask at 37°C in a 5% carbon dioxide incubator until the cells became about 100% confluent. In order to effect collagen coating of the culture flask, 25 ml of a collagen solution (Cellmatrix type I-C, manufactured by Iwaki) whose concentration has been adjusted to 0.3 mg/ml with 1 mM HCl was added to each 175 $cm^2$ culture flask, the collagen solution was recovered after 1 hour of standing at room temperature and then the thus treated flask was washed once with 20 to 50 ml of PBS. Transfection was effected by mixing 20 ml of IMDM solution containing 250 μg/ml of DEAE-dextran (manufactured by Pharmacia), 60 μM of chloroquine (manufactured by Sigma) and 10% (v/v) of Nu-Serum (manufactured by Collaborative) with the plasmid pHTP1 (40 μg) which has been dissolved in 500 μl of HBS, adding the resulting mixture to the COS 1 cells described above contained in one 175 $cm^2$ culture flask, which have been washed once with IMDM just before the transfection, and then culturing the cells for 3 hours at 37°C in a 5% carbon dioxide incubator. Thereafter, the culture supernatant was removed by suction, and the cells were washed once with IMDM, mixed with 50 ml of a serum-free medium and then cultured for 5 days at 37°C in the 5% carbon dioxide incubator to recover the culture supernatant. In one operation, 100 to 260 culture flasks of 175 $cm^2$ were used and 5 to 13 liters of the culture supernatant was recovered. The serum-free medium was prepared by supplementing IMDM with 5 μg/ml of insulin (manufactured by Sigma), 5 μg/ml of transferrin (manufactured by Sigma), 10 μM of monoethanolamine (manufactured by Wako Pure Chemical Industries), 25 nM of sodium selenite (manufactured by Sigma) and 200 μg/ml of BSA (fatty acid-free high purity bovine albumin, manufactured by Nichirei).

⟨Example 36⟩

Purification of human full-length TPO (derived from expression plasmid pHTP1, P1 clone) expressed in COS 1 cells and its biological activity

The following describes examples of the activity and purification of human full-length TPO (derived from the expression plasmid pHTP1) expressed in COS 1 cells. A partially purified product was firstly prepared in order to examine platelet increasing activity and then the purification was carried out to obtain a high purity product. The M-07e assay system was mainly used for the measurement of TPO activity in the following preparation steps. TPO activity was similarly found in the rat CFU-MK assay system. In carrying out these assays, human serum albumin (HSA) was added to each sample to a final concentration of 0.02 to 0.05%.

Firstly, COS 1 cells transfected with the plasmid pHTP1 according to the method of Ohashi and Sudo (Hideya Ohashi and Tadashi Sudo, *Biosci. Biotech. Biochem.*, 58(4), 758 - 759, 1994) were cultured for 5 days at 37°C in a 5% $CO_2$ using a serum-free IMDM culture medium containing, 0.2 g of BSA, 5 mg of bovine insulin, 5 mg of human transferrin, 0.02 mM of monoethanolamine and 25 nM of sodium selenite, in 1000ml, thereby obtaining about 7 liters of serum-free culture supernatant. To the serum-free culture supernatant was added proteolytic enzyme inhibitors p-APMSF and Pefabloc SC (4-(2-aminoethyl)-benzenesulfonyl fluoride hydrochloride, manufactured by Merk, catalog No. 24839) to a final concentration of about 1 mM, followed by filtration using a 0.2 µm filter to recover the supernatant. This was concentrated by a factor of about 10 using an ultrafiltration unit (Omega Ultrasette, nominal molecular weight cutoff of 8,000, manufactured by Filtron; or PLGC Pellicon Cassette, nominal molecular weight cutoff of 10,000, manufactured by Millipore), 723 ml of the thus concentrate (protein concentration, 3.38 mg/ml; total protein, 2,445 mg; relative activity, 43,000; and total activity, 105,100,000), was mixed with 1.6 moles per 1,000 ml of ammonium sulfate (288 g in total) to obtain 804 ml of the solution containing 1.5 M in final concentration of ammonium sulfate, and then the thus obtained solution was applied at a flow rate of 10 ml/min to Macro-Prep Methyl HIC column (5 cm in diameter and 9 cm in bed height, manufactured by Bio-Rad, catalog No. 156-0080) which has been equilibrated in advance with 20 mM sodium citrate buffer (pH 5.2) containing 1.5 M ammonium sulfate. After the sample loading, elution was carried out with 20 mM sodium citrate buffer (pH 5.2) containing 1.25 M ammonium sulfate to obtain passed-through fraction F1 (2,384 ml; protein concentration, 0.864 mg/ml; total protein, 2,061 mg; and relative activity, 6,000).

Next, the eluting solution was changed to 20 mM Na citrate, pH 5.8, to collect fraction F2 (1,092 ml; protein concentration, 0.776 mg/ml; total protein, 847 mg; and relative activity, 150,000).

The thus obtained Macro-Prep Methyl HIC column fraction F2 (1,081 ml) was applied at a flow rate of 10 ml/min to SP Sepharose Fast Flow column (3 cm in diameter and 10 cm in bed height, manufactured by Pharmacia Biotech, catalog No. 17-0729-01) which has been equilibrated in advance with 20 mM sodium citrate buffer (pH 5.8). After the sample loading, elution was carried out with 20 mM sodium citrate buffer (pH 5.6) containing 110 mM NaCl to obtain fraction F1 (2,262 ml; protein concentration, 0.270 mg/ml; total protein, 610 mg; and relative activity, 30,000). Next, the eluting solution was changed to 20 mM sodium citrate buffer (pH 5.4) containing 400 mM NaCl to collect fraction F2 (856 ml; protein concentration, 0.189 mg/ml; total protein, 162 mg; and relative activity, 300,000). Next, the eluting solution was changed to 20 mM sodium citrate buffer (pH 5.2) containing 1,000 mM NaCl to collect eluted fraction F3 (370 ml; protein concentration, 0.034 mg/ml; total protein, 12.6 mg; and relative activity, 150,000).

The main TPO active fraction F2 (845 ml) of the SP Sepharose Fast Flow column step was mixed with about 10% in final concentration of 1-propanol and applied at a flow rate of 3 ml/min to LA-WGA column (2 cm in diameter and 14 cm in bed height, manufactured by Honen, catalog No. WG-007) which has been equilibrated in advance with 20 mM sodium citrate buffer (pH 5.4) containing 400 mM NaCl. After the sample loading, elution was carried out using a mixture of 20 mM sodium citrate buffer (pH 5.4) containing 400 mM NaCl with 1-propanol (9:1) to obtain fraction F1 (64.4 ml; protein concentration, 0.0178 mg/ml; total protein, 1.15 mg; and relative activity, 17,220). Next, the eluting solution was changed to 20 mM sodium citrate buffer (pH 6.1) containing 0.4 M GlcNAc and 10% 1-propanol to collect eluted fraction F2 (45 ml; protein concentration, 0.0104 mg/ml; total protein, 0.470 mg; and relative activity, 675,000).

The main TPO active fraction F2 (340 ml) of the LA-WGA column operation was mixed with about 0.005% in final concentration of TFA and subjected to YMC-Pack CN-AP (6 mm in diameter and 250 mm in bed height, manufactured by YMC, (catalog No. AP-513) column chromatography. That is, using 0.1% TFA as a developing solvent A and 1-propanol containing 0.05% TFA as a developing solvent B, the sample was applied at a flow rate of 0.6 ml/min to the YMC-Pack CN-AP column which has been equilibrated in advance with 15% B. After the sample loading, the propanol concentration was increased from 15% B to 25% B, and the elution was carried out with a linear gradient of from 25% B to 50% B for 65 minutes while collecting the eluates in 1.5 ml (2.5 min) portions in polypropylene tubes.

A 0.5 µl portion (1/3,000 fraction) of the eluate in each tube was mixed with HSA and concentrated by ultrafiltration to obtain 0.25 ml of IMDM assay culture solution containing 0.05% HSA for use in the identification of TPO active fractions by the assay. As the result, strong TPO activity (relative activity of about 630,000 to 4,800,000) was found in tube Nos. 24 to 30 (within the range of 35.0 and 42.0% as propanol concentration) which were pooled as TPO active fraction FA (13.5 ml).

The fraction FA obtained by the YMC-Pack CN-AP column step was subjected to Capcell Pak Cl 300A (4.6 mm in diameter and 150 mm in bed height, manufactured by Shiseido, catalog No. Cl TYPE:SG300A) column chromatography using 0.1 % TFA as a developing solvent A and 1-propanol containing 0.05% TFA as a developing solvent B. A portion (8.9 ml) of the fraction FA obtained by the YMC-Pack CN-AP column operation was mixed with 0.3 ml of glycerol, concentrated by centrifugation evaporation and then mixed with about 2.5 ml of 10% B, and the resulting solution was subsequently applied at a flow rate of 0.4 ml/min to the Capcell Pak Cl 300A column which has been equilibrated in advance with 20% B. After the sample loading, the elution was carried out firstly with 20% B for 5 minutes and then with a linear gradient of from 20% B to 40% B for 50 minutes while collecting the eluates in 1 ml (2.5 min) portions in polypropylene tubes.

A 1 µl portion (1/1,000 fraction) of the eluate in each tube was mixed with HSA and concentrated by ultrafiltration to obtain 0.25 ml of IMDM assay culture solution containing 0.05% HSA for use in the identification of TPO active fractions by the assay. As the result, strong TPO activity (relative activity of about 3,000,000 to 22,500,000) was found in tube Nos. 20 to 23 (within the range of 28.5 and 32.5% as propanol concentration) which were pooled as a high activity fraction.

〈Example 37〉

Molecular weight measurement of human TPO expressed in COS 1 cells

It was assumed that molecular weight of the human complete length TPO (derived from the expression plasmid pHTP1, F1 clone) expressed in COS 1 cells would be larger than the molecular weight presumable from the size of the peptide chain because of the addition of sugar chain. In consequence, as a first step, a partially purified TPO fraction was prepared in the following manner from a culture supernatant containing the human complete length TPO (derived from the expression plasmid pHTP1, F1 clone) expressed in COS 1 cells. That is, using a developing solvent A (0.1 % trifluoroacetic acid (TFA)) and a developing solvent B (1-propanol containing 0.05% TFA), a 0.3 ml portion of the culture supernatant was applied to YMC-Pack PROTEIN-RP (0.46 cm in diameter and 15 cm in bed height, manufactured by YMC, catalog No. A-PRRP-33-46-25) column which has been equilibrated in advance with 25% B, 25% B was passed through the column for 5 minutes at a flow rate of 0.4 ml/min and then fractionation was effected by a linear density gradient of from 25% B to 50% B spending 50 minutes. TPO activity was found in eluates within the range of 34.5% and 43.5% as propanol concentration, and these eluates were evaporated to dryness by centrifugation evaporation to obtain a partially purified sample.

Next, TPO activity was examined by the M-07e assay system after extracting protein from a gel of SDS-PAGE electrophoresis carried out under a non-reducing condition as described in Example 1, or molecular weight was measured based on reduction-treated DPCIII markers by means of a western analysis which will be described later in Example 45. As a result, TPO activity was found within a broad apparent molecular weight range of approximately from 69,000 to 94,000, thus confirming variation in the molecular weight. Also, apparent molecular weight of TPO based on reduction-treated DPCIII markers after its N-glycosidic linkage type sugar chain digestion with N-glycanase (manufactured by Genzyme, catalog No. 1472-00) was measured to be 36,000 to 40,000 which was larger than the molecular weight of about 35,000 presumable from the size of the peptide chain, thus strongly indicating the presence of an O-glycosidic linkage type sugar chain too.

In the same manner, apparent molecular weight of the human complete length TPO (derived from the expression plasmid pHTP1, P1 clone) expressed in COS 1 cells was found to be 63,000 to 83,000.

〈Example 38〉

Biological characteristics of human TPO

Effects of human TPO on human and rat hematopoietic cells were examined, mainly using culture supernatant from COS 1 cells transfected with pHTF1.

In the colony assay using $CD34^+DR^+$ cell fraction prepared from human cord blood, human TPO stimulated the formation of significant number of megakaryocyte colonies. For example, 11.5 megakaryocyte colonies were formed by 6,000 $CD34^+DR^+$ cells in the presence of 10% (v/v) of culture supernatant from COS1 cells transfected with pHT1-231. To examine the effect of human TPO on human megakaryocyte progenitor cells in peripheral blood, $CD34^+$ cells were prepared from human peripheral blood as follows. A leukocyte fraction obtained from human peripheral blood by a Ficoll-Paque density gradient was depleted of cells adherent to plastic dishes. The nonadherent cells, subsequently, were depleted of SBA (soybean agglutinin) positive cells by a panning using AIS Microselecter SBA(Asahi Medical). The resultant cells were incubated on AIS Microselecter CD 34, and the adsorbed cells. CD $34^+$ cells, were collected when $CD3^+$ cells were cultured for 10 days in the presence of culture supernatant from transfected COS 1 cells in a liquid medium, selective proliferation of large size GpIIb/IIIa+ cells was observed, and increase in the ploidy was found in these GpIIb/IIIa+ cells. These results strongly suggested that human TPO exerts its selective action on progenitor cells of human megakaryocyte lineage and stimulates their proliferation and differentiation.

In the colony assay using GpIIb/IIIa+ cells highly enriched for CFU-MK from rat bone marrow cells and plastic nonadherent cells obtained at the preceding step of GpIIb/IIIa+ cells, human TPO induced the formation of a significant number of megakaryocyte colonies. For example, 34.5 megakaryocyte colonies were formed by 1,000 GpIIb/IIIa+ cells and 28.5 megakaryocyte colonies were formed by 20,000 plastic nonadherent cells in the presence of 20% (v/v) of culture supernatant from transfected COS 1 cells. In addition, although the plastic nonadherent cell fraction contains various hematopoietic progenitor cells other than CFU-MK, only megakaryocyte colonies were formed in the presence of human TPO, strongly suggesting that human TPO exerts its selective action on progenitor cells of the megakaryocyte lineage. When the GpIIb/IIIa+ cells obtained from rat bone marrow was cultured for 3 to 5 days in the presence of 20% (v/v) of culture supernatant from transfected COS 1 cells in a liquid culture, increase in the ploidy was observed clearly on 5 days of the culture.

⟨Example 39⟩

Construction of vector for use in the expression of a fusion protein (to be referred to as "GST-TPO(1-174)" hereinafter) of glutathione-S-transferase(GST) and human TPO (amino acid residues 1 to 174) in *E. coli*

In order to facilitate expression of human TPO in *E. coli*, an artificial gene which encodes human TPO and contains an *E. coli* preference codon was prepared. The nucleotide sequence of this DNA contains an amino-terminal methionine codon (ATG) at the -1 position for use in the translation initiation in *E. coli*.

Synthetic oligonucleotides 1 to 12:

1: 5'-CTAGAAAAAACCAAGGAGGTAATAAATAATGAGCCC
GGCTCCGCCAGCTTGTGACCTTCGTGTTCTGTCT-3' (SEQ ID NO:83);
2: 5'-CAGTTTAGACAGAACACGAAGGTCACAAGCTGGCGG
AGCCGGGCTCATTATTTATTACCTCCTTGGTTTTTT-3' (SEQ ID NO:84);
3: 5'-AAACTGCTTCGCGACTCTCACGTGCTGCACTCTCG
TCTGTCCCAGTGCCCGGAAGTTCACCCGCTGCCG-3' (SEQ ID NO:85);
4: 5'-CGGGGTCGGCAGCGGGTGAACTTCCGGGCACTGGG
ACAGACGAGAGTGCAGCACGTGAGAGTCGCGAAG-3' (SEQ ID NO:86);
5: 5'-ACCCCGGTTCTGCTTCCGGCTGTCGACTTCTCCCT
GGGTGAATGGAAAACCCAGATGGAAGAGACCAAA-3' (SEQ ID NO:87);
6: 5'-CTGAGCTTTGGTCTCTTCCATCTGGGTTTTCCATT
CACCCAGGGAGAAGTCGACAGCCGGAAGCAGAAC-3' (SEQ ID NO:88);
7: 5'-GCTCAGGACATCCTGGGTGCAGTAACTCTGCTTCT
GGAAGGCGTTATGGCTGCACGTGGCCAGCTTGGC-3' (SEQ ID NO:89);
8: 5'-GGTCGGGCCAAGCTGGCCACGTGCAGCCATAACGC
CTTCCAGAAGCAGAGTTACTGCACCCAGGATGTC-3' (SEQ ID NO:90);

9: 5'-CCGACCTGCCTGTCTTCCCTGCTTGGCCAGCTGTC
TGGCCAGGTTCGTCTGCTGCTCGGCGCTCTGCAG-3' (SEQ ID NO:91);

10: 5'-CAGAGACTGCAGAGCGCCGAGCAGACGAACCTGGC
CAGACAGCTGGCCAAGCAGGGAAGACAGGCA-3' (SEQ ID NO:92);

11: 5'-TCTCTGCTTGGCACCCAGCTGCCGCCACAGGGCCG
TACCACTGCTCACAAGGATCCGAACGCTATCTTCC-3' (SEQ ID NO:93); and

12: 5'-AAGACAGGAAGATAGCGTTCGGATCCTTGTGAGCA
GTGGTACGGCCCTGTGGCGGCAGCTGGGTGCCAAG-3' (SEQ ID NO:94)

were prepared and each of the synthetic oligonucleotides 2 to 11 was phosphorylated with T4 kinase (manufactured by Pharmacia) in a solution composed of 1 mM ATP, 10 mM Tris-acetate, 10 mM Mg-acetate and 50 mM K-acetate. In order to make these synthetic oligonucleotides into 6 double-stranded DNA fragments, these synthetic oligonucleotides were made into combinations of 1 and 2, 3 and 4, 5 and 6, 7 and 8, 9 and 10 and 11 and 12, and each combination was subjected to annealing in a solution composed of 10 mM Tris/HCl (pH 7.5), 10 mM $MgCl_2$ and 50 mM NaCl. Next, 3 double-stranded DNA fragments of 1 and 2, 3 and 4, and 5 and 6 and the other 3 double-stranded DNA fragments of 7 and 8, 9 and 10 and 11 and 12 were respectively treated with T4 ligase (manufactured by Life Technology), and these 2 reaction solutions were again treated with T4 ligase in the same manner. The DNA fragment obtained by the ligation reaction was digested with *Bam*HI (manufactured by Boehringer-Mannheim) and subjected to 2% agarose gel electrophoresis to recover a fragment of about 390 to 400 bp in size which was subsequently purified using Prep-A-Gene DNA Purification Kit and sub-cloned into pUC18 which has been digested in advance with *Xba*I and *Bam*HI (*E. coli* DH5α was used as the host). From the clones thus obtained, a clone having the artificial gene which encodes human TPO and contains the following *E. coli* preference codons was selected by nucleotide sequence analysis and named pUC18(XB)(1-123). DNA sequence of the coding chain is shown in the Sequence Listing (SEQ ID NO:9).

```
        10  1       20        30        40        50        60
CTAGAAAAAA CCAAGGAGGT AATAAATAAT GAGCCCGGCT CCGCCAGCTT GTGACCTTCG
    TTTTTT GGTTCCTCCA TTATTTATTA CTCGGGCCGA GGCGGTCGAA CACTGGAAGC
XbaI          2
        70        80   3    90       100       110       120
TGTTCTGTCT AAACTGCTTC GCGACTCTCA CGTGCTGCAC TCTCGTCTGT CCCAGTGCCC
ACAAGACAGA TTTGACGAAG CGCTGAGAGT GCACGACGTG AGAGCAGACA GGGTCACGGG
                       4
       130       140       150    5  160       170       180
GGAAGTTCAC CCGCTGCCGA CCCCGGTTCT GCTTCCGGCT GTCGACTTCT CCCTGGGTGA
CCTTCAAGTG GGCGACGGCT GGGGCCAAGA CGAAGGCCGA CAGCTGAAGA GGGACCCACT
                                  6
       190       200       210       220    7   230       240
ATGGAAAACC CAGATGGAAG AGACCAAAGC TCAGGACATC CTGGGTGCAG TAACTCTGCT
TACCTTTTGG GTCTACCTTC TCTGGTTTCG AGTCCTGTAG GACCCACGTC ATTGAGACGA
                                             8
       250       260       270       280       290    9   300
TCTGGAAGGC GTTATGGCTG CACGTGGCCA GCTTGGCCCG ACCTGCCTGT CTTCCCTGCT
AGACCTTCCG CAATACCGAC GTGCACCGGT CGAACCGGGC TGGACGGACA GAAGGGACGA
                                                        10
       310       320       330       340       350       360
TGGCCAGCTG TCTGGCCAGG TTCGTCTGCT GCTCGGCGCT CTGCAGTCTC TGCTTGGCAC
ACCGGTCGAC AGACCGGTCC AAGCAGACGA CGAGCCGCGA GACGTCAGAG ACGAACCGTG

   11  370       380       390       400       410       420
CCAGCTGCCG CCACAGGGCC GTACCACTGC TCACAAGGAT CCGAACGCTA TCTTCC
GGTCGACGGC GGTGTCCCGG CATGGTGACG AGTGTTCCTA GGCTTGCGAT AGAAGGACAG AA
   12                                        BamHI
```

Single-stranded cDNA was synthesized from 1 μg of human normal liver-derived poly $(A)^+$ RNA using oligo dT primer, and PCR was carried out using 0.1 volume of the cDNA synthesis reaction solution as a template. The PCR reaction (incubation at 96°C for 2 minutes, repetition of a total of 30 cycles of reaction, each cycle consisting of incubations at 95°C for 1 minute, at 58°C for 1 minute and at 72°C for 1 minute, and final incubation at 72°C for 7 minutes) was carried out with a volume of 100 μl using hTPO-C and hTPO-Z(*Eco*RI) as primers. The human TPO cDNA fragment obtained in this way was digested with *Bam*HI and *Eco*RI and subjected to 2% agarose gel electrophoresis to recover a fragment of about 600 bp in size which was subsequently purified using Prep-A-Gene DNA Purification Kit and sub-cloned into pUC18 which has been digested in advance with *Eco*RI and *Bam*HI (*E. coli* DH5α was used as the host). A clone encoding correct human TPO nucleotide sequence was selected by nucleotide sequence analysis and named pUC18(BE)(124-332). Oligonucleotide sequences of the primers used in the PCR reaction are as follows:

hTPO-C: 5'-GGA GGA GAC CAA GGC ACA GGA-3' (SEQ ID NO:95) (positions 329 to 349 of pHTF1 clone); and

hTPO-Z(*Eco*RI): 5'-CCG GAA TTC TTA CCC TTC CTG AGA CAG ATT-3' (SEQ ID NO:96) (prepared by adding an *Eco*RI recognition sequence to an antisense primer corresponding to positions 1,143 to 1,163 of pHTF1 clone).

The clone pUC18(BE)(124-332) was digested with *Bam*HI and *Eco*RI and subjected to 2% agarose gel electrophoresis to recover a human TPO cDNA C-terminal side fragment of about 600 bp in size which was subsequently purified using Prep-A-Gene DNA Purification Kit and sub-cloned into pUC18(XB)(1-123) which has been digested in advance with *Eco*RI and *Bam*HI (*E. coli* DH5α was used as the host). A clone obtained in this manner was named pUC18(XE)(1-332).

Since the presence of human TPO activity in the human TPO peptide fragment of 1 to 163 position amino acid residues had been confirmed by an example in which various C-terminal deletion constructs of human TPO cDNA were prepared and expressed to measure *in vitro* human TPO activity, an expression vector containing this peptide fragment was constructed. In this case, expression of the DNA sequence which encodes GST-TPO(1-174) was carried out.

Since a nucleotide sequence corresponding to positions 681 to 686 (amino acid residues of positions 173 and 174) of the pHTF1 clone is recognized by a restriction enzyme *Sac*I, two terminal codons were introduced using 2 synthetic oligonucleotides making use of the recognition site of this restriction enzyme. That is, two synthetic oligonucleotides SSE1 and SSE2 were annealed in a solution composed of 10 mM Tris/HCl (pH 7.5), 10 mM $MgCl_2$ and 50 mM NaCl, and, making use of DNA Ligation Kit (manufactured by Takara Shuzo), the thus obtained double-stranded DNA fragment was introduced into pUC18(XE)(1-332) from which the human TPO cDNA C-terminal fragment of about 480 bp has been removed by its digestion with *Sac*I and *Eco*RI, thereby obtaining a clone pUC18(XS)(1-174) (*E. coli* DH5α was used as the host). Sequences of the synthetic oligonucleotides used herein are as follows:

```
SSE1:  CTAATGAG (SEQ ID NO:97);
SSE2:  AATTCTCATTAGAGCT (SEQ ID NO:98);

SacI      SSE1     EcoRI
    5'-CTAATGAG-3'  (SEQ ID NO:99)
3'-TCGAGATTACTCTTAA-5'  (SEQ ID NO:100)
          SSE2
```

The clone pUC18(XS)(1-174) obtained above was digested with *Xba*I and *Eco*RI and subjected to 2% agarose gel electrophoresis to recover a fragment of about 600 bp in size which encodes amino acid residues 1 to 174 of human TPO, and the fragment was subsequently purified using Prep-A-Gene DNA Purification Kit and sub-cloned into pBluescript IISK$^+$ (manufactured by Stratagene) which had been digested in advance with *Xba*I and *Eco*RI (*E. coli* DH5α was used as the host). A clone obtained in this way was named pBL(XS)(1-174). In the same manner, the clone pBL(XS)(1-174) was digested with *Xba*I and *Hin*dIII to recover a fragment of about 550 bp in size which encodes amino acid residues 1 to 174 of human TPO, and the fragment was subsequently purified using Prep-A-Gene DNA Purification Kit and sub-cloned into pCFM536 (EP-A-136490) which had been digested in advance with *Xba*I and *Hin*dIII (*E. coli* DH5α was used as the host). A clone obtained in this way was named pCFM536/hT(1-174).

The following experiment was carried out in order to effect expression of GST-TPO(1-174) making use of pGEX-2T (manufactured by Pharmacia) which is an expression vector of glutathione-S-transferase (GST) fusion protein. PCR reaction (incubation at 96°C for 2 minutes, repetition of a total of 22 cycles of reaction, each cycle consisting of incubations at 95°C for 1 minute, at 41°C for 1 minute and at 72°C for 1 minute, and final incubation at 72°C for 7 minutes) was carried out using pCFM536/hT(1-174) as a template and two PCR primers GEZ1 and GEX3. The human TPO encoding fragment obtained in this way was digested with *Nae*I and *Eco*RI and subjected to 2% agarose gel electrophoresis to recover a fragment of about 550 bp in size, the fragment was subsequently purified using Prep-A-Gene DNA Purification Kit and cloned into pGEX-2T which has been digested in advance with *Eco*RI and *Sma*I (*E. coli* DH5 was used as the host) and then a clone, named pGEX-2T/hT(1-174), which encodes correct human TPO nucleotide sequence was selected by nucleotide sequence analysis to prepare a transformant for use in the expression of GST-TPO-(1-174). Oligonucleotide sequences of the primers used in the PCR reaction are as follows:

GEX1: 5'-ATC GCC GGC TCC GCC AGC TTG TGA C-3' (SEQ ID NO:101) (prepared by adding a *Nae*I recognition sequence to the sequence of positions 21 to 39 in SEQ ID NO:10); and

GEX3: 5'-GCC GAA TTC TCA TTA GAG CTC GTT CAG TGT-3' (SEQ ID NO:102) (an antisense primer corresponding to the sequence of positions 523 to 549 in SEQ ID NO:10).

This expression plasmid contains a DNA sequence which encodes GST protein followed by a thrombin recognition peptide and a human TPO (amino acid residues 1 to 174). The DNA sequence which encodes thrombin recognition peptide and human TPO (amino acid residues 1 to 174) are shown in the Sequence Listing (SEQ ID NO:10).

⟨Example 40⟩

Expression of GST-TPO(1-174) in *E. coli*

The transformant prepared in Example 39 was cultured overnight at 37°C on a shaker using 60 ml of LB medium containing 50 μg/ml of ampicillin, and 25 ml portion of the resulting culture broth was added to 1,000 ml of LB medium containing 50 μg/ml of ampicillin and cultured at 37°C on the shaker until OD at $A_{600}$ reached 0.7 to 0.8. Thereafter, IPTG was added to the culture broth to a final concentration of 0.1 mM and the shaking culture was continued for additional 3 hours in order to induce expression of GST-TPO(1-174).

⟨Example 41⟩

Purification of GST-TPO(1-174) expressed in *E. coli.* and confirmation of its biological activity

A 5.9 g portion of frozen cells of the recombinant strain which produces GST-TPO(1-174) derived from human TPO nucleotide sequence-encoding clone pGEX-2T/hT(1-174) were suspended in 10 ml of water and disruptured using a high pressure disintegrator. By subjecting the suspension to centrifugation, GST-TPO(1-174) was recovered in the precipitated fraction and most parts of contaminated proteins, cell components and the like were removed. Next, the thus recovered precipitated fraction containing GST-TPO-(1-174) was suspended in 5 ml of water to which were subsequently added, with stirring, 6 ml of 1 M Tris buffer (pH 8.5), 120 ml of 10 M urea and 16 ml of water. After 5 minutes of stirring to solubilize the contents, the resulting solution was evenly divided into 4 portions to carry out the following steps (1) to (4).

(1) One of the divided samples was diluted by a factor of 10 with 20 mM Tris buffer having a pH value of 8.5. To this were added reduced type glutathione and oxidized type glutathione to respective final concentrations of 5 mM and 0.5 mM, followed by overnight incubation at 4°C. The thus prepared mixture was subjected to centrifugation to recover GST-TPO(1-174) as the supernatant fluid which was subsequently diluted by a factor of 2 with 20 mM sodium citrate buffer having a pH value of 5.5 and then adjusted to pH 5.5 with acetic acid. GST-TPO(1-174) in the solution was applied to SP Sepharose Fast Flow (manufactured by Pharmacia Biotech, catalog No. 17-0729-01) cation exchange column which has been equilibrated in advance with 20 mM sodium citrate buffer pH 5.5. After washing the column with 20 mM sodium citrate buffer pH 5.5, elution of GST-TPO(1-174) was effected using 20 mM sodium citrate buffer pH 5.5 containing 500 mM sodium chloride. A 129 ml portion of the eluate was mixed with 2.6 ml of 1 M Tris buffer pH 8.5, and the resulting mixture having a pH value of 8.1 was applied to Glutathione Sepharose 4B (manufactured by Pharmacia Biotech, catalog No. 17-0756-01) column to adsorb GST-TPO(1-174). After washing the column with PBS, elution of GST-TPO(1-174) was effected using 20 mM Tris buffer pH 8.5 containing 10 mM of reduced type glutathione. The resulting eluate was mixed with 37 NIH units of thrombin, allowed to stand for 4 hours at room temperature, diluted with PBS by a factor of 10 and then applied to Glutathione Sepharose 4B column to adsorb digested GST and to recover TPO(1-174) in the non-adsorbed fraction. The thus recovered non-adsorbed fraction was diluted by a factor of 3 with 20 mM sodium citrate buffer pH 5.5 and applied to SP Sepharose Fast Flow cation exchange column which has been equilibrated in advance with the same buffer, and the compound of interest was eluted with a linear density gradient of 0 to 500 mM sodium chloride dissolved in the same buffer.

(2) All operations of the step (1) were carried out in the presence of 0.1% polysorbate 80.

(3) One of the divided samples was diluted by a factor of 10 with 20 mM Tris buffer having a pH value of 8.5. To this were added reduced type glutathione and oxidized type glutathione to respective final concentrations of 5 mM and 0.5 mM, followed by overnight standing at 4°C. The thus prepared mixture was subjected to centrifugation to recover GST-TPO(1-174) as the supernatant fluid which was subsequently diluted by a factor of 2 with 20 mM sodium citrate buffer having a pH value of 5.5 and then adjusted to pH 5.5 with acetic acid. GST-TPO(1-174) in the solution was applied to SP Sepharose Fast Flow cation exchange resin which has been equilibrated in advance with 20 mM sodium citrate buffer pH 5.5. After washing the resin with 20 mM sodium citrate buffer pH 5.5, elution of GST-TPO(1-174) was effected using 20 mM sodium citrate buffer pH 5.5 containing 500 mM sodium chloride. The eluate was adjusted to pH 8 with 1 M Tris buffer pH 8.5, mixed with 320 NIH units of thrombin to remove GST polypeptide sequences via cleavage at a thrombin recognition peptide linker, allowed to stand for 4 hours at room temperature, diluted by a factor of 5 with 20 mM sodium citrate buffer pH 5.5 and then applied to SP Sepharose Fast Flow cation exchange column which has been equilibrated in advance with the same buffer, and the compound of interest was subsequently eluted with a linear density gradient of 0 to 500 mM sodium chloride sissolved in the same buffer.

(4) All operations of the step (3) were carried out in the presence of 0.1 % polysorbate 80.

Each of the fractions eluted at sodium chloride densities of about 200 to 400 mM by the SP Sepharose Fast Flow cation exchange column chromatography was thoroughly dialyzed against IMDM culture medium and then evaluated by the rat CFU-MK assay system. When the fraction was analyzed by SDS-PAGE in the presence of a reducing agent, a band corresponding to a molecular weight of 19 kd was detected (purity, 1 to 20%) as one of the main protein bands of the fraction. When N-terminal sequence analysis of this band was carried out by subjecting it to SDS-PAGE and PVDF membrane transfer in accordance with the procedure described in Example 1, it was confirmed that this band contains a TPO sequence to be expressed as the pGEX-2T/hT(1-174)-derived fusion protein. The deduced amino acid sequence of the resulting TPO was [$Gly^{-1}$]TPO given the sequence of the thrombin recognition peptide linker and the known activity of thrombin on the linker.

〈Example〉 42

Construction of vector for use in *E. coli* expression of human TPO (amino acid residues 1 to 163) having substitutions in the position 1 (Ser → Ala) and position 3 (Ala → Val)

The clone pUC18(XE)(1-332) prepared in Example 39 was digested with *Xba*I and *Eco*RI and subjected to 2% agarose gel electrophoresis to recover a fragment of about 1,000 bp in size which encodes amino acid residues 1 to 332 of human TPO, and the fragment was subsequently purified using Prep-A-Gene DNA Purification Kit and sub-cloned into pBluescript II $SK^+$ (manufactured by Stratagene) which has been digested in advance with *Xba*I and *Eco*RI (*E. coli* DH5α was used as the host). A clone obtained in this way was named pBL(XE)(1-332). Next, a region of the clone pBL(XE)(1-332) from its *Bam*HI recognition sequence to position 163 amino acid (positions 366 to 489) was changed to *E. coli* dominant codons. Synthetic oligonucleotides 13 to 20 shown below were prepared, and each pair of synthetic oligonucleotides 13 and 14, 15 and 16 and 17 and 18 were phosphorylated in the same tube containing a solution composed of 0.1 mM ATP, 10 mM Tris-acetate, 10 mM Mg-acetate and 50 mM K-acetate. After further addition of 1/10 volume of a solution composed of 100 mM Tris/HCl (pH 7.5), 100 mM $MgCl_2$ and 500 mM NaCl, the resulting solution was heated in a boiling water bath for 3 minutes and then allowed to cool to room temperature to obtain a double-stranded DNA fragment. Next, the thus obtained three pairs of double-stranded DNA fragments consisting of 13 and 14, 15 and 16 and 17 and 18 were ligated using DNA Ligation Kit (manufactured by Takara Shuzo), and PCR was carried out using the thus ligated product as a template and the synthetic oligonucleotides 19 and 20 as primers. The thus obtained PCR product was digested with *Bam*HI and *Hin*dIII and subjected to 2% agarose gel electrophoresis to recover a fragment of about 130 bp in size using Prep-A-Gene DNA Purification Kit. This was sub-cloned into pBL(XE)(1-332) which has been digested in advance with *Bam*HI and *Hin*dIII (*E. coli* DH5 was used as the host). A clone having the following nucleotide sequences was selected from the thus obtained clones by sequence analysis and named pBL(XH)(1-163):

13: 5'-GATCCGAACGCTATCTTCCTGTCTTTCCAGCACCTGCTGCGT-3' (SEQ ID NO:103);

14: 5'-TTTGCCACGCAGCAGGTGCTGGAAAGACAGGAAGATAGCGTTCG-3' (SEQ ID NO:104);

15: 5'-GGCAAAGTTCGTTTCCTGATGCTGGTTGGCGGTTCTACCCTG-3' (SEQ ID NO:105);

16: 5'-ACGCACAGGGTAGAACCGCCAACCAGCATCAGGAAACGAAC-3' (SEQ ID NO:106);

17: 5'-TGCGTTCGTCGGGCGCCGCCAACCACTGCTGTTCCGTCTTAATGAA-3' (SEQ ID NO:107);

18: 5'-AGCTTTCATTAAGACGGAACAGCAGTGGTTGGCGGCGCCCGACGA-3' (SEQ ID NO:108);

19: 5'-AAGGATCCGAACGCTATCTTCCTG-3' (SEQ ID NO:109); and

20: 5'-AGAAGCTTTCATTAAGACGGAACA-3' (SEQ ID NO:110).

For the purpose of increasing expression quantity of human TPO (positions 1 to 163) and preventing hydrolysis of N-terminal by protease, an expression vector was constructed for use in the expression of a mutation type human TPO (positions 1 to 163) (to be referred to as "h6T(1-163)" hereinafter) having substitutions in the position 1 (Ser to Ala) and position 3 (Ala to Val) of human TPO (positions 1 to 163) (-[$Ala^1$, $Val^3$]TPO(1-163)) and, at the same time, encoding Lys at the -1 position and Met at the -2 position (-[$Met^{-2}$,$Lys^{-1}$,$Ala^1$,$Val^3$]TPO(1-163)). Four synthetic oligonucleotides shown below were prepared, and synthetic oligonucleotides 2-9 and 3-3 were phosphorylated with T4 kinase (manufactured by Pharmacia) in a solution composed of 1 mM ATP, 10 mM Tris-acetate, 10 mM Mg-acetate and 50 mM K-acetate. In order to make these synthetic oligonucleotides into double-stranded DNA fragments, each pair of the single-stranded DNA fragments 1-9 and 2-9 and 3-3 and 4-3 were annealed in a solution composed of 10 mM Tris/HCl (pH 7.5), 10 mM $MgCl_2$ and 50 mM NaCl. Next, the thus obtained two pairs of double-stranded DNA fragments consisting of 1-9 and 2-9 and 3-3 and 4-3 were treated with DNA Ligation Kit (manufactured by Takara Shuzo). The DNA fragment thus obtained by the ligation reaction was sub-cloned into pBL(XH)(1-163) which has been digested in advance with *Xba*I and *Nru*I, and a clone correctly substituted by the following synthetic oligonucleotide was selected by sequence analysis (*E. coli* DH5α was used as the host) and named pBL(XH)h6T(1-163):

```
1-9:  5'-CTAGAAAAAACCAAGGAGGTAATAAATAATGAAAGCACCTGTACCA-3'
                                                   (SEQ ID NO:111);
2-9:  5'-CAGGTGGTACAGGTGCTTTCATTATTTATTACCTCCTTGGTTTTTT-3'
                                                   (SEQ ID NO:112);
3-3:  5'-CCTGCATGTGATTTACGGGTCCTGTCTAAACTGCTGCG-3'
                                                   (SEQ ID NO:113);
4-3:  5'-CGCAGCAGTTTAGACAGGACCCGTAAATCACATG-3'
                                                   (SEQ ID NO:114);

          10    1-9   20         30         40
CTAGAAAAAA CCAAGGAGGT AATAAATAAT GAAAGCACCT
    TTTTTT GGTTCCTCCA TTATTTATTA CTTTCGTGGA
XbaI            2-9

                50       60  3-3    70         80
                  GTACCACCTG CATGTGATTT ACGGGTCCTG TCTAAACTGC TGCG
                  CATGGTGGAC GTACACTAAA TGCCCAGGAC AGATTTGACG ACGC
                                4-3

                                                   (SEQ ID NO:115)
```

The clone pBL(XH)(1-163) was digested with *Xba*I and *Hin*dIII to recover a fragment of about 500 bp in size which encodes amino acid residues 1 to 163 of the mutation type human TPO, and the fragment was subsequently purified using Prep-A-Gene DNA Purification Kit and cloned into pCFM536 (EP-A-136490) which has been digested in advance with *Xba*I and *Hin*dIII (*E. coli* JM109 which has been transformed in advance with pMW1 (ATCC No. 39933) was used as the host). A clone obtained in this way was named pCFM536/h6T(1-163), and an *E. coli* strain having this expression vector was used as a transformant for use in the expression of the mutation type human TPO, namely h6T(1-163). This expression plasmid contains the DNA sequence shown in the Sequence Listing (SEQ ID NO:11).

⟨Example 43⟩

Expression of h6T(1-163) in *E. coli*

Expression of the expression plasmid pCFM536 is controlled by λPL promoter which by itself is under control of cI857 repressor gene. The transformant obtained in ⟨Example 42⟩ was cultured overnight at 30°C on a shaker using 60 ml of LB medium containing 50 μg/ml of ampicillin and 12.5 μg/ml of tetracycline, and 25 ml portion of the resulting culture broth was added to 1,000 ml of LB medium containing 50 μg/ml of ampicillin and cultured at 37°C on the shaker until OD at $A_{600}$ reached 1.0 to 1.2. Thereafter, about 330 ml of LB medium heated to 65°C was added to the culture broth to adjust the final medium temperature to 42°C, and the shaking culture was continued for 3 hours at 42°C to induce expression of h6T(1-163).

⟨Example 44⟩

Purification of h6T(1-163) expressed in *E. coli,* and confirmation of its biological activity

A 3.6 g portion of frozen cells of the h6T(1-163) producing recombinant strain were suspended in 10 ml of water and disruptured using a high pressure disintegrator. By subjecting the suspension to centrifugation, the precipitated fraction was recovered and most parts of contaminated proteins, cell components and the like were removed. The thus recovered precipitated fraction containing h6T(1-163) was suspended in 7 ml of water, and 3 ml of 1 M Tris buffer (pH 8.5) was added to the suspension with stirring, followed by further addition of urea (final concentration, 8 M), guanidine hydrochloride (final concentration, 6 M) and sodium N-lauroyl sarcosinate (final concentration, 2%). After 5 to 20 minutes of stirring at room temperature to solubilize the contents, the resulting solution was diluted 10 times with 20 mM Tris buffer having a pH value of 8.5 and subjected to protein refolding by overnight oncubation at 4°C. In this case, glutathione and copper sulfate were used as additives in addition to air oxidation. By centrifugation, h6T(1-163) was recovered in the supernatant fluid. When each of the recovered fractions was analyzed by SDS-PAGE in the presence of a reducing agent, a band corresponding to a molecular weight of 18 kd was detected (purity, 30 to 40%) as the main protein band of each fraction. When N-terminal sequence analysis of this band was carried out by subjecting it to SDS-PAGE and PVDF membrane transfer according to the procedure described in Example 1, it was confirmed that this band contains a TPO sequence to be expressed as the pCMF536/h6T(1-163)-derived mutation type TPO. When each fraction was thoroughly dialyzed against IMDM culture medium and evaluated by the rat CFU-MK assay system, TPO activity was found in all fractions in a dose-dependent fashion.

⟨Example 45⟩

Preparation of anti-TPO peptide antibody and detection of TPO by SDS-PAGE and western analysis

Success in preparing anti-TPO peptide antibody will render possible detection of TPO protein by immunological means. In consequence, three regions which appear to be relatively useful as antigens (see below) were selected from the confirmed TPO amino acid sequences, a quadruple-stranded multiple antigen peptide (MAP) type peptide was synthesized in accordance with the procedure of Tam (*Proc. Natl. Acad. Sci. USA*, 85, 5409 - 5413, 1988) using each of the selected regions, and then each of 2 rabbits was immunized 8 times with 100 $\mu$g of each of the thus synthesized peptides to obtain respective antiserum samples.

Amino acid sequences contained in the synthesized peptide antigens

(a) Rat TPO(9-28) (Peptide RT1 region)
PRLLNKLLRDSYLLHRRLSQ (SEQ ID NO:116)
(R at Position 24 is originally S in the amino acid residues determined from the gene.)
(b) Rat TPO(46-66) (Peptide RT2 region)
FSLGEWKTQTEQSKAQDILGA (SEQ ID NO:117)
(c) Rat TPO(163-180) (Peptide RT4 region)
SRTSQLLTLNKFPNRLLD (SEQ ID NO:118)
(LLD at Positions 178-180 is originally TSG in the amino acid residues determined from the gene.)

Next, of these antiserum samples, anti-RTI peptide and anti-RT2 peptide were firstly subjected to a protein A (PROSEP-A; manufactured by Bioprocessing Ltd., catalog No. 8427) column chromatography to prepare IgG fractions (2,344 mg and 1,920 mg, respectively), and 54 mg and 32 mg portions of the respective fractions were biotinylated by their coupling with active type biotin (NHS-LC-Biotin II; manufactured by PIERCE, catalog No. 21336). A recombinant TPO-containing sample was subjected to SDS-PAGE and then to electroblotting on PVDF or nitrocellulose membrane in the same manner as described in Example 1, and western analysis was carried out in the usual way using these biotinylated antibodies as first antibodies.

That is, the membrane after blotting was washed with a solution composed of 20 mM Tris-HCl and 0.5 M NaCl (pH 7.5) (TBS) for 5 minutes and twice with 0.1% Tween 20-containing TBS (TTBS) for 5 minutes each and then treated with a blocking agent (BlockAce; manufactured by Dainippon Pharmaceutical. catalog No. uk-B25) for 60 minutes. Next, the membrane was treated for 60 minutes with TIBS solution containing 10 $\mu$g/ml of biotinylated anti-TPO peptide antibody, 0.05% BSA and 10% Block-Ace and then washed twice with TTBS for 5 minutes each. Thereafter, the membrane was treated for 30 minutes with a solution prepared by diluting alkaline phosphatase-labeled avidin (manufactured by Leinco Technologies, catalog No. A108) by a factor of 5,000 with TTBS solution containing 10% BlockAce and washed twice with TTBS for 5 minutes each and with TBS for 5 minutes, and then color development was effected by the use of an alkaline phosphatase substrate (manufactured by Bio-Rad, catalog No. 170-6432). The western analysis described above was carried out at room temperature.

As the results, it was able to recognize not only various recombinant rat TPO proteins expressed in COS 1 cells but also various recombinant human TPO proteins expressed in COS 1 cells and *E. coli* cells (illustratively, plasmid pHTP1- or plasmid pHTF1-derived human TPO expressed in COS 1 cells and N-glycosidic linkage-cleaved product thereof, GST-TPO(1-174) expressed in *E. coli* and thrombin-digested product thereof and h6T(1-163) which is a mutation type TPO expressed in *E. coli*, which have been described in Examples). In addition, the results also have rendered possible analysis of these various types of recombinant human TPO.

In addition to the above, possibility for the preparation of anti-human TPO peptide antibodies was confirmed. Antibodies obtained by these techniques can be applied not only to western analysis but also to purification of TPO by antibody columns and every usually used antibody-aided immunological means.

Six regions in the human TPO amino acid sequence shown in SEQ ID NO:7, which were expected to have relatively suitable antigenicity, were selected (see Table 4). Tetrameric Multiple Antigen Peptide (MAP) type peptides, which correspond to the regions, respectively, were synthesized. Each peptide was immunized to two rabbits 8 times in an amount of 100 mg/time.

Separately from this, a monomeric peptide in which a cysteine residue had been bonded to the C-terminus of each peptide region shown in Table 4 was synthesized too. This peptide was then used as a test antigen to determine an antibody titer using enzyme immunoassay. As a result, the increase of the antibody titer was confirmed for the above prepared sera, so these sera were employed as antisera.

The antibodies were prepared by immunizing each antigenic peptide to two rabbits to produce antisera directed against the peptide. The resultant two anti-HT1 peptide antibodies are distinguishably referred to as anti-HT1-1 peptide antibody and anti-HT1-2 peptide antibody depending on the rabbit individuals.

An example of the purification of the anti-HT1-1 peptide antibodies will be illustrated below.

First, 30 mg of a monomeric peptide of HT1 to which has been bonded a cystein residue was coupled to 12 ml of Sulfo-Link coupling gel (Pierce, Catalogue No. 44895). Briefly, a peptide solution containing the antigen was coupled for 15 minutes to the gel equilibrated with a coupling buffer (50 mM Tris, 5 mM EDTA-Na pH 8.5) of 6 volumes relative to the gel volume. Then, after incubation for 30 minutes, the gel was washed with the coupling buffer of 3 volumes relative to the gel volume. The coupling buffer containing 0.05 M L-cystein-HCl was added to the gel in an amount of 1 ml/ml gel to block unreacted radicals over 15 minutes. After incubation for 30 minutes, the gel was washed with the coupling buffer of 8 volumes relative to the gel volume. All the coupling reactions were carried out at room temperature. Thus the peptide was covalently bonded to the gel with a coupling efficiency of 28.3%, and then an antigen column with 0.8 mg of the peptide/ml gel was prepared.

76.7 ml (protein content 3620 mg) of the antiserum containing the anti-HT1-1 peptide antibody which had been bled from one of the rabbits with a total amount of 78.4 ml, was applied to the antigen column pre-equilibrated with 50 mM phosphate buffer (pH 8) containing 150 mM NaCl and 0.05% sodium azid, and then washed with the same buffer to afford 105.9 ml of a flow-through fraction (protein content 3680 mg). Then the adsorbed fraction was eluted with 0.1 M citrate buffer (pH 3.0) and immediately neutralized with 21.1 ml of 0.1 M carbonate buffer (pH 9.9), followed by concentration by means of ultrafiltration (using Amicon YM 30 membran) to give the purified anti-HT1-1 peptide antibody in a solution of 50 mM phosphate buffer (pH 8) containing 150 mM NaCl and 0.05% $NaN_3$.

Similarly, the following antibodies were prepared: anti-HT1-2 peptide antibody (60.0 mg); anti-HT2-1 peptide antibody (18.8 mg); anti-HT2-2 peptide antibody (8.2 mg); and the like. Also, anti-HT3 to -HT6 peptide antibodies could be prepared in the similar manner.

Three mg of affinity-purified anti-HT1-1 peptide antibody, anti-HT1-2 peptide antibody, anti-HT2-1 peptide antibody or anti-HT2-2 peptide antibody was biotinylated by coupling to an activated biotin (Pierce, NHS-LC-Biotin II; Catalogue No. 21336).

All the above purified antibodies were found to recognize and detect human TPO by Western blotting (on nitrocellulose filter or PVDF) following SDS-PAGE of a recombinant human TPO standard partially purified from the culture supernatant of CHO cells wherein a gene encoding the amino acid sequence shown in Table 4 had been introduced and expressed.

Table 4

Human TPO amino acid sequences included in the tetrameric MAP type synthetic peptide antigen

Human TPO (amino acid numbers 8-28) peptide HT1 region:
DLRVLSKLLRDSHVLHSRLSQ (SEQ ID NO:119)

Human TPO (amino acid numbers 47-62) peptide HT2 region:
SLGEWKTQMEETKAQD (SEQ ID NO:120)

Human TPO (amino acid numbers 108-126) peptide HT3 region:
LGTQLPPQGRTTAHKDPNA (SEQ ID NO:121)

Human TPO (amino acid numbers 172-190) peptide HT4 region:
NELPNRTSGLLETNFTASA (SEQ ID NO:122)

Human TPO (amino acid numbers 262-284) peptide HT5 region:
SLPPNLQPGYSPSPTHPPTGQYT (SEQ ID NO:123)

Human TPO (amino acid numbers 306-332) peptide HT6 region:
PSAPTPTPTSPLLNTSYTHSQNLSQEG (SEQ ID NO:124)

⟨Example 46⟩

Preparation of anti-TPO peptide antibody column

Since the anti-rat TPO peptide antibody obtained in Example 45 was able to recognize rat and human TPO molecules, immunoglobulin (IgG) fractions of the anti-RT1 peptide antibody and anti-RT2 peptide antibody were linked to a chemically activated gel support in the following manner to prepare anti-TPO peptide antibody columns. Each of the two antibodies used as the materials was prepared from sera of two rabbits separately. That is, the anti-RT1 peptide antibody prepared from two rabbits was respectively named anti-RT1-1 peptide antibody and anti-RT1-2 peptide antibody, and the anti-RT2 peptide antibody prepared from other two rabbits was respectively named anti-RT2-1 peptide antibody and anti-RT2-2 peptide antibody. Since these antibodies were separately used for the antibody column preparation, a total of 5 columns were obtained, namely two anti-RT1 peptide antibody columns (to be referred to as "anti-RT1-1 antibody column" and "anti-RT1-2 antibody column" hereinafter), two anti-RT2 peptide antibody columns (to be referred to as "anti-RT2-1 antibody column" and "anti-RT2-2 antibody column" hereinafter) and one antibody column (anti-RT1-2 + 2-1 mix antibody column) which was prepared by mixing anti-RT1-2 peptide antibody with anti-RT2-1 peptide antibody and coupling the mixture with a gel.

Each antibody was dissolved in a solution of 50 mM Na phosphate and 0.15 M NaCl (pH 8.0) to a final concentration of 5 mg/ml (or 2.5 mg/ml each of evenly mixed anti-RT1-2 peptide antibody and anti-RT2-1 peptide antibody in the case of the anti-RT1 + 2 mix antibody column), and a 2.31 ml portion of the resulting solution was mixed with 1.54 ml in volume of a swelled formyl-activated gel (Formyl-Cellulofine, manufactured by Chisso) and subjected to 2 hours of coupling reaction at 4°C. To this was added 1.1 ml of a 10 mg/ml solution of a reducing agent (trimethylamine borane (TMAB); manufactured by Seikagaku Kogyo, catalog No. 680246), followed by 6 hours of additional coupling reaction and subsequent centrifugation to recover the gel portion. A 10 ml portion of purified water was added to the gel portion which was recovered again by centrifugation, and unreacted antibody molecules were removed by repeating this step 4 times. Next, the resulting gel was mixed with 4.6 ml of a blocking solution (0.2 M Na phosphate and 1 M ethanol amine, pH 7.0) and 1.1 ml of the reducing agent solution and treated at 4°C for at least 2 hours to perform blocking of active groups of unreacted gel. Thereafter, the gel was washed with purified water and DPBS using a centrifuge, packed in a small column tube, washed with a 3 M potassium thiocyanate solution and a 0.1 M glycine-HCl (pH 2.5) solution, equilibrated with DPBS and then stored.

In the 5 anti-TPO peptide antibody gels of the anti-RT1-1 antibody column, anti-RT1-2 antibody column, anti-RT2-1 antibody column, anti-RT2-2 antibody column and anti-RT1-2 + 2-1 mix antibody column, the coupling efficiency of respective IgG fractions reached 97.4 %, 95.4%, 98.4%, 98.3% and 99.4%. In addition, amounts of IgG fractions coupled per gel volume were 5.6 mg/ml gel, 5.8 mg/ml gel, 5.7 mg/ml gel, 5.7 mg/ml gel and 2.9 mg/ml gel, respectively. In consequence, since it was confirmed that the coupling efficiency and coupling quantity do not significantly vary depending on the antibody origins and difference in the antigens, the experiments shown in the following Example 47 were carried out making use of these antibody columns.

〈Example 47〉

<u>Purification of TPO derived from culture supernatant obtained by transfecting COS 1 cells with expression vector pHTP1, using anti-TPO antibody column and then by reverse phase column chromatography, and confirmation of its biological activity</u>

The M-07e assay system was used as the *in vitro* assay for the evaluation of the following purification samples. Partially purified samples of TPO derived from the culture supernatant obtained in Example 35 by transfected COS 1 cells with the expression vector pHTP1 (fractions other than the main TPO fraction, namely a passed-through fraction F1 and a fraction F3 eluted after F2 by washing with 20 mM Na citrate, pH 5.8, of Macro-Prep Methyl HIC column and fractions F1 and F3 of SP Sepharose Fast Flow column) were combined to prepare a TPO subpool fraction (5,463.79 ml; protein concentration, 0.490 mg/ml; total protein, 2,676 mg; relative activity, 12,100; and total activity, 32,380,000) and concentrated using an ultrafiltration unit (Omega Ultraset, nominal molecular weight cutoff of 8,000, manufactured by Filtron), solvent of the concentrated fraction was exchanged with DPBS and then the small-volumed sample was treated with an YM-10 membrane-equipped ultrafiltration unit (manufactured by Amicon) to make it finally into 120.2 ml of a DPBS solution containing 0.05% sodium azide. Next, all of the 5 anti-TPO peptide antibody gels prepared in Example 46 were mixed and made into a single antibody column (1.6 cm in diameter and 4.8 cm in bed height, to be referred to as anti-RT1 + 2 mix antibody column), and the TPO subpool fraction was applied to the column at a flow rate of 0.033 ml/min. After completion of the sample loading, elution was carried out with DPBS to collect eluates until UV absorption became sufficiently small, and the thus collected eluates were concentrated using the YM-10 membrane-equipped ultrafiltration unit (manufactured by Amicon) to obtain a passed-through fraction F1 (82.62 ml; protein concentration, 14.3 mg/ml; total protein, 1,184 mg; relative activity, 67,500; and total activity, 79,900,000). Next, a column-adsorbed fraction F2 (92.97 ml; protein concentration, 0.12 mg/ml; total protein, 11.1 mg; relative activity, 257,100; and total activity, 2,860,000) was eluted with an acidic eluting solution (0.1 M glycine-HCl, pH 2.5). Through the passed-through fraction F1 still contained a TPO activity, the antibody column-adsorbed TPO was further purified because it showed about 20 times increase in the relative activity. That is, using 0.1% TFA as a developing solvent A and 1-propanol containing 0.05% TFA as a developing solvent B, Capcell Pak Cl 300A (manufactured by Shiseido, catalog No. Cl TYPE;SG300A; 4.6 mm in diameter and 150 mm in bed height, connected with a precolumn of 4.6 mm in diameter and 35 mm in bed height) column chromatography was carried out, by mixing the fraction F2 obtained from the antibody column with 1/10 volume of the developing solvent B and applying the mixture at a flow rate of 0.4 ml/min to the Capcell Pak Cl 300A column which has been equilibrated in advance with 20% B. After completion of the sample loading, elution was carried out for 5 minutes with 20% B and then for 50 minutes with a linear density gradient of from 20% B to 40% B, and the eluates were collected in polypropylene tubes in 1 ml (2.5 min) portions.

A 2 µl portion (1/500 fraction) of the eluate in each tube was mixed with HSA and concentrated by ultrafiltration to obtain 0.25 ml of IMDM assay culture solution containing 0.02% HSA for use in the identification of TPO active fractions by the assay. As the result, strong TPO activity was found in samples of tube Nos. 20 to 23 (within the range of 28.5 and 32.5% as propanol concentration). In addition, when these samples were subjected to western analysis in accordance with the procedure described in ⟨Example 45⟩, the presence of TPO was confirmed which had an apparent molecular weight of 60,000 to 70,000 when measured under a reduced condition based on DPC III molecular weight markers, as well as the presence of other molecules having respective molecular weights of 32,000 to 43,000 and 20,000 to 30,000.

⟨Example 48⟩

Confirmation of biological activity of TPO derived from culture supernatant obtained by transfecting Cos 1 cells with expression vector pHTP1 and purified up to the step of Capcell Pak Cl 300A column

The TPO active fractions purified in Example 36, namely tube Nos. 20 to 23 (within the range of 28.5 and 32.5% as propanol concentration) of Capcell Pak Cl 300A column, were pooled, mixed with 0.21 ml of glycerol and then concentrated by centrifugation evaporation. This was mixed with 0.21 ml of 6 M guanidine hydrochloride solution, diluted to a volume of 1 ml with DPBS, buffer-exchanged with DPBS solution containing 0.01% HSA by Sephadex G25 column (NAP-10, manufactured by Pharmacia Biotech, catalog No. 17-0854-01) and then mixed with 1.1 ml of DPBS solution containing 0.01% HSA, thereby finally obtaining a TPO active fraction FA (2.6 ml). In order to examine biological TPO activity of this sample, *in vivo* assay was carried out. That is, the active fraction was subcutaneously administered to ICR male mice (8 weeks of age), each group including 4 animals, daily for 5 days at a dose of 100 µl (having a total activity of 110,000 measured by M-07e assay system) per animal. As a control, 100 µl of DPBS containing 0.01% of HSA was subcutaneously administered in the same manner. Just before the administration and on the next day of the final administration, blood was collected from the eyeground to measure platelet counts using a microcell counter (F800, manufactured by Toa Iyo Denshi). In the TPO-treated group, platelet counts increased by a factor of about 1.42 in average after completion of the administration in comparison with the value prior to the administration and was 1.23 times higher than the case of the control group with a significant difference ($p < 0.05$, Student t-test) between them. On the basis of these results, it was confirmed that the aforementioned human TPO produced by mammalian cells has the ability to increase platelet counts in vivo.

⟨Example 49⟩

Confirmation of biological activity of crude TPO fraction derived from 33 liters of culture supernatant obtained by transfecting COS 1 cells with expression vector pHTP1 and partially purified by cation exchange column

(1) The M-07e assay system was used as the *in vitro* assay for the evaluation of the following purification samples. In the same manner as described in Example 36, a total of 33 liters of serum-free culture supernatant was obtained by transfecting COS 1 cells with the expression vector pHTP1 and filtered through a 0.22 µm filter to recover the supernatant. This was concentrated by a factor of about 10 with an ultrafiltration unit (PLGC pellicon Cassette, nominal molecular weight cutoff of 10,000, manufactured by Millipore) and mixed with about 1 mM in final concentration of a protease inhibitor p-APMSF to obtain a sample of 2,018 ml in volume (protein concentration, 3.22 mg/ml; total protein, 6,502 mg; relative activity, 66,000; total activity, 429,100,000). Next, this was concentrated repeatedly using an ultrafiltration unit (Omega Ultraset, nominal molecular weight cutoff of 30,000; manufactured by Filtron), thereby obtaining a fraction of 30,000 or more in molecular weight (volume, 1,190 ml; protein concentration, 2.54 mg/ml; total protein, 3,020 mg; relative activity, 82,500; total activity, 249,000,000) and a fraction of 30,000 or less in molecular weight (volume, 2,947 ml; protein concentration, 0.471 mg/ml; total protein, 1,402 mg; relative activity, 4,500; total activity, 6,310,000). The presence of TPO activity in the fraction of 30,000 or less in molecular weight indicates a possibility that the culture supernatant contains TPO whose molecular weight has been reduced during its expression in or secretion from the animal cells. On the other hand, the fraction of 30,000 or more in molecular weight was buffer-exchanged with 20 mM sodium citrate buffer (pH 6.1) and applied at a flow rate of 5 ml/min to SP Sepharose Fast Flow (2.6 cm in diameter and 29 cm in bed height, manufactured by Pharmacia Biotech, catalog No. 17-0729-01) column which has been equilibrated in advance with 20 mM sodium citrate buffer (pH 6.1). After completion of the sample loading, the column was washed and eluted with 20 mM sodium citrate buffer (pH 6.1). Next, using 20 mM sodium citrate buffer (pH 6.1) as a developing solvent A and a solution composed of the developing solvent A and 1 M NaCl as a developing solvent B, elution was carried out at a flow rate of 3 ml/min with a linear gradient of from 0% B to 50% B for 215 minutes and then from 50% B to 100% B for 20 minutes, and the eluates were collected in polypropylene tubes in 30 ml (10 min) portions. When a portion of each of these eluates was checked by the M-07e assay system to examine distribution of the activity, elution of TPO activity was found within a broad range. In consequence, fractions eluted with a NaCl concentration of 50 mM or lower, including the passed-through fraction, were pooled as a fraction F1, and the main TPO fractions eluted with 50 to 1,000 mM NaCl as a fraction F2. Volume of the fraction F1 was 1,951 ml (protein concentration, 2.05 mg/ml; total protein, 3,994 mg; relative activity, 13,500; total activity, 53,900,000) and that of F2 was 649.8 ml (protein concentration, 1.11 mg/ml; total protein, 721 mg; relative activity, 268,000; total activity, 193,000,000).

(2) Confirmation of biological activity of SP Sepharose Fast Flow fraction F2

A 2.5 ml portion of the SP Sepharose Fast Flow fraction F2 separated in the above step (1) was buffer-exchanged with 3.5 ml of DPBS solution containing 0.01% HSA by Sephadex G25 column (NAP-25, manufactured by Pharmacia Biotech, catalog No. 17-0852-01), and biological TPO activity of this sample (protein concentration, 1.46 mg/ml) was examined in an *in vivo* assay. That is, the active fraction was subcutaneously administered to ICR male mice (8-weeks age), each group including 4 animals, daily for 5 consecutive days at a dose of 100 µl (having a total activity of 123,000 measured by the M-07e assay system) per animal. As a control, 100 µl of DPBS containing 0.01% of HSA was subcutaneously administered in the same manner. Just before the administration and on the next day of final administration, blood was collected from the eyeground to measure platelet counts using a microcell counter (F800, manufactured by Toa Iyo Denshi). In the TPO-administered group, platelet counts increased by a factor of about 1.29 in average after completion of the administration in comparison with the value prior to the administration and were 1.12 times higher than in the control group with a significant difference ($p < 0.05$, Student t-test) between them. These results demonstrated that the aforementioned human TPO produced by mammalian cells has the ability to increase platelet counts.

〈Example 50〉

Construction of recombinant vector, pDEF202-ghTPO, for use in the expression of human TPO chromosomal DNA in CHO cells

A vector pDEF202 was digested with restriction enzymes *Kpn*I and *Spe*I and then subjected to agarose gel electrophoresis to isolate a fragment containing mouse DHFR mini gene and SV40 polyadenylation signal, and the expression vector pDEF202-ghTPO was obtained by ligating, using T4 DNA ligase (manufactured by Takara Shuzo), the thus obtained fragment with a vector DNA which has been prepared by removing an SV40 polyadenylation signal-containing region from a plasmid pEFHGTE by its treatment with restriction enzymes *Kpn*I and *Spe*I. This plasmid contains SV40 replication origin, human elongation factor 1-α promoter, SV40 early polyadenylation region for transcription of human TPO gene, mouse DHFR mini gene, pUC 18 replication origin and β-lactamase gene ($Amp^r$), and human TPO chromosomal DNA is connected to a downstream site of the human elongation factor 1-α promoter.

〈Example 51〉

Expression of human TPO chromosomal DNA in CHO cells

CHO cells (a $dhfr^-$ strain; Urlaub and Chasin, *Proc. Natl. Acad. Sci. USA*, vol.77, p.4216, 1980) were grown by culturing them in an α-minimum essential medium (α-MEM(-), supplemented with thymidine and hypoxanthine) containing 10% fetal bovine serum using a plate of 6 cm in diameter (manufactured by Falcon), and the resulting cells were subjected to transfection by means of a calcium phosphate method (CellPhect, manufactured by Pharmacia).

That is, 10 µg of the plasmid pDEF202-ghTPO prepared in Example 50 was mixed with 120 µl of buffer A and 120 µl of $H_2O$, and the resulting mixture was incubated at room temperature for 10 minutes. This solution was further mixed with 120 µl of buffer B and incubated at room temperature for additional 30 minutes. The thus prepared DNA solution was put in the plate and cultured for 6 hours in a $CO_2$ incubator.

After removing the medium and washing the resulting plate twice with α-MEM(-), α-MEM(-) containing 10% dimethyl sulfoxide was added to the plate and treated for 2 minutes at room temperature. Next, non-selection medium (α-MEM(-) op. cit., supplemented with hypoxanthine and thymidine) containing 10% dialyzed fetal bovine serum was added and cultured for 2 days, and then selection was carried out using a selection medium (α-MEM(-), free from hypoxanthine and thymidine) containing 10% dialyzed fetal bovine serum. Selection was carried out by treating the cells with trypsin, dispensing the treated cells in one plate of 6 cm in diameter into 5 plates of 10 cm in diameter or 20 plates of 24 well plates and continuing culturing of the cells while changing the selection medium every 2 days. The presence of human TPO activity was confirmed in the culture supernatant in the plates or wells in which proliferation of the cells was observed, when the human TPO activity was measured by the Ba/F3 assay.

In this instance, transfection of CHO cells may also be effected by carrying out co-transfection of CHO cells with pEFHGTE and pMG1.

⟨Example 52⟩

Construction of vector for use in the expression in *E. coli* of human TPO protein (amino acid residues of positions 1 to 163) (to be referred to as "hMKT(1-163)" hereinafter) in which a Lys residue and a Met residue were respectively added to the -1 and -2 positions, and confirmation of its expression

In order to effect expression of a protein whose 1- and 3-position amino acid residues are the same as those of human TPO protein (amino acid residues of positions 1 to 163), a vector pCFM536/hMKT(1-163) was constructed for use in the expression of hMKT(1-163) - also referred to as [$Met^{-2}$, $Lys^{-1}$]TPO(1-163) - in *E. coli*, in the same manner as described in Example 42 using the following synthetic oligonucleotides 1-13, 2-13, 3-3 and 4-3:

1-13: 5'-CTAGAAAAAACCAAGGAGGTAATAAATAATGAAATCTCCTGCACCA-3' (SEQ ID NO:125);

2-13: 5'-CAGGTGGTGCAGGAGATTTCATTATTTATTACCTCCTTGGTTTTTT-3' (SEQ ID NO:126);

3-3: 5'-CCTGCATGTGATTTACGGGTCCTGTCTAAACTGCTGCG-3' (SEQ ID NO:127);

4-3: 5'-CGCAGCAGTTTAGACAGGACCCGTAAATCACATG-3 (SEQ ID NO:128);

```
          10    1-13 20          30          40
 CTAGAAAAAA CCAAGGAGGT AATAAATAAT GAAATCTCCT
     TTTTTT GGTTCCTCCA TTATTTATTA CTTTAGAGGA
 XbaI             2-13

                                      50          60   3-3    70          80
                              GCACCACCTG CATGTGATTT ACGGGTCCTG TCTAAACTGC TGCG
                              CGTGGTGGAC GTACACTAAA TGCCCAGGAC AGATTTGACG ACGC
                                                    4-3
                                                      (SEQ ID NO:129)
```

This expression plasmid contains a DNA sequence shown in the Sequence Listing (SEQ ID NO:12). Thereafter, expression of hMKT(1-163) was induced in the same manner as described in Example 43.

When the thus obtained expression protein was subjected to SDS-PAGE, transferred on a PVDF membrane and then subjected to N-terminal amino acid sequence analysis, it was confirmed that this protein contains the amino acid sequence of hMKT(1-163) to be expressed.

⟨Example 53⟩

Refolding of human TPO, h6T(1-163), derived from human TPO expressed in *E. coli*, using guanidine hydrochloride and glutathione, and purification and confirmation of its biological activity

A 1.2 g portion of frozen cells of the h6T(1-163)-producing recombinant strain prepared in Example 43 were suspended in 3 ml of water and disrupted using a high pressure disintegrator. By subjecting the suspension to centrifugation, the precipitated fraction was recovered and most parts of contaminated proteins, cell components and the like were removed. The thus recovered precipitated fraction containing h6T(1-163) was suspended in 4 ml in final volume of water. A 1 ml portion of 1 M Tris buffer (pH 8.5) was added to the suspension with stirring, followed by further addition of 20 ml of 8 M guanidine hydrochloride and subsequent 5 minutes of stirring at room temperature to solubilize the contents. The resulting solution was diluted 10 times with 20 mM Tris buffer (pH 8.5), 5 mM of reduced type glutathione and 0.5 mM of oxidized type glutathione were dissolved in the diluted solution with stirring and then the thus prepared solution was incubated overnight at 4°C. By centrifugation, h6T(1-163) was recovered in the supernatant fluid. A 160 ml portion of the thus obtained supernatant fluid was concentrated using YM10 ultrafiltration membrane (manufactured by Amicon) and subjected to buffer exchange with DPBS to obtain 3.4 ml in final volume of a fraction (protein concentration, 2.18 mg/ml). When this fraction was thoroughly dialyzed against IMDM culture medium and evaluated by the rat CFU-MK assay system, strong TPO activity (relative activity, 58,000) was found.

The TPO active fraction prepared above was subjected to an *in vivo* assay. That is, the active fraction was subcutaneously administered to ICR male mice (7 weeks of age), each group including 4 animals, daily for 5 consecutive days with a dose of 170 µl (having a total activity 22,000 measured by the rat CFU-MK assay system) per animal. As a control, 170 µl of DPBS was subcutaneously administered to each of 6 animals of the control group in the same manner. Just before the administration and on the next day of and 3 days after the completion of the administration, blood was collected from the eyeground to measure platelet counts using a microcell counter (F800, manufactured by Toa Iyo Denshi). In the TPO-administered group, platelet counts increased by a factor of about 2.15 in average on the next day of the completion of the administration in comparison with the value prior to the administration, and such an effect remained unchanged even after 3 days of the completion of the administration showing a 2.13 times higher value. Platelet counts on the next day of and 3 days after the completion of the administration was 1.73 and 1.80 times higher in TPO-treated group than in the control group with a significant difference ($p < 0.001$, Student t-test) between them. These results demonstrated that the human TPO produced by *E. coli* and prepared by the above method has the ability to increase platelet counts in vivo.

⟨Example 54⟩

Refolding of human TPO, h6T(1-163) expressed in *E. coli*, using sodium N-lauroyl sarcosinate and copper sulfate, and purification and confirmation of its biological activity

A 0.6 g portion of frozen cells of the h6T(1-163)-producing recombinant strain prepared in Example 43 were suspended in 3 ml of water, disrupted using a high pressure disintegrator and then subjected to centrifugation to recover the precipitated fraction. After suspending the precipitated fraction in 3.1 ml of water, 0.19 ml of 1 M Tris buffer (pH 9.2), 11.25 µl of 1 M DTT, 38 µl of 0.5 M EDTA and 0.38 ml of 10% sodium deoxycholate solution were added to the suspension with stirring, and the resulting mixture was stirred at room temperature for 40 minutes. This was then subjected to centrifugation to recover the precipitated fraction and remove most parts of contaminated proteins, cell components and the like. The resulting precipitate was suspended in 4 ml of water and subjected to centrifugation to recover the precipitated fraction containing h6T(1-163). The thus recovered precipitated fraction was suspended in 3.8 ml of water, and 0.2 ml of 1 M Tris buffer (pH 8) and 1 ml of 10% sodium N-lauroyl sarcosinate were added to the suspension with stirring, followed by 20 minutes of stirring at room temperature to solubilize the contents. The resulting solution was mixed with 5 µl of 1 % copper sulfate and stirred overnight (about 20 hours) at room temperature. The resulting solution was subjected to centrifugation to recover the supernatant fraction containing h6T(1-163), and a 5 ml portion of the supernatant was mixed with 5 ml of water and 10 ml of 20 mM Tris buffer (pH 7.7) and then with 2.6 g of 20 to 50 mesh Dowex 1-x4 chloride form ion exchange resin, followed by 90 minutes of stirring. The ion exchange resin was removed using a glass filter to recover the resin-nonadsorbed fraction which was subsequently subjected to centrifugation to recover the supernatant fluid. The thus obtained supernatant fluid was applied to an SP Sepharose Fast Flow cation exchange column which has been equilibrated in advance with 20 mM Tris buffer (pH 7.7) and eluted with the same buffer by a linear gradient of from 0 to 500 mM NaCl. When each of the eluted fractions of the SP Sepharose Fast Flow cation exchange column chromatography was thoroughly dialyzed against IMDM culture medium and evaluated by the rat CFU-MK assay system, strong TPO activity (relative activity, 19,000,000) was found in a fraction eluted with a NaCl concentration of about 100 mM. This fraction was concentrated 1.6 fold (2.5 ml; protein concentration, 25 µg/ml) using an ultrafiltration unit (Ultra Free CL, nominal molecular cutoff of 5,000, manufactured by Millipore, catalog No. UFC4LCC25). When the thus concentrated fraction was analyzed by SDS-PAGE in the presence of a reducing agent, a band corresponding to TPO was detected as the main band (purity, 70 to 80%).

The TPO active fraction prepared by the above procedure was examined in an *in vivo* assay. That is, the active fraction was subcutaneously administered to ICR male mice (8 weeks of age), each group including 4 animals, daily for 5 consecutive days at a dose of 100 µl (having a total activity of 47,500 measured by the rat CFU-MK assay system) per animal. As a control, 100 µl of 20 mM Tris buffer (pH 7.7) containing 100 mM NaCl was subcutaneously administered in the same manner. Just before the administration and on the next day of the completion of the administration, blood samples were collected from the eyeground to measure platelet counts using a microcell counter (F800, manufactured by Toa Iyo Denshi). In the TPO-administered group, platelet counts increased by a factor of about 1.73 in average after completion of the administration, in comparison with the number prior to the administration and were 1.59 times higher than in the control group with a significant difference ($p < 0.01$, Student t-test) between them. These results demonstrated that the human TPO produced by *E. coli* and prepared by the above procedure has the ability to increase platelet counts in vivo.

⟨Example 55⟩

Large scale culture of CHO cells

The large scale cultivation of the human TPO producing CHO cell line (CHO 28-30 cell, resistant to 25 nM MTX) which had been obtained by transfecting the human TPO expression plasmid pDEF202-hTPO-P1 into CHO cells in Example 32, was carried out as follows. The CHO cell line was cultured and proliferated in DMEM/F-12 culture medium (GIBCO) containing 25 nM MTX and 10% FCS. After the cells were detached with trypsin solution, $1 \times 10^7$ cells were inoculated in a roller bottle (ex Falcon, Falcon 3000) containing 200 ml of the same medium therein and then cultured at 37°C at a rolling speed of 1 rpm for 3 days. After 3 days culture, the culture supernatant was removed by suction and the adherent CHO cells were then rinsed with 100 ml of PBS. 200 ml of the DMEM/F-12 medium (GIBCO) containing neither 25 nM MTX nor 10% FCS was added to the bottle and the cells were cultured at 37°C at a rolling speed of 1 rpm for 7 days. After 7 days culture, the culture supernatant was recovered as a starting material for subsequent purification procedure. The above mentioned procedures were carried out in 500 roller bottles to obtain 100 L of the culture supernatant.

⟨Example 56⟩

Purification of human TPO from human TPO producing CHO cell line

(1) About 100 L of the serum free culture supernatant obtained in Example 55 was filtered through a 0.22 µm filter to obtain its filtrate which was then concentrated on an ultrafiltration unit (PLTK Pellicon cassette, nominal molecular weight 30,000 cutoff, ex Millipore). After the replacement of the solvent of the concentrate with pure water, p-APMSF (Wako Pure Chemicals) and Pefabloc SC (Merck), which are proteinase inhibitors, were added to the resulting aqueous solution at final concentrations of 1 mM and 0.35 mM respectively to obtain 3628 ml of the fraction of molecular weight 30,000 or greater (protein concentration: 1.60 mg/ml; total protein: 5805 mg; relative activity; 1,230,000; total activity: 7,149,000,000). The fraction was subsequently subjected to the Western blot analysis as described in Example 45. As a result, the presence of TPO protein was revealed at a molecular weight ranging between 66,000 and 100,000. In examining in more detail, it was found that lower molecular weight TPO species than that of the above TPO were contained in the flow-through fraction.

The ultrafiltrate that contained the TPO with a molecular weight of not more than 30,000 was separately concentrated using an ultrafiltration unit (PLGC Pellicon cassette, nominal molecular weight 10,000 cutoff, Millipore), to obtain 1901 ml of the low molecular weight TPO fraction (protein concentration: 0.36 mg/ml; total protein: 684 mg; relative activity: 245,500; total activity: 167,900,000). This reveals that the low molecular weight TPO species were generated during cell culture.

Next, to 3614 ml of the fraction containing the TPO having a molecular weight of 30,000 or greater were added 764 g of ammonium sulfate and 144.5 ml of 0.5 M sodium citrate buffer (pH 5.5) to make 4089 ml of the solution containing 1.41 M (final concentration) ammonium sulfate and 17.7 mM (final concentration) sodium citrate buffer. After insoluble substances were removed from the solution by centrifugation, the clear solution was obtained.

The clear solution was then applied to Macro-Prep Methyl HIC column (Bio-Rad. Catalog No. 156-0080; diameter 5 cm, bed height 24.5 cm) which had been previously equilibrated with 20 mM sodium citrate buffer (pH 5.5) containing 1.2 M ammonium sulfate at a flow rate of 25 ml/min. After the completion thereof, the elution was carried out with 20 mM sodium citrate buffer (pH 5.5) containing 1.2 M ammonium sulfate. The eluted fraction was subsequently concentrated on an ultrafiltration unit (ex Filtron, Omega Ultrasette, nominal molecular weight 8,000 cutoff) to obtain a flow-through fraction F1 (4455 ml; protein concentration: 0.400 mg/ml; total protein: 1780 mg; relative activity: 1,831,000).

Next, 20 mM Na citrate (pH 6.0) as an eluting buffer was passed through the column to obtain an eluate which was then concentrated using the same ultrafiltration unit (i.e., Omega Ultrasette, nominal molecular weight 8,000 cutoff) to collect a fraction F2 (1457 ml; protein concentration: 0.969 mg/ml; total protein: 1411 mg; relative activity: 1,715,000). In the F2 the presence of a protein having a molecular weight from 66,000 to 100,000 was confirmed on SDS-PAGE. As a result of Western analysis, it was revealed that the protein was TPO.

Next, the F2 eluted from the Macro-Prep Methyl HIC column (1443 ml) was applied to SP Sepharose Fast Flow (Pharmacia Biotech, Catalog No. 17-0729-01; diameter 5 cm, bed height 12 cm) which has been previously equilibrated with 20 mM sodium citrate buffer (pH 6.0) at a flow rate of 15 ml/min. After the completion thereof, the elution was carried out with 20 mM sodium citrate buffer (pH 6.0) containing 50 mM NaCl. The eluate was collected and named fraction F1 (3,007 ml; protein concentration: 0.226 mg/ml; total protein 679 mg; relative activity: 88,830). Thereafter, the eluting buffer was replaced with 20 mM sodium citrate buffer (pH 5.4) containing 750 mM NaCl to collect fraction F2 eluted (931 ml; protein concentration: 0.763 mg/ml; total protein: 710 mg; relative activity: 5,558,000) which was then concentrated to 202 ml using ultrafiltration units (Filtron, Omega Ultrasette, nominal molecular weight 8,000 cutoff; and Amicon, YM 3 membrane).

Next, the concentrated TPO activity-containing fraction F2 (197 ml) was applied to a Sephacryl S-200HR gel filtration column (Pharmacia Biotech. Catalog No. 17-0584-05; diameter 7.5 cm, bed height 100 cm) at a flow rate of 3 ml/min. Elution volumes 1,200-1,785 ml, 1,785-2,010 ml, 2,010-2,280 ml and 2,280-3,000 ml were separately collected to obtain fractions F1 (585 ml; protein concentration: 1.00 mg/ml, total protein: 589 mg; relative activity: 4,118,000), F2 (225 ml; protein concentration: 0.263 mg/ml; total protein: 59.2 mg; relative activity 2,509,000). F3 (270 ml; protein concentration: 0.119 mg/ml, total protein; 32.1 mg; relative activity: 2,535,000) and F4 (720 ml; protein concentration: 0.0467 mg/ml; total protein: 33.6 mg; relative activity: 1,155,000), respectively. Thus, the fractionated TPO activity had a broad molecular weight range as determined by gel filtration. After SDS-PAGE of the fractions followed by Western blot analysis, it was found that F1 had a TPO molecule species with a molecular weight of 66,000 to 1,000,000 predominantly; F2 a TPO molecule species with a molecular weight of 32,000 to 60,000; and F3 a TPO species with a molecular weight of 32,000 to 42,000. All the TPO molecules possessed TPO activity.

On the basis of the result of N-terminal amino acid sequencing of the TPO molecule having a molecular weight of 66,000 to 100,000, we confirmed that the TPO molecule had the amino acid sequence of a human TPO gene encoded protein.

In addition, the TPO molecule having a molecular weight 66,000-100,000 was subjected to enzymatic digestion experiments using the glycosidase enzymes consisting of N-glycanase (ex Genzyme, Catalog No. 1472-00), neuraminidase (Nakarai-Tesqu, Catalog No. 242-29SP), endo-a-N-acetylgalactosaminidase ( Seikagaku Kogyo, Catalog No. 100453) and O-glycosidase (Böehringer Mannheim Biochemica, Catalog No. 1347101) singly or in combination, and then to SDS-PAGE analysis. As a result; the polypeptide portion of the TPO was found to have a molecular weight of about 36,000 as expected from its theoretical molecular weight and to be a glycoprotein with both N- and O-linked sugar chains.

⟨Example 57⟩

Purification of human TPO from human TPO producing CHO cell line

(1) To 1 L of the serum free culture supernatant of the CHO cells obtained in the same manner as in Example 55 was added 211.4 g of ammonium sulfate, after which the mixture was filtered through a 0.2

μm filter (ex Gelman Science, Catalog No. 12992). The obtained filtrate was applied to Macro-Prep Methyl HIC column (Bio-Rad, Catalog No. 156-0081, diameter 50 mm, bed height 90 mm) which had been previously equilibrated with 20 mM sodium acetate buffer (pH 5.6) containing 1.2 M ammonium sulfate at a flow rate of 15 ml/min. After the completion thereof, the elution was carried out with 450 ml of 20 mM sodium acetate buffer (pH 5.6) containing 1.2 M ammonium sulfate. The eluted fraction was then applied to a reverse phase Vydac C4 column (The Separations Group, Catalog No. 214BTP54, diameter 4.6 mm, bed height 250 mm) at a flow rate of 0.75 ml/min. After the column was washed with 10 mM Tris buffer (pH 6.4) containing 5% ethanol (referred to as "development solvent A") for 15 min, the elution was carried out with a 66-min linear gradient of from the development solvent A to 10 mM Tris buffer (pH 6.4) containing 94% ethanol (referred to as "development solvent B"). The chromatogram obtained is shown in Fig. 15. As a result of SDS-PAGE analysis, the molecule having a molecular weight 65,000-100,000 which was expected to be TPO and which corresponded to the fraction of retention time 68-72 min after addition of the sample, appeared as a single band on the SDS-PAGE gel (see Fig. 16). Western analysis further revealed that the obtained protein is TPO.

The N-terminal amino acid analysis of the protein sample revealed that the protein had the amino acid sequence of a human TPO gene encoded protein.

〈Example 58〉

Preparation of recombinant virus for human TPO-expression within insect cells

The plasmid pHTP1 prepared in Example 30 was digested with restriction enzymes EcoRI and NotI and then subjected to 1% agarose gel electrophoresis to obtain an about 1200-bp band which was subsequently purified using Prep-A-Gene DNA purification kit. The purified DNA was then ligated to a transfer vector pVL 1393 (Invitrogen) pretreated with the same restriction enzymes, followed by transformation into Competent-High E. coli DH5 (Toyo Boseki). From the colonies obtained was selected a clone pVL1393/hTPO which contained a full length coding region of human TPO cDNA. The plasmid DNA was then prepared from the clone by the method described in *Molecular Cloning* (Sambrook et al., Cold Spring Harbor Laboratory Press, 1989) followed by transfection into insect cell Sf 21 ( Invitrogen) using BaculoGold™ Transfection kit (Farmingen), and the transfected cells were cultured in Sf-900 medium ( Lifetechnology) at 27°C for 4 days to recover the supernatant containing the virus. The supernatant was then diluted to $1{:}10^9 \sim 1{:}10^5$, and about $7 \times 10^5$ Sf 21 cells were infected with 1 ml of the diluted supernatant at 27°C for 1 hr in a 35-mm (in diameter) shale. After removal of the supernatant, 1% hot agarose in Sf-900 medium was placed into the culture and allowed to solidify, followed by 6-day cultivation at 27°C in a moisturized atmosphere. A single plaque formed was picked up, from which the virus was released in 200 μl of Sf-900 medium. Sf 21 cells were infected with the obtained viral clone in a 24-well plate to proliferate the virus. A portion of the virus-containing liquid from the single plaque was treated with phenol/chloroform followed by ethanol precipitation to recover the viral DNA which was then used as a template to carry out PCR using primers specific for human TPO cDNA. The recombinant virus containing human TPO cDNA was selected based on amplification of the specific DNA fragment. Then, Sf 21 cells were infected with the supernatant containing the recombinant virus carrying human TPO cDNA to proliferate the virus.

〈Example 59〉

Expression of human TPO in Sf 21 insect cells and identification of TPO activity

Sf 21 cells were cultured in a 175 $cm^2$ culture flask to about 80% confluence was attained, followed by infection with the human TPO cDNA carrying recombinant virus prepared in Example 58 at 27°C for 1 hr, after which the obtained cells were cultured in Sf-900 medium at 27°C for 4 days to recover the supernatant of the culture. The obtained culture supernatant was substituted by IMDM culture medium on NAP™-5 column ( Pharmacia). Significant TPO activity in the resultant supernatants was detected in a dose-dependent manner in both rat CFU-MK assay and M-07e assay. Also, the recombinant human TPO expressed in Sf 21 cells was identified by means of the Western analysis as described in Example 45.

⟨Example 60⟩

## Refolding of variant human TPO, h6T(1-163) derived from clone pCFM536/h6T(1-163) carrying a human TPO base sequence via its expression in E. coli, by using sodium N-lauroyl sarcosinate and cupric sulfate, and purification of the h6T(1-163)

Thirty g of the frozen h6T(1-163)-producing recombinant microorganism prepared in Example 43 was suspended in 300 ml of water, disrupted in a high pressure disrupting apparatus (10,000 psi, Rannie High Pressure Laboratory), and then centrifuged to recover a sedimented fraction. The sediment fraction was suspended in 90 ml of water to which water was then added to the total amount of 150 ml with stirring. Subsequently, to the mixture was added 9 ml of 1 M Tris buffer (pH 9.2), 540 µl of 1 M DTT, 1.8 ml of 0.5 M EDTA and 18 ml of 10% sodium deoxycholic acid with stirring, followed by 30 min-stirring at room temperature. The sedimented fraction was recovered by centrifugation and most of the contaminated proteins and microorganism components were removed. To the sediment was added 180 ml of 5 mM DDT to obtain a suspension which was then centrifuged to recover the sediment fraction containing h6T(1-163). To the resultant sediment was added 300 ml of water to obtain a suspension to which water was then added to the liquid amount of 570 ml with stirring. Thirty ml of 1 M Tris buffer (pH 8), 150 ml of 10% sodium N-lauroyl sarcosine were added to the mixture at room temperature with 20-min stirring in order to solubilize the h6T(1-163). To this was further added 750 µl of 1% cupric sulfate and the mixture gas stirred overnight (about 20 hr) at room temperature. After the supernatant fraction containing h6T(1-163) was recovered by centrifugation, to 750 ml of the supernatant was added 750 ml of water and 1,500 ml of 20 mM Tris buffer (ph 7.7) followed by addition of 3 ml of polysorbate 80 (Nikko Chemicals) with stirring. To the obtained solution was added 600 g of Dowex 1-X4 (20-50 mesh, chloride form) ion-exchange resin followed by 90-min stirring at room temperature. Following recovery of the non-adsorbed fraction using a glass filter, the ion-exchange resin was washed with 750 ml of 20 mM Tris buffer (pH 7.7). The combined solution of the non-adsorbed and wash fraction was adjusted to pH 9.2 with 2 N sodium hydroxide after which it was applied to Q Sepharose Fast Flow anion-exchange column (ID 5 cm x 10 cm) equilibrated with 20 mM Tris buffer (pH 9.2) containing 0.1 % polysorbate 80 to recover a non-adsorbed fraction. The pH of the obtained non-adsorbed fraction was adjusted to 7.2 with hydrochloric acid; applied to SP Sepharose Fast Flow cation exchange column (ID 5 cm X 10 cm) equilibrated with 20 mM Tris buffer (pH 7.2) containing 0.1% polysorbate 80, and then eluted with the linear gradient from 0 M to 500 mM NaCl in the same buffer. The eluted fractions were subjected to SDS-PAGE analysis to collect a TPO fraction to which trifluoroacetic acid was then added to a concentration of 0.1%. After the solution obtained was applied to Capcell Pak 5 µm 300 A C1 column (ID 2.1 cm X 5 cm X 2, Shiseido), a reverse phase HPLC was carried out by the linear gradient elution method with increasing 1-propanol concentration in 0.1% trifluoroacetic acid. The eluate was analyzed on SDS-PAGE in the absence of a reducing agent. As a result, three fractions were fractionated based upon the elution position in the reverse phase HPLC, each fraction of which was diluted three times with 0.1% trifluoro-acetic acid. Each fraction diluted was applied to the above reverse phase HPLC column in the similar manner. The resultant three TPO fractions which were designated as Fr. S-a, Fr. S-b and Fr. S-c depending in the order of elution on the reverse phase HPLC (see Fig. 17), were analyzed on SDS-PAGE in the absence of a reducing agent. As a result, a single band was detected at a molecular weight of about 18 kDa (Fr. S-a), about 19 kDa (Fr. S-b) or 18 kDa (Fr. S-c) (see Fig. 18). Following their amino acid analyses, each of the amino acid compositions determined was almost consistent with the corresponding theoretical value estimated from the sequence information. Additionally, the results of N-terminal amino acid analysis showed that they were the expected sequences. The protein amount of each fraction determined by amino acid analysis was 0.64 mg (Fr. S-a), 1.81 mg (Fr. S-b) or 3.49 mg (Fr. S-c). After full dialysis of each fraction against IMDM medium, M-07e assay was carried out. As a result, the TPO relative activity of each fraction was about 1,620,000 (Fr. s-a), 23,500,000 (Fr. S-b) or 746,000,000 (Fr. S-c).

〈Example 61〉

Refolding of variant human TPO, h6T(1-163) derived from clone pCFM536/h6T carryinga human TPO base sequence via its expression in E. coli, by using guanidine hydrochloride and cystein-cystin, and purification of the h6T(1-163)

Fifty g of the h6T(1-163)-producing frozen recombinant microorganism prepared in Example 43 was added to 500 ml of water and suspended, disrupted using a high pressure disrupting apparatus (10,000 psi, Rannie High Pressure Laboratory), and then centrifuged to recover a sediment fraction. The sediment fraction was suspended in water and its liquid amount was then adjusted to 250 ml by addition of water with stirring. To this, thereafter, 15 ml of 1 M Tris buffer (pH 9.2), 900 $\mu$l of 1 M DTT, 3 ml of 0.5 M EDTA and 30 ml of 10% sodium deoxycholic acid were added and stirred at room temperature for 30 min. After centrifugation, the sediment fraction was recovered while removing most of the contaminated proteins, microorganism components and the like. To the sediment was added 300 ml of 5 mM DTT and suspended, after which the sedimented fraction containing h6T(1-163) was recovered by means of centrifugation. The recovered sedimented fraction containing h6T(1-163) was suspended in water in a total volume of 104 ml. To the suspension was added 20 ml of 1M Tris buffer (pH 8.5) and then 376 ml of 8 M guanidine hydrochloride with stirring, and subsequently stirred at room temperature for 10 min in order to solubilize the h6T (1-163). To this was added 2,500 ml of 20 mM Tris buffer (pH 8.5) containing 0.1% polysorbate 80 and then 2,000 ml of 20 mM Tris buffer (pH 8.5) containing 1 M guanidine hydrochloride with stirring, followed by addition of 5 mM cystein and 0.5 mM cystin. The thus obtained solution was incubated overnight at 4°C and then centrifuged to recover h6T(1-163) in the supernatant which was then concentrated using Prep Scale UF cartridge PLDC ultrafiltration membrane (Millipore). The buffer of the concentrate was replaced by 20 mM Tris buffer (ph 9.2) containing 0.1% polysorbate 80 until reaching a final volume of 1,000 ml. The obtained solution was applied to Q Sepharose Fast Flow anionic exchange column (ID 5 cm X 10 cm) equilibrated with 20 mM Tris buffer (pH 9.2) containing 0.1 % polysorbate 80, and then eluted with a linear gradient from 0 M to 500 mM NaCl in the same buffer, by which the fraction (240 ml) eluted at about 20 mM - about 150 mM NaCl was recovered. The resultant fraction was diluted four times with 20 mM Tris buffer (pH 7.2) to attain a total volume of 960 ml, and then adjusted to pH 7.2 with acetic acid, applied to SP Sepharose Fast Flow cation exchange column (ID 5 cm X 10 cm) equilibrated with 20 mM Tris buffer (pH 7.2) containing 0.1 % polysorbate 80, and then eluted with a linear gradient from 0 M to 500 mM NaCl in the same buffer. The eluate was analyzed on SDS-PAGE to collect a TPO fraction. To the TPO fraction was added trifluoroacetic acid until its final volume of 0.1%. The resultant solution was applied to Capcell Pak 5 $\mu$m 300A C1 column (ID 2.1 cm X 5 cm X 2, Shiseido), and reverse phase HPLC was subsequently conducted by linear gradient elution method with increasing 1-propanol concentration in 0.1% trifluoroacetic acid. The eluate was analyzed by SDS-PAGE in the absence of a reducing agent and fractionated into two fractions depending on the eluted position in the reverse phase HPLC. The two TPO fractions were designated as Fr. G-a and Fr. G-d based on their order eluted in the reverse phase HPLC (see Fig. 17). Each fraction was then analyzed on SDS-PAGE in the absence of a reducing agent. As a result, a single band was detected at a molecular weight of about 18 kDa (Fr. G-a) or about 32 kDa (Fr. G-d) (see Fig. 18). Furthermore, the SDS-PAGE analysis of the above fractions in the presence of a reducing agent showed that in both the fractions a single band was detected at a molecular weight of about 20 kDa. The result of the amino acid analysis of the fractions showed that each of the amino acid compositions thereof was almost consistent with the corresponding theoretical value estimated from the sequence information. Additionally, the results of the N-terminal amino acid analysis showed that they were the expected sequences. The protein amount of each fraction determined from the results of the amino acid analysis was 2.56 mg (Fr. G-a) or 1.16 mg (Fr. G-d). Following full dialysis of each fraction against IMDM medium, M-07e assay was carried out. As a result, the fraction had TPO relative activity of about 3,960,000 (Fr. G-a) or of about 7,760,000 (Fr. G-d).

〈Example 62〉

Construction of recombinant vector pDEF202-hTPO163 for expression of a partial length human TPO (amino acids 1 to 163) (hereinafter referred to as "hTPO163") within CHO cells

The vector pDEF202 constructed in Example 31 was treated with restriction enzymes EcoRI and SpeI followed by recovery of a larger vector fragment using agarose gel electrophoresis. The fragment was ligated by T4DNA ligase (Takara-Shuzo) with an hTPO163 cDNA which had been prepared by treating a plasmid pEF18S-hTPO163 containing the hTPO163 cDNA encoding the amino acids -21 to 163 (SEQ ID NO:13) with restriction enzymes EcoRI and SpeI, to give an expression vector pDEF202-hTPO163. This plasmid contained an origin of replication of SV40, a human elongation factor 1-$\alpha$-promoter, an SV40 early polyadenylation site, a murine DHFR minigene, an origin of replication of pUC18 and a $\beta$-lactamase gene ($Amp^r$), in which the hTPO163 cDNA is linked at a site downstream of the human elongation factor 1-$\alpha$-promoter.

〈Example 63〉

Expression of hTPO163 in CHO cells

A CHO cell line ($dhfr^-$strain, Urlaub and Chasin, Proc. Natl. Acad. Sci. USA, 77, p 4216, 1980) was grown in ( alpha- minimum essential medium ($\alpha$-MEM(-) with thymidine and hypoxanthine) containing 10% bovine fetal serum in 6 cm dish (Falcon), and then transformed with a pDEF202-hTPO163 plasmid by the transfectum method (Seikagaku Kogyo K.K.).

Briefly, 10 $\mu$g of the plasmid pDEF202-hTPO163 prepared in Example 62 was mixed with 240 $\mu$l of 0.3 M NaCl then with a mixture of 20 $\mu$l of transfectum and 220 $\mu$l of $H_2O$. This DNA solution was added dropwise to the dish followed by culture for 6 hr in a $CO_2$ incubator. The medium was removed from the dish which was then washed twice with $\alpha$-MEM(-) followed by addition of 10% DMSO containing $\alpha$-MEM(-) before incubation for 2 minutes at room temperature. Thereafter, to the dish was added 10% dialyzed bovine fetal serum-containing non-selection medium ($\alpha$-MEM(-) with hypoxanthine and thymidine) followed by two day culture, then selection in 10% dialized bovine fetal serum-containing selection medium ($\alpha$-MEM(-) without hypoxanthine and thymidine). The selection was conducted by trypsinizing the cells, dividing them into five 10 cm dishes or twenty 24 well dishes per the above 6 cm dish, and continuing to culture while the medium was exchanged with the fresh selection medium at intervals of two days. The supernatants from plates or wells were assayed for TPO activity using Ba/F3 assay, thereby the TPO activity being observed. The cells in which TPO activity was observed in the culture supernatants were then transferred to fresh plates or wells after divided to a cell concentration of 1:15 with the selection medium containing 25 nM methotrexate, and subsequently recultured for growth and cloning of cells resistant to methotrexate.

Alternatively, the transformation of the CHO cells can be carried out by co-transfection of pEF18S-hTPO163 and pMG1 into the CHO cells.

CHO strain (CHO-DUKXB11) transfected with the plasmid pDEF202-hTPO163 has been deposited by the present Inventors on January 31, 1995, under the deposit No. FERM BP-4989, in the National Institute of Bioscience and Human Technology, Agency of Industrial Science and Technology, Ministry of International Trade and Industry, Japan.

〈Example 64〉

Large-scale culture of CHO cells

The large-scale culture of the hTPO163-producing CHO cell line (CHO 109 cells, obtained by the above-mentioned selection in 10% dialyzed bovine fetal serum-containing selection medium [$\alpha$-$MEM^-$ without hypoxanthine and thymidine]) which had been obtained by transfection of the hTPO163-expression plasmid pDEF202-hTPO163 into CHO cells in Example 63 was carried out as follows. The CHO 109 cells were grown in DMEM/F-12 medium (GIBCO) with 10% FCS. After the cells were harvested (or trypsinized) using a trypsin solution, ten millions cells were inoculated into a Falcon roller bottle (Falcon 3000) containing 200 ml of the same medium and then cultured at a rolling speed of 1 rpm at 37°C for 3 days. The culture medium was removed under suction and the surface of the cell culture was rinsed with 100 ml of PBS. Then, to the cell culture was added 200 ml of DMEM/F-12 medium (GIBCO) without 10% FCS followed by 7 day culture at 37°C at 1 rpm. The culture supernatant harvested was used as a starting material for the subsequent purification step. The similar procedures were conducted in 300 roller bottles to yield 60 L of the serum-free culture supernatant.

〈Example 65〉

Purification of hTPO163 from hTPO163-producing CHO cell line

(1) 60 L of the serum-free culture supernatant obtained in Example 64 was filtered through a 0.22 µm filtration filter to collect a filtrate which was then concentrated using an ultrafiltration unit (Filtron, molecular weight 10,000 cutoff) to obtain a concentrated fraction (600 ml, 11.2 protein/ml, total protein 6430 mg). By Western analysis of this fraction using the anti-HT1 peptide antibody, the presence of hTPO163 protein expressed at the range of an apparent molecular weight from 20,000 to 26,000 was shown.

The resulting concentrated culture supernatant (537 ml) was treated on a Sephadex G-25 Fine column (Pharmacia-Biotech, Catalogue No. 17-0032-02; diameter 10 cm and bed height 30 cm) pre-equilibrated with 10 mM sodium phosphate buffer (pH 6.8) to obtain a protein fraction F1 (938 ml, protein concentration 4.9 mg/ml, total protein 4594 mg) as a solution in 10 mM sodium phosphate buffer (pH 6.8).

This protein fraction (929 ml) from Sephadex G-25 Fine column was applied at a flow rate of 15 ml/min, to a SP Sepharose Fast Flow column (Farmacia-Biotech, Catalogue No. 17-0729-01; diameter 5 cm and bed height 12 cm) pre-equilibrated with 10 mM sodium phosphate buffer (pH 6.8) followed by elution with 10 mM sodium phosphate buffer (pH 6.8) then the same buffer containing 10% ethanol. The eluates were combined as a fraction F1 (1608 ml, protein concentration 2.13 mg/ml, total protein 3426 mg). Then, the second elution was carried out with 10 mM sodium phosphate buffer (pH 6.8) containing 750 mM NaCl and 25% ethanol to collect a main hTPO163 eluate of fraction F2 (651 ml, protein concentration 1.67 mg/ml, total protein 1087 mg).

To the TPO activity-containing fraction F2 (200 ml) from SP Sepharose Fast Flow column were added ethanol and purified water to prepare 300 ml of 45% (final conc.) ethanol solution. Insoluble materials which resulted from the addition of ethanol were removed by centrifugation. The supernatant was injected into a SOURCE 15RPC column (Pharmacia-Biotech, Catalogue No. 17-0727-02; diameter 2 cm and bed height 20 cm) pre-equilibrated with 50% of solvent A (10 mM sodium acetate buffer, pH 6.7) plus 50% of solvent B (10 mM sodium acetate buffer, pH 6.7, containing 90% ethanol) at a flow rate of 2 ml/min. Then the column was washed with 55% solvent A plus 45% solvent B until unadsorbed substances were almost completely eluted, after which the elution at a flow rate of 1.5 ml/min was carried out according to the following elution profile: 50% B for 5 minutes; linear gradient from 50% B to 100% B over 140 minutes; and then 100% B for 35 minutes. The fractions were collected at intervals of 5 minutes (corresponding to 7.5 ml volume). All the fractions were subjected to SDS-PAGE then to Western analysis for examining an elution range of hTPO163. As a result, the highly purified hTPO163 with an apparent molecular weight from approximately 20,000 to 26,000 was found to be eluted at the range from 66% to 87% ethanol. Among these hTPO163 proteins, the hTPO163 having a higher molecular weight was eluted earlier from the reverse phase column, suggesting that the glycosylated hTPO163 molecule has an increased hydrophilicity.

To 88.8 ml of the hTPO163 elution fraction (i.e. hTPO163 fraction (90 ml) eluted at the range from 68% to 86.5% ethanol) from SOURCE 15RPC column was added CHAPS, and the mixture was concentrated and washed to obtain 2.5 ml of a concentrate containing about 5% ethanol and 4 mM CHAPS. This concentrate was subsequently applied to a Superdex 75 pg column (Pharmacia-Biotech, Catalogue No. 17-1070-01; diameter 2.6 cm and bed height 60 cm) equilibrated with 10 mM sodium phosphate buffer (pH 6.8) containing 10% ethanol at a flow rate of 1.5 ml/min. From the time of 60 minutes after application, the elution fractions were collected in an amount of 6 ml each (i.e. every 4 min). As a result, the hTPO163 was eluted in the fractions from tube number 16 to at least tube number 31 as determined by SDS-PAGE. This elution position is corresponding to the range of a molecular weight from approximately 44,000 to approximately 6,000, as determined by gel filtration using a standard molecular weight marker (mixture of Bio-Rad, Gel Filtration Standard. Catalogue No. 151-1901; and Calbiochem, insulin, Catalogue No. 407696). Additionally, these hTPO163 molecules appeared to be eluted in the order of decreasing degree of glycosylation. All the hTPO163 species of the range from tube numbers 16 to 31 (elution volume: 180 to 276 ml) were collected as fraction FA. In addition to the fraction FA, the fractions of the tube Nos. 16 to 18 (elution volume: 180 to 198 ml), 19 to 24 (elution volume: 198 to 234 ml) and 25 to 31 (elution volume: 234 to 276 ml) were separately collected and designated as fractions FH, FM and FL, respectively. Also, the fraction FA was prepared by combination of the portions of the fractions FH, FM and FL. The above is shown in Fig. 19.

(2) Next, the hTPO163 elution fractions FH, FM, FL and FA from the Superdex 75 pg column set forth in (1) will be illustrated in more detail. The N-terminal amino acid sequences of the hTPO163 species obtained above were determined by the same method as in Example 1, but most of the N-terminal Ser residues could not be identified. This suggests that an O-linked sugar is added to the N-terminal Ser. Furthermore, the sequence following the N-terminal Ser was confirmed to be an amino acid sequence expected from the gene sequence of hTPO. The concentrations of the hTPO163 proteins in the FH, FM, FL and FA were 10.2 ng/ml, 6.2 ng/ml, 0.84 ng/ml and 3.2 ng/ml as determined by amino acid analysis (AccQ. Tag method, Wasters), respectively, provided that the concentrations were of the peptide moieties not containing any sugar chain. These fractions (100 ng each) were subjected to SDS-PAGE under unreducing conditions using a multigel 15/25 (Dai-ichi Kagaku Yakuhin, 15 to 25% precast polyacrylamide gel) or under reducing conditions using DTT, followed by silver-staining (Daiichi Pure Chemicals). As a result, each of these fractions was found to contain highly pure hTPO163. The apparent molecular weights of the hTPO163 in the FH, FM, FL and FA, as calculated using DPCIII molecular weight marker (Daiichi Pure Chemicals) as a standard under the reducing conditions, were 24,000 to 21,500, 23,000 to 21,000, 23,000 to 20,500 and 23,500 to 20,500, respectively (see Fig. 20). Additionally, the apparent molecular weights of the hTPO in the FH, FM, FL and FA, as calculated using biotin-labelled SDS-PAGE standard (Bio-Rad, Biotynylated SDS-PAGE Standards, Broad Range: Catalogue No. 161-0319) under the reducing conditions on Western analysis, were 26,000 to 22,000, 25,500 to 22,000, 26,000 to 21,000 and 26,000 to 21,000, respectively. The heterogeneity among the molecular weights of FH, FM, FL and FA may be due to the heterogeneity of the O-linked sugar chains. Therefore, each fraction of the FH, FM, FL and FA was digested with neuraminidase (Neuraminidase, Nacalai tesque, Catalogue No. 242-29SP), reduced with DTT, and then analyzed on SDS-PAGE. As a result, in all the fractions, the apparent molecular weight was around 19,000, revealing that the heterogeneity of the molecular weight of hTPO is mainly due to the heterogeneity of the amount of sialic acid in the sugar chains coupled with the hTPO163 protein and that the hTPO163 obtained was expressed as a glycoprotein in CHO cells.

(3) The fractions FH, FM, FL and FA obtained in (2) were assayed for their in vitro activity by M-07e assay system. As a result, the relative specific activity were 511,000,000, 775,000,000, 1,150,000,000 and 715,000,000/mg hTPO163 protein (weight of the peptide moiety not containing any sugar weight).

⟨Example 66⟩

## Construction of E. coli vector for expression of human TPO (amino acids 1 to 332) in which Lys is added at the position-1 and Met at the position-2 respectively (hereinafter referred to as "hMKT(1-332)"), and expression of the hMKT(1-332)

To express the full length amino acid sequence of human TPO in E. coli, the use codons of from amino acid 164 to amino acid 332 were changed to E. coli preference codons as described below.

Table 5

21 : 5'-CTCCCGAACCGTACCAGCGGCCTGCTGGAAACCAACTTTACCGCGAG-3' (SEQ ID NO:130)

22 : 5'-GGTAAAGTTGGTTTCCAGCAGGCCGCTGGTACGGTTCGGGAGCTCGT-3' (SEQ ID NO:131)

23 : 5'-CGCGCGTACCACCGGCAGCGGCCTGCTGAAATGGCAGCAGGGCTTTCGT-3' (SEQ ID NO:132)

24 : 5'-AGCCCTGCTGCCATTTCAGCAGGCCGCTGCCGGTGGTACGCGCGCTCGC-3' (SEQ ID NO:133)

25 : 5'-GCGAAAATCCCGGGCCTGCTGAACCAGACCAGCCGTAGCCTGGATCAGAT-3' (SEQ ID NO:134)

26 : 5'-ATCCAGGCTACGGCTGGTCTGGTTCAGCAGGCCCGGGATTTTCGCACGAA-3' (SEQ ID NO:135)

27 : 5'-CCCGGGCTATCTGAACCGTATCCATGAACTGCTGAACGGCACCCGTG-3' (SEQ ID NO:136)

28 : 5'-GTGCCGTTCAGCAGTTCATGGATACGGTTCAGATAGCCCGGGATCTG-3' (SEQ ID NO:137)

29 : 5'-GCCTGTTTCCGGGCCCGAGCCGTCGCACCCTGGGCGCGCCGGATATCAG-3' (SEQ ID NO:138)

30 : 5'-ATCCGGCGCGCCCAGGGTGCGACGGCTCGGGCCCGGAAACAGGCCACGG-3' (SEQ ID NO:139)

31 : 5'-ATCAGCTCTGGCACCAGCGATACCGGCAGCCTGCCGCCGAACCTGCAGCC-3' (SEQ ID NO:140)

32 : 5'-CAGGTTCGGCGGCAGGCTGCCGGTATCGCTGGTGCCAGAGCTGATATCCG-3 (SEQ ID NO:141)

33 : 5'-GGGCTATAGCCCGAGCCCGACCCATCCGCCGACCGGCCAGTATACCCTGTT-3' (SEQ ID NO:142)

34 : 5'-GGTATACTGGCCGGTCGGCGGATGGGTCGGGCTCGGGCTATAGCCCGGCTG-3' (SEQ ID NO:143)

35 : 5'-TCCGCTGCCGCCGACCCTGCCGACCCCGGTGGTTCAGCTGCATCCGCTGC-3' (SEQ ID NO:144)

36 : 5'-GGATGCAGCTGAACCACCGGGGTCGGCAGGGTCGGCGGCAGCGGAAACAG-3' (SEQ ID NO:145)

37 : 5'-TGCCGGATCCGAGCGCGCCGACCCCGACCCCGACCAGCCCGCTGCTGAACA-3' (SEQ ID NO:146)

38 : 5'-AGCAGCGGGCTGGTCGGGGTCGGGGTCGGCGCGCTCGGATCCGGCAGCAGC-3' (SEQ ID NO:147)

39 : 5'-CCAGCTATACCCATAGCCAGAACCTGAGCCAGGAAGGCTAATGAAGCTTGA-3' (SEQ ID NO:148)

40 : 5'-CTTCATTAGCCTTCCTGGCTCAGGTTCTGGCTATGGGTATAGCTGGTGTTC-3' (SEQ ID NO:149)

41 : 5'-ACGAGCTCCCGAACCGTACCA-3' (SEQ ID NO:150)

42 : 5'-CTGATATCCGGCGCGCCCAGG-3' (SEQ ID NO:151)

43 : 5'-CGGATATCAGCTCTGGCACCA-3' (SEQ ID NO:152)

44 : 5'-TCAAGCTTCATTAGCCTTCCT-3' (SEQ ID NO:153)

The synthetic oligonucleotides: 21 and 22; 23 and 24; 25 and 26; 27 and 28; 29 and 30; 31 and 32; 33 and 34; 35 and 36; 37 and 38; or 39 and 40, were phosphorylated in a solution of 0.1 mM ATP, 10 mM Tris-acetate, 10 mM Mg-acetate, 50 mM K-acetate using T4 kinase (Pharmacia) in a same tube. Then 1/10 volumes of a solution of 100 mM Tris/HCl (pH 7.5), 100 mM $MgCl_2$, 500 mM NaCl was added to the reaction mixture, boiled for 3 minutes in a water bath, and allowed to cool to form a double stranded DNA.

Four sets of the double stranded DNAs, i.e. oligonucleotides 21 and 22/23 and 24 (combination-A); oligonucleotides 25 and 26/27 and 28 (combination-B); oligonucleotides 31 and 32/33 and 34 (combination-C); and oligonucleotides 35 and 36/37 and 38/39 and 40 (combination-D), were separately ligated with a DNA ligation kit (Takara-Shuzo), and thereafter the combination-A was ligated together with the combination-B and similarly the combination-C with the combination-D to produce ligation-1 and ligation-2 respectively. Using the ligations-1 and -2 as templates, PCR were carried out wherein the oligonucleotides-41 and -42 for ligation-1, as well as the oligonucleotides-43 and -44 for ligation-2, were employed as primers. The products from the PCR of the ligations-1 and -2 were digested with SacI and EcoRV and with EcoRV and HindIII respectively, followed by electrophoresis on 2% agorose gel and then purification using a Prep-A-Gene DNA Purification Kit to recover about 240-bp and 250-bp fragments respectively. The two fragments thus obtained were subcloned into pBluescript II KS+ (Stratagene) predigested with SacI and HindIII (E. coli DH5 was employed as a host.). Of the resulting colones, the clone having the base sequence shown in Table 6 was selected through sequencing and named pBL(SH)(174-332) (see SEQ ID NO:154).

## Table 6

| | | | | | | | | | | | | | | | |
|---|---|---|---|---|---|---|---|---|---|---|---|---|---|---|---|
| CTC | CCG | AAC | CGT | ACC | AGC | GGC | CTG | CTG | GAA | ACC | AAC | TTT | ACC | GCG | AGC |
| Leu | Pro | Asn | Arg | Thr | Ser | Gly | Leu | Leu | Glu | Thr | Asn | Phe | Thr | Ala | Ser |
| 174 | | | | | | 180 | | | | | | | | | |
| GCG | CGT | ACC | ACC | GGC | AGC | GGC | CTG | CTG | AAA | TGG | CAG | CAG | GGC | TTT | CGT |
| Ala | Arg | Thr | Thr | Gly | Ser | Gly | Leu | Leu | Lys | Trp | Gln | Gln | Gly | Phe | Arg |
| 190 | | | | | | | | | | 200 | | | | | |
| GCG | AAA | ATC | CCG | GGC | CTG | CTG | AAC | CAG | ACC | AGC | CGT | AGC | CTG | GAT | CAG |
| Ala | Lys | Ile | Pro | Gly | Leu | Leu | Asn | Gln | Thr | Ser | Arg | Ser | Leu | Asp | Gln |
| | | | | 210 | | | | | | | | | | | 220 |
| ATC | CCG | GGC | TAT | CTG | AAC | CGT | ATC | CAT | GAA | CTG | CTG | AAC | GGC | ACC | CGT |
| Ile | Pro | Gly | Tyr | Leu | Asn | Arg | Ile | His | Glu | Leu | Leu | Asn | Gly | Thr | Arg |
| | | | | | | | | 230 | | | | | | | |
| GGC | CTG | TTT | CCG | GGC | CCG | AGC | CGT | CGC | ACC | CTG | GGC | GCG | CCG | GAT | ATC |
| Gly | Leu | Phe | Pro | Gly | Pro | Ser | Arg | Arg | Thr | Leu | Gly | Ala | Pro | Asp | Ile |
| | | 240 | | | | | | | | | | 250 | | | |
| AGC | TCT | GGC | ACC | AGC | GAT | ACC | GGC | AGC | CTG | CCG | CCG | AAC | CTG | CAG | CCG |
| Ser | Ser | Gly | Thr | Ser | Asp | Thr | Gly | Ser | Leu | Pro | Pro | Asn | Leu | Gln | Pro |
| | | | | | | 260 | | | | | | | | | |
| GGC | TAT | AGC | CCG | AGC | CCG | ACC | CAT | CCG | CCG | ACC | GGC | CAG | TAT | ACC | CTG |
| Gly | Tyr | Ser | Pro | Ser | Pro | Thr | His | Pro | Pro | Thr | Gly | Gln | Tyr | Thr | Leu |
| 270 | | | | | | | | | | 280 | | | | | |
| TTT | CCG | CTG | CCG | CCG | ACC | CTG | CCG | ACC | CCG | GTG | GTT | CAG | CTG | CAT | CCG |
| Phe | Pro | Leu | Pro | Pro | Thr | Leu | Pro | Thr | Pro | Val | Val | Gln | Leu | His | Pro |
| | | | | 290 | | | | | | | | | | | 300 |
| CTG | CTG | CCG | GAT | CCG | AGC | GCG | CCG | ACC | CCG | ACC | CCG | ACC | AGC | CCG | CTG |
| Leu | Leu | Pro | Asp | Pro | Ser | Ala | Pro | Thr | Pro | Thr | Pro | Thr | Ser | Pro | Leu |
| | | | | | | | | 310 | | | | | | | |
| CTG | AAC | ACC | AGC | TAT | ACC | CAT | AGC | CAG | AAC | CTG | AGC | CAG | GAA | GGC | TAA |
| Leu | Asn | Thr | Ser | Tyr | Thr | His | Ser | Gln | Asn | Leu | Ser | Gln | Glu | Gly | |
| | | 320 | | | | | | | | | | 330 | | 332 | |
| TGA | AGC | TTG | A | | | | | | | | | | | | |

Next, the pBL(XH)h6T(1-163) prepared in Example 42 as a template was subjected to PCR in the presence of the following synthetic oligonucleotides 45 and 46 as primers, and the PCR product was

digested with BamHI and SacI followed by electrophoresis on 6% polyacrylamide gel to recover an about 160-bp fragment from the gel.

45: 5'-AAGGATCCGAACGCTATCTTCCTG-3' (SEQ ID NO:155)

46: 5'-GGGAGCTCGTTCAGGGTCAGAACCAGA
GAGGTACGAGACGGAACAGCAGTGGTTGG-3' (SEQ ID NO:156)

Also, the pBL(SH)(174-332) was digested with SacI and HindIII and the digest was then purified using the Prep-A-Gene DNA Purification Kit to give an about 480-bp fragment. The two fragments prepared above were subcloned into pBluescript II KS+ (Stratagene) predigested with BamHI and HindIII (E. coli DH5 was employed as a host.).

Of the resulting clones, the clone having the base sequence shown in Table 7 was selected through sequencing and named pBL(BH)(123-332) (see SEQ ID NO:157).

Table 7

```
  G GAT CCG AAC GCT ATC TTC CTG TCT TTC CAG CAC CTG CTG CGT GGC
    Asp Pro Asn Ala Ile Phe Leu Ser Phe Gln His Leu Leu Arg Gly
    123                             130
AAA GTT CGT TTC CTG ATG CTG GTT GGC GGT TCT ACC CTG TGC GTT CGT
Lys Val Arg Phe Leu Met Leu Val Gly Gly Ser Thr Leu Cys Val Arg
        140                                     150
CGG GCG CCG CCA ACC ACT GCT GTT CCG TCT CGT ACC TCT CTG GTT CTG
Arg Ala Pro Pro Thr Thr Ala Val Pro Ser Arg Thr Ser Leu Val Leu
                        160
ACC CTG AAC GAG CTC
Thr Leu Asn Glu Leu
170             174
```

The pBL(XH)h6T(1-163) prepared in Example 42 was digested with BamHI and HindIII, and the pBL-(BH)(123-332) prepared above was similarly digested to produce a fragment of about 640 bp after which the two fragments were ligated together to obtain a clone pBL(XH)h6T(1-334). Further, the PCFM536/hMKT(1-163) prepared in Example 52 was digested with XbaI and SfiI to afford an about 270-bp fragment which was subsequently ligated with a pBL(XH)h6T(1-334) digested with the same restriction enzymes to produce a clone pBL(XH)hMKT(1-334). After the pBL(XH)hMKT(1-334) was digested with XbaI and HindIII, an about 1040-bp fragment which codes for the mutation type human TPO amino acids 1-332 was recovered and purified, and then cloned into pCFM536 (EP-A-136490) predigested with XbaI and HindIII (E. coli JM109 previously transformed with pMW1 (ATCC No. 39933) was employed as a host.). The resultant clone was named pCFM536/hMKT(1-332), and the E. coli strain carrying this expression vector was employed as a transformant for the expression of the mutation type protein hMKT(1-332) protein. The expression plasmid pCFM536/hMKT(1-332) contains a DNA sequence shown in SEQ ID NO:14.

The above transformant was cultured in 60 ml of LB medium containing 50 μg/ml of ampicillin and 12.5 μg/ml of tetracycline overnight at 30°C, and the culture (25 ml) was then added to 1000 ml of LB medium containing 50 μg/ml of ampicillin followed by shaking culture at 36°C until the $OD_{600}$ reaches 1.0 to 1.2. Then, after about 330 ml of LB medium at 65°C was added to the culture so that the final temperature of the culture became 42°C, the shaking culture was continued for further 3 hours at 42°C in order to induce expression of the variant human TPO, hMKT(1-332) protein - also referred to as [$Met^{-2}$,$Lys^{-1}$]TPO(1-332) -.

The obtained culture was directly subjected to SDS-PAGE analysis. In the SDS-PAGE analysis, Multigel 15/25 (Dai-ichi Chemical Co.) was employed. After electrophoresis and Coomassie Blue staining, the protein specific for the induction of expression was detected at a molecular weight of about 35 KD on the gel with regard to the transformant that the expression of the hMKT(1-332) protein was induced. In addition, following SDS-PAGE and electroblotting (using nitrocellulose membrane), the above expressed protein was reacted with the anti-HT1 peptide antibody prepared in Example 45. After staining, a band was detected at a molecular weight of approximately 35 kD thus indicating that the hMKT(1-332) was expressed.

〈Example 67〉

Preparation of human TPO substitution derivatives, their expression in COS7 cells, and identification of their activity

According to the following procedures, whether several derivatives that the amino acids of human TPO were partially substituted with an other amino acid(s) have TPO activity was studied.

The vector pSMT201 for expression in animal cells, for use in this Example, was constructed as follows.

First, the expression plasmid pXM-mEPORn which contains a cDNA for murine erythropoietin receptor (obtained from Dr. D'Andrea in Dana Farbor Cancer Institute; Cell: 57, 277-285 (1989)) was treated with restriction enzymes KpnI and EcoRI, and then electrophoresed on agarose gel to recover a pXM vector fragment wherein the cDNA for murine erythropoietin receptor was removed. Next, two oligonucleotides for introducing various cloning restriction sites into the above expression vector were synthesized using a DNA synthesizer (ABI). The oligonucleotides synthesized have the following basic sequences:

primer-1:

5'-CCTCGAGGAATTCCTGCAGCCCGGGACTAGTATCGGCTACCCCTACGACGTCCCCGACTACG
CCGGCGTCCATCACCATCACCATCACTGAGCGGCCGCC-3' (SEQ ID NO:158)

primer-2:

5'-AATTGGCGGCCGCTCAGTGATGGTGATGGTGATGAGCGCCGGCGTAGTCGGGGACGTCGTAG
GGGTAGCCGATACTAGTCCCGGGCTGCAGGAATTCCTCGAGGGTAC-3' (SEQ ID NO:159)

The two oligonucleotides were mixed and annealed to form a double stranded oligonucleotide which was thereafter ligated with the pXM vector recovered above in the presence of T4 DNA ligase (Takara-Shuzo) to give an expression vector pDMT201. To remove the murine DHFR cDNA contained in pDMT201 from the pDMT201, the vectors pDMT201 and pEF18S were separately digested with NotI and HpaI followed by agarose gel electrophoresis to recover a larger fragment from the pDMT201 vector DNA and a smaller fragment from the pEF18S vector DNA. Then, these fragments were ligated together using T4 DNA ligase (Takara-Shuzo) to produce an expression vector pSMT201. The pSMT201, as shown in Fig. 21, has an origin of replication of SV40, an enhancer sequence, adenovirus major late promoter sequence, an adenovirus tripatite leader sequence, a splice signal sequence, an SV 40 early polyadenylation site sequence, an adenovirus VA RNA gene sequence, an origin of replication of pUC18 and a $\beta$-lactamase gene ($Amp^r$), together with the following restriction sites for ligation of a desired gene: BglII, PstI, KpnI, XhoI, EcoRI, SmaI, SpeI and NotI sites. The pHTP illustrated in Example 30 carrying the full length human TPO cDNA was digested with EcoRI and SpeI to give a full length human TPO cDNA fragment which was subsequently subcloned into a vector pSMT201 predigested similarly to produce a plasmid pSMT201-hTPO.

Using the thus obtained plasmid pSMT201-hTPO as a template, the plasmid $\beta$GL-TPO/pBlue which was employed as a template for the preparation of derivatives of interest was firstly constructed as follows.

In the $\beta$GL-TPO/pBlue the addition of the rabbit $\beta$-globin leader sequence to the 5'-non-translational site of human TPO was attempted by the Annweiler's method (Annweiler et al., Nucleic Acids Research, 19: 3750, 1991). For this purpose, the following two DNA strands which were chemically synthesized:

5'-AATTCCAAGATCTCACACTTGCTTTTGACACAAC
TGTGTTTACTTGCAATCCCCCAAAACAGACAGACCC-3' (SEQ ID NO:160); and

5'-GGGTCTGTCTGTTTTGGGGGATTGCAAGTAAACA
CAGTTGTGTCAAAAGCAAGTGTGAGATCTTGG-3' (SEQ ID NO:161),

were ligated with a fragment which had been obtained by digesting the vector Bluescript II SK+ (Toyoboseki) with EcoRI and SmaI to produce a vector $\beta$GL/pBlue in which the leader sequence was inserted.

PCR was conducted using the following primer sequences:
Sma-ATG: 5'-GGCCCGGGATGGAGCTGACTGAATTGCTC-3' (SEQ ID NO:162) (i.e., 102-122 sequence of SEQ ID NO: 7 to which SmaI sequence has been linked); and
end: 5'-TCAAGCTTACTAGTCCCTTCCTGAGACAGATTCTG-3' (SEQ ID NO:163) (i.e., antisense of the 1140-1160 sequence of SEQ ID NO:7, to which SpeI and HindIII sequences had been linked).

The PCR was carried out using 1 ng of the plasmid pSMT201-hTPO DNA as a template together with 10 $\mu$M the synthetic primers. Using TaKaRa PCR Amplification Kit (Takara-Shuzo) and Programmable Thermal Controller (MJ Research), the PCR was conducted in a volume of 100 $\mu$l under the following conditions: 3 cycles of 94°C 1 min, 55°C 2 min and 72°C 2 min/cycle; then 20 cycles of 94°C 45 sec, 55°C 1 min and 72°C 1 min/cycle. The PCR product was extracted with chloroform and then ethanol-precipitated twice followed by suspension in 100 $\mu$l of TE buffer.

Subsequently, the PCR product was subjected to the restriction digestion with SmaI and HindIII, extracted with phenol/chloroform, and precipitated with ethanol. The resultant precipitate was dissolved in 10 $\mu$l of TE buffer, after which it was subcloned into the vector $\beta$GL/pBlue which was previously digested with the same restriction enzymes (Competent High E. coli JM109 (Toyo-boseki) was used as a host). Of the transformant cells obtained, twenty clones were selected from which plasmid DNA's were prepared essentially as described in Sambook et al., *Molecular Cloning*, Cold Spring Harbor Laboratory Press (1989). The purified plasmid DNA was identified through sequencing using Taq Dye Deoxy™ Terminater Cycle Sequencing Kit (Applied Biosystems, Inc.) and 373A DNA sequencer (Applied Biosystems, Inc.). As a result, it was confirmed that the plasmid encoding the $\beta$GL-TPO/pBlue had the expected TPO cDNA sequence without any substitution over the full length thereof. Then, after the $\beta$GL-TPO/pBlue was digested with BglII and SpeI, the digest was extracted with phenol/chloroform and then ethanol-precipitated. The precipitate obtained was dissolved in 10 $\mu$l of TE buffer, and it was subcloned into the vector pSMT201 which has been digested with the same enzymes (Compitent High E. coli JM109 (Toyo-boseki) was used as a host.). In accordance with the method described in the *Molecular Cloning* (Sambook et al., supra), the plasmid DNA was prepared from the transformant cells. The resulting expression vector pSMT/$\beta$GL-TPO had the structure that the $\beta$-globin leader sequence and the human TPO cDNA were linked at the BglII/SpeI restriction site downstream of the sprice signal sequence of the pSMT201 vector as shown in Fig. 21. This pSMT/$\beta$GL-TPO vector was employed for its transfection into COS cells.

For the preparation of human TPO substitution derivatives, PCR was applied in which the Ito's method was employed (Ito et al., Gene, 102:67-70, 1991). In particular, two derivatives of human TPO that the Arg-25 and His-33 of the human TPO are substituted by Asn and Thr respectively were illustratively prepared. Respectively, these derivaticves can be referred to as [$Asn^{25}$]TPO and [$Thr^{33}$]TPO. In PCR the following primers were employed:
T7: 5'-TAATACGACTCACTATAGGGCG-3' (SEQ ID NO:164) (corresponding to T7 promoter region of Bluescript II SK+);
ΔBglII: 5'-AATTCCAAGATCACACACTTGC-3' (SEQ ID NO:165) (for substitution of BglII recognition sequence);
end: 5'-TCAAGCTTACTAGTCCCTTCCTGAGACAGATTCTG-3' (SEQ ID NO:166) (antisense of the 1140-1160 of SEQ ID NO:7, to which SpeI and HindIII sequences have been linked);
N3: 5'-TGGGCACTGGCTCAGGTTGCTGTGAAGGACATGGG-3' (SEQ ID NO:167) (Arg 25 -> Asn of SEQ ID NO:7); and
O9: 5'-TGTAGGCAAAGGGGTAACCTCTGGGCA-3' (SEQ ID NO:168) (His-33 -> Thr of SEQ ID NO:7).

The first step PCR was carried out using 1 ng of the plasmid $\beta$GL-TPO/pBlue DNA as a template together with 10 $\mu$g each of the synthetic primers. The combinations of the primers were: [1] ΔBglII and "end" primers; [2] T7 and N3; and [3] T7 and O9. Using TaKaRa PCR Amplification Kit (Takara-Shuzo) and Programmable Thermal Controller (MJ Research), the PCR was conducted in a volume of 100 $\mu$l under the following conditions: 3 cycles of 94°C 1 min, 55°C 2 min and 72°C 2 min/cycle; then 17 cycles of 94°C 45 sec, 55°C 1 min and 72°C 1 min/cycle. Each of the PCR products was extracted with chloroform and precipitated twice with ethanol, and the precipitate was dissolved in 100 $\mu$l of TE buffer. The resulting PCR products (1 $\mu$l each) were subsequently subjected to a second PCR. The combinations of the templates were: PCR products of [1]/[2] and PCR products of [1]/[3]. With regard to the primers; the combination of T7 and end was employed in two cases. After incubation at 94°C for 10 min and addition of TaKaRa Taq, the PCR was carried out under the following conditions: 3 cycles of 94°C 1 min, 55°C 2 min and 72°C 2 min/cycle; then 9 cycles of 94°C 45 sec, 55 °C 1 min and 72°C 1 min/cycle. To each of the resulting PCR products were added 1 $\mu$l of proteinase K (5 mg/ml), 2 $\mu$l of 0.5 M EDTA and 2 $\mu$l of 20% SDS, incubated at 37°C for 30 min to inactivate Taq, extracted with phenol/chloroform and ethanol-precipitated. Thereafter, the precipitate dissolved in 20 $\mu$l of sterile water was digested with BglII and SpeI, followed by

phenol/chloroform extraction and ethanol-precipitation. The precipitate thus obtained was dissolved in 10 μl of TE buffer, and subsequently subcloned into the expression vector pSMT201 which has been previously digested with the same restriction enzymes and treated with calf intestine alkaline phosphatase (Böhringer-Mannheim) (Competent•High E. coli: JM109 was employed as a host.). Of the transformant cells obtained, in each case two clones were selected from which the plasmid DNAs were prepared essentially as described in the *Molecular Cloning* (Sambook et al., supra). The purified plasmid DNAs were sequenced using 373A DNA sequencer and Taq Dye Deoxy™ Terminater Cycle Sequencing Kit (both, Applied Biosystems, Inc.).

The plasmid prepared by PCR with the use of the primers [1] and [3] contained a cDNA encoding a TPO substitution derivative (O9/TPO). The amino acid substitution of His33(CAC) by Thr(ACC) and the extension of the original C-terminus (Gly 332) by the addition of the amino acid sequence, TSIGYPYDVPDYAGVHHHHHH (SEQ ID NO:169), were confirmed. This derivative was referred to as [$Thr^{33}$, $Thr^{333}$, $Ser^{334}$, $Ile^{335}$, $Gly^{336}$, $Tyr^{337}$, $Pro^{338}$, $Tyr^{339}$, $Asp^{340}$, $Val^{341}$, $Pro^{342}$, $Asp^{343}$, $Tyr^{344}$, $Ala^{345}$, $Gly^{346}$, $Val^{347}$, $His^{348}$, $His^{349}$, $His^{350}$, $His^{351}$, $His^{352}$, $His^{353}$]TPO.

On the other hand, the plasmid prepared by PCR with the use of the primers [1] and [2] contained a cDNA encoding a TPO substitution derivative (N3/TPO). The amino acid substitutions of Arg25(AGA) by Asn(AAC) and Glu231(GAA) by Lys (AAA) and the extension of the original C-terminus(Gly332) by the addition of the amino acid sequence, TSIGYPYDVPDYAGVHHHHHH, were confirmed. This derivative was referred to as [$Asn^{25}$, $Lys^{231}$, $Thr^{333}$, $Ser^{334}$, $Ile^{335}$, $Gly^{336}$, $Tyr^{337}$, $Pro^{338}$, $Tyr^{339}$, $Asp^{340}$, $Val^{341}$, $Pro^{342}$, $Asp^{343}$, $Tyr^{344}$, $Ala^{345}$, $Gly^{346}$, $Val^{347}$, $His^{348}$, $His^{349}$, $His^{350}$, $His^{351}$, $His^{352}$, $His^{353}$]TPO.

The transfection into COS7 cells of each clone obtained was carried out by the DEAE-dextran method including chloroquine treatment as described in Example 35. Briefly, 40 μg each of the plasmid DNA's were used in transfection, and, after 5 days, the culture supernatant was recovered which were subsequently concentrated to a volume of 1/20 using Centricon-30 (Amicon) in order to evaluate their TPO activity by the M-O7e assay. As a result, in the culture supernatants of COS7 cells into which plasmids containing cDNAs encoding the human TPO substitution derivatives N3/TPO and O9/TPO were transfected respectively, the TPO activity was detected in a dose-dependent manner (see Fig. 22).

〈Example 68〉

Preparation of insertion or deletion derivatives of human TPO, expression thereof in COS7 cells and identification of TPO activity

This Example makes evident whether insertion or deletion derivatives of hTPO163 have a TPO activity.

Derivatives prepared were a His33-deletion derivative ([$\Delta His^{33}$]TPO(1-163)); a Gly116-deletion derivative ([$\Delta Gly^{116}$]TPO(1-163)); an Arg117-deletion derivative ([$\Delta Arg^{117}$]TPO(1-163)); a Thr-insertion derivative (-[$His^{33}$, $Thr^{33'}$, $Pro^{34}$]TPO(1-163)), an Ala-insertion derivative ([$His^{33}$, $Ala^{33'}$, $Pro^{34}$]TPO(1-163)) and a Gly-insertion derivative ([$His^{33}$, $Gly^{33'}$, $Pro^{34}$, $Ser^{38}$]TPO(1-163)) in which Thr, Ala and Gly have been independently inserted between His33 and Pro34; and an Asn-insertion derivative ([$Gly^{116}$, $Asn^{116'}$, $Arg^{117}$]TPO(1-163)), an Ala-insertion derivative ([$Gly^{116}$, $Ala^{116'}$, $Arg^{117}$]TPO(1-163)) and a Gly-insertion derivative ([$Gly^{116}$, $Gly^{116'}$, $Arg^{117}$]TPO(1-163)) in which Asn, Ala and Gly have been independently inserted between Gly116 and Arg117, all the sequences being based on SEQ ID NO:13.

Among the derivatives, T33' and N116' are intended to introduce mucin type and Asn-binding type sugar chains, respectively.

The above mentioned derivatives were prepared using PCR by the method described by Ito et al (Gene, 102: 67-70, 1991). Primer sequences used in PCR are as follows:

dH33: 5'-TGTAGGCAAAGGAACCTCTGGGCA-3' (SEQ ID NO:172) (for the preparation of His33-deletion derivative based on the sequence shown in SEQ ID NO:7);

T33': 5'-TGTAGGCAAAGGAGTGTGAACCTCTGG-3' (SEQ ID NO:173) (for the preparation of Thr-insertion derivative with Thr inserted between His33 and Pro34 in the sequence shown in SEQ ID NO:7);

A33': 5'-TGTAGGCAAAGGAGCGTGAACCTCTGG-3' (SEQ ID NO:174) (for the preparation of Ala-insertion derivative with Ala inserted between His33 and Pro34 in the sequence shown in SEQ ID NO:7);

G33': 5'-TGTAGGCAAAGGTCCGTGAACCTCTGG-3' (SEQ ID NO:175) (for the preparation of Gly-insertion derivative with Gly inserted between His33 and Pro34 in the sequence shown in SEQ ID NO:7);

dG116: 5'-AGCTGTGGTCCTCTGTGGAGGAAG-3' (SEQ ID NO:176) (for the preparation of Gly116-deletion derivative based on the sequence shown in SEQ ID NO:7);

dR117: 5'-GTGAGCTGTGGTGCCCTGTGGAGG-3' (SEQ ID NO:177) (for the preparation of Arg117-deletion derivative based on the sequence shown in SEQ ID NO:7);

N116': 5'-AGCTGTGGTCCTGTTGCCCTGTGGAGG-3' (SEQ ID NO:178) (for the preparation of Asn-insertion derivative with Asn inserted between Gly116 and Arg117 in the sequence shown in SEQ ID NO:7);
A116': 5'-AGCTGTGGTCCTAGCGCCCTGTGGAGG-3' (SEQ ID NO:179) (for the preparation of Ala-insertion derivative with Ala inserted between Gly116 and Arg 117 in the sequence shown in SEQ ID NO:7);
G116': 5'-AGCTGTGGTCCTTCCGCCCTGTGGAGG-3' (SEQ ID NO:180) (for the preparation of Gly-insertion derivative with Gly inserted between Gly116 and Arg117 in the sequence shown in SEQ ID NO:7);
dRI: 5'-CGGGCTGCAGGATATCCAAGATCTCA-3' (SEQ ID NO:181) (for the substitution of a restriction enzyme EcoRI-recognition sequence);
T7: 5'-TAATACGACTCACTATAGGGCG-3' (SEQ ID NO:182) (corresponding to T7 promoter region of BluescriptIISK$^+$); and
endNotI: 5'-GGGCGGCCGCTCAGCTGGGGACAGCTGTGGTGGG-3' (SEQ ID NO:183) (antisense of the 633-653 nucleotide sequence of SEQ ID NO:7, to which a termination codon and a NotI recognition sequence have been added).

A first step PCR was carried out using, as a template, 1 ng of the plasmid $\beta$GL-TPO/pBlue DNA prepared in Example 67 and using 10 $\mu$M synthetic primers. The combination of the primers was: [1] dRI and endNotI or [2] T7 and mutated primers (dH33, A33', G33', T33', dG116, dR117, A116', G116' and N116'). In PCR, TaKaRa PCR Amplification Kit (Takara-Shuzo) and Programmable Thermal Controller (MJ Research) were employed, and the PCR was carried out in a final volume of 100 $\mu$l. In the first PCR reaction of [1], one cycle was 94°C 1 min, 45°C 2 min and 72°C 2 min and 3 cycles were conducted, followed by 17 cycles in which one cycle was 94°C 45 sec, 45°C 1 min and 72°C 1 min. On the other hand, the first PCR reaction of [2] was carried out under the conditions of 94°C 1 min, 55°C 2 min and 72°C 2 min for 3 cycles followed by 94°C 45 sec, 55°C 1 min and 72°C 1 min for 17 cycles. Each of the PCR products was extracted with chloroform before twice ethanol precipitation and then the resultant precipitate was dissolved in 100 $\mu$l of TE buffer.

Next, a second step of the PCR was conducted using 1 $\mu$l of each of the PCR products of [1] and [2]. The primers used were a combination of T7, and endNotI. After incubation at 94°C for 10 min, to each PCR reaction mixture was added TaKaRa Taq. The second PCR was carried out under the conditions of 94°C 1 min, 55°C 2 min and 72°C 2 min for 3 cycles followed by 94°C 45 sec, 55°C 1 min and 72°C 1 min for 9 cycles. To each PCR product thus obtained were added 1 $\mu$l of 5 mg/ml proteinase K, 2 $\mu$l of 0.5 M EDTA and 2 $\mu$l of 20% SDS, and the mixture was kept at a temperature of 37°C for 30 min to inactivate Taq. The PCR product was extracted with phenol/chloroform followed by ethanol-precipitation, and the resultant precipitate was then dissolved in 20 $\mu$l of sterile water. After digested with EcoRI and NotI, the solution was extracted with phenol/chloroform and then precipitated with ethanol. The obtained precipitate was dissolved in 10 $\mu$l of TE buffer, and subcloned into an expression vector pEF18S that has been previously digested with the same restriction enzymes and treated with alkaline phosphatase (from calf intestine, Boehringer-Mannheim). A host cell employed was Competent-High E. coli JM109 (Toyo-Boseki). From the transformant thus obtained, a plasmid DNA was prepared essentially by the method described in *Molecular Cloning* - (Sambook et al., Cold Spring Harbor Laboratory Press (1989)). Thereafter, the nucleotide sequence of the purified plasmid DNA was confirmed using Taq Dye Deoxy™ Terminater Cycle Sequencing Kit (Applied Biosystems) and using an Applied Biosystems 373A DNA sequencer. As a result, it was confirmed that all the plasmids encoding dH33, A33', T33', dG116, dR117, A116', G116' and N116' had the expected TPO cDNA sequence without any substitution of a nucleotide sequence at sites other than the intended sites. Moreover, in G33' a base substitution [Pro38 (CCT)-> Ser (TCT)] other than that at the intended site was observed.

The transfection of each clone obtained into COS7 cells was conducted in accordance with the method in Example 11. Briefly, the transfection was carried out using 10 $\mu$g of each plasmid DNA by the DEAE-dextran method including chloroquine treatment, and after 4 to 5 days, the culture supernatant was recovered which was then evaluated by M-07e assay system. As a result, in each supernatant of the COS7 cell cultures transfected with plasmids encoding an amino acid insertion or deletion derivative, the TPO activity was detected in a dose-dependent manner (see Fig. 23). On the contrary, no TPO activity was found in the supernatant of the COS7 cell culture that the expression plasmid pEF18S not containing a cDNA encoding a TPO derivative has been transfected into and expressed.

⟨Example 69⟩

Preparation and purification of anti-human TPO peptide antibodies

(1) Six regions (shown in Table 8) which are considered relatively suitable as antigens were selected from the amino acid sequence of human TPO of SEQ ID NO: 191, from which a four-stranded peptide of a multiple antigen peptide (MAP) type was synthesized by Tam's method (*Proc. Natl. Acad. Sci., USA,* *85*, 5409-5413, 1988). Two rabbits were immunized eight times each with 100 μg of this peptide.

Apart from the above, single-stranded peptides were produced by bonding cysteine residue to the C-terminal of each of the peptide regions shown by SEQ ID NOS: 119-124 (these are referred to as peptide region HT 1 to 6 regions, respectively, which are partial peptides corresponding to 8 to 28, 47 to 62, 108 to 126, 172 to 190, 262 to 284, and 306 to 332, respectively, of the sequence of SEQ ID NO: 191). Using these as test antigens, the antibody values thereto in the sera obtained from the thus-immunized rabbits were measured by enzyme-immunoassay, from which the increase in the antibody value was confirmed in all the sera tested. Thus, these sera were referred to as anti-sera.

In addition, the above-mentioned single-stranded synthetic peptide having the cysteine residue bonded to its C-terminal was used also as the antigen for the affinity purification of the anti-sera in the following (2).

(2) Two rabbits each were immunized with one of the above-mentioned antigen peptides, and the antibody derived from each of all the thus-immunized rabbits was prepared separately. Concretely, for anti-HT1 peptide antibodies obtained were anti-HT1-1 peptide antibody and anti-HT1-2 peptide antibody derived from the two immunized rabbits.

As one example the purification of anti-HT1-1 peptide antibody is illustrated hereunder.

First, 30 mg of the single-stranded peptide of HT1 having cysteine residue bonded thereto were coupled onto 12 ml of Sulfo Link Coupling Gel (produced by Pierce Co., as catalog No. 44895). Precisely, a peptide solution containing the antigen was coupled onto the gel that had been equilibrated with a coupling buffer (50 mM Tris, 5 mM EDTA-Na, pH 8.5) of 6 times by volume the gel, over a period of 15 minutes. Next, after having been left to statically stand as it was for 30 minutes, the gel was washed with the coupling buffer of 3 times by volume the gel. Next, the coupling buffer containing 0.05 M L-cysteine-HCl was added to the gel at a rate of 1 ml/ml-gel, by which the non-treated groups were blocked over a period of 15 minutes. After having been left to statically stand as it was for 30 minutes, the gel was washed with the coupling buffer of 8 times by volume the gel. The above-mentioned coupling operation was conducted at room temperature. In this way, the antigen region-containing peptide was coupled onto the gel by covalent bonding, at a coupling efficiency of 28.3 %, to prepare an antigen peptide gel having 0.8 mg of the peptide coupled onto 1 ml of the gel, in the antigen column. After collecting all blood from each immunized rabbit, 78.4 ml of anti-serum containing anti-HT1-1 peptide antibody were obtained. 76.7 ml of the anti-serum (having a protein content of 3620 mg) were added to the antigen column that had previously been equilibrated with 50 mM phosphate buffer (pH 8) containing 150 mM of NaCl and 0.05 % of sodium azide, then the column was washed with the same buffer. Thus, 105.9 ml of a passing fraction (having a protein content of 3680 mg) were obtained. Next, the fraction adsorbed was eluted with 0.1 M citric acid buffer (pH 3.0), which was then immediately neutralized by adding thereto 21.1 ml of 0.1 M carbonate buffer (pH 9.9), concentrated by ultrafiltration (using YM30 membrane produced by Amicon Co.) and purified in 50 mM phosphate buffer (pH 8) containing 150 mM of NaCl and 0.05 % sodium azide. Thus, 11.2 ml of a purified anti-HT1-1 peptide antibody (having a protein content of 77.7 mg) were obtained in the buffer. In the same manner as above, anti-HT1-2 peptide antibody (60.0 mg), anti-HT2-1 peptide antibody (18.8 mg), anti-HT2-2 peptide antibody (8.2 mg), etc. were obtained.

Anti-HT3 to anti-HT6 peptide antibodies are obtained in the same manner as above.

⟨Example 70⟩

Detection of TPO Western Analysis

(1) To evaluate the anti-sera obtained in Example 69, these were tested in the manner mentioned below.

A standard sample of recombinant human TPO that had been partially purified from the supernatant of the culture of CHO cells, into which a gene coding for the amino acids -21 to 332 of SEQ ID NO:6 had been introduced and expressed, was subjected to SDS-polyacrylamide electrophoresis (SDS-PAGE) according to an ordinary method and then electrically blotted on a PVDF or nitrocellulose membrane. After the blotting, the membrane was washed with 20 mM Tris-HCl and 0.5 M NaCl (pH 7.5) (TBS) for 5 minutes, then washed two times each with 0.1 % Tween 20-containing TBS (TTBS) for 5 minutes, and treated with a blocking agent (Block Ace; produced by Dai-Nippon Pharmaceutical Co., as catalog No. UK-B25) for 60 minutes. The anti-sera each separately containing one of anti-HT1-1 peptide antibody, anti-HT2-1 peptide antibody, anti-HT3-2 peptide antibody, anti-HT4-1 peptide antibody, anti-HT5-2 peptide antibody and anti-HT6-1 peptide antibody each were diluted with TTBS solution containing 0.05 % BSA and 10 % Block Ace to 1/1000 dilutions. The thus-diluted antibodies were employed for Western assay as the primary antibodies. Precisely, the recombinant human TPO sample processed as above was treated with TTBS solution containing the anti-TPO peptide antibody, 0.05 % BSA and 10 % Block Ace for 60 minutes and then bashed with TTBS for 5 minutes. Next, this was treated with TTBS solution containing anti-rabbit(ab') goat biotinated antibody (produced by Caltag Co., as catalog No. L43015), as the secondary antibody, along with 0.05 % BSA and 10 % Block Ace, for 60 minutes, and then washed two times each with TTBS for 5 minutes. Next, this was treated with a 1/5000 dilution of alkaline phosphatase-labeled avidin (produced by Leinco Technologies Co., as catalog No. A108) diluted with 10 % Block Ace-containing TTBS solution, for 30 minutes, and thereafter washed two times each with TTBS for 5 minutes and then with TBS for 5 minutes. Then, this was developed, using an alkaline phosphatase substrate (produced by Bio-Rad Co., as catalog No. 170-6432). The above-mentioned Western assay was conducted at room temperature, which verified that the human TPO was recognized and detected by the anti-sera.

(2) 3 mg of each of anti-HT1-1 peptide antibody, anti-HT1-2 peptide antibody, anti-HT2-1 peptide antibody and anti-HT2-2 peptide antibody that had been purified by affinity chromatography in Example 69 were weighed and biotinated by coupling them onto an activated biotin (NHS-LC-Biotin II, produced by Pierce Co., as catalog No. 21336). The same standard sample of recombinant human TPO as that used in the foregoing (1) was subjected to SDS-PAGE, then electrically blotted on a PVDF or nitrocellulose membrane, and subjected to ordinary Western assay using each of these biotinated antibodies as the primary antibodies. Immediately after the blotting, the membrane was washed with TBS for 5 minutes and then washed two times each with TTBS for 5 minutes, and thereafter this was treated with a blocking agent (Block Ace) for 60 minutes. Next, this was treated with TTBS solution containing 1 μg/ml of the biotinated anti-TPO peptide antibody, 0.05 % BSA and 10 % Block Ace for 60 minutes and then washed two times each with TTBS for 5 minutes. Next, this was treated with a 1/5000 dilution of alkaline phosphatase-labeled avidin (produced by Leinco Technologies Co., as catalog No. A108) diluted with 10 % Block Ace-containing TTBS solution, for 30 minutes, and thereafter washed two times each with TTBS for 5 minutes and then with TBS for 5 minutes. Then, this was developed, using an alkaline phosphatase substrate (produced by Bio-Rad Co., as catalog No. 170-6432). The above-mentioned Western assay was conducted at room temperature which verified that the human TPO was recognized and detected by the purified antibodies.

⟨Example 71⟩

<u>Preparation of anti-TPO peptide antibody columns and detection of human TPO</u>

The anti-human TPO peptide antibodies obtained in Example 69 were verified to recognize human TPO. These antibodies were separately coupled onto a chromatography carrier to prepare anti-TPO peptide antibody columns. As one example, the preparation of anti-HT1-2 peptide antibody column is illustrated hereinafter.

(1) A solution comprising 50 mM Na phosphate and 0.15 M NaCl (pH 8.0) and containing 5 mg/ml of the antibody was prepared. 0.8 ml of this solution were combined and coupled with 1.54 ml of a swollen formyl-activated gel (Formyl-Gellulofine, produced by Chisso Co.) at 4°C for 2 hours. Next, 1.1 ml of a solution containing 10 mg/liter of a reducing agent (trimethylamine borane (TMAB), produced by Seikagaku Kogyo K.K., as catalog No. 680246) were added thereto and coupled with them for further 4 hours. Then, only the gel part was recovered from the reaction mixture by centrifugation. 10 ml of pure water were added thereto and, only the gel part was recovered from this by centrifugation. This process was repeated four times, by which the non-reacted antibody was removed. Next, the thus-recovered gel was treated with 2.1 ml of a blocking buffer (0.2 M Na phosphate, 1 M ethanolamine, pH 7.0) and 1.1 ml of the solution of reducing agent that had been added thereto, at 4°C for 2 hours, by which the active groups in the non-reacted gel were blocked. Last, the resulting gel was washed with water; 20 mM Tris-HCl and 0.15 M NaCl (pH 8.0), using a centrifuge. This was filled in a small column tube, which was washed with 3 M sodium thiocyanate solution and 0.1 M glycine-HCl (pH 2.5) solution and then again equilibrated with 20 mM Tris-HCl and 0.15 M NaCl (pH 8.0). The thus-equilibrated column was stored.

The anti-TPO peptide antibody gel in the anti-HT1-2 antibody column had a coupling efficiency of up to 94.2 % for each IgG fraction. The amount of the IgG fraction coupled onto the gel per the unit volume of the gel was 1.9 mg/ml-gel. In the same manner as above, a gel was prepared, onto which 21.8 mg, per ml of the gel, of IgG not having TPO as the antigen had been coupled. This was used as a precolumn (hereinafter referred to as a pre-antibody column) for the purpose of removing the non-specifically-bonding molecules from TPO antibody columns, as is mentioned in (2).

(2) Next, another example of preparing an anti-HT1-2 antibody column is illustrated hereinafter.

A standard sample of recombinant human TPO that had been partially purified from the supernatant of the culture of CHO cells into which a gene coding for the amino acid sequence of any of SEQ ID NOS: 119-124 had been introduced and expressed was added to the pre-antibody column (containing 1 ml of gel) prepared in (1), and 10 times by volume the gel of 20 mM Tris-HCl (pH 8.0) and 0.15 M NaCl were passed through the column, whereupon the fraction passing through the column was collected. Next, the fraction adsorbed to the pre-antibody column was eluted with 10 times by volume the gel of an acidic eluent (0.1 M glycine-HCl, pH 2.5). Next, the fraction that had passed through the pre-antibody column was added to the anti-HT1-2 antibody column (containing 2 ml of gel) prepared in (1), and the column was washed with 10 times by volume the gel of 20 mM Tris-HCl (pH 8.0) and 0.15 M NaCl, while collecting the fraction passing through the anti-HT1-2 antibody column. Next, the fraction adsorbed to the anti-HT1-2 antibody column was eluted with 8 times by volume the gel of an acidic eluent (0.1 M glycine-HCl, pH 2.5). These fractions were analyzed by SDS-PAGE, which verified that TPO did not adsorb to the pre-antibody column but was specifically bonded to the anti-HT1-2 antibody column and that almost all TPO in the sample added bonded the latter column. From this, it was confirmed that at least from 200 to 300 µg/ml-gel of TPO was bonded to the gel in the latter column.

⟨Example 72⟩

Effect of TPO for Increasing the Numbers of Platelets

Twenty normal ICR strain male mice, eight weeks of age, (blood was collected from their orbit veins already and the numbers of platelets was measured using a micro cell counter F-800 manufactured by Toa Iyo Denshi) were randomly divided into four groups. One of the four groups (a control-iv group) was injected with 100 microliters of PBS intravenously route once daily for five consecutive days. One of the other groups (TPO-iv group) was injected with 100 microliters of a solution (prepared by substituting the concentrated solution of the TPO active fraction F2 of the SP Sepharose Fast Flow obtained in Example 56 with PSB using an NAP-25 column [Pharmacia Biotech; Catalog No. 17-0852-02] followed by diluting with PBS;containing relative activity, 211,900 by M-07e assay) intravenously once daily for five consecutive days. Another group (control-sc group) was injected with 100 microliters of PBS subcutaneously once daily for five consecutive days while the remaining group (TPO-sc group) was injected with 100 microliters once daily for five consecutive days in the same manner as in the TPO-iv group subcutaneously. After 6, 8, 10, 13 and 15 days from the initiation of the administrations, blood was collected from the orbit vein of each of the mice and the numbers of platelets was counted using a micro cell counter (F-890; manufactured by Toa Iyo Denshi). The changes in the numbers of platelets are given in Fig. 2A. Thus, in the control-iv group, the platelet number increased by 44% as compared with before the administration even on the Day 6 (Day 0 being the day of administration) where the platelet numbers were highest and, in the control-sc group, only a 47% increase was noted even on Day 8 where the numbers were highest. On the other hand, in the TPO-iv group, a 177% increase was noted on Day 6 as compared with the numbers of prior to the administration and, in the TPO-sc group, an increase of 347% was noted already on Day 6 and, on Day 8, the highest increase of 493% was achieved.

From the above results, it has been confirmed that human TPO increases the number of platelets independently of the difference in the type and also of the route of administration.

⟨Example 73⟩

Effect of TPO for Increasing the Number of Platelets

Sixteen normal C3H/HeJ strain male mice, 11 weeks of age, (the blood was already collected from their orbit veins and the number of platelets were measured using a microcell counter type F-800 manufactured by Toa Iyo Denshi) were randomly divided into four groups. One of the four groups (a control group) was injected with 100 microliters of PBS subcutaneously once daily for five consecutive days. Another group (TPO-1 group) was injected subcutaneously with 100 microliters of a solution (prepared by substituting the TPO active fraction of Sepha cryl S-200HR obtained in Example 56 with PBS using NAP-25 followed by diluting with PBS; containing relative activity, 83,000 by the M-07e assay) once daily for five consecutive days. Still another group (TPO-2 group) was injected with 100 microliters of a solution (prepared by diluting the TPO active fraction used in the TPO-1 group with PBS to an extent of 2-fold; containing relative activity, 41,500 by the M-07e assay) subcutaneously once daily for five consecutive days. The last group (TPO-3 group) was injected with 100 microliters of a solution (prepared by diluting the TPO active fraction used in the TPO-2 group to an extent of 2-fold more; containing relative activity, 20,750 by the M-07e assay) subcutaneously once daily for five consecutive days.

Blood was collected from the orbit veins of the mice on Day 6, 8, 10 and 12 after injection and the numbers of platelets were counted using a microcell counter (type F-800; manufactured by Toa Iyo Denshi).

The changes in the numbers of platelets are given in Fig. 25. Thus, in the control group, there was nearly no change in the numbers of platelets while, in the groups to which TPO was injected, there were increases in the numbers of platelets in all of the groups to the extent that the numbers were highest on Day 8 after the injection. In addition, differences in dose-response were noted among the groups. Thus, in the TPO-1 group, about a 200-% increase was noted on Day 6 already, the increase rose to about 270% on Day 8 and then, however, a decrease was noted, even on Day 12, about a 65-% increase was still noted whereupon there was a significant difference from the control group ($p < 0.01$; by a Dunnet multiple comparison test). In the TPO-2 group, the same increase was found and, though the increasing action was smaller than the TPO-1 group, increases of about 140% and about 160% were noted on Days 6 and 8, respectively. On Day 12, the numbers decreased to nearly the same level as the control group. The increasing action of the TPO-3 group was weaker than the TPO-2 group and, on Day 8 where the numbers were highest, about a 110-% increase was noted and, again, there was a significant difference ($p < 0.01$) from the control group.

From the above-mentioned results, it has been confirmed that the human TPO increases the platelet numbers independently of the strain of the mice and that its action has a dose-responding property.

⟨Example 74⟩

An Inhibiting Effect of TPO on the Thrombocytopenia Caused by Administration of Anti-Cancer Agent

Thirty ICR male mice, eight weeks of age, were injected with 200 mg/kg of 5-FU intravenously and randomly divided into two groups (each group comprising 15 mice). From the next day (Day 1), one of the groups (control group) was injected with 100 microliters of PBS subcutaneously once daily for five consecutive days while another group (TPO group) was injected with 100 microliters of TPO active fraction used in Example 72 (containing relative activity, 211,900 by the M-07e assay) subcutaneously once daily for five consecutive days. On Days 4, 6 and 8, each five mice were randomly chosen from each of the groups, then blood was collected from the orbit veins and the numbers of platelet were counted by a microcell counter (type E-2500; manufactured by Toa Iyo Denshi). The changes in the numbers of platelets are given in Fig. 26. Thus, in the control group, the numbers of platelets after the administration of 5-FU decreased as the days passed and, on Day 6, the value was lowest (about 28% of the value prior to the administration of 5-FU) though, on Day 8, an approximately 1.3-fold increase was noted as a reaction of the decrease. Even in the case of a TPO group, the numbers of platelets after the administration of 5-FU decreased as the days passed and, on Day 6, the value was the lowest (about 52 % of the value before the administration of 5-FU). However, the difference between the numbers of platelets on Days 4 and 6 was very small and, on Day 6, a significantly ($p < 0.05$; by a Dunnet multiple comparison test) high value as compared with the control group was maintained. On Day 8, there was an increase of about 200% of the value before the 5-FU administration.

From the above-mentioned result, it has been confirmed that human TPO inhibits the decrease of the platelet number as caused by the anti-cancer agent.

⟨Example 75⟩

A Therapeutic Effect of TPO for Thrombocytopenia

Nimustine hydrochloride (ACNU) (50 mg/kg) was injected intravenously to each of 36 ICR male mice, seven weeks of age, and then the mice were randomly divided into two groups (each group comprising 18 mice). As from the next day (Day 1), one of the groups (control group) was injected with 200 microliters of PBS subcutaneously twice daily for consecutive days while another group (TPO group) was injected with 200 microliters of the TPO active fraction used in Example 73 (without diluting with PBS) to which 0.04% of Tween 80 was added (containing relative activity, 380,000 by the M-07e assay) subcutaneously twice daily for consecutive days.

After the injection, the mice in each of the group were randomly divided into three groups - from one group blood was collected on Days 5 and 12; from another group on Days 8 and 14; and from the remaining group on Day 10. Blood was collected from the orbit veins and the numbers of platelet number were counted by a microcell counter (type F-800; manufactured by Toa Iyo Denshi). The changes in the numbers of platelets are given in Fig. 27. Thus, in the control group, the numbers of platelets after administration of ACNU decreased as the days passed and, on Days 8-10, the value was lowest (about 29% of the value before the ACNU administration) and the recovery was only about 49% and about 74% on Days 12 and 14, respectively. On the other hand, in the TPO group, a decrease to about 38% of the value before the ACNU administration was noted on Day 5 and then the situation changed to an increase. Thus, on Day 8, the value recovered to about 63% of the value before the ACNU administration and, on Day 10 and thereafter, the numbers of platelets were larger than that before the ACNU administration. Thus, on Days 12 and 14, increases of up to about 300% and about 400% were noted, respectively.

As noted hereinabove, on Days 8-10 of which the decrease was noted in the control group, an increase was observed in the TPO group already and, on Day 10, the platelet numbers were more than those before the administration of ACNU. Accordingly, it has been confirmed that human TPO promotes the recovery from the thrombocytopenia caused by the anti-cancer agent.

When the results of Example 74 are taken into consideration as well, it is now expected that human TPO inhibits the decrease in the numbers of platelets and promotes the recovery from thrombocytopenia independently of the type of the anti-cancer agent.

〈Example 76〉

A Therapeutic Effect of TPO on Thrombocytopenia after BMT

Forty-eight male mice of C3H/HeN strain, seven weeks of age, were irradiated with 10 Gy of radioactive radiation on the entire body and, immediately thereafter, they were intravenously injected with 1 x $10^6$ of bone marrow cells from the mice of the same strain. The mice were then randomly divided into two groups and, from the next day (Day 1), one of the groups (control group) was injected with the PBS while another group (TPO group) was injected with the TPO active fraction used in Example 73 (containing relative activity, 44,000 by the M-07e assay) with a dose of 100 microliters each subcutaneously once daily for twenty consecutive days.

After initiation of the injection, each six mice were randomly selected on Days 5, 10, 14 and 21, blood was collected from their orbit veins and the numbers of platelets were counted by a microcell counter (type E-2500; manufactured by Toa Iyo Denshi).

The changes in the numbers of platelets are given in Fig. 28. Thus, in all of the groups, the numbers of platelets decreased as the days passed after applying the BMT thereto and, on Day 10, the numbers were lowest (about 3% of the numbers of before the BMT application). After that, a gradual recovery was noted and, on Day 4, the numbers were about 11% and about 13% in the control group and in the TPO group, respectively. On Day 21, only a 37% recovery was noted in the control group while, in the TPO group, the recovery was about 65% whereupon a significant recovery promoting effect was observed. From the above-mentioned results, it has been confirmed that human TPO prepared as in Example 56 promote the recovery of the numbers of platelets after application of the BMT.

〈Example 77〉

A Therapeutic Effect of TPO for Thrombocytopenia after Irradiation with Radioactive Ray

Thirty-six male mice of ICR strain, eight weeks of age, were irradiated with x-rays of 5 Gy on the entire body and randomly divided into two groups. From the next day (Day 1), one of the groups (control group) was injected with PBS while another group (TPO group) was injected with a TPO active fraction used in Example 73 (containing relative activity, 1,440,000 by the M-07e assay) once daily for three consecutive days subcutaneously and, from the next day, relative activity, 360,000 by the M-07e assay was injected subcutaneously once daily for seven consecutive days. After initiation of the injection, each of the group was randomly divided into three groups - a group from which blood was collected on Days 4, 11 and 21;

another group on Days 7 and 13; and the remaining group on Days 9 and 15. The collection of blood was carried out from the orbit veins and the numbers of platelets were counted by a microcell counter (type F-800; manufactured by Toa Iyo Denshi). The changes in the numbers of platelets are given in Fig. 29. Thus, in the control group, the numbers of platelets decreased as the days passed after irradiation with x-rays and, on Day 9, the numbers were smallest (about 25% of the number before the irradiation with X-rays). On days 11 and 13, the numbers recovered to about 38% and about 67%, respectively and, on Day 15, they recovered to the values before the irradiation with x-rays. In the TPO group, the numbers decreased to about 24 % of those before the irradiation with X-rays on Day 7. After that, an increase was noted and, on Day 9, the numbers recovered to about 82%. On Day 11 and thereafter, the platelet numbers were more than those before irradiation with X-rays and, until Day 21, this tendency was maintained. As mentioned above, on Day 9 on which the numbers tended to decrease in the control group, the numbers of platelets in the TPO group already changed to an increase and, on Day 11, the numbers of platelets were more than those before the irradiation with X-rays while remaining high even thereafter. On the other hand, in the control group, fifteen days were needed for the numbers of platelets to recover from before the irradiation with X-rays. From those results, it has been confirmed that human TPO produced in Example 56 shortens the time necessary to recover from the decrease in the numbers of platelets after irradiation with X-rays and that it exhibits an therapeutic effect for thrombocytopenia after the irradiation with X-rays.

⟨Example 78⟩

Effect of hTPO163 for Increasing the Numbers of Platelets

Fifteen healthy male mice of C3H/HeN strain from whom blood was already collected from their orbit veins and subjected to the number of platelets was measured by a microcell counter (type F-800; manufactured by Toa Iyo Denshi) were randomly divided into four groups - a control group and groups A, B and C. The first group (control group) was injected subcutaneously with PBS containing 0.1% of serum from mouse once daily for five consecutive days. Groups A, B and C were injected with the TPO active fraction of SP Sepharose Fast Flow obtained in Example 65 followed by diluting with PBS containing 0.1% of mouse serum at the doses of about 40,000,000, about 8,000,000 and about 1,600,000/kg body weight/day, respectively, in terms of the relative activity of hTPO163 by the M-07e assay for five consecutive days subcutaneously.

On Days 6, 8, 10 and 12 after the injection, blood was collected from the orbit vein of each mouse and the numbers of platelets were counted by a microcell counter (type F-800; manufactured by Toa Iyo Denshi). The chances in the numbers of platelets are given in Fig. 30.

Thus, in the control group, there was nearly no change in the numbers of platelets while, in the TPO-given groups, increases in the numbers of platelets were noted in all cases giving the highest values on Day 8 after the injection. Among each of the TPO-given groups, a dose-response relationship was noted. Thus, in group A, increases of about 88% and about 100% were noted on Days 6 and 8, respectively and, even on Day 10, an increase of about 97% was maintained whereupon all data showed a significant difference from the control group ($p < 0.01$ by a Dunnet Multiple Comparison Test). Similar increases were noted in group B as well and, though the degree of increase was lower than that of group A, the increases of about 65% and about 84% were noted on Days 6 and 8, respectively showing a significant difference from the control group ($p < 0.01$ or 0.05 by a Dunnet Multiple Comparison Test). The increasing action in group C was lower than that in group B. Still it showed about 31% increase on Day 8 when the highest numbers were noted. From the above-mentioned results, it has been confirmed that the hTPO163 produced in Example 65 increases the numbers of platelets and that its action shows a dose-response relationship.

⟨Example 79⟩

A Therapeutic Effect of hTPO163 for Thrombocytopenia

One hundred male mice of C3H/HeN strain, eight weeks of age, were injected intravenously with 50 mg/kg of nimustine hydrochloride (ACNU) by and then randomly divided into four groups - a control group and groups A, B and C - each comprising 25 mice. As from the next day (Day 1), the control group was injected subcutaneously with 5 ml/kg of PBS once daily for consecutive days while groups A, B and C were injected subcutaneously with the TPO active fraction of the SP Sepharose Fast Flow obtained in Example 65 diluted with PBS at the doses of about 16,000,000, about 48,000,000 and about 160,000,000/kg body weight/day, respectively, in terms of the relative activity of the hTPO163 by the M-07e assay once daily for consecutive days.

After initiation of the injection, mice in each of the groups were randomly divided into five groups and the blood was collected on Days 5, 8, 10, 12 and 14. The collection of blood was carried out from their orbit veins and the numbers of platelets were counted by a microcell counter (type E-2500; manufactured by Toa Iyo Denshi). The changes in the numbers of platelets are given in Fig. 31. Thus, in the control group, the numbers of platelets decreased as the days passed after the administration of ACNU giving the lowest values (about 15-16% of that before the ACNU administration) on Days 8-10 and the extent of the recovery was only as little as about 28% and about 51% on Days 12 and 14, respectively. On the other hand, in group A, the numbers decreased to about 25% of those before the ACNU administration on Day 8. After that, however, they changed to increase and recovery to an extent of about 34% and about 89% were noted on Days 10 and 12, respectively. On Day 14, the number was larger than that of before the ACNU administration.

As mentioned above, the smallest numbers of platelets were noted on Day 8 in all of the groups though, in the groups to which human TPO was given, the decrease was slower than the control group giving rise to the smallest numbers of platelets. In the control group, there was nearly no recovery of the numbers of platelet even on Day 10 while, in the groups given the human TPO, recovery in the numbers of platelets were noted in all of the groups, though their degrees varied. In a group to which 50 micrograms/kg was administered; recovery was noted on Day 12 already giving higher numbers than of those before the ACNU administration. When it is compared with the fact that, in the control group, the recovery was as small as about 51% as compared with the state before the ACNU administration even on Day 14, it is now clear that the hTPO163 produced as in Example 65 promotes the recovery from the decrease in the numbers of platelets. As such, the hTPO163 has been confirmed to have a therapeutic effect on thrombocytopenia caused by anti-cancer agents.

Examples of the pharmaceutical preparations are hereinunder.

〈Example 80〉

The human TPO fraction obtained in Example 56 was concentrated, subjected to aseptic treatment and frozen at -20 °C to give a frozen product to be used as an injectable preparation.

〈Example 81〉

The human TPO fraction obtained in Example 56 was concentrated, 5 ml of it was filled in a 10-ml vial bottle using aseptic operation, freeze-dried at -20 °C and the bottle was stopped with a rubber stopper to give a freeze-dried substance to be used as an injectable preparation.

〈Example 82〉

The human TPO fraction obtained in Example 56 was concentrated, subjected to aseptic filtration and filled in a 10-ml vial bottle to give a product to be used as an injectable preparation.

〈Example 83〉

The hTPO163 fraction obtained in Example 65 was concentrated, subjected to aseptic treatment and freeze-dried at -20 °C to give a frozen product to be used as an injectable preparation.

〈Example 84〉

The hTPO163 fraction obtained in Example 65 was concentrated, 5 ml of it was filled in a 10-ml vial bottle using aseptic operation, freeze-dried at -20 °C and the bottle was sealed with a rubber stopper to give a freeze-dried product to be used as an injectable preparation.

〈Example 85〉

The hTPO163 fraction obtained in Example 65 was concentrated, subjected to aseptic filtration and filled in a 10-ml vial bottle to give a product to be used as an injectable preparation.

⟨Example 86⟩

The human TPO fraction obtained in Example 57 was concentrated, subjected to an aseptic treatment and frozen at -20 °C to give a frozen product to be used as an injectable preparation.

⟨Example 87⟩

The human TPO fraction obtained in Example 57 was concentrated, 5 ml of it was filled in a 10-ml vial bottle using aseptic operation, freeze-dried at -20 °C and the bottle was sealed with a rubber stopper to give a freeze-dried product to be used as an injectable preparation.

⟨Example 88⟩

The human TPO fraction obtained in Example 57 was concentrated, subjected to an aseptic filtration an filled in a 10-ml vial bottle to give a product to be used as an injectable preparation.

⟨Example 89⟩

Aplastic Canine Plasma

Heparinized aplastic canine plasma ("APK9") or normal canine plasma ("NK9") was produced as described [Mazur, E. and South, K. *Exp. Hematol.* **13**:1164-1172 (1985); Arriaga, M., South K., Cohen J.L. and Mazur, E.M. *Blood* **69**: 486-492 (1987); Mazur, E., Basilico, D., Newton, J.L., Cohen, J.L., Charland, C., Sohl, P.A., and Narendran. A. *Blood* **76**: 1771-1782 (1990)] except that 450 rads of total body irradiation were delivered to recipients.

⟨Example 90⟩

Human Megakaryocyte Assay

Standard methods for many of the procedures described in the following examples, or suitable alternative procedures, are provided in widely recognized manuals of molecular biology such as, for example, Sambrook, *et al.*, (Eds), *Molecular Cloning*, Second Edition, ColdSpring Harbor Laboratory Press (1987) and in Ausubel, *et al.*, (Eds), *Current Protocols in Molecular Biology*, Green associates/Wiley Interscience, New York (1990).

APK9 and fractionated APK9 were assayed for the ability to stimulate development of human megakaryocytes from $CD34^+$ progenitor cells. CD34- selected cells were obtained from peripheral blood cells as described (Hokom, M.H., Choi, E., Nichol, J.L., Hornkohl, A., Arakawa, T., and Hunt, P. *Molecular Biology of Haematopiesis* **3**:15-31,1994) and were incubated in the following culture medium: Iscove's modified Dulbecco's medium (IMDM; GIBCO, Grand Island, NY) supplemented with 1% Glutamine Penstrep (Irvine Scientific, Santa Ana, CA) and 10% heparinized, platelet-poor, human AB plasma. Also included were 2-mercaptoethanol ($10^{-4}$ M), pyruvic acid (110 $\mu$g/ml), cholesterol (7.8 $\mu$g/ml), adenosine, guanine, cytidine, uridine, thymidine, 2-deoxycytosine, 2-deoxyadenosine, 2-deoxyguanosine (10 $\mu$g/ml each, Sigma); human recombinant insulin (10 $\mu$g/ml), human transferrin (300 $\mu$g/ml), soybean lipids (1%, Boehringer Mannheim, Indianapolis, IN); human recombinant basic fibroblast growth factor (2 ng/ml), Genzyme, Cambridge, MA); human recombinant epidermal growth factor (15 ng/ml), platelet-derived growth factor (10 ng/ml, Amgen, Inc., Thousand Oaks, CA). CD34-selected cells were plated at $2x10^5$ ml) culture medium, 15 ul final volume, in wells of Terasaki-style microtiter plates (Vanguard, Inc., Neptune, NJ). Cells were incubated at 37 °C for 8 days in humidified boxes in 5% $CO_2$ in air, fixed directly to the culture wells with 1% glutaraldehyde, and incubated with a monoclonal antibody cocktail (anti-GPIb, anti-GPIIB, (Biodesign) and anti-GPIb (Dako, Carpinteria, CA). The immune reaction was developed with a streptavidin-beta-galactosidase detection system (HistoMark, Kirgegaard and Perry). Megakaryocytes, identified by a blue color, were counted with an inverted phase microscope at 100X magnification. Results were presented as the average number of megakaryocytes per well +/- standard error of the mean (SEM). In some cases, data were presented in terms of "megakaryocyte units/ml" where the degree to which a given sample induced megakaryocyte development was normalized to the positive APK9 control for that experiment. One unit is defined as the amount of material that results in the same number of megakaryocytes as 1 ul of APK9 standard. Activity was accepted as due to MPL ligand if it could be blocked with 5-10 ug/ml MPL-X (soluble Mpl receptor).

APK9 has been demonstrated to contain factor(s) that stimulate human megakaryocyte development in this system. CD34-selected cells incubated with 10% NK9 for 8 days show a negligible number of blue-stained megakaryocytes, whereas CD34-selected cells incubated with 10% APK9 for 8 days show a very large number of blue-stained megakaryocytes.

Figure 32 shows that increasing concentrations of Mpl-X added to the human megakaryocyte culture system increasingly block megakaryocyte development. At concentrations of Mpl-X greater than 5 µg/ml, inhibition is complete. In this experiment, CD34-selected cells were stimulated with 5% APK9. This demonstrates that an activity which interacts with Mpl-X (presumptive Mpl ligand) is necessary for human megakaryocyte development, and implies that the Mpl ligand is present in APK9 itself.

It has been further demonstrated herein that the Mpl ligand activity necessary for human megakaryocyte development is present in APK9. APK9 (135 ml) was diluted 6-fold into Iscove's media and applied to an Mpl-X affinity column. Unbound material (flow through was collected and concentrated to the original volume before assay. Bound material was eluted in 10 ml of 1 M NaCl, and 20% of the pool was diafilitered and concentrated 4-fold for assay. CD34-selected cells incubated in media alone did not develop into megakaryocytes. Cells incubated in 5% APK9 (same pool as applied to column) developed into 48 +/- 8 megakaryocytes per well. Cells incubated in 10% of the unbound material did not develop into megakaryocytes. Cells incubated in 10% of the elution pool developed into 120 +/- 44 megakaryocytes per well. Both the column load and the elution pool activities were substantially completely inhibited with 5 µg/ml Mpl-X in the assay.

⟨Example 91⟩

Transfection of murine or human Mpl receptor into a murine cell line

A. Murine Mpl Receptor

The full length murine Mpl receptor cDNA was subcloned into an expression vector containing a transcriptional promoter derived from the LTR of Moloney Murine Sarcome virus. 5 µg of this construct and 1 µg of the selectable marker plasmid pWLNeo (Stratagene) were co-electroporated into an IL-3 dependent murine cell line (32D, cline 23; Greenberger et al., *PNAS* **80**:2931-2936 (1983)). Cells were cultured for 5 days to recover from the procedure, then incubated in selection media including 800 µg/ml Geneticin (G418, Sigma) and 1 ng/ml murine IL-3. The surviving cells were then divided into pools of $2x10^5$ cells and cultured until a population grew out which could be further analyzed. Six populations were tested for surface expression of Mpl receptor by FACS analysis using a polyclonal rabbit antipeptide serum. One population was chosen for FACS sorting using the same antipeptide serum as before. Single-cells clones of the parent cell line were selected by growth in 10% APK9 and Geneticin. After selection in APK9 for 35 days; the cells were maintained in 1 ng/ml murine IL-3. One of the subclones, 1A6.1, was used for this body of work.

B. Human Mpl Receptor

The full length human Mpl receptor sequence (Vigon, I., et al., *PNAS* **89**: 5640-5644 (1992)) was subcloned into an expression vector containing the transcriptional promoter of Maloney Murine Sarcoma virus (same vector as with the murine receptor). Six µg of this construct and 6 µg of an amphotrophic retroviral packaging construct (Landau, N.R., Littman, D.R., *J. Virology* **66**: 5110-5113 (1992)) were transfected into 3 x $10^6$ 293 cells using a $CaPO_4$ mannallan transfection kit Stratagene. The same cells were retransfected after 2 days and again after 4 days. The day after the last transfection the 293 cells were cocultivated with the IL-3 dependent murine cell line (32D, clone 23; Greenberger et al., *PNAS* **80**: 2931-2936 (1983)). After 24 hours, the 32D cells were rescued and banded in a BSA gradient (Path-o-cyte; Mills Inc.). Cells were expanded in 1 ng/ml murine IL-3 and then were selected for growth in 20% APK9. Cells were sorted for cell surface expression of receptor by FACS using a polyclonal rabbit antipeptide serum. These cells were subsequently used in the assays.

⟨Example 92⟩

1A6.1 assay for Mpl ligand

1A6.1 cells were washed free of culture IL-3 and replaced (1000 cells/15 µl total col/well) in Terasaki-style microtiter plates in alpha MEM (Gibco) supplemented with 10% fetal calf serum (FCS), Geneticin (800 µg/ml) and 1% pen/strep (Gibco) in 1:1 serial dilutions of test samples. After 48 hours, the number of viable cells per well was determined microscopically. One unit of activity was defined as that amount of activity that resulted in 200 viable cells per well. Activity was defined as due to Mpl ligand if it could be completely blocked by including 5-10 µg/ml Mpl-X in the assay. Mpl ligand activity in APK9 averaged 4400 +/- 539 units/ml of aplastic plasma. Unless otherwise indicated, units of Mpl ligand activity are defined in the 1A6.1 assay.

Assays with cells transfected with the human Mpl receptor gene were carried out in essentially the same manner as with the 1A6.1 cells.

⟨Example 93⟩

Demonstration that Mpl-ligand is present in aplasticplasma of sera of mouse, dog, pig and human sources

Mpl ligand is present in the aplastic plasma or sera from murine, canine, porcine and human sources (Table 9). Plasma was collected from BDF1 mice pre-irradiation and 12 days post-irradiation (500 rads). Plasma was tested in the 1A6.1 assay where it demonstrated 2000 units/ml activity that was substantially completely inhibitable with Mpl-X (10 µg/ml). Irradiated mouse plasma was also positive in the human megakaryocyte assay where it displayed an activity of 1833 units/ml. Plasma was collected from dogs pre-irradiation and 10 days post-irradiation (450 rads). Plasma was tested in both the 1A6.1 assay and human megakaryocyte assays. Activity was detected and completely inhibited by Mpl-X (10 µg/ml) in both assays. Plasma was collected from pigs pre-irradiation and 10 days post-irradiation (650 rads). Plasma was tested in both the 1A6.1 assay and the human megakaryocyte assays. In both assays it displayed Mpl ligand activity (inhibitable by 10 µg/ml Mpl-X) comparable to that found in aplastic canine plasma. Sera from aplastic humans was obtained. This material was collected from bone marrow transplantation patients. The sera from 6 patients were assayed in the 1A6.1 assay where it showed an activity of 903 units/ml, 88% of which was due to Mpl ligand (inhibitable with 10 µg/ml Mpl-X). Sera from 14 aplastic patients has also been tested in the human megakaryocyte assay. As a group, they displaced substantial activity, 941 meg units/ml, which was completely inhibitable with 10 µg/ml Mpl-X. Murine IL-3 data is included to demonstrate the specificity of the 1A6.1 assay. Although this recombinant cytokine induces growth of the cell line, it is not blocked by 10 µg/ml Mpl-X.

TABLE 9

| Species | 1A6.1 Cell Assay (units/ml) Media | + Mpl-X | Human Meg Assay (meg units/ml) Media | +Mpl-X |
|---|---|---|---|---|
| Normal mouse | 0+/-0 | 0+/-0 | 0+/-0 | 0+/-0 |
| Aplastic mouse | 2000 | 0 | 1833 | not done |
| Normal canine | 0+/-0 | 0+/-0 | 0+/-0 | 0+/-0 |
| Aplistic canine | 4400+/-539 | 0+/-0 | 792+/-128 | 0+/-0 |
| Normal porcine | 0+/-0 | 0+/-0 | 0+/-0 | 0+/-0 |
| Aplastic porcine | 3866+/-1136 | 0+/-0 | 1284+/-182 | 10+/-10 |
| Normal human | 0+/-0 | 0+/-0 | 0+/-0 | 0+/-0 |
| Aplastic human | 903+/-64 | 114+/-33 | 941+/-178 | 0+/-0 |
| murIL3 | 6000+/-565 | 6000+/-565 | not done | not done |

⟨Example 94⟩

The preparation of the human TPO derivatives expressed in an E. coli system

In an attempt to improve the biological activity, stability and solubility, several TPO derivatives were designed and expressed in E. coli. The derivatives, generated using the amino acid sequence set forth in SEQ ID NO: 11, included [$Met^{-2}$, $Lys^{-1}$, $Ala^{1}$, $Val^{3}$, $Arg^{129}$]TPO(1-163) wherein an arginine replaces a leucine at amino acid position 129 (also referred to as L129R), [$Met^{-2}$, $Lys^{-1}$, $Ala^{1}$, $Val^{3}$, $Arg^{133}$]TPO(1-163) wherein an arginine replaces histidine at amino acid position 133 (also referred to as H133R), [$Met^{-2}$, $Lys^{-1}$, $Ala^{1}$, $Val^{3}$, $Arg^{143}$]TPO(1-163) wherein an arginine replaces a methionine at amino acid position 143 (also referred to as M143R), [$Met^{-2}$, $Lys^{-1}$, $Ala^{1}$, $Val^{3}$, $Leu^{82}$]TPO(1-163) wherein a leucine replaces a glycine at amino acid position 82 (also referred to as G82L), [$Met^{-2}$, $Lys^{-1}$, $Ala^{1}$, $Val^{3}$, $Leu^{146}$]TPO(1-163) wherein a leucine replaces glycine at amino acid position 146 (also referred to as G146L), [$Met^{-2}$, $Lys^{-1}$, $Ala^{1}$, $Val^{3}$, $Pro^{148}$]TPO(1-163) wherein a proline replaces a serine at amino acid position 148 (also referred to as S148P), [$Met^{-2}$, $Lys^{-1}$, $Ala^{1}$, $Val^{3}$, $Arg^{59}$]TPO(1-163) wherein an arginine replaces a lysine at amino acid position 59 (also referred to as K59R), [$Met^{-2}$, $Lys^{-1}$, $Ala^{1}$, $Val^{3}$, $Arg^{115}$]TPO(1-163) wherein an arginine replaces a glutamate at amino acid position 115 (also referred to as Q115R).

In making the TPO derivatives, h6T (1-163), described in example 42, was used as the template, and the oligo nucleotides which have the following sequences were synthesized.

L129R:5' -ATCTTCCGTTCTTTCCAGCACCT-3' (for the preparation of Arg-substitution derivative with Leu-129 substituted by Arg in the sequence shown in SEQ ID NO:11)

H133R;5' -TCTTTCCAGCGTCTGCTGCGT-3' (for the preparation of Arg-substitution derivative with His-133 substituted by Arg in the sequence shown in SEQ ID NO:11)

M143R;5' -CGTTTCCTGCGTCTGGTTGGC-3' (for the preparation of Arg-substitution derivative with Met-143 substituted by Arg in the sequence shown in SEQ ID NO:11)

G82L;5' -GGCCAGCTTCTGCCGACCTGCCT-3' (for the preparation of Leu-substitution derivative with Gly-82 substituted by Leu in the sequence shown in SEQ ID NO:11)

G146L;5' -ATGCTGGTTCTGGGTTCTACCCT-3' (for the preparation of Leu-substitution derivative with Gly-146 substituted by Leu in the sequence shown in SEQ ID NO:11)

S148P;5' -GTTGGCGGTCCGACCCTGTGCG-3' (for the preparation of Pro-substitution derivative with Ser-148 substituted by Pro in the sequence shown in SEQ ID NO:11)

K59R;5' -GAAGAGACCCGCGCTCAGGACATCC-3' (for the preparation of Arg-substitution derivative with Lys-59 substituted by Arg in the sequence shown in SEQ ID NO:11)

Q115R;5' -CTGCCGCCACGTGGCCGTACCAC-3' (for the preparation of Arg-substitution derivative with Gln-115 substituted by Arg in the sequence shown in SEQ ID NO:11)

Mutant plasmids were constructed using the Sculptor in vitro mutagenesis kit (Amer sham). The Xba I - Hind III fragment derived from pCFM536/h6T(1-163) described in Example 42 was introduced into Bluescript II (SK(-) phagemid vector (Stratagene, California USA) after digestion with Xba I and Hind III to obtain a plasmid pSKTPO, which was then transformed into E. coli JM109. The single stranded DNA for the mutagenesis was prepared from pSKTPO using helper phage M13KO7 (Takara Shuzo, Japan). Mutations were introduced into the TPO gene according to the supplier's protocols using the oligo nucleotides as listed above. The sequencing analysis was performed with DNA sequencing kit (Applied Biosystems USA) according to the supplier's protocols and the mutations of TPO were confirmed.

The Xba I - Hind III fragments prepared from mutated pSKTPO were introduced into pCFM536 digested with Xba I and Hind III. The plasmids pCFM536 carrying mutated genes were transformed into E. coli #261 in order to obtain the transformant expressing the TPO derivative genes.

The cultivation of E. coli #261 transformed with mutated pCFM 536 was performed according to Example 43 as previously described. The cell pellet of the transformant was collected and stored in the freezer for at least 1 day. The frozen cell pellet (3 g) was suspended in 30 mL of 20 mM Tris buffer (pH 8.5) containing 10 mM EDTA, 10 mM DTT and 1 mM PMSF. The suspension was kept on ice and was sonicated 5 times at 1 minute (at least) intervals. The sonicated cells were collected by centrifugation at 15000 rpm for 10 min.

The pellet was suspended in 30 mL of 10 mM Tris buffer (pH 8.7) containing 8 M guanidium chloride, 5 mM EDTA, 1 mM PMSF and reduced by adding 50 mg of DTT. After stirring for 1-1.5 hour, the pH of the solution was adjusted to 5.0 by using diluted HCl and was then cooled to 4°C before dilution.

The sample solution was gradually diluted in 1.5 L of 10 mM CAPS buffer (pH 10.5) containing 30% glycerol, 3 M urea, 3 mM cystamine and 1 mM L-cysteine overnight. After allowing the sample to stir for at least 2 days, the precipitate was removed by centrifugation at 8000 rpm for 45 min. The pH of the solution was adjusted to 6.8 with 6 M phosphoric acid, and diluted two times. The solution was filtered with 2 sheets of #2 filter paper (∅=90mm, Toyo filter paper, Japan) and then loaded to the column (2.6 × 10 cm) of CM-Sepharose Fast Flow (Pharmacia). The column was washed with 400 mL of 10 mM phosphate buffer (pH 6.8) containing 15% glycerol and 1 M of urea, and equilibrated with 10 mM phosphate buffer (pH 7.2) containing 15% glycerol. The protein was eluted from the column using a linear gradient system from 10 mM phosphate buffer (pH 7.2) containing 15% glycerol to the same buffer containing 0.5 M NaCl at a flow rate of 1.0 mL/min. The protein elution was monitored by the absorbance at 280 nm and the fraction containing TPO derivative was confirmed by SDS-PAGE and collected.

After the TPO fraction (about 30 mL) was concentrated to 2 mL by using Centriprep 10 (Amicon), the mutant TPO was further purified by reversed phase HPLC (Waters) with a column of µBondasphere C4 (3.9 × 150 mm). The protein elution was performed by using a linear gradient from 0.05% TFA in $H_2O$ to 2-propanol containing 30% $CH_3CN$ and 0.02% TFA with a flow rate of 0.5 mL/min for 40 min. Under these conditions, the TPO derivatives were eluted after approximately 30 min.

For the biological assay, the TPO sample thus purified was dialyzed two times against 1 L of 10 mM phosphate buffer for 2 days. After concentrating with Centriprep 10 (Amicon) to approximately 0.1 mg/mL, the biological activity of the derivatives was evaluated by M-07e assay system as previously described. It was found that all mutants have almost identical activity to that of h6T(1-163), the reference sample of TPO (See Figures 33 and 34).

SEQUENCE LISTING

```
(1) GENERAL INFORMATION:

    (i) APPLICANT:
        (A) NAME: Kirin Brewery Company, Limited
        (B) STREET: 26-1, Jingumae 6-chome
        (C) CITY: Shibuya-ku, Tokyo
        (E) COUNTRY: Japan
        (F) POSTAL CODE (ZIP): 150-11

   (ii) TITLE OF INVENTION: PROTEIN HAVING TPO ACTIVITY

  (iii) NUMBER OF SEQUENCES: 197

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

   (vi) CURRENT APPLICATION DATA:
        (A) APPLICATION NUMBER:
        (B) FILING DATE:
        (C) CLASSIFICATION:

  (vii) PRIOR APPLICATION DATA:
        (A) APPLICATION NUMBER: JP 39090/94
        (B) FILING DATE: 14-FEB-1994

  (vii) PRIOR APPLICATION DATA:
        (A) APPLICATION NUMBER: JP 79842/94
        (B) FILING DATE: 25-MAR-1994

  (vii) PRIOR APPLICATION DATA:
        (A) APPLICATION NUMBER: JP 155126/94
        (B) FILING DATE: 01-JUN-1994

  (vii) PRIOR APPLICATION DATA:
        (A) APPLICATION NUMBER: JP 167328/94
        (B) FILING DATE: 15-JUN-1994

  (vii) PRIOR APPLICATION DATA:
        (A) APPLICATION NUMBER: JP 227159/94
        (B) FILING DATE: 17-AUG-1994

  (vii) PRIOR APPLICATION DATA:
        (A) APPLICATION NUMBER: JP 304167/94
        (B) FILING DATE: 01-NOV-1994

  (vii) PRIOR APPLICATION DATA:
        (A) APPLICATION NUMBER: JP UNKNOWN
        (B) FILING DATE: 28-DEC-1994

  (vii) PRIOR APPLICATION DATA:
        (A) APPLICATION NUMBER: JP 193169/94
        (B) FILING DATE: 17-AUG-1994
```

(vii) PRIOR APPLICATION DATA:
(A) APPLICATION NUMBER: JP 298669/94
(B) FILING DATE: 01-DEC-1994

(vii) PRIOR APPLICATION DATA:
(A) APPLICATION NUMBER: JP 193916/94
(B) FILING DATE: 18-AUG-1994

(vii) PRIOR APPLICATION DATA:
(A) APPLICATION NUMBER: US 08/212,164
(B) FILING DATE: 14-MAR-1994

(vii) PRIOR APPLICATION DATA:
(A) APPLICATION NUMBER: US 08/221,020
(B) FILING DATE: 01-APR-1994

(vii) PRIOR APPLICATION DATA:
(A) APPLICATION NUMBER: US 08/278,083
(B) FILING DATE: 20-JUL-1994

(vii) PRIOR APPLICATION DATA:
(A) APPLICATION NUMBER: US 08/320,300
(B) FILING DATE: 11-OCT-1994

(vii) PRIOR APPLICATION DATA:
(A) APPLICATION NUMBER: US 08/361,811
(B) FILING DATE: 22-DEC-1994

(2) INFORMATION FOR SEQ ID NO:1:

(i) SEQUENCE CHARACTERISTICS:
(A) LENGTH: 261 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Rattus norvegicus
(H) CELL LINE: McA-RH8994

(xi) SEQUENCE DESCRIPTION: SEQ ID NO:1:

```
GGTGTACCTG GGTCCTGAAG CCCTTCTTCA CCTGGATAGA TTCCTTGGCC CACCTGTCCC      60

CACCCCACTC TGTGCAGAGG TACAAAAGCT CAAGCCGTCT CCATGGCCCC AGGAAAGATT     120

CAGGGGAGAG GCCCCACACA GGGAGCCACT GCAGTCAGAC ACCCTGGGCA GA             172

ATG GAG CTG ACT GAT TTG CTC CTG GTG GCC ATA CTT CTC CTC ACC GCA       220
Met Glu Leu Thr Asp Leu Leu Leu Val Ala Ile Leu Leu Leu Thr Ala
  1               5                  10                  15

AGA CTA ACT CTG TCC AGC CCA GTT CCT CCC GCC TGT GAC CC                261
Arg Leu Thr Leu Ser Ser Pro Val Pro Pro Ala Cys Asp
             20                  25
```

(2) INFORMATION FOR SEQ ID NO:2:

(i) SEQUENCE CHARACTERISTICS:
(A) LENGTH: 147 amino acids
(B) TYPE: amino acid
(D) TOPOLOGY: linear

(ii) MOLECULE TYPE: protein

(xi) SEQUENCE DESCRIPTION: SEQ ID NO:2:

```
Met Glu Leu Thr Asp Leu Leu Leu Val Ala Ile Leu Leu Leu Thr Ala
-21 -20                 -15                 -10

Arg Leu Thr Leu Ser Ser Pro Val Pro Pro Ala Cys Asp Pro Arg Leu
 -5                   1               5                  10

Leu Asn Lys Leu Leu Arg Asp Ser Tyr Leu Leu His Ser Arg Leu Ser
             15                  20                  25

Gln Cys Pro Asp Val Asn Pro Leu Ser Ile Pro Val Leu Leu Pro Ala
         30                  35                  40

Val Asp Phe Ser Leu Gly Glu Trp Lys Thr Gln Thr Glu Gln Ser Lys
     45                  50                  55

Ala Gln Asp Ile Leu Gly Ala Val Ser Leu Leu Leu Glu Gly Val Met
 60                  65                  70                  75

Ala Ala Arg Gly Gln Leu Glu Pro Ser Cys Leu Ser Ser Leu Leu Gly
                 80                  85                  90

Gln Leu Ser Gly Gln Val Arg Leu Leu Leu Gly Ala Leu Gln Gly Leu
             95                 100                 105

Leu Gly Thr Gln Val Ser Pro Gln Thr Tyr Arg Asn Tyr Pro Leu Thr
        110                 115                 120

Gln Phe Leu
    125
```

(2) INFORMATION FOR SEQ ID NO:3:

(i) SEQUENCE CHARACTERISTICS:
(A) LENGTH: 580 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:3:

```
CTC GTG GTC ATG CTT CTC CTA ACT GCA AGG CTA ACG CTG TCC AGC CCG      48
Leu Val Val Met Leu Leu Leu Thr Ala Arg Leu Thr Leu Ser Ser Pro
  1               5                  10                  15

GCT CCT CCT GCT TGT GAC CTC CGA GTC CTC AGT AAA CTG CTT CGT GAC      96
Ala Pro Pro Ala Cys Asp Leu Arg Val Leu Ser Lys Leu Leu Arg Asp
             20                  25                  30

TCC CAT GTC CTT CAC AGC AGA CTG AGC CAG TGC CCA GAG GTT CAC CCT     144
Ser His Val Leu His Ser Arg Leu Ser Gln Cys Pro Glu Val His Pro
```

```
          35                  40                  45

TTG CCT ACA CCT GTC CTG CTG CCT GCT GTG GAC TTT AGC TTG GGA GAA      192
Leu Pro Thr Pro Val Leu Leu Pro Ala Val Asp Phe Ser Leu Gly Glu
     50                  55                  60

TGG AAA ACC CAG ATG GAG GAG ACC AAG GCA CAG GAC ATT CTG GGA GCA      240
Trp Lys Thr Gln Met Glu Glu Thr Lys Ala Gln Asp Ile Leu Gly Ala
 65                  70                  75                  80

GTG ACC CTT CTG CTG GAG GGA GTG ATG GCA GCA CGG GGA CAA CTG GGA      288
Val Thr Leu Leu Leu Glu Gly Val Met Ala Ala Arg Gly Gln Leu Gly
                 85                  90                  95

CCC ACT TGC CTC TCA TCC CTC CTG GGG CAG CTT TCT GGA CAG GTC CGT      336
Pro Thr Cys Leu Ser Ser Leu Leu Gly Gln Leu Ser Gly Gln Val Arg
            100                 105                 110

CTC CTC CTT GGG GCC CTG CAG AGC CTC CTT GGA ACC CAG NNN NNN NNN      384
Leu Leu Leu Gly Ala Leu Gln Ser Leu Leu Gly Thr Gln Xaa Xaa Xaa
        115                 120                 125

NNN NNN NNN NCC ACA GCT CAC AAG GAT CCC AAT GCC ATC TTC CTG AGC      432
Xaa Xaa Xaa Xaa Thr Ala His Lys Asp Pro Asn Ala Ile Phe Leu Ser
    130                 135                 140

TTC CAA CAC CTG CTC CGA GGA AAG GTG CGT TTC CTG ATG CTT GTA GGA      480
Phe Gln His Leu Leu Arg Gly Lys Val Arg Phe Leu Met Leu Val Gly
145                 150                 155                 160

GGG TCC ACC CTC TGC GTC AGG CGG GCC CCA CCC ACC ACA GCT GTC CCC      528
Gly Ser Thr Leu Cys Val Arg Arg Ala Pro Pro Thr Thr Ala Val Pro
                165                 170                 175

AGC AGA ACC TCT CTA GTC CTC ACA CTG AAC GAG CTC CCA AAC AGG ACT      576
Ser Arg Thr Ser Leu Val Leu Thr Leu Asn Glu Leu Pro Asn Arg Thr
            180                 185                 190

TCT G                                                                580
Ser
```

(2) INFORMATION FOR SEQ ID NO:4:

(i) SEQUENCE CHARACTERISTICS:
(A) LENGTH: 253 amino acids
(B) TYPE: amino acid
(D) TOPOLOGY: linear

(ii) MOLECULE TYPE: protein

(xi) SEQUENCE DESCRIPTION: SEQ ID NO:4:

```
Met Glu Leu Thr Glu Leu Leu Leu Val Val Met Leu Leu Leu Thr Ala
-21 -20                 -15                 -10

Arg Leu Thr Leu Ser Ser Pro Ala Pro Pro Ala Cys Asp Leu Arg Val
 -5                   1               5                  10

Leu Ser Lys Leu Leu Arg Asp Ser His Val Leu His Ser Arg Leu Ser
            15                  20                  25

Gln Cys Pro Glu Val His Pro Leu Pro Thr Pro Val Leu Leu Pro Ala
        30                  35                  40

Val Asp Phe Ser Leu Gly Glu Trp Lys Thr Gln Met Glu Glu Thr Lys
     45                  50                  55
```

```
Ala Gln Asp Ile Leu Gly Ala Val Thr Leu Leu Leu Glu Gly Val Met
 60                 65                  70                  75

Ala Ala Arg Gly Gln Leu Gly Pro Thr Cys Leu Ser Ser Leu Leu Gly
                 80                  85                  90

Gln Leu Ser Gly Gln Val Arg Leu Leu Leu Gly Ala Leu Gln Ser Leu
             95                 100                 105

Leu Gly Thr Gln Leu Pro Pro Gln Gly Arg Thr Thr Ala His Lys Asp
        110                 115                 120

Pro Asn Ala Ile Phe Leu Ser Phe Gln His Leu Leu Arg Gly Lys Val
    125                 130                 135

Arg Phe Leu Met Leu Val Gly Gly Ser Thr Leu Cys Val Arg Arg Ala
140                 145                 150                 155

Pro Pro Thr Thr Ala Val Pro Ser Arg Thr Ser Leu Val Leu Thr Leu
                160                 165                 170

Asn Glu Leu Pro Asn Arg Thr Ser Gly Leu Leu Glu Thr Asn Phe Thr
            175                 180                 185

Ala Ser Ala Arg Thr Thr Gly Ser Gly Leu Leu Lys Trp Gln Gln Gly
        190                 195                 200

Phe Arg Ala Lys Ile Pro Gly Leu Leu Asn Gln Thr Ser Arg Ser Leu
    205                 210                 215

Asp Gln Ile Pro Gly Tyr Leu Asn Arg Ile His Glu Leu
220                 225                 230
```

(2) INFORMATION FOR SEQ ID NO:5:

(i) SEQUENCE CHARACTERISTICS:
(A) LENGTH: 576 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:5:

```
AG GGA TTC AGA GCC AAG ATT CCT GGT CTG CTG AAC CAA ACC TCC AGG      47
   Gly Phe Arg Ala Lys Ile Pro Gly Leu Leu Asn Gln Thr Ser Arg
     1               5                  10                  15

TCC CTG GAC CAA ATC CCC GGA TAC CTG AAC AGG ATA CAC GAA CTC TTG     95
Ser Leu Asp Gln Ile Pro Gly Tyr Leu Asn Arg Ile His Glu Leu Leu
                 20                  25                  30

AAT GGA ACT CGT GGA CTC TTT CCT GGA CCC TCA CGC AGG ACC CTA GGA    143
Asn Gly Thr Arg Gly Leu Phe Pro Gly Pro Ser Arg Arg Thr Leu Gly
             35                  40                  45

GCC CCG GAC ATT TCC TCA GGA ACA TCA GAC ACA GGC TCC CTG CCA CCC    191
Ala Pro Asp Ile Ser Ser Gly Thr Ser Asp Thr Gly Ser Leu Pro Pro
         50                  55                  60

AAC CTC CAG CCT GGA TAT TCT CCT TCC CCA ACC CAT CCT CCT ACT GGA    239
Asn Leu Gln Pro Gly Tyr Ser Pro Ser Pro Thr His Pro Pro Thr Gly
```

```
      65                  70                  75

CAG TAT ACG CTC TTC CCT CTT CCA CCC ACC TTG CCC ACC CCT GTG GTC      287
Gln Tyr Thr Leu Phe Pro Leu Pro Pro Thr Leu Pro Thr Pro Val Val
 80                 85                  90                  95

CAG CTC CAC CCC CTG CTT CCT GAC CCT TCT GCT CCA ACG CCC ACC CCT      335
Gln Leu His Pro Leu Leu Pro Asp Pro Ser Ala Pro Thr Pro Thr Pro
                100                 105                 110

ACC AGC CCT CTT CTA AAC ACA TCC TAC ACC CAC TCC CAG AAT CTG TCT      383
Thr Ser Pro Leu Leu Asn Thr Ser Tyr Thr His Ser Gln Asn Leu Ser
            115                 120                 125

CAG GAA GGG TAAGGTTCTC AGACACTGCC GACATCAGCA TTGTCTCATG              432
Gln Glu Gly
        130

TACAGCTCCC TTCCCTGCAG GGCGCCCCTG GGAGACAACT GGACAAGATT TCCTACTTTC    492

TCCTGAAACC CAAAGCCCTG GTAAAAGGGA TACACAGGAC TGAAAAGGGA ATCATTTTTC    552

ACTGTACATT ATAAACCTTC AGAA                                           576
```

(2) INFORMATION FOR SEQ ID NO:6:

(i) SEQUENCE CHARACTERISTICS:
(A) LENGTH: 353 amino acids
(B) TYPE: amino acid
(D) TOPOLOGY: linear

(ii) MOLECULE TYPE: protein

(xi) SEQUENCE DESCRIPTION: SEQ ID NO:6:

```
Met Glu Leu Thr Glu Leu Leu Leu Val Val Met Leu Leu Leu Thr Ala
-21 -20                 -15                 -10

Arg Leu Thr Leu Ser Ser Pro Ala Pro Pro Ala Cys Asp Leu Arg Val
 -5                  1               5                  10

Leu Ser Lys Leu Leu Arg Asp Ser His Val Leu His Ser Arg Leu Ser
            15                  20                  25

Gln Cys Pro Glu Val His Pro Leu Pro Thr Pro Val Leu Leu Pro Ala
        30                  35                  40

Val Asp Phe Ser Leu Gly Glu Trp Lys Thr Gln Met Glu Glu Thr Lys
    45                  50                  55

Ala Gln Asp Ile Leu Gly Ala Val Thr Leu Leu Leu Glu Gly Val Met
 60                 65                  70                  75

Ala Ala Arg Gly Gln Leu Gly Pro Thr Cys Leu Ser Ser Leu Leu Gly
                80                  85                  90

Gln Leu Ser Gly Gln Val Arg Leu Leu Leu Gly Ala Leu Gln Ser Leu
            95                  100                 105

Leu Gly Thr Gln Leu Pro Pro Gln Gly Arg Thr Thr Ala His Lys Asp
        110                 115                 120

Pro Asn Ala Ile Phe Leu Ser Phe Gln His Leu Leu Arg Gly Lys Val
    125                 130                 135

Arg Phe Leu Met Leu Val Gly Gly Ser Thr Leu Cys Val Arg Arg Ala
140                 145                 150                 155
```

```
Pro Pro Thr Thr Ala Val Pro Ser Arg Thr Ser Leu Val Leu Thr Leu
                160                 165                 170

Asn Glu Leu Pro Asn Arg Thr Ser Gly Leu Leu Glu Thr Asn Phe Thr
            175                 180                 185

Ala Ser Ala Arg Thr Thr Gly Ser Gly Leu Leu Lys Trp Gln Gln Gly
        190                 195                 200

Phe Arg Ala Lys Ile Pro Gly Leu Leu Asn Gln Thr Ser Arg Ser Leu
    205                 210                 215

Asp Gln Ile Pro Gly Tyr Leu Asn Arg Ile His Glu Leu Leu Asn Gly
220                 225                 230                 235

Thr Arg Gly Leu Phe Pro Gly Pro Ser Arg Arg Thr Leu Gly Ala Pro
                240                 245                 250

Asp Ile Ser Ser Gly Thr Ser Asp Thr Gly Ser Leu Pro Pro Asn Leu
            255                 260                 265

Gln Pro Gly Tyr Ser Pro Ser Pro Thr His Pro Pro Thr Gly Gln Tyr
        270                 275                 280

Thr Leu Phe Pro Leu Pro Pro Thr Leu Pro Thr Pro Val Val Gln Leu
    285                 290                 295

His Pro Leu Leu Pro Asp Pro Ser Ala Pro Thr Pro Thr Pro Thr Ser
300                 305                 310                 315

Pro Leu Leu Asn Thr Ser Tyr Thr His Ser Gln Asn Leu Ser Gln Glu
                320                 325                 330

Gly
```

(2) INFORMATION FOR SEQ ID NO:7:

(i) SEQUENCE CHARACTERISTICS:
(A) LENGTH: 1721 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:7:

```
GCGGCACGAG GGGGGTGTCT GGCTGGCGTG GCTCCCTGTT TGGGGCCTCT CCCCTGAATC      60

CTTCCTGGGG CCATGGAGGC CAGACAGACA CCCCGGCCAG A ATG GAG CTG ACT         113
                                              Met Glu Leu Thr
                                              -21 -20

GAA TTG CTC CTC GTG GTC ATG CTT CTC CTA ACT GCA AGG CTA ACG CTG       161
Glu Leu Leu Leu Val Val Met Leu Leu Leu Thr Ala Arg Leu Thr Leu
        -15                 -10                 -5

TCC AGC CCG GCT CCT CCT GCT TGT GAC CTC CGA GTC CTC AGT AAA CTG       209
Ser Ser Pro Ala Pro Pro Ala Cys Asp Leu Arg Val Leu Ser Lys Leu
    1               5                   10                  15

CTT CGT GAC TCC CAT GTC CTT CAC AGC AGA CTG AGC CAG TGC CCA GAG       257
Leu Arg Asp Ser His Val Leu His Ser Arg Leu Ser Gln Cys Pro Glu
```

```
                    20                      25                      30

GTT CAC CCT TTG CCT ACA CCT GTC CTG CTG CCT GCT GTG GAC TTT AGC       305
Val His Pro Leu Pro Thr Pro Val Leu Leu Pro Ala Val Asp Phe Ser
                35                      40                      45

TTG GGA GAA TGG AAA ACC CAG ATG GAG GAG ACC AAG GCA CAG GAC ATT       353
Leu Gly Glu Trp Lys Thr Gln Met Glu Glu Thr Lys Ala Gln Asp Ile
            50                      55                      60

CTG GGA GCA GTG ACC CTT CTG CTG GAG GGA GTG ATG GCA GCA CGG GGA       401
Leu Gly Ala Val Thr Leu Leu Leu Glu Gly Val Met Ala Ala Arg Gly
        65                      70                      75

CAA CTG GGA CCC ACT TGC CTC TCA TCC CTC CTG GGG CAG CTT TCT GGA       449
Gln Leu Gly Pro Thr Cys Leu Ser Ser Leu Leu Gly Gln Leu Ser Gly
 80                      85                      90                      95

CAG GTC CGT CTC CTC CTT GGG GCC CTG CAG AGC CTC CTT GGA ACC CAG       497
Gln Val Arg Leu Leu Leu Gly Ala Leu Gln Ser Leu Leu Gly Thr Gln
                    100                     105                     110

CTT CCT CCA CAG GGC AGG ACC ACA GCT CAC AAG GAT CCC AAT GCC ATC       545
Leu Pro Pro Gln Gly Arg Thr Thr Ala His Lys Asp Pro Asn Ala Ile
                115                     120                     125

TTC CTG AGC TTC CAA CAC CTG CTC CGA GGA AAG GTG CGT TTC CTG ATG       593
Phe Leu Ser Phe Gln His Leu Leu Arg Gly Lys Val Arg Phe Leu Met
            130                     135                     140

CTT GTA GGA GGG TCC ACC CTC TGC GTC AGG CGG GCC CCA CCC ACC ACA       641
Leu Val Gly Gly Ser Thr Leu Cys Val Arg Arg Ala Pro Pro Thr Thr
        145                     150                     155

GCT GTC CCC AGC AGA ACC TCT CTA GTC CTC ACA CTG AAC GAG CTC CCA       689
Ala Val Pro Ser Arg Thr Ser Leu Val Leu Thr Leu Asn Glu Leu Pro
160                     165                     170                     175

AAC AGG ACT TCT GGA TTG TTG GAG ACA AAC TTC ACT GCC TCA GCC AGA       737
Asn Arg Thr Ser Gly Leu Leu Glu Thr Asn Phe Thr Ala Ser Ala Arg
                    180                     185                     190

ACA ACT GGC TCT GGG CTT CTG AAG TGG CAG CAG GGA TTC AGA GCC AAG       785
Thr Thr Gly Ser Gly Leu Leu Lys Trp Gln Gln Gly Phe Arg Ala Lys
                195                     200                     205

ATT CCT GGT CTG CTG AAC CAA ACC TCC AGG TCC CTG GAC CAA ATC CCC       833
Ile Pro Gly Leu Leu Asn Gln Thr Ser Arg Ser Leu Asp Gln Ile Pro
            210                     215                     220

GGA TAC CTG AAC AGG ATA CAC GAA CTC TTG AAT GGA ACT CGT GGA CTC       881
Gly Tyr Leu Asn Arg Ile His Glu Leu Leu Asn Gly Thr Arg Gly Leu
        225                     230                     235

TTT CCT GGA CCC TCA CGC AGG ACC CTA GGA GCC CCG GAC ATT TCC TCA       929
Phe Pro Gly Pro Ser Arg Arg Thr Leu Gly Ala Pro Asp Ile Ser Ser
240                     245                     250                     255

GGA ACA TCA GAC ACA GGC TCC CTG CCA CCC AAC CTC CAG CCT GGA TAT       977
Gly Thr Ser Asp Thr Gly Ser Leu Pro Pro Asn Leu Gln Pro Gly Tyr
                    260                     265                     270

TCT CCT TCC CCA ACC CAT CCT CCT ACT GGA CAG TAT ACG CTC TTC CCT      1025
Ser Pro Ser Pro Thr His Pro Pro Thr Gly Gln Tyr Thr Leu Phe Pro
                275                     280                     285

CTT CCA CCC ACC TTG CCC ACC CCT GTG GTC CAG CTC CAC CCC CTG CTT      1073
```

```
Leu Pro Pro Thr Leu Pro Thr Pro Val Val Gln Leu His Pro Leu Leu
        290                 295                 300

CCT GAC CCT TCT GCT CCA ACG CCC ACC CCT ACC AGC CCT CTT CTA AAC      1121
Pro Asp Pro Ser Ala Pro Thr Pro Thr Pro Thr Ser Pro Leu Leu Asn
    305                 310                 315

ACA TCC TAC ACC CAC TCC CAG AAT CTG TCT CAG GAA GGG TAAGGTTCTC       1170
Thr Ser Tyr Thr His Ser Gln Asn Leu Ser Gln Glu Gly
320                 325                 330

AGACACTGCC GACATCAGCA TTGTCTCGTG TACAGCTCCC TTCCCTGCAG GGCGCCCCTG    1230

GGAGACAACT GGACAAGATT TCCTACTTTC TCCTGAAACC CAAAGCCCTG GTAAAAGGGA    1290

TACACAGGAC TGAAAAGGGA ATCATTTTTC ACTGTACATT ATAAACCTTC AGAAGCTATT    1350

TTTTTAAGCT ATCAGCAATA CTCATCAGAG CAGCTAGCTC TTTGGTCTAT TTTCTGCAGA    1410

AATTTGCAAC TCACTGATTC TCTACATGCT CTTTTTCTGT GATAACTCTG CAAAGGCCTG    1470

GGCTGGCCTG GCAGTTGAAC AGAGGGAGAG ACTAACCTTG AGTCAGAAAA CAGAGAAAGG    1530

GTAATTTCCT TTGCTTCAAA TTCAAGGCCT TCCAACGCCC CCATCCCCTT TACTATCATT    1590

CTCAGTGGGA CTCTGATCCC ATATTCTTAA CAGATCTTTA CTCTTGAGAA ATGAATAAGC    1650

TTTCTCTCAG AAATGCTGTC CCTATACACT AGACAAAACT GAAAAAAAAA AAAAAAAAAA    1710

AAAAAAAAAA A                                                         1721
```

(2) INFORMATION FOR SEQ ID NO:8:

(i) SEQUENCE CHARACTERISTICS:
(A) LENGTH: 4506 base pairs
(B) TYPE: nucleic acid
(C) STRANDEDNESS: double
(D) TOPOLOGY: linear

(ii) MOLECULE TYPE: Genomic DNA

(vi) ORIGINAL SOURCE:
(A) ORGANISM: Homo Sapiens
(G) CELL TYPE: peripheral leukocytes

(xi) SEQUENCE DESCRIPTION: SEQ ID NO:8:

```
GAATTCAGGG CTTTGGCAGT TCCAGGCTGG TCAGCATCTC AAGCCCTCCC CAGCATCTGT      60

TCACCCTGCC AGGCAGTCTC TTCCTAGAAA CTTGGTTAAA TGTTCACTCT TCTTGCTACT     120

TTCAGGATAG ATTCCTCACC CTTGGCCCGC CTTTGCCCCA CCCTACTCTG CCCAGAAGTG     180

CAAGAGCCTA AGCCGCCTCC ATGGCCCCAG GAAGGATTCA GGGGAGAGGC CCCAAACAGG     240

GAGCCACGCC AGCCAGACAC CCCGGCCAGA ATG GAG CTG ACT  G GTGAGAACAC       293
                                 Met Glu Leu Thr
                                 -21 -20

ACCTGAGGGG CTAGGGCCAT ATGGAAACAT GACAGAAGGG GAGAGAGAAA GGAGACACGC     353

TGCAGGGGGC AGGAAGCTGG GGGAACCCAT TCTCCCAAAA ATAAGGGGTC TGAGGGGTGG     413

ATTCCCTGGG TTTCAGGTCT GGGTCCTGAA TGGGAATTCC TGGAATACCA GCTGACAATG     473

ATTTCCTCCT CATCTTTCAA CCTCACCTCT CCTCATCTAA G  AA TTG CTC CTC         525
                                               Glu Leu Leu Leu
```

```
                                                                 -15
GTG GTC ATG CTT CTC CTA ACT GCA AGG CTA ACG CTG TCC AGC CCG GCT       573
Val Val Met Leu Leu Leu Thr Ala Arg Leu Thr Leu Ser Ser Pro Ala
            -10                     -5                  1

CCT CCT GCT TGT GAC CTC CGA GTC CTC AGT AAA CTG CTT CGT GAC TCC       621
Pro Pro Ala Cys Asp Leu Arg Val Leu Ser Lys Leu Leu Arg Asp Ser
    5                  10                  15

CAT GTC CTT CAC AGC AGA CTG GTGAGAACTC CCAACATTAT CCCCTTTATC          672
His Val Leu His Ser Arg Leu
 20                 25

CGCGTAACTG GTAAGACACC CATACTCCCA GGAAGACACC ATCACTTCCT CTAACTCCTT     732

GACCCAATGA CTATTCTTCC CATATTGTCC CCACCTACTG ATCACACTCT CTGACAAGGA     792

TTATTCTTCA CAATACAGCC CGCATTTAAA AGCTCTCGTC TAGAGATAGT ACTCATGGAG     852

GACTAGCCTG CTTATTAGGC TACCATAGCT CTCTCTATTT CAGCTCCCTT CTCCCCCCAC     912

CAATCTTTTT CAACAG AGC CAG TGC CCA GAG GTT CAC CCT TTG CCT ACA         961
                  Ser Gln Cys Pro Glu Val His Pro Leu Pro Thr
                                  30                  35

CCT GTC CTG CTG CCT GCT GTG GAC TTT AGC TTG GGA GAA TGG AAA ACC      1009
Pro Val Leu Leu Pro Ala Val Asp Phe Ser Leu Gly Glu Trp Lys Thr
        40                  45                  50

CAG ATG GTAAGAAAGC CATCCCTAAC CTTGGCTTCC CTAAGTCCTG TCTTCAGTTT       1065
Gln Met
    55

CCCACTGCTT CCCATGGATT CTCCAACATT CTTGAGCTTT TTAAAAATAT CTCACCTTCA    1125

GCTTGGCCAC CCTAACCCAA TCTACATTCA CCTATGATGA TAGCCTGTGG ATAAGATGAT    1185

GGCTTGCAGG TCCAATATGT GAATAGATTT GAAGCTGAAC ACCATGAAAA GCTGGAGAGA    1245

AATCGCTCAT GGCCATGCCT TTGACCTATT CCTGTTCAGT CTTCTTAAAT TGGCATGAAG    1305

AAGCAAGACT CATATGTCAT CCACAGATGA CACAAAGCTG GGAAGTACCA CTAAAATAAC    1365

AAAAGACTGA ATCAAGATTC AAATCACTGA AAGACTAGGT CAAAAACAAG GTGAAACAAC    1425

AGAGATATAA ACTTCTACAT GTGGGCCGGG GGCTCACGCC TGTAATCCCA GCACTTTGGG    1485

AGGCCGAGGC AGGCAGATCA CCTGAGGGCA GGAGTTTGAG AGCAGCCTGG CCAACATGGC    1545

GAAACCCCGT CTCTACTAAG AATACAAAAT TAGCCGGGCA TGGTAGTGCA TGCCTGTAAT    1605

CCCAGCTACT TGGAAGGCTG AAGCAGGAGA ATCCCTTGAA CCCAGGAGGT GGAGGTTGTA    1665

GTGAGCTGAG ATCATGCCAA TGCACTCCAG CCTGGGTGAC AAGAGCAAAA CTCCGTCTCA    1725

AAAAGAAAAA AAAATTCTAC ATGTGTAAAT TAATGAGTAA AGTCCTATTC CAGCTTTCAG    1785

GCCACAATGC CCTGCTTCCA TCATTTAAGC CTCTGGCCCT AGCACTTCCT ACGAAAAGGA    1845

TCTGAGAGAA TTAAATTGCC CCCAAACTTA CCATGTAACA TTACTGAAGC TGCTATTCTT    1905

AAAGCTAGTA ATTCTTGTCT GTTTGATGTT TAGCATCCCC ATTGTGGAAA TGCTCGTACA    1965

GAACTCTATT CCGAGTGGAC TACACTTAAA TATACTGGCC TGAACACCGG ACATCCCCCT    2025

GAAGACATAT GCTAATTTAT TAAGAGGGAC CATATTAAAC TAACATGTGT CTAGAAAGCA    2085
```

```
GCAGCCTGAA CAGAAAGAGA CTAGAAGCAT GTTTTATGGG CAATAGTTTA AAAAACTAAA    2145
ATCTATCCTC AAGAACCCTA GCGTCCCTTC TTCCTTCAGG ACTGAGTCAG GGAAGAAGGG    2205
CAGTTCCTAT GGGTCCCTTC TAGTCCTTTC TTTTCATCCT TATGATCATT ATGGTAGAGT    2265
CTCATACCTA CATTTAGTTT ATTTATTATT ATTATTTGAG ACGGAGTCTC ACTCTATCCC    2325
CCAGGCTGGA GTGCAGTGGC ATGATCTCAA CTCACTGCAA CCTCAGCCTC CCGGATTCAA    2385
GCGATTCTCC TGCCTCAGTC TCCCAAGTAG CTGGGATTAC AGGTGCCCAC CGCCATGCCC    2445
AGCTAATTTT TGTATTTTTG GTAGAGATGG GGTTTCACCA TGTTGGCCAG GCTGATCTTG    2505
AACTCCTGAC CTCAGGTGAT CCACCTGCCT CAGCCTCCCA AAGTGCTGGG ATTACAGGCG    2565
TGAGCCACTG CACCCAGCCT TCATTCAGTT TAAAAATCAA ATGATCCTAA GGTTTTGCAG    2625
CAGAAAGAGT AAATTTGCAG CACTAGAACC AAGAGGTAAA AGCTGTAACA GGGCAGATTT    2685
CAGCAACGTA AGAAAAAAGG AGCTCTTCTC ACTGAAACCA AGTGTAAGAC CAGGCTGGAC    2745
TAGAGGACAC GGGAGTTTTT GAAGCAGAGG CTGATGACCA GCTGTCGGGA GACTGTGAAG    2805
GAATTCCTGC CCTGGGTGGG ACCTTGGTCC TGTCCAGTTC TCAGCCTGTA TGATTCACTC    2865
TGCTGGCTAC TCCTAAGGCT CCCCACCCGC TTTTAGTGTG CCCTTTGAGG CAGTGCGCTT    2925

CTCTCTTCCA TCTCTTTCTC AG GAG GAG ACC AAG GCA CAG GAC ATT CTG GGA    2977
                         Glu Glu Thr Lys Ala Gln Asp Ile Leu Gly
                                              60                  65

GCA GTG ACC CTT CTG CTG GAG GGA GTG ATG GCA GCA CGG GGA CAA CTG    3025
Ala Val Thr Leu Leu Leu Glu Gly Val Met Ala Ala Arg Gly Gln Leu
                70                  75                  80

GGA CCC ACT TGC CTC TCA TCC CTC CTG GGG CAG CTT TCT GGA CAG GTC    3073
Gly Pro Thr Cys Leu Ser Ser Leu Leu Gly Gln Leu Ser Gly Gln Val
            85                  90                  95

CGT CTC CTC CTT GGG GCC CTG CAG AGC CTC CTT GGA ACC CAG            3115
Arg Leu Leu Leu Gly Ala Leu Gln Ser Leu Leu Gly Thr Gln
        100                 105                 110

GTAAGTCCCC AGTCAAGGGA TCTGTAGAAA CTGTTCTTTT CTGACTCAGT CCCCCTAGAA    3175
GACCTGAGGG AAGAAGGGCT CTTCCAGGGA GCTCAAGGGC AGAAGAGCTG ATCTACTAAG    3235
AGTGCTCCCT GCCAGCCACA ATGCCTGGGT ACTGGCATCC TGTCTTTCCT ACTTAGACAA    3295
GGGAGGCCTG AGATCTGGCC CTGGTGTTTG GCCTCAGGAC CATCCTCTGC CCTCAG       3351

CTT CCT CCA CAG GGC AGG ACC ACA GCT CAC AAG GAT CCC AAT GCC ATC    3399
Leu Pro Pro Gln Gly Arg Thr Thr Ala His Lys Asp Pro Asn Ala Ile
            115                 120                 125

TTC CTG AGC TTC CAA CAC CTG CTC CGA GGA AAG GTG CGT TTC CTG ATG    3447
Phe Leu Ser Phe Gln His Leu Leu Arg Gly Lys Val Arg Phe Leu Met
        130                 135                 140

CTT GTA GGA GGG TCC ACC CTC TGC GTC AGG CGG GCC CCA CCC ACC ACA    3495
Leu Val Gly Gly Ser Thr Leu Cys Val Arg Arg Ala Pro Pro Thr Thr
    145                 150                 155

GCT GTC CCC AGC AGA ACC TCT CTA GTC CTC ACA CTG AAC GAG CTC CCA    3543
Ala Val Pro Ser Arg Thr Ser Leu Val Leu Thr Leu Asn Glu Leu Pro
160                 165                 170                 175
```

```
AAC AGG ACT TCT GGA TTG TTG GAG ACA AAC TTC ACT GCC TCA GCC AGA      3591
Asn Arg Thr Ser Gly Leu Leu Glu Thr Asn Phe Thr Ala Ser Ala Arg
                180                 185                 190

ACT ACT GGC TCT GGG CTT CTG AAG TGG CAG CAG GGA TTC AGA GCC AAG      3639
Thr Thr Gly Ser Gly Leu Leu Lys Trp Gln Gln Gly Phe Arg Ala Lys
            195                 200                 205

ATT CCT GGT CTG CTG AAC CAA ACC TCC AGG TCC CTG GAC CAA ATC CCC      3687
Ile Pro Gly Leu Leu Asn Gln Thr Ser Arg Ser Leu Asp Gln Ile Pro
        210                 215                 220

GGA TAC CTG AAC AGG ATA CAC GAA CTC TTG AAT GGA ACT CGT GGA CTC      3735
Gly Tyr Leu Asn Arg Ile His Glu Leu Leu Asn Gly Thr Arg Gly Leu
    225                 230                 235

TTT CCT GGA CCC TCA CGC AGG ACC CTA GGA GCC CCG GAC ATT TCC TCA      3783
Phe Pro Gly Pro Ser Arg Arg Thr Leu Gly Ala Pro Asp Ile Ser Ser
240                 245                 250                 255

GGA ACA TCA GAC ACA GGC TCC CTG CCA CCC AAC CTC CAG CCT GGA TAT      3831
Gly Thr Ser Asp Thr Gly Ser Leu Pro Pro Asn Leu Gln Pro Gly Tyr
                260                 265                 270

TCT CCT TCC CCA ACC CAT CCT CCT ACT GGA CAG TAT ACG CTC TTC CCT      3879
Ser Pro Ser Pro Thr His Pro Pro Thr Gly Gln Tyr Thr Leu Phe Pro
            275                 280                 285

CTT CCA CCC ACC TTG CCC ACC CCT GTG GTC CAG CTC CAC CCC CTG CTT      3927
Leu Pro Pro Thr Leu Pro Thr Pro Val Val Gln Leu His Pro Leu Leu
        290                 295                 300

CCT GAC CCT TCT GCT CCA ACG CCC ACC CCT ACC AGC CCT CTT CTA AAC      3975
Pro Asp Pro Ser Ala Pro Thr Pro Thr Pro Thr Ser Pro Leu Leu Asn
    305                 310                 315

ACA TCC TAC ACC CAC TCC CAG AAT CTG TCT CAG GAA GGG TAAGGTTCTC       4024
Thr Ser Tyr Thr His Ser Gln Asn Leu Ser Gln Glu Gly
320                 325                 330

AGACACTGCC GACATCAGCA TTGTCTCATG TACAGCTCCC TTCCCTGCAG GGCGCCCCTG    4084

GGAGACAACT GGACAAGATT TCCTACTTTC TCCTGAAACC CAAAGCCCTG GTAAAAGGGA    4144

TACACAGGAC TGAAAAGGGA ATCATTTTTC ACTGTACATT ATAAACCTTC AGAAGCTATT    4204

TTTTTAAGCT ATCAGCAATA CTCATCAGAG CAGCTAGCTC TTTGGTCTAT TTTCTGCAGA    4264

AATTTGCAAC TCACTGATTC TCTACATGCT CTTTTTCTGT GATAACTCTG CAAAGGCCTG    4324

GGCTGGCCTG GCAGTTGAAC AGAGGGAGAG ACTAACCTTG AGTCAGAAAA CAGAGAAAGG    4384

GTAATTTCCT TTGCTTCAAA TTCAAGGCCT TCCAACGCCC CCATCCCCTT TACTATCATT    4444

CTCAGTGGGA CTCTGATCCC ATATTCTTAA CAGATCTTTA CTCTTGAGAA ATGAATAAGC    4504

TT                                                                   4506
```

(2) INFORMATION FOR SEQ ID NO:9:

(i) SEQUENCE CHARACTERISTICS:
- (A) LENGTH: 416 base pairs
- (B) TYPE: nucleic acid
- (C) STRANDEDNESS: double
- (D) TOPOLOGY: linear

(ii) MOLECULE TYPE: synthetic DNA

(xi) SEQUENCE DESCRIPTION: SEQ ID NO:9:

```
CTAGAAAAAA CCAAGGAGGT AATAAATAAT GAGCCCGGCT CCGCCAGCTT GTGACCTTCG     60
TGTTCTGTCT AAACTGCTTC GCGACTCTCA CGTGCTGCAC TCTCGTCTGT CCCAGTGCCC    120
GGAAGTTCAC CCGCTGCCGA CCCCGGTTCT GCTTCCGGCT GTCGACTTCT CCCTGGGTGA    180
ATGGAAAACC CAGATGGAAG AGACCAAAGC TCAGGACATC CTGGGTGCAG TAACTCTGCT    240
TCTGGAAGGC GTTATGGCTG CACGTGGCCA GCTTGGCCCG ACCTGCCTGT CTTCCCTGCT    300
TGGCCAGCTG TCTGGCCAGG TTCGTCTGCT GCTCGGCGCT CTGCAGTCTC TGCTTGGCAC    360
CCAGCTGCCG CCACAGGGCC GTACCACTGC TCACAAGGAT CCGAACGCTA TCTTCC         416
```

(2) INFORMATION FOR SEQ ID NO:10:

(i) SEQUENCE CHARACTERISTICS:
(A) LENGTH: 179 amino acids
(B) TYPE: amino acid
(D) TOPOLOGY: linear

(ii) MOLECULE TYPE: protein

(xi) SEQUENCE DESCRIPTION: SEQ ID NO:10:

```
Leu Val Pro Arg Gly Ser Pro Ala Pro Pro Ala Cys Asp Leu Arg Val
 -5                  1               5                  10

Leu Ser Lys Leu Leu Arg Asp Ser His Val Leu His Ser Arg Leu Ser
             15                  20                  25

Gln Cys Pro Glu Val His Pro Leu Pro Thr Pro Val Leu Leu Pro Ala
         30                  35                  40

Val Asp Phe Ser Leu Gly Glu Trp Lys Thr Gln Met Glu Glu Thr Lys
     45                  50                  55

Ala Gln Asp Ile Leu Gly Ala Val Thr Leu Leu Leu Glu Gly Val Met
 60                  65                  70                  75

Ala Ala Arg Gly Gln Leu Gly Pro Thr Cys Leu Ser Ser Leu Leu Gly
                 80                  85                  90

Gln Leu Ser Gly Gln Val Arg Leu Leu Leu Gly Ala Leu Gln Ser Leu
             95                 100                 105

Leu Gly Thr Gln Leu Pro Pro Gln Gly Arg Thr Thr Ala His Lys Asp
        110                 115                 120

Pro Asn Ala Ile Phe Leu Ser Phe Gln His Leu Leu Arg Gly Lys Val
    125                 130                 135

Arg Phe Leu Met Leu Val Gly Gly Ser Thr Leu Cys Val Arg Arg Ala
140                 145                 150                 155

Pro Pro Thr Thr Ala Val Pro Ser Arg Thr Ser Leu Val Leu Thr Leu
                160                 165                 170

Asn Glu Leu
```

(2) INFORMATION FOR SEQ ID NO:11:

(i) SEQUENCE CHARACTERISTICS:
(A) LENGTH: 535 base pairs
(B) TYPE: nucleic acid

(C) STRANDEDNESS: double
(D) TOPOLOGY: linear

(ii) MOLECULE TYPE: synthetic DNA

(vi) ORIGINAL SOURCE:
(A) ORGANISM: Homo Sapiens

(xi) SEQUENCE DESCRIPTION: SEQ ID NO:11:

```
CTAGAAAAAA CCAAGGAGGT AATAAATA ATG AAA GCA CCT GTA CCA CCT GCA        52
                               Met Lys Ala Pro Val Pro Pro Ala
                                -2       1               5 TGT GAT TTA CGG GTC CTG TCT AAA CTG CTG CGC GAC TCT CAC GTG CTG      100
Cys Asp Leu Arg Val Leu Ser Lys Leu Leu Arg Asp Ser His Val Leu
            10                  15                  20

CAC TCT CGT CTG TCC CAG TGC CCG GAA GTT CAC CCG CTG CCG ACC CCG      148
His Ser Arg Leu Ser Gln Cys Pro Glu Val His Pro Leu Pro Thr Pro
        25                  30                  35

GTT CTG CTT CCG GCT GTC GAC TTC TCC CTG GGT GAA TGG AAA ACC CAG      196
Val Leu Leu Pro Ala Val Asp Phe Ser Leu Gly Glu Trp Lys Thr Gln
    40                  45                  50

ATG GAA GAG ACC AAA GCT CAG GAC ATC CTG GGT GCA GTA ACT CTG CTT      244
Met Glu Glu Thr Lys Ala Gln Asp Ile Leu Gly Ala Val Thr Leu Leu
 55                  60                  65                  70

CTG GAA GGC GTT ATG GCT GCA CGT GGC CAG CTT GGC CCG ACC TGC CTG      292
Leu Glu Gly Val Met Ala Ala Arg Gly Gln Leu Gly Pro Thr Cys Leu
                75                  80                  85

TCT TCC CTG CTT GGC CAG CTG TCT GGC CAG GTT CGT CTG CTG CTC GGC      340
Ser Ser Leu Leu Gly Gln Leu Ser Gly Gln Val Arg Leu Leu Leu Gly
            90                  95                 100

GCT CTG CAG TCT CTG CTT GGC ACC CAG CTG CCG CCA CAG GGC CGT ACC      388
Ala Leu Gln Ser Leu Leu Gly Thr Gln Leu Pro Pro Gln Gly Arg Thr
        105                 110                 115

ACT GCT CAC AAG GAT CCG AAC GCT ATC TTC CTG TCT TTC CAG CAC CTG      436
Thr Ala His Lys Asp Pro Asn Ala Ile Phe Leu Ser Phe Gln His Leu
    120                 125                 130

CTG CGT GGC AAA GTT CGT TTC CTG ATG CTG GTT GGC GGT TCT ACC CTG      484
Leu Arg Gly Lys Val Arg Phe Leu Met Leu Val Gly Gly Ser Thr Leu
135                 140                 145                 150

TGC GTT CGT CGG GCG CCG CCA ACC ACT GCT GTT CCG TCT TAATGAAAGC        533
Cys Val Arg Arg Ala Pro Pro Thr Thr Ala Val Pro Ser
                155                 160

TT                                                                    535
```

(2) INFORMATION FOR SEQ ID NO:12:

(i) SEQUENCE CHARACTERISTICS:
(A) LENGTH: 535 base pairs
(B) TYPE: nucleic acid
(C) STRANDEDNESS: double
(D) TOPOLOGY: linear

(ii) MOLECULE TYPE: synthetic DNA

(vi) ORIGINAL SOURCE:

(A) ORGANISM: Homo Sapiens

(xi) SEQUENCE DESCRIPTION: SEQ ID NO:12:

```
CTAGAAAAAA CCAAGGAGGT AATAAATA ATG AAA TCT CCT GCA CCA CCT GCA        52
                               Met Lys Ser Pro Ala Pro Pro Ala
                                -2       1               5 TGT GAT TTA CGG GTC CTG TCT AAA CTG CTG CGC GAC TCT CAC GTG CTG      100
Cys Asp Leu Arg Val Leu Ser Lys Leu Leu Arg Asp Ser His Val Leu
            10                  15                  20

CAC TCT CGT CTG TCC CAG TGC CCG GAA GTT CAC CCG CTG CCG ACC CCG      148
His Ser Arg Leu Ser Gln Cys Pro Glu Val His Pro Leu Pro Thr Pro
        25                  30                  35

GTT CTG CTT CCG GCT GTC GAC TTC TCC CTG GGT GAA TGG AAA ACC CAG      196
Val Leu Leu Pro Ala Val Asp Phe Ser Leu Gly Glu Trp Lys Thr Gln
    40                  45                  50

ATG GAA GAG ACC AAA GCT CAG GAC ATC CTG GGT GCA GTA ACT CTG CTT      244
Met Glu Glu Thr Lys Ala Gln Asp Ile Leu Gly Ala Val Thr Leu Leu
 55                  60                  65                  70

CTG GAA GGC GTT ATG GCT GCA CGT GGC CAG CTT GGC CCG ACC TGC CTG      292
Leu Glu Gly Val Met Ala Ala Arg Gly Gln Leu Gly Pro Thr Cys Leu
                75                  80                  85

TCT TCC CTG CTT GGC CAG CTG TCT GGC CAG GTT CGT CTG CTG CTC GGC      340
Ser Ser Leu Leu Gly Gln Leu Ser Gly Gln Val Arg Leu Leu Leu Gly
            90                  95                 100

GCT CTG CAG TCT CTG CTT GGC ACC CAG CTG CCG CCA CAG GGC CGT ACC      388
Ala Leu Gln Ser Leu Leu Gly Thr Gln Leu Pro Pro Gln Gly Arg Thr
        105                 110                 115

ACT GCT CAC AAG GAT CCG AAC GCT ATC TTC CTG TCT TTC CAG CAC CTG      436
Thr Ala His Lys Asp Pro Asn Ala Ile Phe Leu Ser Phe Gln His Leu
    120                 125                 130

CTG CGT GGC AAA GTT CGT TTC CTG ATG CTG GTT GGC GGT TCT ACC CTG      484
Leu Arg Gly Lys Val Arg Phe Leu Met Leu Val Gly Gly Ser Thr Leu
135                 140                 145                 150

TGC GTT CGT CGG GCG CCG CCA ACC ACT GCT GTT CCG TCT TAATGAAAGC       533
Cys Val Arg Arg Ala Pro Pro Thr Thr Ala Val Pro Ser
                155                 160

TT                                                                   535
```

(2) INFORMATION FOR SEQ ID NO:13:

(i) SEQUENCE CHARACTERISTICS:
(A) LENGTH: 555 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:13:

```
ATG GAG CTG ACT GAA TTG CTC CTC GTG GTC ATG CTT CTC CTA ACT GCA       48
```

```
Met Glu Leu Thr Glu Leu Leu Leu Val Val Met Leu Leu Leu Thr Ala
-21 -20                 -15                 -10 AGG CTA ACG CTG TCC AGC CCG GCT CCT CCT GCT TGT GAC CTC CGA GTC       96
Arg Leu Thr Leu Ser Ser Pro Ala Pro Pro Ala Cys Asp Leu Arg Val
 -5                   1               5                  10 CTC AGT AAA CTG CTT CGT GAC TCC CAT GTC CTT CAC AGC AGA CTG AGC      144
Leu Ser Lys Leu Leu Arg Asp Ser His Val Leu His Ser Arg Leu Ser
             15                  20                  25 CAG TGC CCA GAG GTT CAC CCT TTG CCT ACA CCT GTC CTG CTG CCT GCT      192
Gln Cys Pro Glu Val His Pro Leu Pro Thr Pro Val Leu Leu Pro Ala
         30                  35                  40 GTG GAC TTT AGC TTG GGA GAA TGG AAA ACC CAG ATG GAG GAG ACC AAG      240
Val Asp Phe Ser Leu Gly Glu Trp Lys Thr Gln Met Glu Glu Thr Lys
     45                  50                  55 GCA CAG GAC ATT CTG GGA GCA GTG ACC CTT CTG CTG GAG GGA GTG ATG      288
Ala Gln Asp Ile Leu Gly Ala Val Thr Leu Leu Leu Glu Gly Val Met
 60                  65                  70                  75 GCA GCA CGG GGA CAA CTG GGA CCC ACT TGC CTC TCA TCC CTC CTG GGG      336
Ala Ala Arg Gly Gln Leu Gly Pro Thr Cys Leu Ser Ser Leu Leu Gly
                 80                  85                  90 CAG CTT TCT GGA CAG GTC CGT CTC CTC CTT GGG GCC CTG CAG AGC CTC      384
Gln Leu Ser Gly Gln Val Arg Leu Leu Leu Gly Ala Leu Gln Ser Leu
             95                 100                 105 CTT GGA ACC CAG CTT CCT CCA CAG GGC AGG ACC ACA GCT CAC AAG GAT      432
Leu Gly Thr Gln Leu Pro Pro Gln Gly Arg Thr Thr Ala His Lys Asp
        110                 115                 120 CCC AAT GCC ATC TTC CTG AGC TTC CAA CAC CTG CTC CGA GGA AAG GTG      480
Pro Asn Ala Ile Phe Leu Ser Phe Gln His Leu Leu Arg Gly Lys Val
    125                 130                 135 CGT TTC CTG ATG CTT GTA GGA GGG TCC ACC CTC TGC GTC AGG CGG GCC      528
Arg Phe Leu Met Leu Val Gly Gly Ser Thr Leu Cys Val Arg Arg Ala
140                 145                 150                 155 CCA CCC ACC ACA GCT GTC CCC AGC TGA                                  555
Pro Pro Thr Thr Ala Val Pro Ser
                160
```

(2) INFORMATION FOR SEQ ID NO:14:

(i) SEQUENCE CHARACTERISTICS:
- (A) LENGTH: 1043 base pairs
- (B) TYPE: nucleic acid
- (C) STRANDEDNESS: double
- (D) TOPOLOGY: linear

(ii) MOLECULE TYPE: synthetic DNA

(vi) ORIGINAL SOURCE:
- (A) ORGANISM: Homo Sapiens

(xi) SEQUENCE DESCRIPTION: SEQ ID NO:14:

```
CTAGAAAAAA CCAAGGAGGT AATAAATA ATG AAA AGT CCT GCA CCA CCT GCA        52
                               Met Lys Ser Pro Ala Pro Pro Ala
                                -2       1               5 TGT GAT TTA CGG GTC CTG TCT AAA CTG CTG CGC GAC TCT CAC GTG CTG      100
```

```
Cys Asp Leu Arg Val Leu Ser Lys Leu Leu Arg Asp Ser His Val Leu
            10                  15                  20

CAC TCT CGT CTG TCC CAG TGC CCG GAA GTT CAC CCG CTG CCG ACC CCG        148
His Ser Arg Leu Ser Gln Cys Pro Glu Val His Pro Leu Pro Thr Pro
        25                  30                  35

GTT CTG CTT CCG GCT GTC GAC TTC TCC CTG GGT GAA TGG AAA ACC CAG        196
Val Leu Leu Pro Ala Val Asp Phe Ser Leu Gly Glu Trp Lys Thr Gln
    40                  45                  50

ATG GAA GAG ACC AAA GCT CAG GAC ATC CTG GGT GCA GTA ACT CTG CTT        244
Met Glu Glu Thr Lys Ala Gln Asp Ile Leu Gly Ala Val Thr Leu Leu
55                  60                  65                  70

CTG GAA GGC GTT ATG GCT GCA CGT GGC CAG CTT GGC CCG ACC TGC CTG        292
Leu Glu Gly Val Met Ala Ala Arg Gly Gln Leu Gly Pro Thr Cys Leu
                75                  80                  85

TCT TCC CTG CTT GGC CAG CTG TCT GGC CAG GTT CGT CTG CTG CTC GGC        340
Ser Ser Leu Leu Gly Gln Leu Ser Gly Gln Val Arg Leu Leu Leu Gly
            90                  95                  100

GCT CTG CAG TCT CTG CTT GGC ACC CAG CTG CCG CCA CAG GGC CGT ACC        388
Ala Leu Gln Ser Leu Leu Gly Thr Gln Leu Pro Pro Gln Gly Arg Thr
        105                 110                 115

ACT GCT CAC AAG GAT CCG AAC GCT ATC TTC CTG TCT TTC CAG CAC CTG        436
Thr Ala His Lys Asp Pro Asn Ala Ile Phe Leu Ser Phe Gln His Leu
    120                 125                 130

CTG CGT GGC AAA GTT CGT TTC CTG ATG CTG GTT GGC GGT TCT ACC CTG        484
Leu Arg Gly Lys Val Arg Phe Leu Met Leu Val Gly Gly Ser Thr Leu
135                 140                 145                 150

TGC GTT CGT CGG GCG CCG CCA ACC ACT GCT GTT CCG TCT CGT ACC TCT        532
Cys Val Arg Arg Ala Pro Pro Thr Thr Ala Val Pro Ser Arg Thr Ser
                155                 160                 165

CTG GTT CTG ACC CTG AAC GAG CTC CCG AAC CGT ACC AGC GGC CTG CTG        580
Leu Val Leu Thr Leu Asn Glu Leu Pro Asn Arg Thr Ser Gly Leu Leu
            170                 175                 180

GAA ACC AAC TTT ACC GCG AGC GCG CGT ACC ACC GGC AGC GGC CTG CTG        628
Glu Thr Asn Phe Thr Ala Ser Ala Arg Thr Thr Gly Ser Gly Leu Leu
        185                 190                 195

AAA TGG CAG CAG GGC TTT CGT GCG AAA ATC CCG GGC CTG CTG AAC CAG        676
Lys Trp Gln Gln Gly Phe Arg Ala Lys Ile Pro Gly Leu Leu Asn Gln
    200                 205                 210

ACC AGC CGT AGC CTG GAT CAG ATC CCG GGC TAT CTG AAC CGT ATC CAT        724
Thr Ser Arg Ser Leu Asp Gln Ile Pro Gly Tyr Leu Asn Arg Ile His
215                 220                 225                 230

GAA CTG CTG AAC GGC ACC CGT GGC CTG TTT CCG GGC CCG AGC CGT CGC        772
Glu Leu Leu Asn Gly Thr Arg Gly Leu Phe Pro Gly Pro Ser Arg Arg
                235                 240                 245

ACC CTG GGC GCG CCG GAT ATC AGC TCT GGC ACC AGC GAT ACC GGC AGC        820
Thr Leu Gly Ala Pro Asp Ile Ser Ser Gly Thr Ser Asp Thr Gly Ser
            250                 255                 260

CTG CCG CCG AAC CTG CAG CCG GGC TAT AGC CCG AGC CCG ACC CAT CCG        868
Leu Pro Pro Asn Leu Gln Pro Gly Tyr Ser Pro Ser Pro Thr His Pro
        265                 270                 275
```

```
CCG ACC GGC CAG TAT ACC CTG TTT CCG CTG CCG CCG ACC CTG CCG ACC        916
Pro Thr Gly Gln Tyr Thr Leu Phe Pro Leu Pro Pro Thr Leu Pro Thr
    280                 285                 290

CCG GTG GTT CAG CTG CAT CCG CTG CTG CCG GAT CCG AGC GCG CCG ACC        964
Pro Val Val Gln Leu His Pro Leu Leu Pro Asp Pro Ser Ala Pro Thr
295                 300                 305                 310

CCG ACC CCG ACC AGC CCG CTG CTG AAC ACC AGC TAT ACC CAT AGC CAG       1012
Pro Thr Pro Thr Ser Pro Leu Leu Asn Thr Ser Tyr Thr His Ser Gln
                315                 320                 325

AAC CTG AGC CAG GAA GGC TAATGAAGCT TGA                                1043
Asn Leu Ser Gln Glu Gly
            330
```

(2) INFORMATION FOR SEQ ID NO:15:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:15:

AACTGGAAGA ATTCGCGGCC GCAGGAATTT TTTTTTTTTT TTTTT 45

(2) INFORMATION FOR SEQ ID NO:16:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:16:

AATTCGGCAC GAG 13

(2) INFORMATION FOR SEQ ID NO:17:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:17:

CTCGTGCCG 9

(2) INFORMATION FOR SEQ ID NO:18:

(i) SEQUENCE CHARACTERISTICS:
(A) LENGTH: 12 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:18:

```
Ile Pro Val Pro Pro Ala Cys Asp Pro Arg Leu Leu
 1               5                   10
```

(2) INFORMATION FOR SEQ ID NO:19:

(i) SEQUENCE CHARACTERISTICS:
(A) LENGTH: 12 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:19:

```
Thr Pro Val Pro Pro Ala Cys Asp Pro Arg Leu Leu
 1               5                   10
```

(2) INFORMATION FOR SEQ ID NO:20:

(i) SEQUENCE CHARACTERISTICS:
(A) LENGTH: 12 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:20:

```
Ser Pro Val Pro Pro Ala Cys Asp Pro Arg Leu Leu
 1               5                   10
```

(2) INFORMATION FOR SEQ ID NO:21:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(ix) FEATURE:
(A) NAME/KEY: modified_base
(B) LOCATION: 12 & 15
(D) OTHER INFORMATION: /mod_base= i

(xi) SEQUENCE DESCRIPTION: SEQ ID NO:21:

ACGCTGGGGG CNGANGA 17

(2) INFORMATION FOR SEQ ID NO:22:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(ix) FEATURE:
(A) NAME/KEY: modified_base
(B) LOCATION: 12 & 15

(D) OTHER INFORMATION: /mod_base= i

(xi) SEQUENCE DESCRIPTION: SEQ ID NO:22:

ACACTAGGAT CNAANAA 17

(2) INFORMATION FOR SEQ ID NO:23:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(ix) FEATURE:
(A) NAME/KEY: modified_base
(B) LOCATION: 12 & 15
(D) OTHER INFORMATION: /mod_base= i

(xi) SEQUENCE DESCRIPTION: SEQ ID NO:23:

ACGCTGGGTG CNGANGA 17

(2) INFORMATION FOR SEQ ID NO:24:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(ix) FEATURE:
(A) NAME/KEY: modified_base
(B) LOCATION: 12 & 15
(D) OTHER INFORMATION: /mod_base= i

(xi) SEQUENCE DESCRIPTION: SEQ ID NO:24:

ACGCTGGGCG CNGANGA 17

(2) INFORMATION FOR SEQ ID NO:25:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:25:

GGGGGGCGGA CGCTGGG 17

(2) INFORMATION FOR SEQ ID NO:26:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:26:

GGAGGACGAA CACTAGG 17

(2) INFORMATION FOR SEQ ID NO:27:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:27:

GGTGGTCGTA CGCTGGG 17

(2) INFORMATION FOR SEQ ID NO:28:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:28:

GGCGGCCGCA CGCTGGG 17

(2) INFORMATION FOR SEQ ID NO:29:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:29:

GGATCTTGGT TCATTCTCAA G 21

(2) INFORMATION FOR SEQ ID NO:30:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:30:

CCTCAGACAG TGGTTCAAAG 20

(2) INFORMATION FOR SEQ ID NO:31:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:31:

CGAGGGTGTA CCTGGGTCCT G 21

(2) INFORMATION FOR SEQ ID NO:32:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:32:

CAGAGTTAGT CTTGCGGTGA G 21

(2) INFORMATION FOR SEQ ID NO:33:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:33:

CCTGTCCTGC TGCCTGCTGT G 21

(2) INFORMATION FOR SEQ ID NO:34:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:34:

TGAAGTTCGT CTCCAACAAT C 21

(2) INFORMATION FOR SEQ ID NO:35:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:35:

ATGGAGCTGA CTGATTTGCT C 21

(2) INFORMATION FOR SEQ ID NO:36:

(i) SEQUENCE CHARACTERISTICS:
(A) LENGTH: 21 base pairs
(B) TYPE: nucleic acid

(C) STRANDEDNESS: single
(D) TOPOLOGY: linear

(ii) MOLECULE TYPE: Other nucleic acid

(xi) SEQUENCE DESCRIPTION: SEQ ID NO:36:

TGAAGTTCGT CTCCAACAAT C 21

(2) INFORMATION FOR SEQ ID NO:37:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:37:

TTGTGACCTC CGAGTCCTCA G 21

(2) INFORMATION FOR SEQ ID NO:38:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:38:

TGACGCAGAG GGTGGACCCT C 21

(2) INFORMATION FOR SEQ ID NO:39:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:39:

AGCAGAACCT CTCTAGTCCT C 21

(2) INFORMATION FOR SEQ ID NO:40:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:40:

ACACTGAACG AGCTCCCAAA C 21

(2) INFORMATION FOR SEQ ID NO:41:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:41:

AACTACTGGC TCTGGGCTTC T 21

(2) INFORMATION FOR SEQ ID NO:42:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:42:

AGGGATTCAG AGCCAAGATT C 21

(2) INFORMATION FOR SEQ ID NO:43:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:43:

TAGCGGCCGC TTTTTTTTTT TTTTTTTGGG G 31

(2) INFORMATION FOR SEQ ID NO:44:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:44:

TAGCGGCCGC TTTTTTTTTT TTTTTTTAAA A 31

(2) INFORMATION FOR SEQ ID NO:45:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:45:

TAGCGGCCGC TTTTTTTTTT TTTTTTTCTT T 31

(2) INFORMATION FOR SEQ ID NO:46:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:46:

TAGCGGCCGC TTTTTTTTTT TTTTTTTGCC C 31

(2) INFORMATION FOR SEQ ID NO:47:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:47:

TAGCGGCCGC TTTTTTTTTT T 21

(2) INFORMATION FOR SEQ ID NO:48:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:48:

TTGTGACCTC CGAGTCCTCA G 21

(2) INFORMATION FOR SEQ ID NO:49:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:49:

AGGATGGGTT GGGGAAGGAG A 21

(2) INFORMATION FOR SEQ ID NO:50:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:50:

TTGTGACCTC CGAGTCCTCA G 21

(2) INFORMATION FOR SEQ ID NO:51:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:51:

AGGGAAGAGC GTATACTGTC C 21

(2) INFORMATION FOR SEQ ID NO:52:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:52:

GGCCAGCCAG ACACCCCGGC C 21

(2) INFORMATION FOR SEQ ID NO:53:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:53:

ATGGGAGTCA CGAAGCAGTT T 21

(2) INFORMATION FOR SEQ ID NO:54:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:54:

TGCGTTTCCT GATGCTTGTA G 21

(2) INFORMATION FOR SEQ ID NO:55:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:55:

AACCTTACCC TTCCTGAGAC A 21

(2) INFORMATION FOR SEQ ID NO:56:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:56:

TTTGAATTCG GCCAGCCAGA CACCCCGGCC 30

(2) INFORMATION FOR SEQ ID NO:57:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:57:

TTTGCGGCCG CTCATTAGCT GGGGACAGCT GTGGTGGGT 39

(2) INFORMATION FOR SEQ ID NO:58:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:58:

TTTGCGGCCG CTCATTACAG TGTGAGGACT AGAGAGGTTC TG 42

(2) INFORMATION FOR SEQ ID NO:59:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:59:

TTTGCGGCCG CTCATTATCT GGCTGAGGCA GTGAAGTTTG TC 42

(2) INFORMATION FOR SEQ ID NO:60:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:60:

TTTGCGGCCG CTCATTACAG ACCAGGAATC TTGGCTCTGAA T 42

(2) INFORMATION FOR SEQ ID NO:61:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:61:

TTTGAATTCG GCCAGCCAGA CACCCCGGCC 30

(2) INFORMATION FOR SEQ ID NO:62:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:62:

TTTGCGGCCG CTCATTAGAG GGTGGACCCT CCTACAAGCA T 41

(2) INFORMATION FOR SEQ ID NO:63:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:63:

TTTGCGGCCG CTCATTAGCA GAGGGTGGAC CCTCCTACAA 40

(2) INFORMATION FOR SEQ ID NO:64:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:64:

TTTGCGGCCG CTCATTACCT GACGCAGAGG GTGGACCC 38

(2) INFORMATION FOR SEQ ID NO:65:

(i) SEQUENCE CHARACTERISTICS:
(A) LENGTH: 38 base pairs
(B) TYPE: nucleic acid

(C) STRANDEDNESS: single
(D) TOPOLOGY: linear

(ii) MOLECULE TYPE: Other nucleic acid

(xi) SEQUENCE DESCRIPTION: SEQ ID NO:65:

TTTGCGGCCG CTCATTACCG CCTGACGCAG AGGGTGGA 38

(2) INFORMATION FOR SEQ ID NO:66:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:66:

TTTGCGGCCG CTCATTAGGC CCGCCTGACG CAGAGGGT 38

(2) INFORMATION FOR SEQ ID NO:67:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:67:

TTTGCGGCCG CTCATTATGG GGCCCGCCTG ACGCAGAG 38

(2) INFORMATION FOR SEQ ID NO:68:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:68:

TTTGCGGCCG CTCATTAGGG TGGGGCCCGC CTGACGCA 38

(2) INFORMATION FOR SEQ ID NO:69:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:69:

TTTGAATTCG GCCAGCCAGA CACCCCGGCC 30

(2) INFORMATION FOR SEQ ID NO:70:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:70:

TTTGCGGCCG CTCATTATTC GTGTATCCTG TTCAGGTATC C 41

(2) INFORMATION FOR SEQ ID NO:71:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:71:

TTTGCGGCCG CTCATTAGCT GGGGACAGCT GTGGTGGGT 39

(2) INFORMATION FOR SEQ ID NO:72:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:72:

AGTAAACTGC TTCGTGACTC CCATGTCCTT CACAGCAGAC TGAGCCAGTG 50

(2) INFORMATION FOR SEQ ID NO:73:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:73:

CATGGGAGTC ACGAAGCAGT TTACTGGACA GCGTTAGCCT TGCAGTTAG 49

(2) INFORMATION FOR SEQ ID NO:74:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:74:

TGTGACCTCC GAGTCCTCAG TAAACTGCTT CGTGACTCCC ATGTCCTTC 49

(2) INFORMATION FOR SEQ ID NO:75:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:75:

TTTACTGAGG ACTCGGAGGT CACAGGACAG CGTTAGCCTT GCAGTTAG 48

(2) INFORMATION FOR SEQ ID NO:76:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:76:

GACCTCCGAG TCCTCAGTAA ACTGCTTCGT GACTCCCATG TCCTTCACA 49

(2) INFORMATION FOR SEQ ID NO:77:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:77:

CAGTTTACTG AGGACTCGGA GGTCGGACAG CGTTAGCCTT GCAGTTAG 48

(2) INFORMATION FOR SEQ ID NO:78:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:78:

TTGTGACCTC CGAGTCCTCA G 21

(2) INFORMATION FOR SEQ ID NO:79:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:79:

CAGGTATCCG GGGATTTGGT C 21

(2) INFORMATION FOR SEQ ID NO:80:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:80:

TGCGTTTCCT GATGCTTGTA G 21

(2) INFORMATION FOR SEQ ID NO:81:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:81:

GAGAGAGCGG CCGCTTACCC TTCCTGAGAC AGATT 35

(2) INFORMATION FOR SEQ ID NO:82:

(i) SEQUENCE CHARACTERISTICS:
(A) LENGTH: 6 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:82:

GAGAGA 6

(2) INFORMATION FOR SEQ ID NO:83:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:83:

CTAGAAAAAA CCAAGGAGGT AATAAATAAT GAGCCCGGCT CCGCCAGCTT GTGACCTTCG 60

TGTTCTGTCT 70

(2) INFORMATION FOR SEQ ID NO:84:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:84:

CAGTTTAGAC AGAACACGAA GGTCACAAGC TGGCGGAGCC GGGCTCATTA TTTATTACCT 60

CCTTGGTTTT TT 72

(2) INFORMATION FOR SEQ ID NO:85:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:85:

AAACTGCTTC GCGACTCTCA CGTGCTGCAC TCTCGTCTGT CCCAGTGCCC GGAAGTTCAC 60

CCGCTGCCG 69

(2) INFORMATION FOR SEQ ID NO:86:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:86:

CGGGGTCGGC AGCGGGTGAA CTTCCGGGCA CTGGGACAGA CGAGAGTGCA GCACGTGAGA 60

GTCGCGAAG 69

(2) INFORMATION FOR SEQ ID NO:87:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:87:

ACCCCGGTTC TGCTTCCGGC TGTCGACTTC TCCCTGGGTG AATGGAAAAC CCAGATGGAA 60

GAGACCAAA 69

(2) INFORMATION FOR SEQ ID NO:88:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:88:

CTGAGCTTTG GTCTCTTCCA TCTGGGTTTT CCATTCACCC AGGGAGAAGT CGACAGCCGG 60

AAGCAGAAC 69

(2) INFORMATION FOR SEQ ID NO:89:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:89:

GCTCAGGACA TCCTGGGTGC AGTAACTCTG CTTCTGGAAG GCGTTATGGC TGCACGTGGC 60

CAGCTTGGC 69

(2) INFORMATION FOR SEQ ID NO:90:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:90:

GGTCGGGCCA AGCTGGCCAC GTGCAGCCAT AACGCCTTCC AGAAGCAGAG TTACTGCACC 60

CAGGATGTC 69

(2) INFORMATION FOR SEQ ID NO:91:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:91:

CCGACCTGCC TGTCTTCCCT GCTTGGCCAG CTGTCTGGCC AGGTTCGTCT GCTGCTCGGC 60

GCTCTGCAG 69

(2) INFORMATION FOR SEQ ID NO:92:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:92:

CAGAGACTGC AGAGCGCCGA GCAGACGAAC CTGGCCAGAC AGCTGGCCAA GCAGGGAAGA 60

CAGGCA 66

(2) INFORMATION FOR SEQ ID NO:93:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:93:

TCTCTGCTTG GCACCCAGCT GCCGCCACAG GGCCGTACCA CTGCTCACAA GGATCCGAAC 60

GCTATCTTCC 70

(2) INFORMATION FOR SEQ ID NO:94:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:94:

AAGACAGGAA GATAGCGTTC GGATCCTTGT GAGCAGTGGT ACGGCCCTGT GGCGGCAGCT 60

GGGTGCCAAG 70

(2) INFORMATION FOR SEQ ID NO:95:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:95:

GGAGGAGACC AAGGCACAGG A 21

(2) INFORMATION FOR SEQ ID NO:96:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:96:

CCGGAATTCT TACCCTTCCT GAGACAGATT 30

(2) INFORMATION FOR SEQ ID NO:97:

(i) SEQUENCE CHARACTERISTICS:
(A) LENGTH: 8 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:97:

CTAATGAG 8

(2) INFORMATION FOR SEQ ID NO:98:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:98:

AATTCTCATT AGAGCT 16

(2) INFORMATION FOR SEQ ID NO:99:

(i) SEQUENCE CHARACTERISTICS:
(A) LENGTH: 8 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:99:

CTAATGAG 8

(2) INFORMATION FOR SEQ ID NO:100:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:100:

AATTCTCATT AGAGCT 16

(2) INFORMATION FOR SEQ ID NO:101:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:101:

ATCGCCGGCT CCGCCAGCTT GTGAC 25

(2) INFORMATION FOR SEQ ID NO:102:

(i) SEQUENCE CHARACTERISTICS:
(A) LENGTH: 30 base pairs
(B) TYPE: nucleic acid

(C) STRANDEDNESS: single
(D) TOPOLOGY: linear

(ii) MOLECULE TYPE: Other nucleic acid

(xi) SEQUENCE DESCRIPTION: SEQ ID NO:102:

GCCGAATTCT CATTAGAGCT CGTTCAGTGT 30

(2) INFORMATION FOR SEQ ID NO:103:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:103:

GATCCGAACG CTATCTTCCT GTCTTTCCAG CACCTGCTGC GT 42

(2) INFORMATION FOR SEQ ID NO:104:

(i) SEQUENCE CHARACTERISTICS:
(A) LENGTH: 44 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:104:

TTTGCCACGC AGCAGGTGCT GGAAAGACAG GAAGATAGCG TTCG 44

(2) INFORMATION FOR SEQ ID NO:105:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:105:

GGCAAAGTTC GTTTCCTGAT GCTGGTTGGC GGTTCTACCC TG 42

(2) INFORMATION FOR SEQ ID NO:106:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:106:

ACGCACAGGG TAGAACCGCC AACCAGCATC AGGAAACGAA C 41

(2) INFORMATION FOR SEQ ID NO:107:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:107:

TGCGTTCGTC GGGCGCCGCC AACCACTGCT GTTCCGTCTT AATGAA 46

(2) INFORMATION FOR SEQ ID NO:108:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:108:

AGCTTTCATT AAGACGGAAC AGCAGTGGTT GGCGGCGCCC GACGA 45

(2) INFORMATION FOR SEQ ID NO:109:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:109:

AAGGATCCGA ACGCTATCTT CCTG 24

(2) INFORMATION FOR SEQ ID NO:110:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:110:

AGAAGCTTTC ATTAAGACGG AACA 24

(2) INFORMATION FOR SEQ ID NO:111:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:111:

CTAGAAAAAA CCAAGGAGGT AATAAATAAT GAAAGCACCT GTACCA 46

(2) INFORMATION FOR SEQ ID NO:112:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:112:

CAGGTGGTAC AGGTGCTTTC ATTATTTATT ACCTCCTTGG TTTTTT 46

(2) INFORMATION FOR SEQ ID NO:113:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:113:

CCTGCATGTG ATTTACGGGT CCTGTCTAAA CTGCTGCG 38

(2) INFORMATION FOR SEQ ID NO:114:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:114:

CGCAGCAGTT TAGACAGGAC CCGTAAATCA CATG 34

(2) INFORMATION FOR SEQ ID NO:115:

(i) SEQUENCE CHARACTERISTICS:
(A) LENGTH: 84 base pairs
(B) TYPE: nucleic acid
(C) STRANDEDNESS: double
(D) TOPOLOGY: linear

(ii) MOLECULE TYPE: Other nucleic acid

(xi) SEQUENCE DESCRIPTION: SEQ ID NO:115:

CTAGAAAAAA CCAAGGAGGT AATAAATAAT GAAAGCACCT GTACCACCTG CATGTGATTT 60

ACGGGTCCTG TCTAAACTGC TGCG 84

(2) INFORMATION FOR SEQ ID NO:116:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:116:

Pro Arg Leu Leu Asn Lys Leu Leu Arg Asp Ser Tyr Leu Leu His Arg
1 5 10 15

Arg Leu Ser Gln
20

(2) INFORMATION FOR SEQ ID NO:117:

(i) SEQUENCE CHARACTERISTICS:
(A) LENGTH: 21 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:117:

Phe Ser Leu Gly Glu Trp Lys Thr Gln Thr Glu Gln Ser Lys Ala Gln
1 5 10 15

Asp Ile Leu Gly Ala
20

(2) INFORMATION FOR SEQ ID NO:118:

(i) SEQUENCE CHARACTERISTICS:
(A) LENGTH: 18 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:118:

Ser Arg Thr Ser Gln Leu Leu Thr Leu Asn Lys Phe Pro Asn Arg Leu
1 5 10 15

Leu Asp

(2) INFORMATION FOR SEQ ID NO:119:

(i) SEQUENCE CHARACTERISTICS:
(A) LENGTH: 21 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:119:

Asp Leu Arg Val Leu Ser Lys Leu Leu Arg Asp Ser His Val Leu His
1 5 10 15

Ser Arg Leu Ser Gln
20

(2) INFORMATION FOR SEQ ID NO:120:

(i) SEQUENCE CHARACTERISTICS:
(A) LENGTH: 16 amino acids
(B) TYPE: amino acid
(C) STRANDEDNESS:

(D) TOPOLOGY: linear

(ii) MOLECULE TYPE: peptide

(xi) SEQUENCE DESCRIPTION: SEQ ID NO:120:

```
Ser Leu Gly Glu Trp Lys Thr Gln Met Glu Glu Thr Lys Ala Gln Asp
 1               5                  10                  15
```

(2) INFORMATION FOR SEQ ID NO:121:

(i) SEQUENCE CHARACTERISTICS:
(A) LENGTH: 19 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:121:

```
Leu Gly Thr Gln Leu Pro Pro Gln Gly Arg Thr Thr Ala His Lys Asp
 1               5                  10                  15

Pro Asn Ala
```

(2) INFORMATION FOR SEQ ID NO:122:

(i) SEQUENCE CHARACTERISTICS:
(A) LENGTH: 19 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:122:

```
Asn Glu Leu Pro Asn Arg Thr Ser Gly Leu Leu Glu Thr Asn Phe Thr
 1               5                  10                  15

Ala Ser Ala
```

(2) INFORMATION FOR SEQ ID NO:123:

(i) SEQUENCE CHARACTERISTICS:
(A) LENGTH: 23 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:123:

```
Ser Leu Pro Pro Asn Leu Gln Pro Gly Tyr Ser Pro Ser Pro Thr His
 1               5                  10                  15

Pro Pro Thr Gly Gln Tyr Thr
            20
```

(2) INFORMATION FOR SEQ ID NO:124:

(i) SEQUENCE CHARACTERISTICS:
(A) LENGTH: 27 amino acids
(B) TYPE: amino acid
(C) STRANDEDNESS:

(D) TOPOLOGY: linear

(ii) MOLECULE TYPE: peptide

(xi) SEQUENCE DESCRIPTION: SEQ ID NO:124:

```
Pro Ser Ala Pro Thr Pro Thr Pro Thr Ser Pro Leu Leu Asn Thr Ser
  1               5                  10                  15

Tyr Thr His Ser Gln Asn Leu Ser Gln Glu Gly
            20                  25
```

(2) INFORMATION FOR SEQ ID NO:125:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:125:

CTAGAAAAAA CCAAGGAGGT AATAAATAAT GAAATCTCCT GCACCA 46

(2) INFORMATION FOR SEQ ID NO:126:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:126:

CAGGTGGTGC AGGAGATTTC ATTATTTATT ACCTCCTTGG TTTTTT 46

(2) INFORMATION FOR SEQ ID NO:127:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:127:

CCTGCATGTG ATTTACGGGT CCTGTCTAAA CTGCTGCG 38

(2) INFORMATION FOR SEQ ID NO:128:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:128:

CGCAGCAGTT TAGACAGGAC CCGTAAATCA CATG 34

(2) INFORMATION FOR SEQ ID NO:129:

(i) SEQUENCE CHARACTERISTICS:
(A) LENGTH: 84 base pairs
(B) TYPE: nucleic acid
(C) STRANDEDNESS: double
(D) TOPOLOGY: linear

(ii) MOLECULE TYPE: Other nucleic acid

(xi) SEQUENCE DESCRIPTION: SEQ ID NO:129:

CTAGAAAAAA CCAAGGAGGT AATAAATAAT GAAATCTCCT GCACCACCTG CATGTGATTT 60

ACGGGTCCTG TCTAAACTGC TGCG 84

(2) INFORMATION FOR SEQ ID NO:130:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:130:

CTCCCGAACC GTACCAGCGG CCTGCTGGAA ACCAACTTTA CCGCGAG 47

(2) INFORMATION FOR SEQ ID NO:131:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:131:

GGTAAAGTTG GTTTCCAGCA GGCCGCTGGT ACGGTTCGGG AGCTCGT 47

(2) INFORMATION FOR SEQ ID NO:132:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:132:

CGCGCGTACC ACCGGCAGCG GCCTGCTGAA ATGGCAGCAG GGCTTTCGT 49

(2) INFORMATION FOR SEQ ID NO:133:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:133:

AGCCCTGCTG CCATTTCAGC AGGCCGCTGC CGGTGGTACG CGCGCTCGC 49

(2) INFORMATION FOR SEQ ID NO:134:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:134:

GCGAAAATCC CGGGCCTGCT GAACCAGACC AGCCGTAGCC TGGATCAGAT 50

(2) INFORMATION FOR SEQ ID NO:135:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:135:

ATCCAGGCTA CGGCTGGTCT GGTTCAGCAG GCCCGGGATT TTCGCACGAA 50

(2) INFORMATION FOR SEQ ID NO:136:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:136:

CCCGGGCTAT CTGAACCGTA TCCATGAACT GCTGAACGGC ACCCGTG 47

(2) INFORMATION FOR SEQ ID NO:137:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:137:

GTGCCGTTCA GCAGTTCATG GATACGGTTC AGATAGCCCG GGATCTG 47

(2) INFORMATION FOR SEQ ID NO:138:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:138:

GCCTGTTTCC GGGCCCGAGC CGTCGCACCC TGGGCGCGCC GGATATCAG 49

(2) INFORMATION FOR SEQ ID NO:139:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:139:

ATCCGGCGCG CCCAGGGTGC GACGGCTCGG GCCCGGAAAC AGGCCACGG 49

(2) INFORMATION FOR SEQ ID NO:140:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:140:

ATCAGCTCTG GCACCAGCGA TACCGGCAGC CTGCCGCCGA ACCTGCAGCC 50

(2) INFORMATION FOR SEQ ID NO:141:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:141:

CAGGTTCGGC GGCAGGCTGC CGGTATCGCT GGTGCCAGAG CTGATATCCG 50

(2) INFORMATION FOR SEQ ID NO:142:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:142:

GGGCTATAGC CCGAGCCCGA CCCATCCGCC GACCGGCCAG TATACCCTGT T 51

(2) INFORMATION FOR SEQ ID NO:143:

(i) SEQUENCE CHARACTERISTICS:
(A) LENGTH: 51 base pairs
(B) TYPE: nucleic acid

(C) STRANDEDNESS: single
(D) TOPOLOGY: linear

(ii) MOLECULE TYPE: Other nucleic acid

(xi) SEQUENCE DESCRIPTION: SEQ ID NO:143:

GGTATACTGG CCGGTCGGCG GATGGGTCGG GCTCGGGCTA TAGCCCGGCT G 51

(2) INFORMATION FOR SEQ ID NO:144:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:144:

TCCGCTGCCG CCGACCCTGC CGACCCCGGT GGTTCAGCTG CATCCGCTGC 50

(2) INFORMATION FOR SEQ ID NO:145:

(i) SEQUENCE CHARACTERISTICS:
(A) LENGTH: 50 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:145:

GGATGCAGCT GAACCACCGG GGTCGGCAGG GTCGGCGGCA GCGGAAACAG 50

(2) INFORMATION FOR SEQ ID NO:146:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:146:

TGCCGGATCC GAGCGCGCCG ACCCCGACCC CGACCAGCCC GCTGCTGAAC A 51

(2) INFORMATION FOR SEQ ID NO:147:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:147:

AGCAGCGGGC TGGTCGGGGT CGGGGTCGGC GCGCTCGGAT CCGGCAGCAG C 51

(2) INFORMATION FOR SEQ ID NO:148:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:148:

CCAGCTATAC CCATAGCCAG AACCTGAGCC AGGAAGGCTA ATGAAGCTTG A 51

(2) INFORMATION FOR SEQ ID NO:149:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:149:

CTTCATTAGC CTTCCTGGCT CAGGTTCTGG CTATGGGTAT AGCTGGTGTT C 51

(2) INFORMATION FOR SEQ ID NO:150:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:150:

ACGAGCTCCC GAACCGTACC A 21

(2) INFORMATION FOR SEQ ID NO:151:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:151:

CTGATATCCG GCGCGCCCAG G 21

(2) INFORMATION FOR SEQ ID NO:152:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:152:

CGGATATCAG CTCTGGCACC A 21

(2) INFORMATION FOR SEQ ID NO:153:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:153:

```
TCAAGCTTCA TTAGCCTTCC T                                          21
```

(2) INFORMATION FOR SEQ ID NO:154:

(i) SEQUENCE CHARACTERISTICS:
(A) LENGTH: 490 base pairs
(B) TYPE: nucleic acid
(C) STRANDEDNESS: double
(D) TOPOLOGY: linear

(ii) MOLECULE TYPE: Other nucleic acid

(xi) SEQUENCE DESCRIPTION: SEQ ID NO:154:

```
CTC CCG AAC CGT ACC AGC GGC CTG CTG GAA ACC AAC TTT ACC GCG AGC    48
Leu Pro Asn Arg Thr Ser Gly Leu Leu Glu Thr Asn Phe Thr Ala Ser
 1               5                  10                  15

GCG CGT ACC ACC GGC AGC GGC CTG CTG AAA TGG CAG CAG GGC TTT CGT    96
Ala Arg Thr Thr Gly Ser Gly Leu Leu Lys Trp Gln Gln Gly Phe Arg
            20                  25                  30

GCG AAA ATC CCG GGC CTG CTG AAC CAG ACC AGC CGT AGC CTG GAT CAG   144
Ala Lys Ile Pro Gly Leu Leu Asn Gln Thr Ser Arg Ser Leu Asp Gln
        35                  40                  45

ATC CCG GGC TAT CTG AAC CGT ATC CAT GAA CTG CTG AAC GGC ACC CGT   192
Ile Pro Gly Tyr Leu Asn Arg Ile His Glu Leu Leu Asn Gly Thr Arg
    50                  55                  60

GGC CTG TTT CCG GGC CCG AGC CGT CGC ACC CTG GGC GCG CCG GAT ATC   240
Gly Leu Phe Pro Gly Pro Ser Arg Arg Thr Leu Gly Ala Pro Asp Ile
65                  70                  75                  80

AGC TCT GGC ACC AGC GAT ACC GGC AGC CTG CCG CCG AAC CTG CAG CCG   288
Ser Ser Gly Thr Ser Asp Thr Gly Ser Leu Pro Pro Asn Leu Gln Pro
                85                  90                  95

GGC TAT AGC CCG AGC CCG ACC CAT CCG CCG ACC GGC CAG TAT ACC CTG   336
Gly Tyr Ser Pro Ser Pro Thr His Pro Pro Thr Gly Gln Tyr Thr Leu
            100                 105                 110

TTT CCG CTG CCG CCG ACC CTG CCG ACC CCG GTG GTT CAG CTG CAT CCG   384
Phe Pro Leu Pro Pro Thr Leu Pro Thr Pro Val Val Gln Leu His Pro
        115                 120                 125

CTG CTG CCG GAT CCG AGC GCG CCG ACC CCG ACC CCG ACC AGC CCG CTG   432
Leu Leu Pro Asp Pro Ser Ala Pro Thr Pro Thr Pro Thr Ser Pro Leu
    130                 135                 140

CTG AAC ACC AGC TAT ACC CAT AGC CAG AAC CTG AGC CAG GAA GGC TAA   480
Leu Asn Thr Ser Tyr Thr His Ser Gln Asn Leu Ser Gln Glu Gly
145                 145                 150

TGAAGCTTGA                                                        490
```

(2) INFORMATION FOR SEQ ID NO:155:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:155:

AAGGATCCGA ACGCTATCTT CCTG 24

(2) INFORMATION FOR SEQ ID NO:156:

(i) SEQUENCE CHARACTERISTICS:
(A) LENGTH: 56 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:156:

GGGAGCTCGT TCAGGGTCAG AACCAGAGAG GTACGAGACG GAACAGCAGT GGTTGG 56

(2) INFORMATION FOR SEQ ID NO:157:

(i) SEQUENCE CHARACTERISTICS:
(A) LENGTH: 157 base pairs
(B) TYPE: nucleic acid
(C) STRANDEDNESS: double
(D) TOPOLOGY: linear

(ii) MOLECULE TYPE: Other nucleic acid

(xi) SEQUENCE DESCRIPTION: SEQ ID NO:157:

```
G GAT CCG AAC GCT ATC TTC CTG TCT TTC CAG CAC CTG CTG CGT GGC          46
  Asp Pro Asn Ala Ile Phe Leu Ser Phe Gln His Leu Leu Arg Gly
    1               5                  10                  15

AAA GTT CGT TTC CTG ATG CTG GTT GGC GGT TCT ACC CTG TGC GTT CGT          94
Lys Val Arg Phe Leu Met Leu Val Gly Gly Ser Thr Leu Cys Val Arg
                20                  25                  30

CGG GCG CCG CCA ACC ACT GCT GTT CCG TCT CGT ACC TCT CTG GTT CTG         142
Arg Ala Pro Pro Thr Thr Ala Val Pro Ser Arg Thr Ser Leu Val Leu
            35                  40                  45

ACC CTG AAC GAG CTC                                                     157
Thr Leu Asn Glu Leu
        50
```

(2) INFORMATION FOR SEQ ID NO:158:

(i) SEQUENCE CHARACTERISTICS:
(A) LENGTH: 100 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:158:

CCTCGAGGAA TTCCTGCAGC CCGGGACTAG TATCGGCTAC CCCTACGACG TCCCCGACTA 60

CGCCGGCGTC CATCACCATC ACCATCACTG AGCGGCCGCC 100

(2) INFORMATION FOR SEQ ID NO:159:

(i) SEQUENCE CHARACTERISTICS:
(A) LENGTH: 108 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:159:

AATTGGCGGC CGCTCAGTGA TGGTGATGGT GATGAGCGCC GGCGTAGTCG GGGACGTCGT 60

AGGGGTAGCC GATACTAGTC CCGGGCTGCA GGAATTCCTC GAGGGTAC 108

(2) INFORMATION FOR SEQ ID NO:160:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:160:

AATTCCAAGA TCTCACACTT GCTTTTGACA CAACTGTGTT TACTTGCAAT CCCCCAAAAC 60

AGACAGACCC 70

(2) INFORMATION FOR SEQ ID NO:161:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:161:

GGGTCTGTCT GTTTTGGGGG ATTGCAAGTA AACACAGTTG TGTCAAAAGC AAGTGTGAGA 60

TCTTGG 66

(2) INFORMATION FOR SEQ ID NO:162:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:162:

GGCCCGGGAT GGAGCTGACT GAATTGCTC 29

(2) INFORMATION FOR SEQ ID NO:163:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:163:

TCAAGCTTAC TAGTCCCTTC CTGAGACAGA TTCTG 35

(2) INFORMATION FOR SEQ ID NO:164:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:164:

TAATACGACT CACTATAGGG CG 22

(2) INFORMATION FOR SEQ ID NO:165:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:165:

AATTCCAAGA TCACACACTT GC 22

(2) INFORMATION FOR SEQ ID NO:166:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:166:

TCAAGCTTAC TAGTCCCTTC CTGAGACAGA TTCTG 35

(2) INFORMATION FOR SEQ ID NO:167:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:167:

TGGGCACTGG CTCAGGTTGC TGTGAAGGAC ATGGG 35

(2) INFORMATION FOR SEQ ID NO:168:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:168:

TGTAGGCAAA GGGGTAACCT CTGGGCA 27

(2) INFORMATION FOR SEQ ID NO:169:

(i) SEQUENCE CHARACTERISTICS:
(A) LENGTH: 21 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:169:

Thr Ser Ile Gly Tyr Pro Tyr Asp Val Pro Asp Tyr Ala Gly Val His
1 5 10 15

Xaa Xaa Xaa Xaa Xaa
20

(2) INFORMATION FOR SEQ ID NO:170:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:170:

TTTGAATTCG GCCAGCCAGA CACCCCGGCC 30

(2) INFORMATION FOR SEQ ID NO:171:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:171:

TTTGCGGCCG CTCATTATTC GTGTATCCTG TTCAGGTATC C 41

(2) INFORMATION FOR SEQ ID NO:172:

(i) SEQUENCE CHARACTERISTICS:
(A) LENGTH: 24 base pairs
(B) TYPE: nucleic acid

(C) STRANDEDNESS: single
(D) TOPOLOGY: linear

(ii) MOLECULE TYPE: Other nucleic acid

(xi) SEQUENCE DESCRIPTION: SEQ ID NO:172:

TGTAGGCAAA GGAACCTCTG GGCA 24

(2) INFORMATION FOR SEQ ID NO:173:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:173:

TGTAGGCAAA GGAGTGTGAA CCTCTGG 27

(2) INFORMATION FOR SEQ ID NO:174:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:174:

TGTAGGCAAA GGAGCGTGAA CCTCTGG 27

(2) INFORMATION FOR SEQ ID NO:175:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:175:

TGTAGGCAAA GGTCCGTGAA CCTCTGG 27

(2) INFORMATION FOR SEQ ID NO:176:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:176:

AGCTGTGGTC CTCTGTGGAG GAAG 24

(2) INFORMATION FOR SEQ ID NO:177:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:177:

GTGAGCTGTG GTGCCCTGTG GAGG 24

(2) INFORMATION FOR SEQ ID NO:178:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:178:

AGCTGTGGTC CTGTTGCCCT GTGGAGG 27

(2) INFORMATION FOR SEQ ID NO:179:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:179:

AGCTGTGGTC CTAGCGCCCT GTGGAGG 27

(2) INFORMATION FOR SEQ ID NO:180:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:180:

AGCTGTGGTC CTTCCGCCCT GTGGAGG 27

(2) INFORMATION FOR SEQ ID NO:181:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:181:

CGGGCTGCAG GATATCCAAG ATCTCA 26

(2) INFORMATION FOR SEQ ID NO:182:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:182:

TAATACGACT CACTATAGGG CG 22

(2) INFORMATION FOR SEQ ID NO:183:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: Other nucleic acid

(xi) SEQUENCE DESCRIPTION: SEQ ID NO:183:

GGGCGGCCGC TCAGCTGGGG ACAGCTGTGG TGGG 34

(2) INFORMATION FOR SEQ ID NO:184:

(i) SEQUENCE CHARACTERISTICS:
(A) LENGTH: 7 amino acids
(B) TYPE: amino acid
(C) STRANDEDNESS:
(D) TOPOLOGY: linear

(ii) MOLECULE TYPE: peptide

(ix) FEATURE:
(A) NAME/KEY: misc_feature
(D) OTHER INFORMATION:
/note= "The amino acid at position 2 is Ala, Ser, Gly, Met or Gln."
(ix) FEATURE:
(A) NAME/KEY: misc_feature
(D) OTHER INFORMATION:
/note= "The amino acid at position 7 is Glu or Lys."

(xi) SEQUENCE DESCRIPTION: SEQ ID NO:184:

Lys Xaa Tyr Tyr Glu Ser Xaa
1 5

(2) INFORMATION FOR SEQ ID NO:185:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(ix) FEATURE:
(A) NAME/KEY: misc_feature
(D) OTHER INFORMATION:

/note= "The amino acid at position 6 is Glu or Asp."

(xi) SEQUENCE DESCRIPTION: SEQ ID NO:185:

Lys Glx Arg Ala Ala Xaa
1 5

(2) INFORMATION FOR SEQ ID NO:186:

(i) SEQUENCE CHARACTERISTICS:
(A) LENGTH: 7 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:186:

Lys Ala Gly Xaa Cys Ser Gly
1 5

(2) INFORMATION FOR SEQ ID NO:187:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(ix) FEATURE:
(A) NAME/KEY: misc_feature
(D) OTHER INFORMATION:
/note= "The amino acid at position 2 is Ile, Thr or Ser."

(xi) SEQUENCE DESCRIPTION: SEQ ID NO:187:

Lys Xaa Pro Val Pro Pro Ala Cys Asp Pro Arg Leu Leu
1 5 10

(2) INFORMATION FOR SEQ ID NO:188:

(i) SEQUENCE CHARACTERISTICS:
(A) LENGTH: 9 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:188:

Lys Asp Ser Phe Leu Ala Asp Val Lys
1 5

(2) INFORMATION FOR SEQ ID NO:189:

(i) SEQUENCE CHARACTERISTICS:
(A) LENGTH: 9 amino acids
(B) TYPE: amino acid
(C) STRANDEDNESS:

(D) TOPOLOGY: linear

(ii) MOLECULE TYPE: peptide

(ix) FEATURE:
(A) NAME/KEY: misc_feature
(D) OTHER INFORMATION:
/note= "The amino acid at position 1 is Lysine or Arginine."

(xi) SEQUENCE DESCRIPTION: SEQ ID NO:189:

```
Xaa Thr Leu Pro Thr Xaa Ala Val Pro
 1               5
```

(2) INFORMATION FOR SEQ ID NO:190:

(i) SEQUENCE CHARACTERISTICS:
(A) LENGTH: 15 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO:190:

```
Lys Asp Ser Phe Leu Ala Asp Val Lys Gln Tyr Tyr Glu Ser Glu
 1               5                  10                  15
```

(2) INFORMATION FOR SEQ ID NO:191:

(i) SEQUENCE CHARACTERISTICS:
(A) LENGTH: 332 amino acids
(B) TYPE: amino acid
(D) TOPOLOGY: linear

(ii) MOLECULE TYPE: protein

(vi) ORIGINAL SOURCE:
(A) ORGANISM: Homo Sapiens

(xi) SEQUENCE DESCRIPTION: SEQ ID NO:191:

```
Ser Pro Ala Pro Pro Ala Cys Asp Leu Arg Val Leu Ser Lys Leu Leu
 1               5                  10                  15

Arg Asp Ser His Val Leu His Ser Arg Leu Ser Gln Cys Pro Glu Val
            20                  25                  30

His Pro Leu Pro Thr Pro Val Leu Leu Pro Ala Val Asp Phe Ser Leu
        35                  40                  45

Gly Glu Trp Lys Thr Gln Met Glu Glu Thr Lys Ala Gln Asp Ile Leu
    50                  55                  60

Gly Ala Val Thr Leu Leu Leu Glu Gly Val Met Ala Ala Arg Gly Gln
65                  70                  75                  80

Leu Gly Pro Thr Cys Leu Ser Ser Leu Leu Gly Gln Leu Ser Gly Gln
                85                  90                  95

Val Arg Leu Leu Leu Gly Ala Leu Gln Ser Leu Leu Gly Thr Gln Leu
            100                 105                 110

Pro Pro Gln Gly Arg Thr Thr Ala His Lys Asp Pro Asn Ala Ile Phe
        115                 120                 125
```

```
Leu Ser Phe Gln His Leu Leu Arg Gly Lys Val Arg Phe Leu Met Leu
    130                 135                 140

Val Gly Gly Ser Thr Leu Cys Val Arg Arg Ala Pro Pro Thr Thr Ala
145                 150                 155                 160

Val Pro Ser Arg Thr Ser Leu Val Leu Thr Leu Asn Glu Leu Pro Asn
                165                 170                 175

Arg Thr Ser Gly Leu Leu Glu Thr Asn Phe Thr Ala Ser Ala Arg Thr
            180                 185                 190

Thr Gly Ser Gly Leu Leu Lys Trp Gln Gln Gly Phe Arg Ala Lys Ile
        195                 200                 205

Pro Gly Leu Leu Asn Gln Thr Ser Arg Ser Leu Asp Gln Ile Pro Gly
    210                 215                 220

Tyr Leu Asn Arg Ile His Glu Leu Leu Asn Gly Thr Arg Gly Leu Phe
225                 230                 235                 240

Pro Gly Pro Ser Arg Arg Thr Leu Gly Ala Pro Asp Ile Ser Ser Gly
                245                 250                 255

Thr Ser Asp Thr Gly Ser Leu Pro Pro Asn Leu Gln Pro Gly Tyr Ser
            260                 265                 270

Pro Ser Pro Thr His Pro Pro Thr Gly Gln Tyr Thr Leu Phe Pro Leu
        275                 280                 285

Pro Pro Thr Leu Pro Thr Pro Val Val Gln Leu His Pro Leu Leu Pro
    290                 295                 300

Asp Pro Ser Ala Pro Thr Pro Thr Pro Thr Ser Pro Leu Leu Asn Thr
305                 310                 315                 320

Ser Tyr Thr His Ser Gln Asn Leu Ser Gln Glu Gly
                325                 330
```

(2) INFORMATION FOR SEQ ID NO:192:

(i) SEQUENCE CHARACTERISTICS:
(A) LENGTH: 1086 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens

(xi) SEQUENCE DESCRIPTION: SEQ ID NO:192:

```
GGCCAGCCAG ACACCCCGGC CAGA ATG GAG CTG ACT GAA TTG CTC CTC GTG       51
                           Met Glu Leu Thr Glu Leu Leu Leu Val
                           -21 -20                 -15

GTC ATG CTT CTC CTA ACT GCA AGG CTA ACG CTG TCC AGC CCG GCT CCT      99
Val Met Leu Leu Leu Thr Ala Arg Leu Thr Leu Ser Ser Pro Ala Pro
    -10                 -5                  1

CCT GCT TGT GAC CTC CGA GTC CTC AGT AAA CTG CTT CGT GAC TCC CAT     147
Pro Ala Cys Asp Leu Arg Val Leu Ser Lys Leu Leu Arg Asp Ser His
  5                 10                  15                  20

GTC CTT CAC AGC AGA CTG AGC CAG TGC CCA GAG GTT CAC CCT TTG CCT     195
```

```
Val Leu His Ser Arg Leu Ser Gln Cys Pro Glu Val His Pro Leu Pro
                25                  30                  35

ACA CCT GTC CTG CTG CCT GCT GTG GAC TTT AGC TTG GGA GAA TGG AAA      243
Thr Pro Val Leu Leu Pro Ala Val Asp Phe Ser Leu Gly Glu Trp Lys
            40                  45                  50

ACC CAG ATG GAG GAG ACC AAG GCA CAG GAC ATT CTG GGA GCA GTG ACC      291
Thr Gln Met Glu Glu Thr Lys Ala Gln Asp Ile Leu Gly Ala Val Thr
        55                  60                  65

CTT CTG CTG GAG GGA GTG ATG GCA GCA CGG GGA CAA CTG GGA CCC ACT      339
Leu Leu Leu Glu Gly Val Met Ala Ala Arg Gly Gln Leu Gly Pro Thr
    70                  75                  80

TGC CTC TCA TCC CTC CTG GGG CAG CTT TCT GGA CAG GTC CGT CTC CTC      387
Cys Leu Ser Ser Leu Leu Gly Gln Leu Ser Gly Gln Val Arg Leu Leu
85                  90                  95                 100

CTT GGG GCC CTG CAG AGC CTC CTT GGA ACC CAG CTT CCT CCA CAG GGC      435
Leu Gly Ala Leu Gln Ser Leu Leu Gly Thr Gln Leu Pro Pro Gln Gly
                105                 110                 115

AGG ACC ACA GCT CAC AAG GAT CCC AAT GCC ATC TTC CTG AGC TTC CAA      483
Arg Thr Thr Ala His Lys Asp Pro Asn Ala Ile Phe Leu Ser Phe Gln
            120                 125                 130

CAC CTG CTC CGA GGA AAG GTG CGT TTC CTG ATG CTT GTA GGA GGG TCC      531
His Leu Leu Arg Gly Lys Val Arg Phe Leu Met Leu Val Gly Gly Ser
        135                 140                 145

ACC CTC TGC GTC AGG CGG GCC CCA CCC ACC ACA GCT GTC CCC AGC AGA      579
Thr Leu Cys Val Arg Arg Ala Pro Pro Thr Thr Ala Val Pro Ser Arg
    150                 155                 160

ACC TCT CTA GTC CTC ACA CTG AAC GAG CTC CCA AAC AGG ACT TCT GGA      627
Thr Ser Leu Val Leu Thr Leu Asn Glu Leu Pro Asn Arg Thr Ser Gly
165                 170                 175                 180

TTG TTG GAG ACA AAC TTC ACT GCC TCA GCC AGA ACT ACT GGC TCT GGG      675
Leu Leu Glu Thr Asn Phe Thr Ala Ser Ala Arg Thr Thr Gly Ser Gly
                185                 190                 195

CTT CTG AAG TGG CAG CAG GGA TTC AGA GCC AAG ATT CCT GGT CTG CTG      723
Leu Leu Lys Trp Gln Gln Gly Phe Arg Ala Lys Ile Pro Gly Leu Leu
            200                 205                 210

AAC CAA ACC TCC AGG TCC CTG GAC CAA ATC CCC GGA TAC CTG AAC AGG      771
Asn Gln Thr Ser Arg Ser Leu Asp Gln Ile Pro Gly Tyr Leu Asn Arg
        215                 220                 225

ATA CAC GAA CTC TTG AAT GGA ACT CGT GGA CTC TTT CCT GGA CCC TCA      819
Ile His Glu Leu Leu Asn Gly Thr Arg Gly Leu Phe Pro Gly Pro Ser
    230                 235                 240

CGC AGG ACC CTA GGA GCC CCG GAC ATT TCC TCA GGA ACA TCA GAC ACA      867
Arg Arg Thr Leu Gly Ala Pro Asp Ile Ser Ser Gly Thr Ser Asp Thr
245                 250                 255                 260

GGC TCC CTG CCA CCC AAC CTC CAG CCT GGA TAT TCT CCT TCC CCA ACC      915
Gly Ser Leu Pro Pro Asn Leu Gln Pro Gly Tyr Ser Pro Ser Pro Thr
                265                 270                 275

CAT CCT CCT ACT GGA CAG TAT ACG CTC TTC CCT CTT CCA CCC ACC TTG      963
His Pro Pro Thr Gly Gln Tyr Thr Leu Phe Pro Leu Pro Pro Thr Leu
            280                 285                 290
```

```
CCC ACC CCT GTG GTC CAG CTC CAC CCC CTG CTT CCT GAC CCT TCT GCT     1011
Pro Thr Pro Val Val Gln Leu His Pro Leu Leu Pro Asp Pro Ser Ala
        295                 300                 305

CCA ACG CCC ACC CCT ACC AGC CCT CTT CTA AAC ACA TCC TAC ACC CAC     1059
Pro Thr Pro Thr Pro Thr Ser Pro Leu Leu Asn Thr Ser Tyr Thr His
    310                 315                 320

TCC CAG AAT CTG TCT CAG GAA GGG TAA                                 1086
Ser Gln Asn Leu Ser Gln Glu Gly
325                 330
```

(2) INFORMATION FOR SEQ ID NO:193:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:193:

```
Lys Gln Tyr Tyr Glu Ser Glu
  1               5
```

(2) INFORMATION FOR SEQ ID NO:194:

(i) SEQUENCE CHARACTERISTICS:
(A) LENGTH: 1663 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Rattus norvegicus
(H) CELL LINE: McA-RH8994

(xi) SEQUENCE DESCRIPTION: SEQ ID NO:194:

```
GGTGTACCTG GGTCCTGAAG CCCTTCTTCA CCTGGATAGA TTCCTTGGCC CACCTGTCCC     60

CACCCCACTC TGTGCAGAGG TACAAAAGCT CAAGCCGTCT CCATGGCCCC AGGAAAGATT    120

CAGGGGAGAG GCCCCACACA GGGAGCCACT GCAGTCAGAC ACCCTGGGCA GA ATG        175
                                                          Met
                                                          -21

GAG CTG ACT GAT TTG CTC CTG GTG GCC ATA CTT CTC CTC ACC GCA AGA     223
Glu Leu Thr Asp Leu Leu Leu Val Ala Ile Leu Leu Leu Thr Ala Arg
-20                 -15                 -10                  -5

CTA ACT CTG TCC AGC CCA GTT CCT CCC GCC TGT GAC CCC AGA CTC CTA     271
Leu Thr Leu Ser Ser Pro Val Pro Pro Ala Cys Asp Pro Arg Leu Leu
                     1               5                  10

AAT AAA CTG CTT CGT GAC TCC TAC CTC CTT CAC AGC CGA CTG AGT CAG     319
Asn Lys Leu Leu Arg Asp Ser Tyr Leu Leu His Ser Arg Leu Ser Gln
         15                  20                  25

TGT CCT GAC GTC AAC CCT TTG TCT ATC CCT GTC CTG CTG CCT GCT GTG     367
Cys Pro Asp Val Asn Pro Leu Ser Ile Pro Val Leu Leu Pro Ala Val
    30                  35                  40
```

```
GAC TTT AGC CTG GGA GAA TGG AAA ACC CAG ACG GAA CAG AGC AAG GCA       415
Asp Phe Ser Leu Gly Glu Trp Lys Thr Gln Thr Glu Gln Ser Lys Ala
 45                 50                  55                  60

CAG GAC ATT CTA GGG GCA GTG TCC CTT CTA CTG GAG GGG GTG ATG GCA       463
Gln Asp Ile Leu Gly Ala Val Ser Leu Leu Leu Glu Gly Val Met Ala
                65                  70                  75

GCA CGA GGA CAG TTG GAA CCC TCC TGC CTC TCA TCC CTC CTG GGA CAG       511
Ala Arg Gly Gln Leu Glu Pro Ser Cys Leu Ser Ser Leu Leu Gly Gln
            80                  85                  90

CTT TCT GGT CAG GTT CGC CTC CTC TTG GGA GCC CTG CAG GGC CTC CTA       559
Leu Ser Gly Gln Val Arg Leu Leu Leu Gly Ala Leu Gln Gly Leu Leu
        95                  100                 105

GGA ACC CAG GTA AGT CCC CAG ACC TAT AGA AAC TAC CCT CTT ACT CAG       607
Gly Thr Gln Val Ser Pro Gln Thr Tyr Arg Asn Tyr Pro Leu Thr Gln
    110                 115                 120

TTC CTC TAAGGACCTG GGAAAAGACA AGGGATTCTA GATTCTAGGT GTCTTCAGTG       663
Phe Leu
125

TATGAAAGCT GGTCTATACG GAGTGATGCT TCTCAGCCAC AATACCTGGG TGCTGGCAGT     723

AAATCTTTCC ACCTTAGTGA GAAGAGGCCT GATATGTGGG CCAACTCACT GGCCTCAGGC     783

CCATCCTCTG CCTTCAGCTT CCTCCACAGG GCAGGACCAC AGCTCACAAG GACCCCAGTG     843

CCCTCTTCTT GAGCTTGCAA CAACTGCTTC GGGGAAAGGT GCGCTTCCTG CTGCTGGTAG     903

AAGGTCCCGC CCTCTGTGTC AGACGGACCC TTCCCACCAC AGCTGTCCCA AGCAGAACCT     963

CTCAACTCCT CACACTAAAC AAGTTCCCAA ACAGACTTCT GGATTGTTGG AGACGAACTT    1023

CAGTGTTGTA GCCAGAACTG CTGGCCCTGG ACTTCTGAAC AGGCTTCAAG GATTCAGAGC    1083

CAAGATTATT CCTGGTCAGC TAAATCAAAC CTCCGGGTCC TTAGACCAAA TCCCTGGATA    1143

CCTGAACGGG ACACACGAAC CTGTGAATGG AACTCATGGG CTCTTTGCTG GGACCTCACT    1203

ACAGACCCTG GAAGCCCCAG ACGTTGTGCC AGGAGCTTTC AACAAAGGCT CCTTGCCACT    1263

CAACCTCCAG AGTGGACTTC CTCCTATCCC AAGCCTTGCT GCTGATGGAT ACACACTTTT    1323

CCCTCCTTCA CCTACCTTCC CCACCCCTGG GTCTCCACCC CAGCTCCCCC CCGTTTCCTG    1383

ACCCCTCCAC CACCATACCT AACTCTACCA ACCCTCATCC AGGACTTGGT CTCAGTAAGC    1443

GTCCCGTGCA CTGGCACGGA GCGCGATCGT CTGCAACATC TCTCAGGGGC AAGCTTCCTC    1503

AGGAAGGCTC TGAGGCAGCT CACTAGACAT CCTGCTCTCG CCTAACGGGC CCTGGGAAAG    1563

GGATACACAG GCCAGGACAC TGTACAACCT TAGGAGCGAT TTTTTTCTTA ACCTATCAAC    1623

AATATTCATC AGAGCAAAAA AAAAAAAAAA AAAAAAAAAA                          1663
```

(2) INFORMATION FOR SEQ ID NO:195:

(i) SEQUENCE CHARACTERISTICS:
- (A) LENGTH: 861 base pairs
- (B) TYPE: nucleic acid
- (C) STRANDEDNESS: double
- (D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:195:

```
GGCCAGCCAG ACACCCCGGC CAGA ATG GAG CTG ACT GAA TTG CTC CTC GTG      51
                           Met Glu Leu Thr Glu Leu Leu Leu Val
                           -21 -20                 -15

GTC ATG CTT CTC CTA ACT GCA AGG CTA ACG CTG TCC AGC CCG GCT CCT     99
Val Met Leu Leu Leu Thr Ala Arg Leu Thr Leu Ser Ser Pro Ala Pro
        -10                 -5                  1

CCT GCT TGT GAC CTC CGA GTC CTC AGT AAA CTG CTT CGT GAC TCC CAT    147
Pro Ala Cys Asp Leu Arg Val Leu Ser Lys Leu Leu Arg Asp Ser His
  5                  10                  15                  20

GTC CTT CAC AGC AGA CTG AGC CAG TGC CCA GAG GTT CAC CCT TTG CCT    195
Val Leu His Ser Arg Leu Ser Gln Cys Pro Glu Val His Pro Leu Pro
                 25                  30                  35

ACA CCT GTC CTG CTG CCT GCT GTG GAC TTT AGC TTG GGA GAA TGG AAA    243
Thr Pro Val Leu Leu Pro Ala Val Asp Phe Ser Leu Gly Glu Trp Lys
             40                  45                  50

ACC CAG ATG GAG GAG ACC AAG GCA CAG GAC ATT CTG GGA GCA GTG ACC    291
Thr Gln Met Glu Glu Thr Lys Ala Gln Asp Ile Leu Gly Ala Val Thr
         55                  60                  65

CTT CTG CTG GAG GGA GTG ATG GCA GCA CGG GGA CAA CTG GGA CCC ACT    339
Leu Leu Leu Glu Gly Val Met Ala Ala Arg Gly Gln Leu Gly Pro Thr
     70                  75                  80

TGC CTC TCA TCC CTC CTG GGG CAG CTT TCT GGA CAG GTC CGT CTC CTC    387
Cys Leu Ser Ser Leu Leu Gly Gln Leu Ser Gly Gln Val Arg Leu Leu
 85                  90                  95                 100

CTT GGG GCC CTG CAG AGC CTC CTT GGA ACC CAG CTT CCT CCA CAG GGC    435
Leu Gly Ala Leu Gln Ser Leu Leu Gly Thr Gln Leu Pro Pro Gln Gly
                105                 110                 115

AGG ACC ACA GCT CAC AAG GAT CCC AAT GCC ATC TTC CTG AGC TTC CAA    483
Arg Thr Thr Ala His Lys Asp Pro Asn Ala Ile Phe Leu Ser Phe Gln
            120                 125                 130

CAC CTG CTC CGA GGA AAG GTG CGT TTC CTG ATG CTT GTA GGA GGG TCC    531
His Leu Leu Arg Gly Lys Val Arg Phe Leu Met Leu Val Gly Gly Ser
        135                 140                 145

ACC CTC TGC GTC AGG CGG GCC CCA CCC ACC ACA GCT GTC CCC AGC AGA    579
Thr Leu Cys Val Arg Arg Ala Pro Pro Thr Thr Ala Val Pro Ser Arg
    150                 155                 160

ACC TCT CTA GTC CTC ACA CTG AAC GAG CTC CCA AAC AGG ACT TCT GGA    627
Thr Ser Leu Val Leu Thr Leu Asn Glu Leu Pro Asn Arg Thr Ser Gly
165                 170                 175                 180

TTG TTG GAG ACA AAC TTC ACT GCC TCA GCC AGA ACT ACT GGC TCT GGG    675
Leu Leu Glu Thr Asn Phe Thr Ala Ser Ala Arg Thr Thr Gly Ser Gly
                185                 190                 195

CTT CTG AAG TGG CAG CAG GGA TTC AGA GCC AAG ATT CCT GGT CTG CTG    723
Leu Leu Lys Trp Gln Gln Gly Phe Arg Ala Lys Ile Pro Gly Leu Leu
            200                 205                 210

AAC CAA ACC TCC AGG TCC CTG GAC CAA ATC CCC GGA TAC CTG AAC AGG    771
```

```
Asn Gln Thr Ser Arg Ser Leu Asp Gln Ile Pro Gly Tyr Leu Asn Arg
        215                 220                 225

ATA CAC GAA CTC TTAAAAAAAA AAAAAAAAAA AAAAAAAAAA AAAAAAAAA        823
Ile His Glu Leu
    230

AAAAAAAAAA AAAAAAAAAA AAAAAAAAAA AAAAAAAA                         861
```

(2) INFORMATION FOR SEQ ID NO:196:

(i) SEQUENCE CHARACTERISTICS:
(A) LENGTH: 1267 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Homo Sapiens
(F) TISSUE TYPE: liver

(xi) SEQUENCE DESCRIPTION: SEQ ID NO:196:

```
GGCCAGCCAG ACACCCCGGC CAGA ATG GAG CTG ACT GAA TTG CTC CTC GTG     51
                           Met Glu Leu Thr Glu Leu Leu Leu Val
                           -21 -20                 -15

GTC ATG CTT CTC CTA ACT GCA AGG CTA ACG CTG TCC AGC CCG GCT CCT    99
Val Met Leu Leu Leu Thr Ala Arg Leu Thr Leu Ser Ser Pro Ala Pro
        -10                 -5                  1

CCT GCT TGT GAC CTC CGA GTC CTC AGT AAA CTG CTT CGT GAC TCC CAT   147
Pro Ala Cys Asp Leu Arg Val Leu Ser Lys Leu Leu Arg Asp Ser His
  5                  10                  15                  20

GTC CTT CAC AGC AGA CTG AGC CAG TGC CCA GAG GTT CAC CCT TTG CCT   195
Val Leu His Ser Arg Leu Ser Gln Cys Pro Glu Val His Pro Leu Pro
                 25                  30                  35

ACA CCT GTC CTG CTG CCT GCT GTG GAC TTT AGC TTG GGA GAA TGG AAA   243
Thr Pro Val Leu Leu Pro Ala Val Asp Phe Ser Leu Gly Glu Trp Lys
            40                  45                  50

ACC CAG ATG GAG GAG ACC AAG GCA CAG GAC ATT CTG GGA GCA GTG ACC   291
Thr Gln Met Glu Glu Thr Lys Ala Gln Asp Ile Leu Gly Ala Val Thr
        55                  60                  65

CTT CTG CTG GAG GGA GTG ATG GCA GCA CGG GGA CAA CTG GGA CCC ACT   339
Leu Leu Leu Glu Gly Val Met Ala Ala Arg Gly Gln Leu Gly Pro Thr
    70                  75                  80

TGC CTC TCA TCC CTC CTG GGG CAG CTT TCT GGA CAG GTC CGT CTC CTC   387
Cys Leu Ser Ser Leu Leu Gly Gln Leu Ser Gly Gln Val Arg Leu Leu
 85                  90                  95                 100

CTT GGG GCC CTG CAG AGC CTC CTT GGA ACC CAG CTT CCT CCA CAG GGC   435
Leu Gly Ala Leu Gln Ser Leu Leu Gly Thr Gln Leu Pro Pro Gln Gly
                105                 110                 115

AGG ACC ACA GCT CAC AAG GAT CCC AAT GCC ATC TTC CTG AGC TTC CAA   483
Arg Thr Thr Ala His Lys Asp Pro Asn Ala Ile Phe Leu Ser Phe Gln
            120                 125                 130

CAC CTG CTC CGA GGA AAG GTG CGT TTC CTG ATG CTT GTA GGA GGG TCC   531
His Leu Leu Arg Gly Lys Val Arg Phe Leu Met Leu Val Gly Gly Ser
```

```
        135                 140                 145

ACC CTC TGC GTC AGG CGG GCC CCA CCC ACC ACA GCT GTC CCC AGC AGA        579
Thr Leu Cys Val Arg Arg Ala Pro Pro Thr Thr Ala Val Pro Ser Arg
    150                 155                 160

ACC TCT CTA GTC CTC ACA CTG AAC GAG CTC CCA AAC AGG ACT TCT GGA        627
Thr Ser Leu Val Leu Thr Leu Asn Glu Leu Pro Asn Arg Thr Ser Gly
165                 170                 175                 180

TTG TTG GAG ACA AAC TTC ACT GCC TCA GCC AGA ACT ACT GGC TCT GGG        675
Leu Leu Glu Thr Asn Phe Thr Ala Ser Ala Arg Thr Thr Gly Ser Gly
                185                 190                 195

CTT CTG AAG TGG CAG CAG GGA TTC AGA GCC AAG ATT CCT GGT CTG CTG        723
Leu Leu Lys Trp Gln Gln Gly Phe Arg Ala Lys Ile Pro Gly Leu Leu
            200                 205                 210

AAC CAA ACC TCC AGG TCC CTG GAC CAA ATC CCC GGA TAC CTG AAC AGG        771
Asn Gln Thr Ser Arg Ser Leu Asp Gln Ile Pro Gly Tyr Leu Asn Arg
        215                 220                 225

ATA CAC GAA CTC TTG AAT GGA ACT CGT GGA CTC TTT CCT GGA CCC TCA        819
Ile His Glu Leu Leu Asn Gly Thr Arg Gly Leu Phe Pro Gly Pro Ser
    230                 235                 240

CGC AGG ACC CTA GGA GCC CCG GAC ATT TCC TCA GGA ACA TCA GAC ACA        867
Arg Arg Thr Leu Gly Ala Pro Asp Ile Ser Ser Gly Thr Ser Asp Thr
245                 250                 255                 260

GGC TCC CTG CCA CCC AAC CTC CAG CCT GGA TAT TCT CCT TCC CCA ACC        915
Gly Ser Leu Pro Pro Asn Leu Gln Pro Gly Tyr Ser Pro Ser Pro Thr
                265                 270                 275

CAT CCT CCT ACT GGA CAG TAT ACG CTC TTC CCT CTT CCA CCC ACC TTG        963
His Pro Pro Thr Gly Gln Tyr Thr Leu Phe Pro Leu Pro Pro Thr Leu
            280                 285                 290

CCC ACC CCT GTG GTC CAG CTC CAC CCC CTG CTT CCT GAC CCT TCT GCT       1011
Pro Thr Pro Val Val Gln Leu His Pro Leu Leu Pro Asp Pro Ser Ala
        295                 300                 305

CCA ACG CCC ACC CCT ACC AGC CCT CTT CTA AAC ACA TCC TAC ACC CAC       1059
Pro Thr Pro Thr Pro Thr Ser Pro Leu Leu Asn Thr Ser Tyr Thr His
    310                 315                 320

TCC CAG AAT CTG TCT CAG GAA GGG TAAGGTTCTC AGACACTGCC GACATCAGCA      1113
Ser Gln Asn Leu Ser Gln Glu Gly
325                 330

TTGTCTCATG TACAGCTCCC TTCCCTGCAG GGCGCCCCTG GGAGACAACT GGACAAGATT     1173

TCCTACTTTC TCCTGAAACC CAAAGCCCTG GTAAAAGGGA TACACAGGAC TGAAAAGGGA     1233

ATCATTTTTC ACTGTACATT ATAAACCTTC AGAA                                 1267
```

(2) INFORMATION FOR SEQ ID NO:197:

(i) SEQUENCE CHARACTERISTICS:
- (A) LENGTH: 549 base pairs
- (B) TYPE: nucleic acid
- (C) STRANDEDNESS: double
- (D) TOPOLOGY: linear

(ii) MOLECULE TYPE: synthetic DNA

```
(xi) SEQUENCE DESCRIPTION: SEQ ID NO:197:

CTG GTT CCG CGT GGA TCC CCG GCT CCG CCA GCT TGT GAC CTT CGT GTT        48
Leu Val Pro Arg Gly Ser Pro Ala Pro Pro Ala Cys Asp Leu Arg Val
 -5                  1               5                  10

CTG TCT AAA CTG CTT CGC GAC TCT CAC GTG CTG CAC TCT CGT CTG TCC        96
Leu Ser Lys Leu Leu Arg Asp Ser His Val Leu His Ser Arg Leu Ser
            15                  20                  25

CAG TGC CCG GAA GTT CAC CCG CTG CCG ACC CCG GTT CTG CTT CCG GCT       144
Gln Cys Pro Glu Val His Pro Leu Pro Thr Pro Val Leu Leu Pro Ala
        30                  35                  40

GTC GAC TTC TCC CTG GGT GAA TGG AAA ACC CAG ATG GAA GAG ACC AAA       192
Val Asp Phe Ser Leu Gly Glu Trp Lys Thr Gln Met Glu Glu Thr Lys
    45                  50                  55

GCT CAG GAC ATC CTG GGT GCA GTA ACT CTG CTT CTG GAA GGC GTT ATG       240
Ala Gln Asp Ile Leu Gly Ala Val Thr Leu Leu Leu Glu Gly Val Met
 60                  65                  70                  75

GCT GCA CGT GGC CAG CTT GGC CCG ACC TGC CTG TCT TCC CTG CTT GGC       288
Ala Ala Arg Gly Gln Leu Gly Pro Thr Cys Leu Ser Ser Leu Leu Gly
                80                  85                  90

CAG CTG TCT GGC CAG GTT CGT CTG CTG CTC GGC GCT CTG CAG TCT CTG       336
Gln Leu Ser Gly Gln Val Arg Leu Leu Leu Gly Ala Leu Gln Ser Leu
            95                 100                 105

CTT GGC ACC CAG CTG CCG CCA CAG GGC CGT ACC ACT GCT CAC AAG GAT       384
Leu Gly Thr Gln Leu Pro Pro Gln Gly Arg Thr Thr Ala His Lys Asp
        110                 115                 120 CCC AAT GCC ATC TTC CTG AGC TTC CAA CAC CTG CTC CGA GGA AAG GTG       432
Pro Asn Ala Ile Phe Leu Ser Phe Gln His Leu Leu Arg Gly Lys Val
    125                 130                 135 CGT TTC CTG ATG CTT GTA GGA GGG TCC ACC CTC TGC GTC AGG CGG GCC       480
Arg Phe Leu Met Leu Val Gly Gly Ser Thr Leu Cys Val Arg Arg Ala
140                 145                 150                 155 CCA CCC ACC ACA GCT GTC CCC AGC AGA ACC TCT CTA GTC CTC ACA CTG       528
Pro Pro Thr Thr Ala Val Pro Ser Arg Thr Ser Leu Val Leu Thr Leu
                160                 165                 170

AAC GAG CTC TAATGAGAAT TC                                             549
Asn Glu Leu
```

## Claims

1. A thrombopoietin (TPO) polypeptide having the biological activity of specifically stimulating or increasing platelet production and comprising the amino acid sequence 1 to 332 of SEQ ID NO:6 or derivative thereof.

2. The TPO polypeptide derivative according to claim 1 consisting of the amino acid sequence 1 to 163 of SEQ ID NO:6.

3. The TPO polypeptide derivative according to claims 1 or 2 selected from the group consisting of [$Ala^1$,$Val^3$,$Arg^{129}$]TPO(1-163), [$Ala^1$,$Val^3$,$Arg^{133}$]TPO(1-163), [$Ala^1$,$Val^3$,$Arg^{143}$]TPO(1-163), [$Ala^1$,$Val^3$,$Leu^{82}$]TPO(1-163), [$Ala^1$,$Val^3$,$Leu^{148}$]TPO(1-163), [$Ala^1$,$Val^3$,$Pro^{148}$]TPO(1-163), [$Ala^1$,$Val^3$,$Arg^{58}$]TPO(1-163), [$Ala^1$,$Val^3$,$Arg^{115}$]TPO(1-163), [$Arg^{129}$]TPO(1-163), [$Arg^{133}$]TPO(1-163), [$Arg^{143}$]TPO(1-163), [$Leu^{82}$]TPO(1-163), [$Leu^{148}$]TPO(1-163), [$Pro^{148}$]TPO(1-163), [$Arg^{58}$]TPO(1-163) and [$Arg^{115}$]TPO(1-163).

4. The TPO polypeptide derivative according to claim 1 consisting of the amino acid sequence 1 to 232 of SEQ ID NO:6.

5. The TPO polypeptide derivative according to claim 1 consisting of the amino acid sequence 1 to 151 of SEQ ID NO:6.

6. The TPO polypeptide derivative of claim 1, 2, 3 or 4 having from 1 to 6 amino terminal amino acids deleted.

7. The TPO polypeptide derivative according to claims 1 or 2 selected from the group consisting of [ΔHis$^{33}$]TPO(1-163), [ΔArg$^{117}$]TPO(1-163) and [ΔGly$^{116}$]TPO(1-163).

8. The TPO polypeptide derivative according to claims 1 or 2 selected from the group consisting of [His$^{33}$, Thr$^{33'}$, Pro$^{34}$]TPO(1-163), [His$^{33}$, Ala$^{33'}$, Pro$^{34}$]TPO(1-163),[His$^{33}$, Gly$^{33'}$, Pro$^{34}$, Ser$^{38}$]TPO(1-163), [Gly$^{116}$, Asn$^{116'}$, Arg$^{117}$]TPO(1-163), [Gly$^{116}$, Ala$^{116'}$, Arg$^{117}$]TPO(1-163), [Gly$^{116}$, Gly$^{116'}$, Arg$^{117}$]TPO(1-163) and [Ala$^{1}$, Val$^{3}$]TPO(1-163).

9. The TPO polypeptide derivative according to claim 1 selected from the group consisting of [Thr$^{33}$, Thr$^{333}$, Ser$^{334}$, Ile$^{335}$, Gly$^{336}$, Tyr$^{337}$, Pro$^{338}$, Tyr$^{339}$, Asp$^{340}$, Val$^{341}$, Pro$^{342}$, Asp$^{343}$, Tyr$^{344}$, Ala$^{345}$, Gly$^{346}$, Val$^{347}$, His$^{348}$, His$^{349}$, His$^{350}$, His$^{351}$, His$^{352}$, His$^{353}$]TPO and [Asn$^{25}$, Lys$^{231}$, Thr$^{333}$, Ser$^{334}$, Ile$^{335}$, Gly$^{336}$, Tyr$^{337}$, Pro$^{338}$, Tyr$^{339}$, Asp$^{340}$, Val$^{341}$, Pro$^{342}$, Asp$^{343}$, Tyr$^{344}$, Ala$^{345}$, Gly$^{346}$, Val$^{347}$, His$^{348}$, His$^{349}$, His$^{350}$, His$^{351}$, His$^{352}$, His$^{353}$]TPO.

10. The TPO polypeptide derivative according to claim 1 selected from the group consisting of [Asn$^{25}$]TPO and [Thr$^{33}$]TPO.

11. The TPO polypeptide according to claims 1 to 10 covalently bonded to a polymer.

12. The TPO polypeptide according to claim 11 wherein said polymer is polyethylene glycol.

13. The TPO polypeptide according to any of claims 1 through 12 which further comprises the amino acid Met$^{-2}$-Lys$^{-1}$.

14. The TPO polypeptide according to any of claims 1 through 12 which further comprises Met$^{-1}$.

15. The TPO polypeptide according to any claims 1 through 12 which further comprises Gly$^{-1}$.

16. A polypeptide consisting of the mature sequence of amino acids of SEQ ID NOs: 2, 4 or 6.

17. A DNA encoding a TPO polypeptide according to any of claims 1 through 10, 13, 14 and 16.

18. A DNA sequence for use in securing expression of a TPO polypeptide having the biological activity of specifically stimulating or increasing platelet production, which is selected from the group consisting of:
    (a) the DNA sequences shown in SEQ ID NOS: 194, 195 or 196 or complementary strands thereof; and
    (b) DNA sequences which hybridize under stringent conditions to the DNA sequences as defined in (a) or fragments thereof; and
    (c) DNA sequences which would hybridize to the DNA sequences as defined in (a) and (b), but for the degeneracy of the genetic code.

19. A cDNA sequence according to claim 18.

20. A genomic DNA sequence according to claim 18.

21. A manufactured DNA sequence according to claim 18.

22. A process for producing a TPO polypeptide comprising the steps of:

a) expressing a polypeptide encoded by a DNA according to any of claims 17, 18, 19, 20 or 21 in a suitable host, and
b) isolating said TPO polypeptide.

**23.** The process of claim 22 wherein the TPO polypeptide expressed is a $Met^{-2}$-$Lys^{-1}$ polypeptide and further including the step of cleaving $Met^{-2}$-$Lys^{-1}$ from said isolated TPO polypeptide.

**24.** A DNA sequence for use in securing expression of a TPO polypeptide having the biological activity of specifically stimulating or increasing platelet production, said DNA further encoding a thrombin recognition peptide 5′ to TPO polypeptide encoding sequences and encoding glutathione-S-transferase (GST) 5′ to the thrombin recognition peptide.

**25.** The DNA sequence of claim 24 for use in securing expression of a TPO polypeptide consisting of the amino acid sequence 1 to 174 of SEQ ID NO: 6.

**26.** A process for producing a TPO polypeptide having the biological activity of specifically stimulating or increasing platelet production comprising the steps of:
a) expressing a polypeptide encoded by a DNA according to claims 24 to 26 in a suitable host, and
b) isolating said GST-(thrombin recognition peptide)-TPO polypeptide.
c) treating said isolated GST(thrombin recognition peptide)-TPO polypeptide with thrombin, and
d) isolating a $Gly^{-1}$-TPO polypeptide.

**27.** The process of claim 26 wherein the $Gly^{-1}$-TPO polypeptide is [$Gly^{-1}$]TPO(1-174).

**28.** The process of claim 26 wherein the isolated $Gly^{-1}$-TPO polypeptide is a TPO derivative consisting of the amino acid sequence 1 to 174 of SEQ ID NO:6.

**29.** A protein product obtained by the process of claims 22, 23, 27 or 28.

**30.** A procaryotic or eucaryotic host cell transformed or transfected with the DNA sequence according to any of claims 17, 18, 19, 20, 21 or 24 to 26 in a manner enabling said host cell to express a polypeptide having the biological activity of specifically stimulating or increasing platelet production.

**31.** A pharmaceutical composition comprising an effective amount of the polypeptide according to any of claims 1 through 15 and 29 in combination with a pharmaceutically acceptable carrier.

**32.** A pharmaceutical composition according to claim 31 for use in the treatment of platelet disorders.

**33.** A pharmaceutical composition according to claim 31 for use in the treatment of thrombocytopenia.

**34.** A pharmaceutical composition according to claim 33 for use in the treatment of thrombocytopenia induced by chemotherapy, radiotherapy or bone marrow transplantation.

**35.** Use of the polypeptide according to any of claims 1 through 15 and 29 for manufacturing a medicament for treating platelet disorders.

**36.** Use the polypeptide according to any of claims 1 through 15 and 29 for manufacturing a medicament for treating thrombocytopenia.

**37.** Use according to claim 36 for treating thrombocytopenia induced by chemotherapy, radiotherapy or bone marrow transplantation.

**38.** An antibody specifically immunoreactive with a polypeptide of any claims 1 through 15 and 29.

**39.** Use of the antibody of claim 38 for the isolation of a polypeptide according to any of claims 1 through 15 and 29.

**40.** Use of the antibody of claim 38 for the quantification of a polypeptide according to any of claims 1 through 15 and 29.

Figure 1

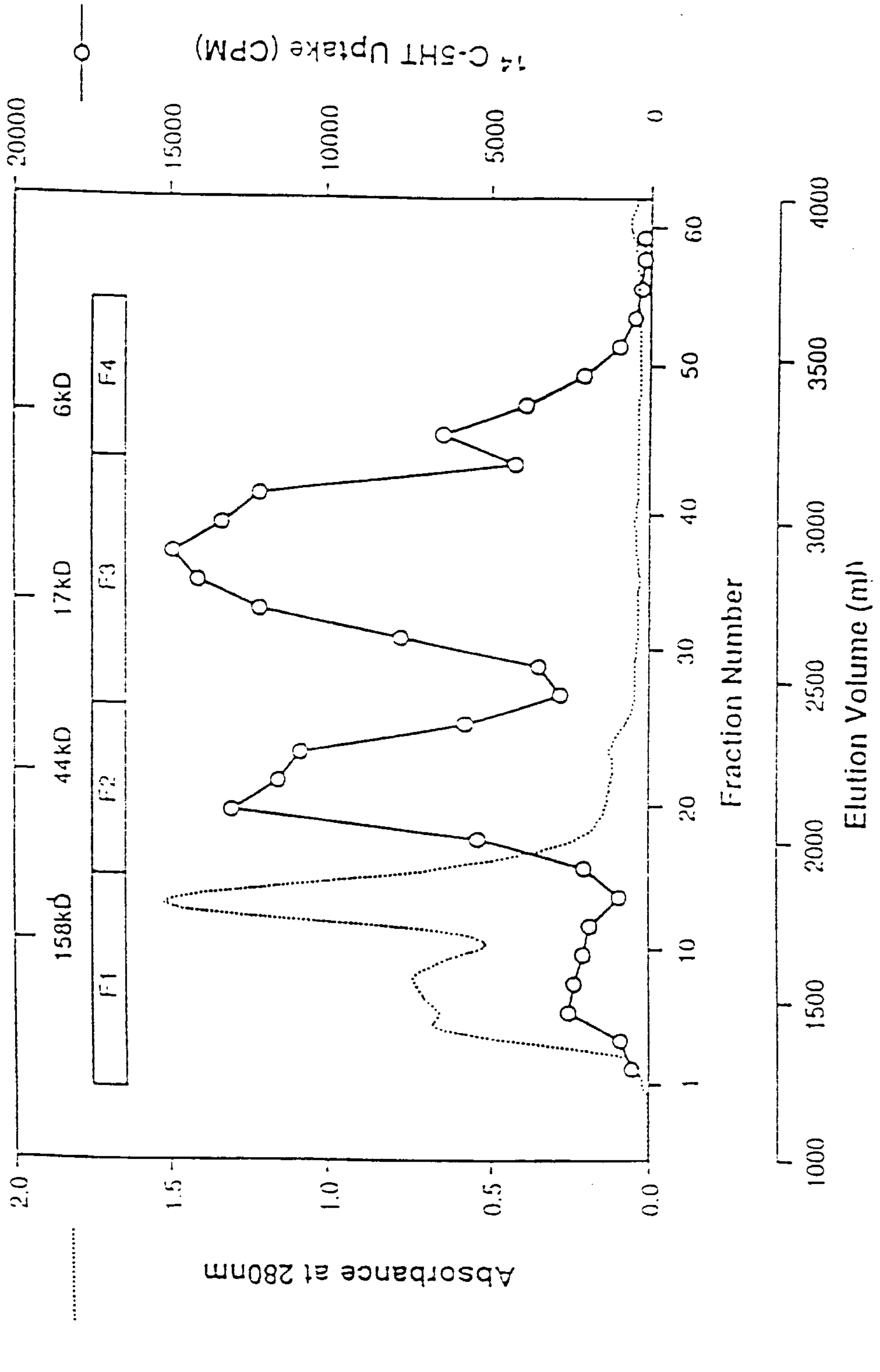
14 C-5HT Uptake (CPM)
20000
15000
10000
5000
0
158kD
44kD
17kD
6kD
F1
F2
F3
F4
Absorbance at 280nm
2.0
1.5
1.0
0.5
0.0
1
10
20
30
40
50
60
Fraction Number
1000
1500
2000
2500
3000
3500
4000
Elution Volume (ml)

Figure 2

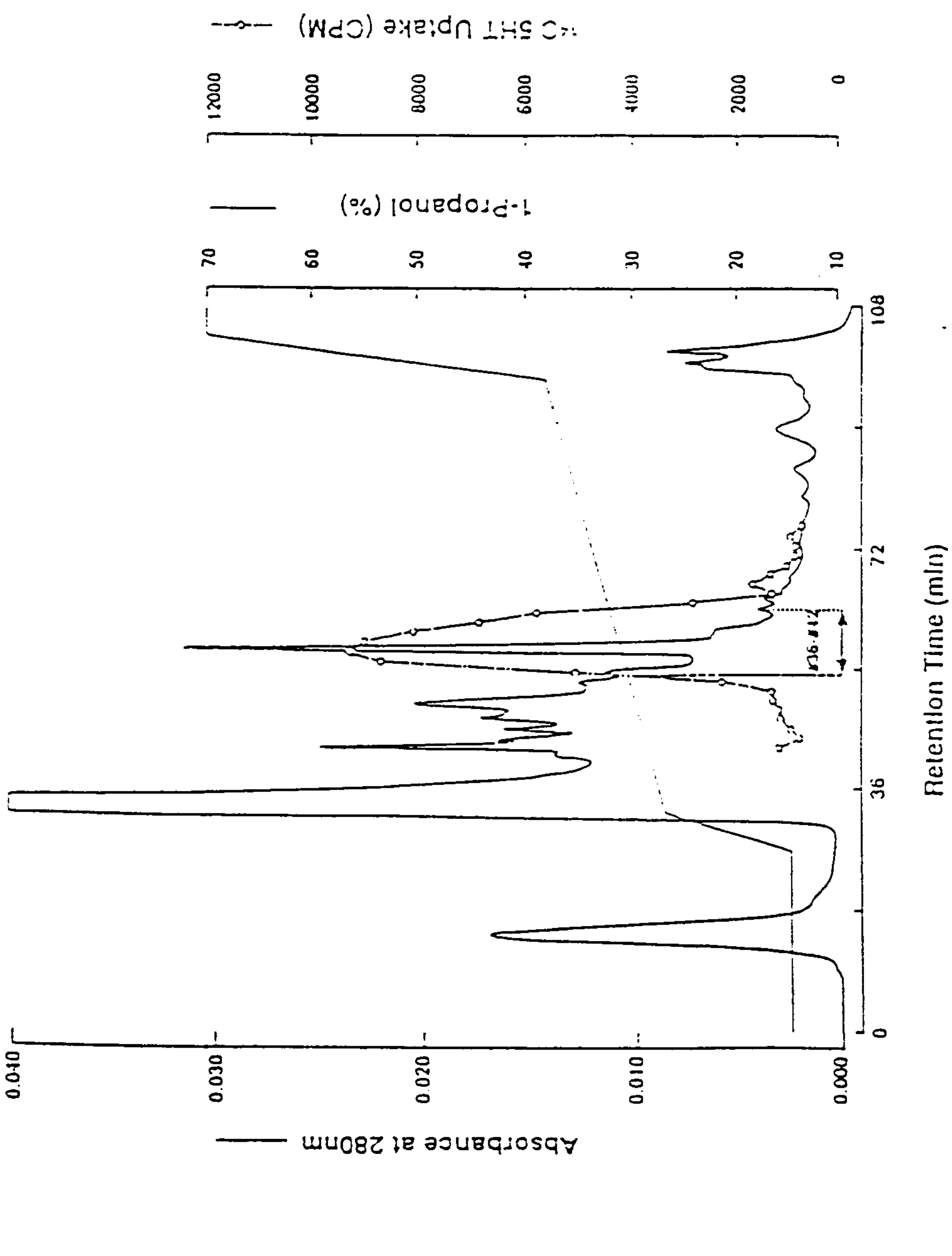
Absorbance at 280nm
1-Propanol (%)
5HT Uptake (CPM)
Retention Time (min)
0
36
72
108
0.000
0.010
0.020
0.030
0.040
10
20
30
40
50
60
70
0
2000
4000
6000
8000
10000
12000

Figure 3

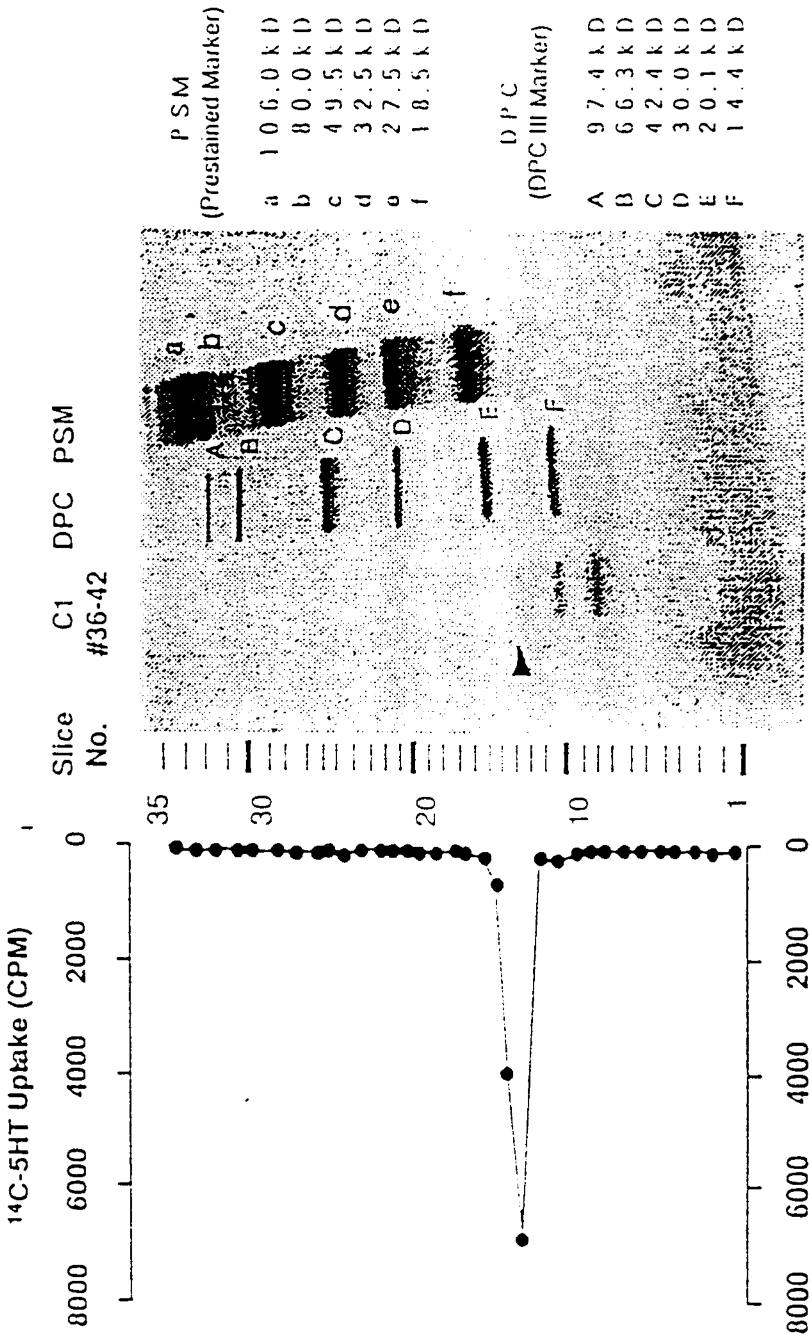

14C-5HT Uptake (CPM)
0
2000
4000
6000
8000
Slice No.
35
30
20
10
1
C1 #36-42
DPC
PSM
P S M (Prestained Marker)
a 106.0 kD
b 80.0 kD
c 49.5 kD
d 32.5 kD
e 27.5 kD
f 18.5 kD
D P C (DPC III Marker)
A 97.4 kD
B 66.3 kD
C 42.4 kD
D 30.0 kD
E 20.1 kD
F 14.4 kD

Figure 4

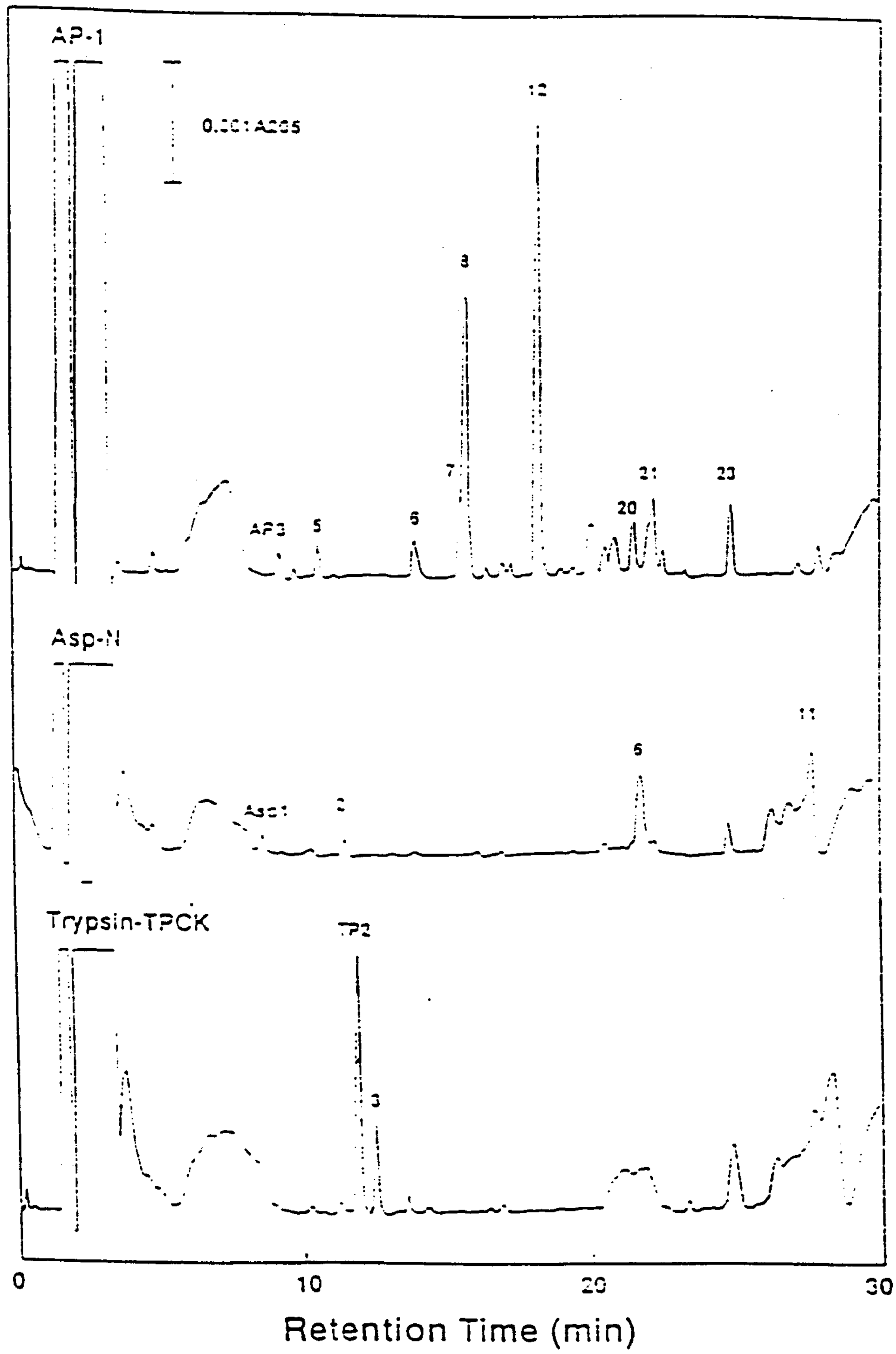
AP-1
0.001 A205
AP3
5
6
7
8
12
20
21
23
Asp-N
Asp1
2
6
11
Trypsin-TPCK
TP2
3
0
10
20
30
Retention Time (min)

Figure 5

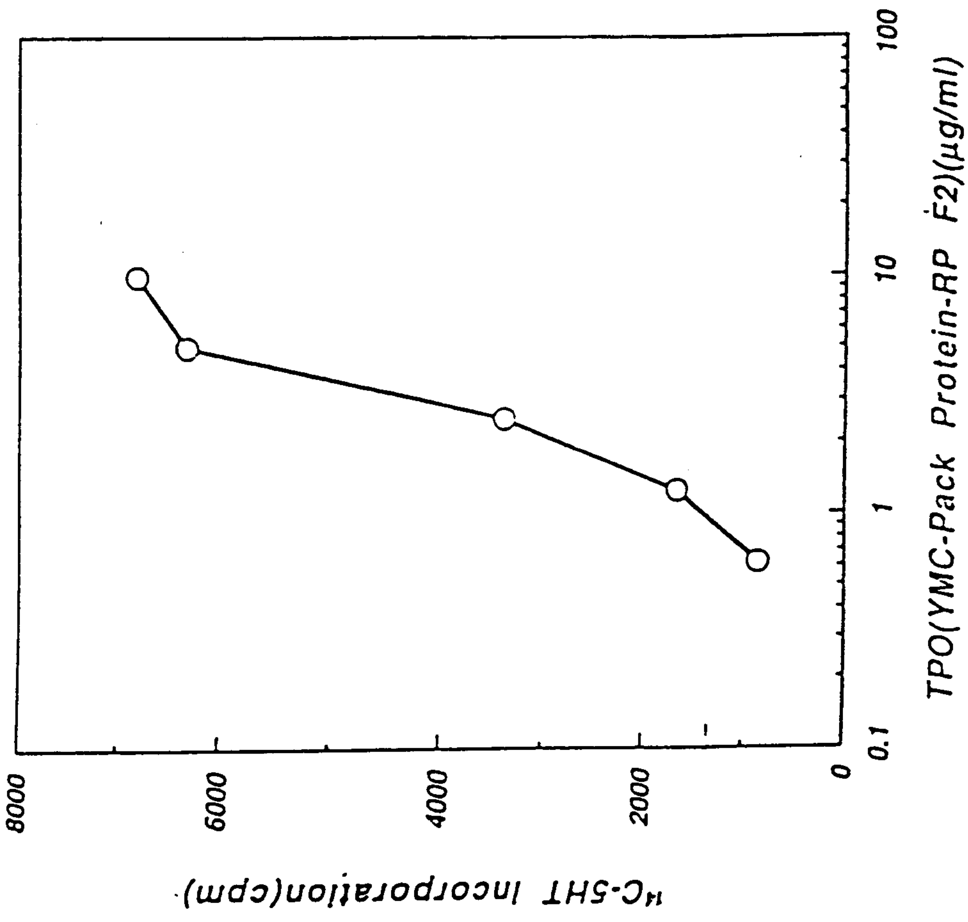

8000
6000
4000
2000
0
$^{14}C$-5HT Incorporation(cpm)
0.1
1
10
100
TPO(YMC-Pack Protein-RP F2)(µg/ml)

Figure 6

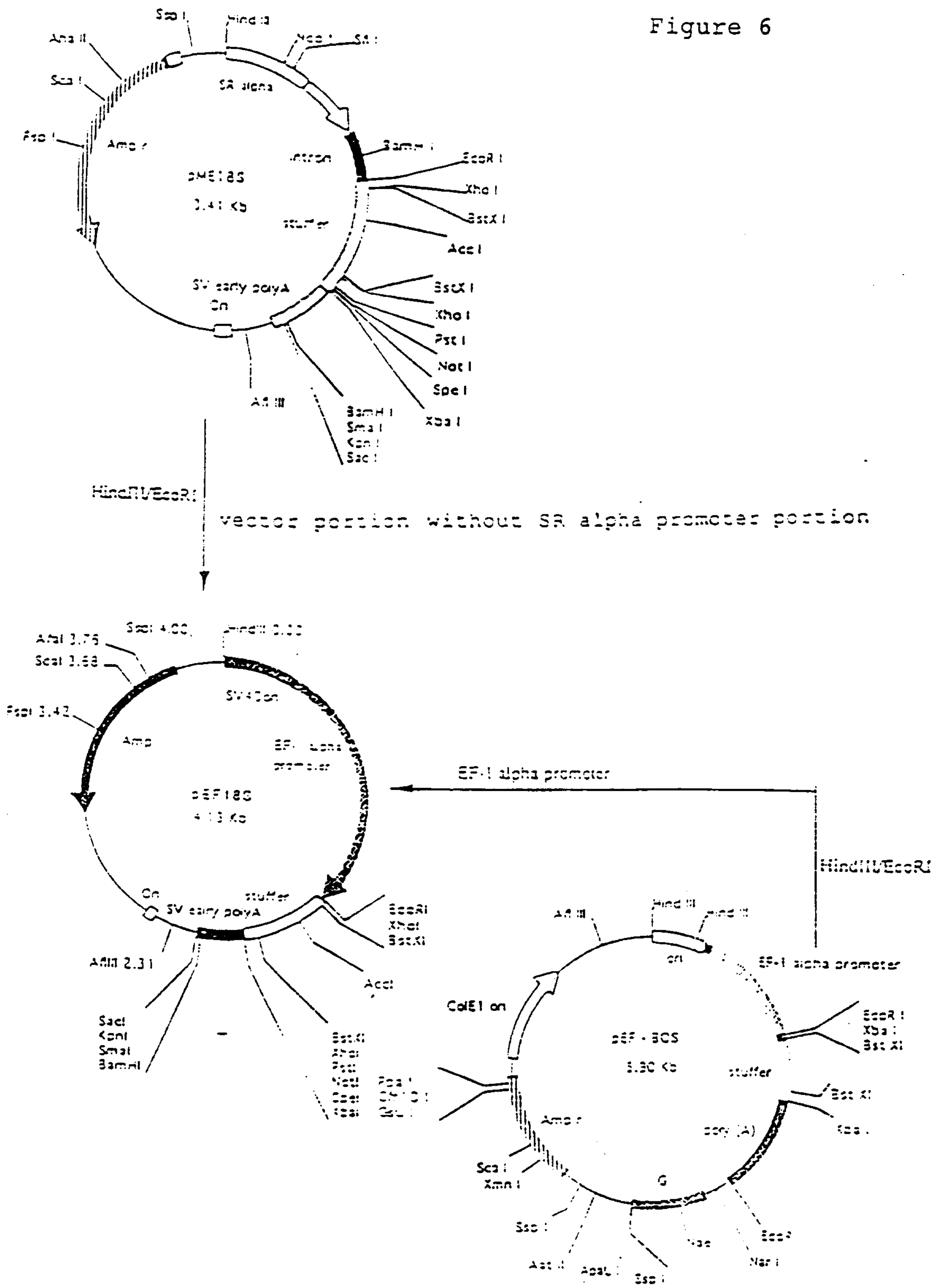
SR alpha
Amp r
intron
stuffer
SV early polyA
Ori
BamH I
EcoR I
Xho I
BstX I
Acc I
Pst I
Not I
Spe I
Xba I
Sma I
Sac I
HindIII/EcoRI
vector portion without SR alpha promoter portion
SV40ori
EF-1 alpha promoter
Amp
stuffer
SV early polyA
AflII 2.31
SacI
KpnI
SmaI
BamHI
AccI
EcoRI
XhoI
BstXI
Fsp I 3.42
HindIII/EcoRI
Hind III
ori
EF-1 alpha promoter
ColE1 ori
pEF-BOS
5.80 Kb
stuffer
Amp r
Sca I
Xmn I
EcoR I
Xba I
Bst XI

Figure 7

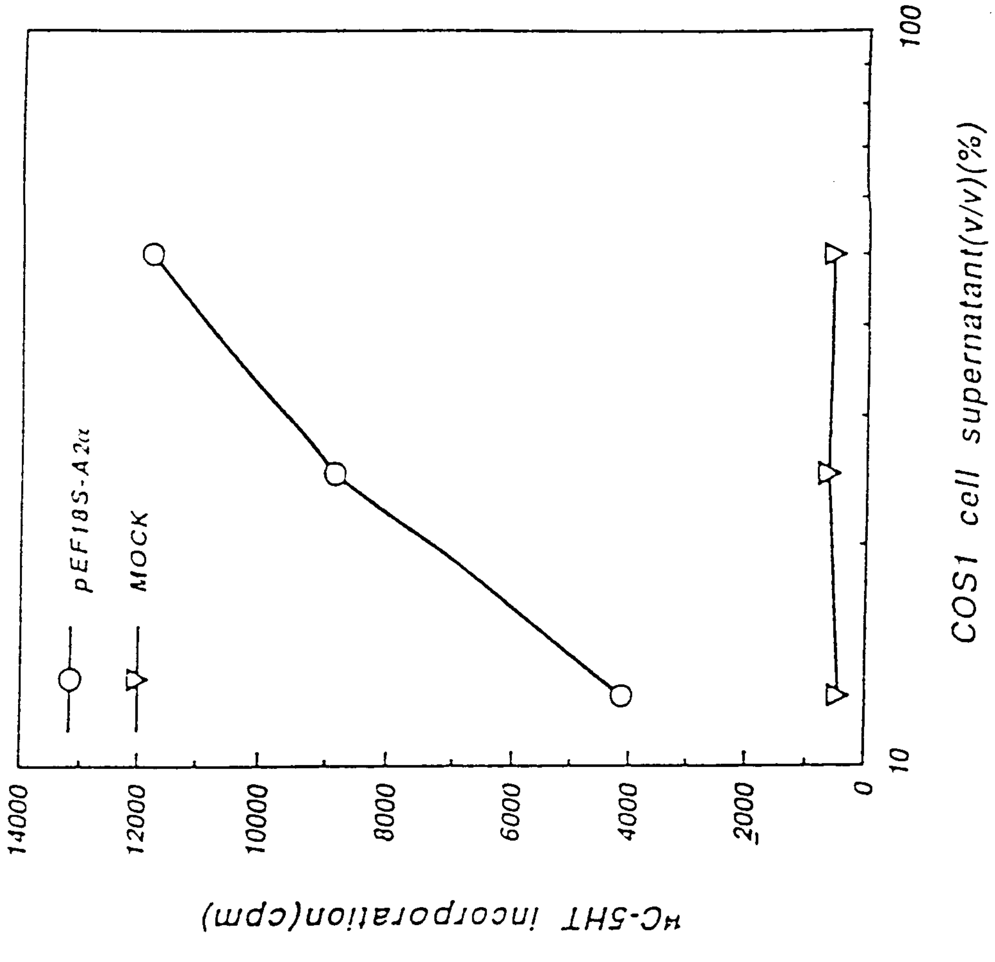
pEF18S-A2α
MOCK
14000
12000
10000
8000
6000
4000
2000
0
10
100
COS1 cell supernatant(v/v)(%)
HC-5HT incorporation(cpm)

Figure 8

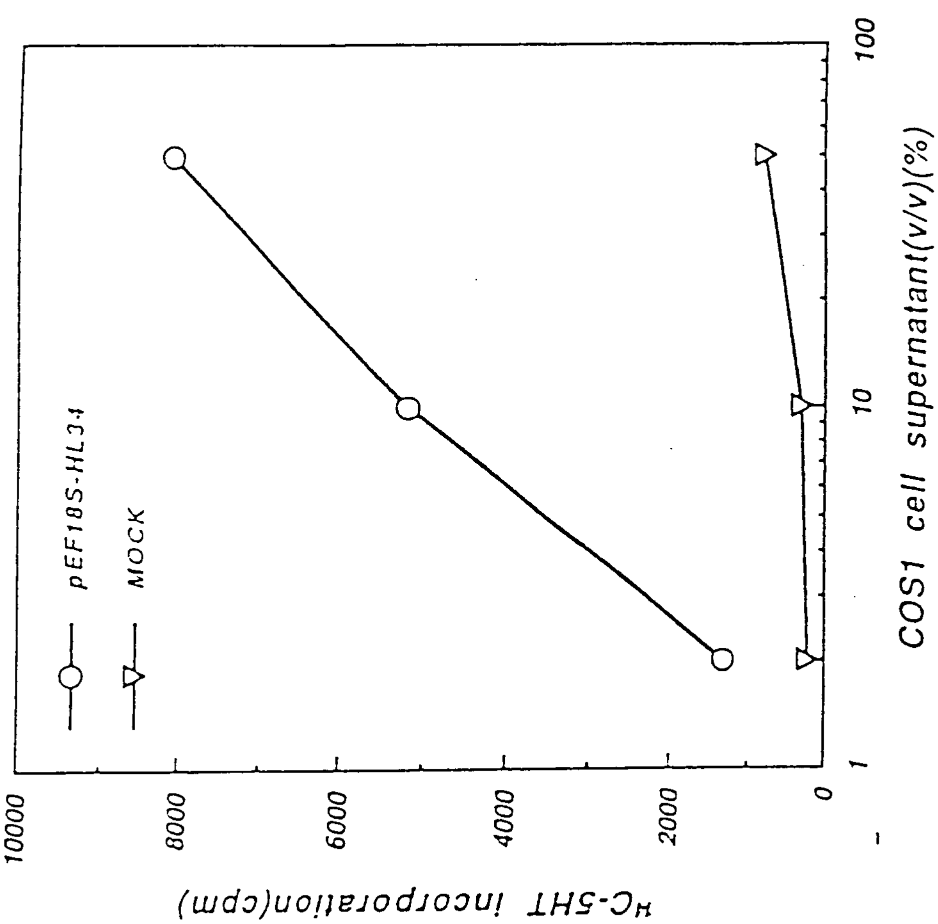
MOCK
10000
8000
6000
4000
2000
0
1
10
100
COS1 cell supernatant(v/v)(%)

Figure 9

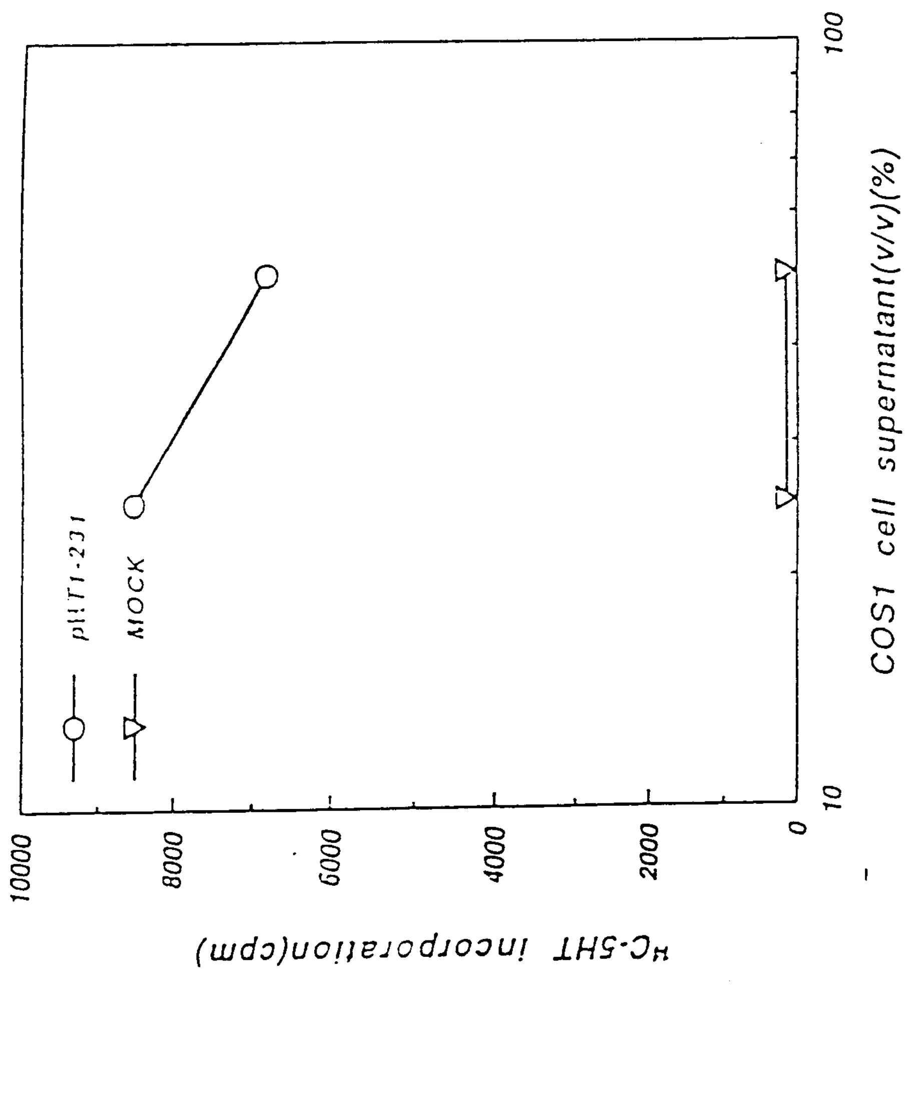
pHT1-231
MOCK
10000
8000
6000
4000
2000
0
10
100
COS1 cell supernatant(v/v)(%)
³H-5HT incorporation(cpm)

Figure 10a

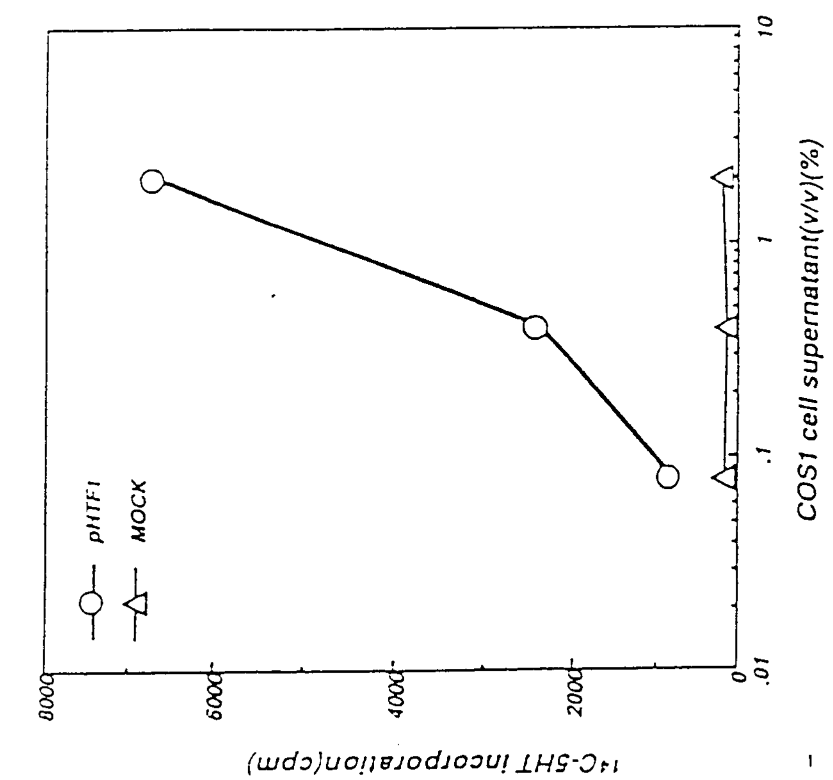


Figure 10b

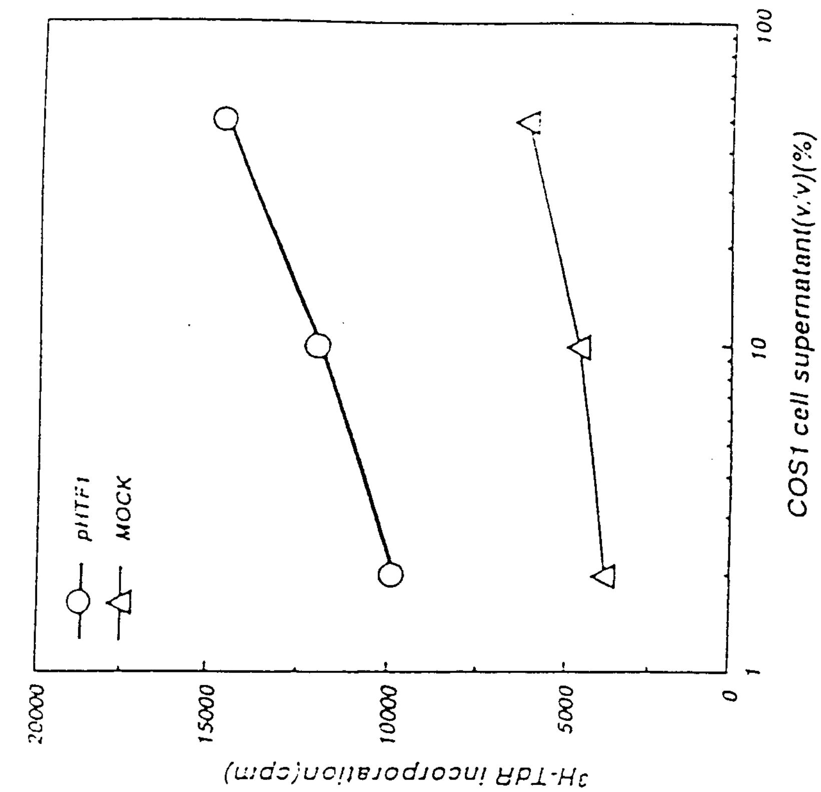

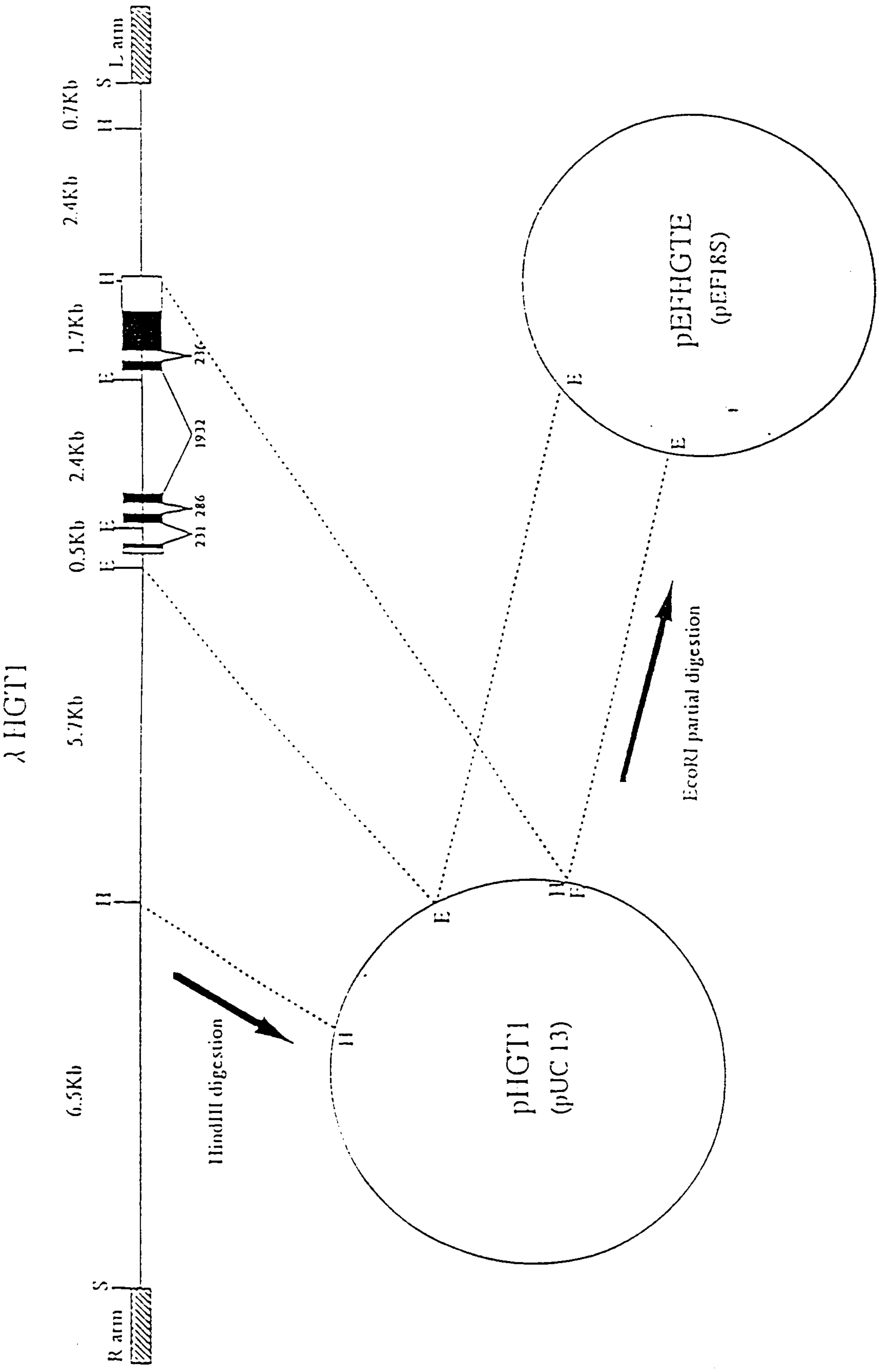
λ HGT1
R arm
S
6.5Kb
H
5.7Kb
0.5Kb
E
E
231 286
2.4Kb
1932
E
1.7Kb
236
H
2.4Kb
0.7Kb
H
S
L arm
HindIII digestion
pHGT1
(pUC 13)
EcoRI partial digestion
pEFHGTE
(pEF18S)

Figure 11

Figure 12a

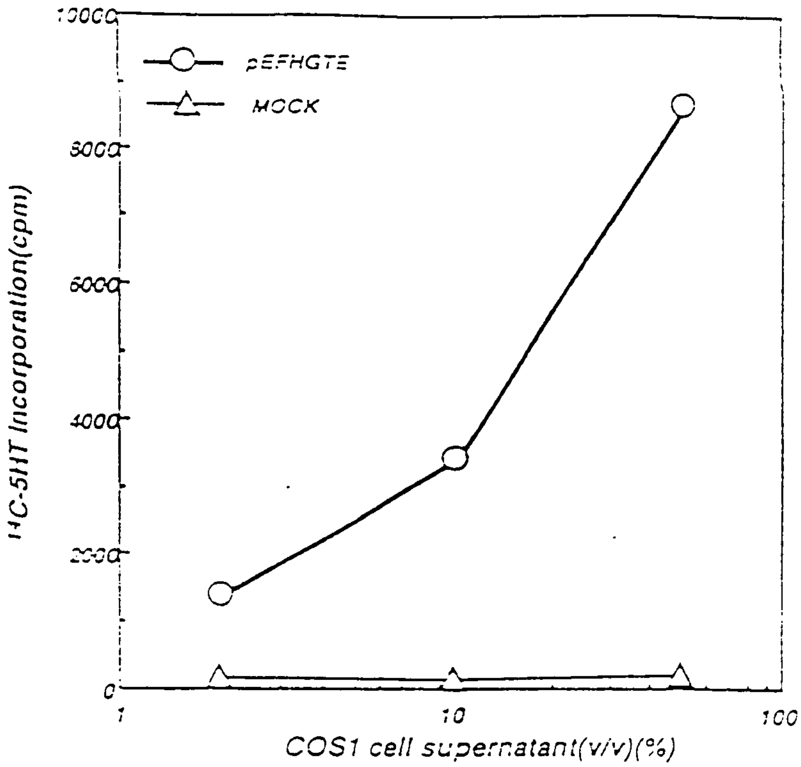


Figure 12b

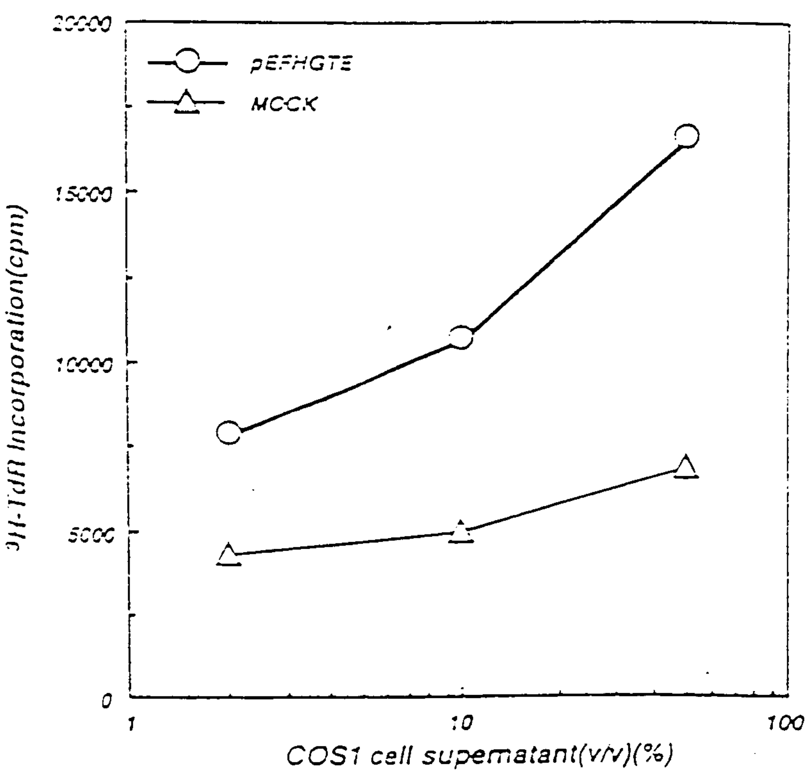

Figure 13a

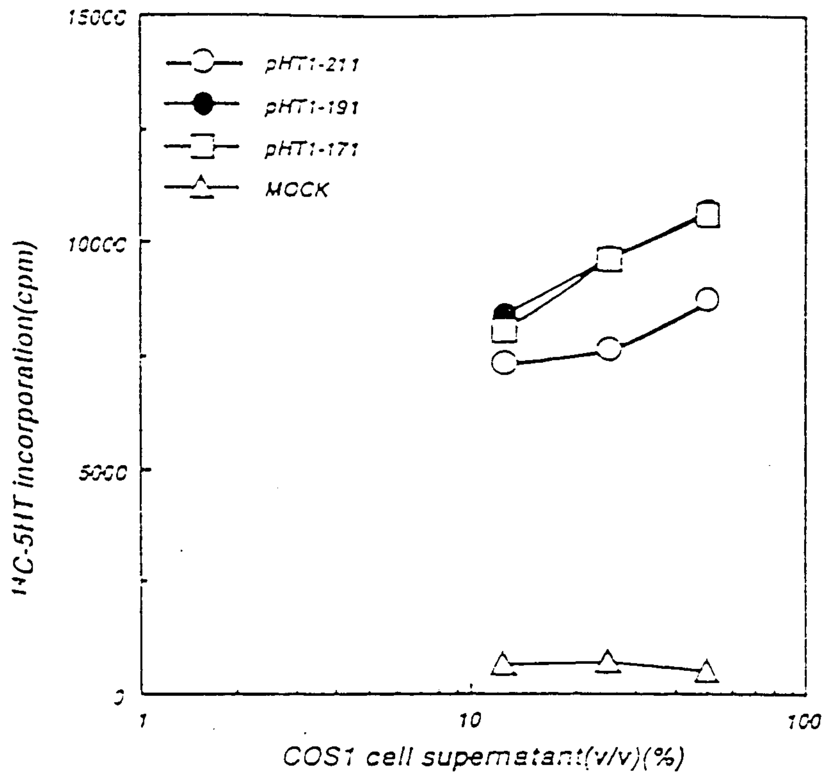


Figure 13b

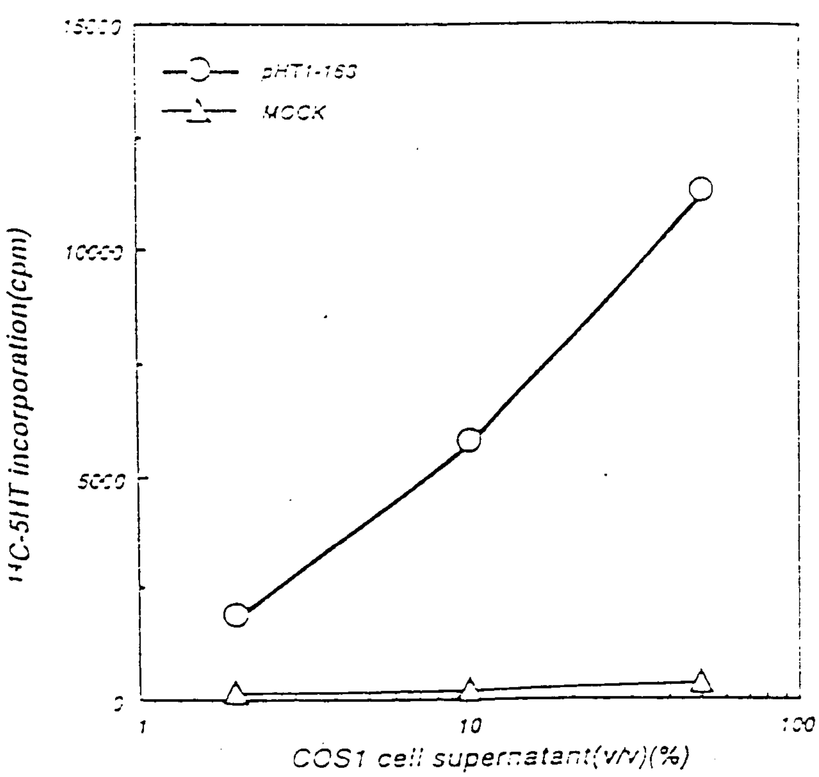

Figure 14

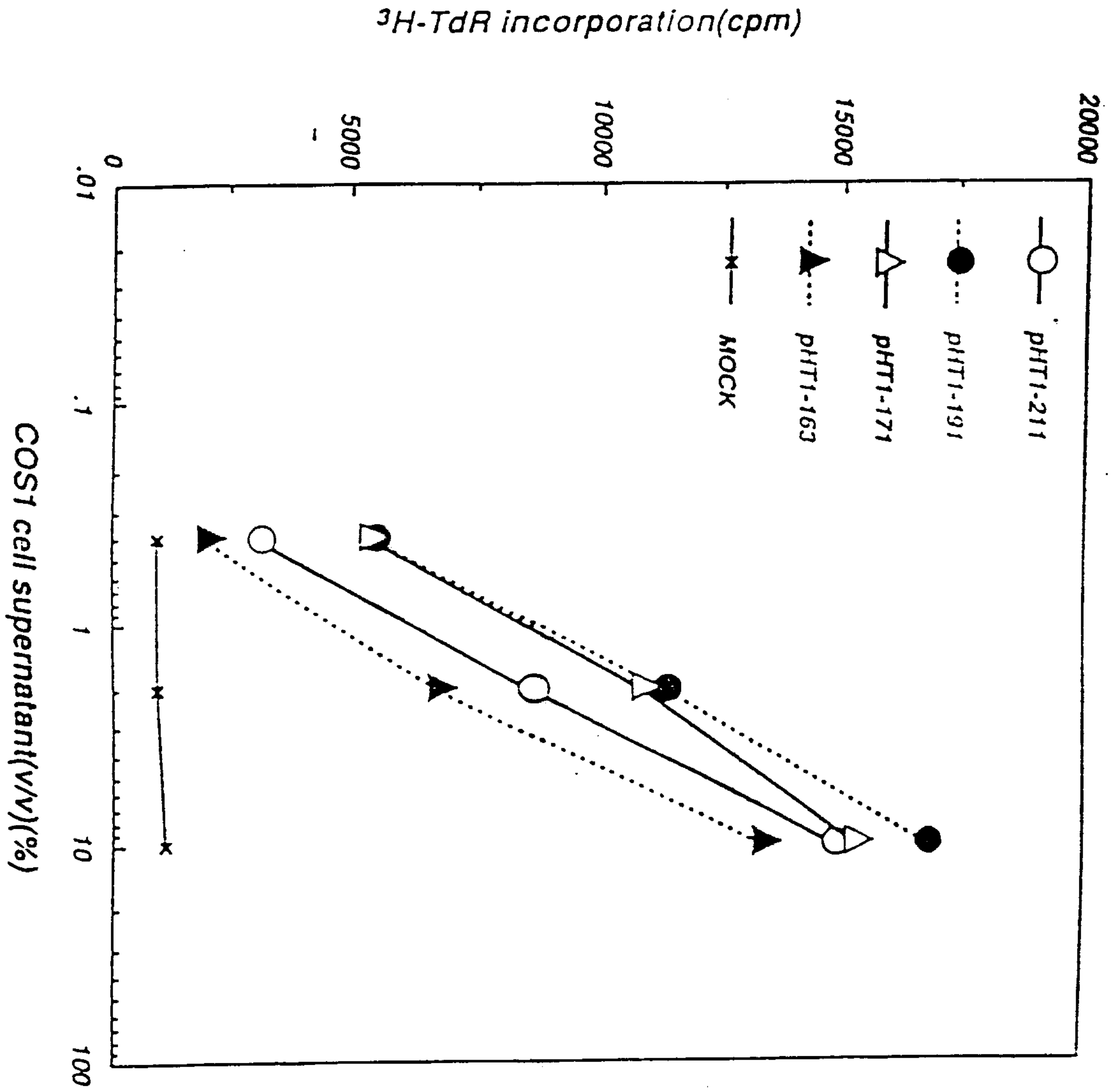

³H-TdR incorporation(cpm)
0
5000
10000
15000
20000
.01
.1
1
10
100
pHT1-211
pHT1-191
pHT1-171
pHT1-163
MOCK
COS1 cell supernatant(v/v)(%)

Figure 15

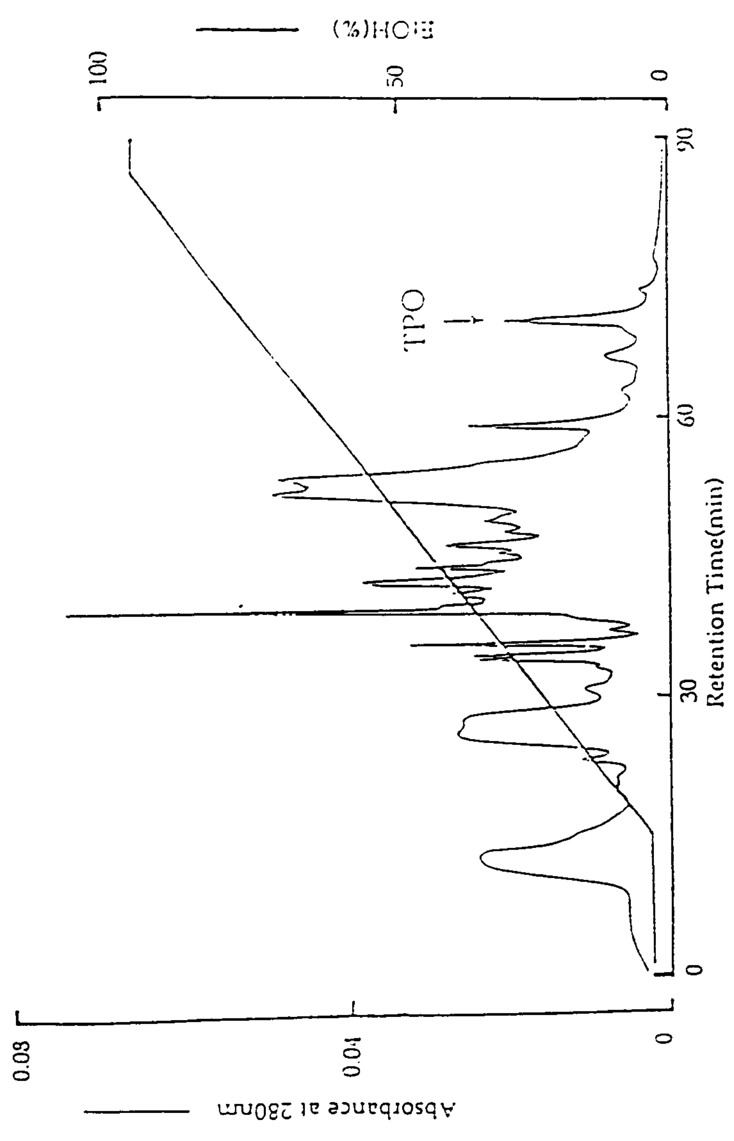


Figure 16

4/20% SDS-PAGE of Vydac C4 Fraction.

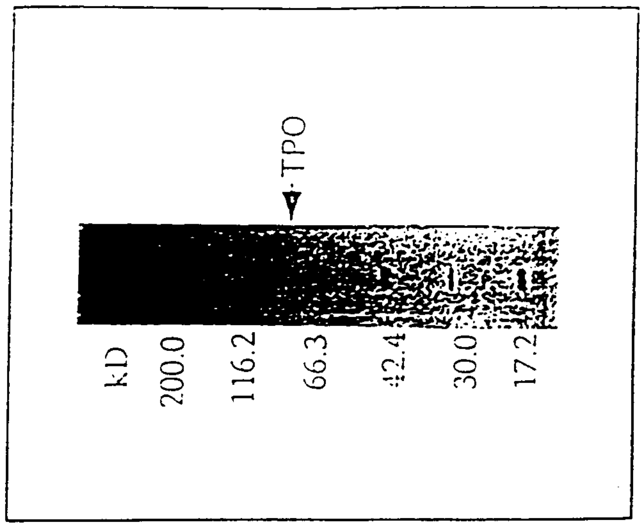

Figure 17

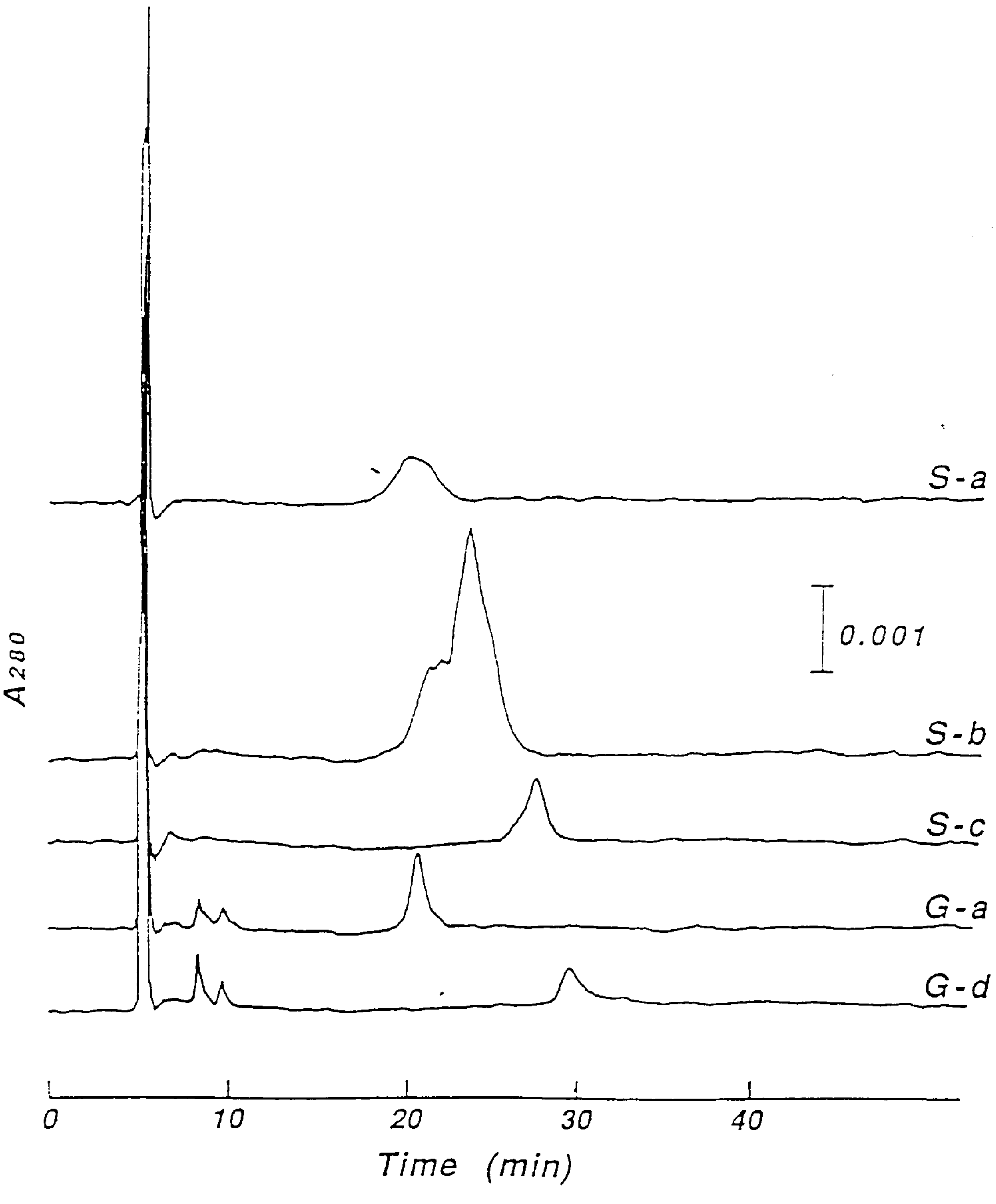

Figure 18

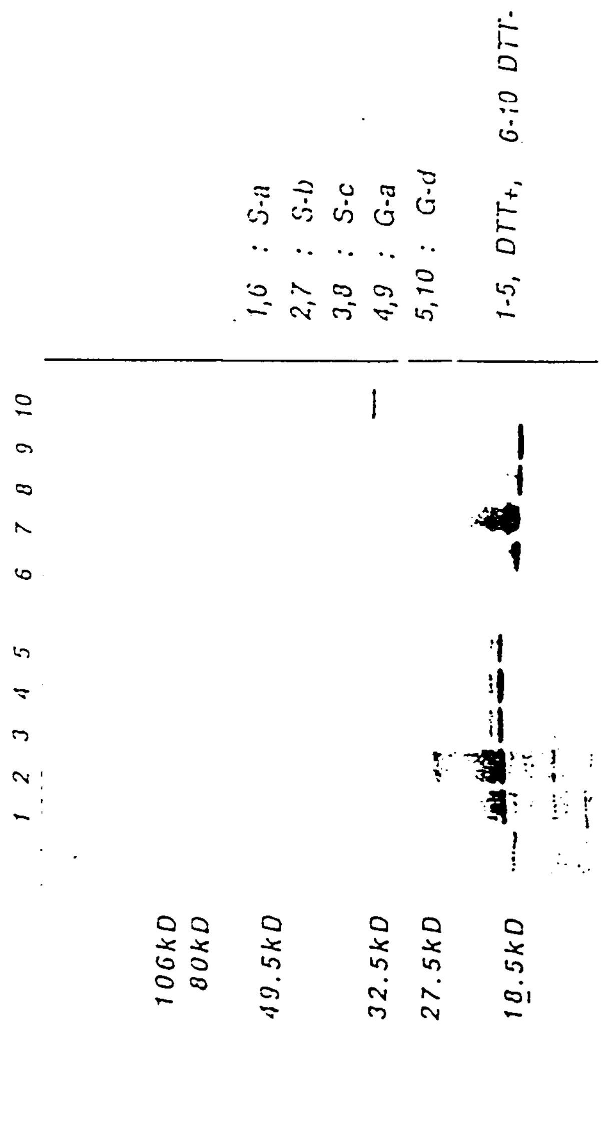
1 2 3 4 5 6 7 8 9 10
1,6 : S-a
2,7 : S-b
3,8 : S-c
4,9 : G-a
5,10 : G-d
1-5, DTT+, 6-10 DTT-
106kD
80kD
49.5kD
32.5kD
27.5kD
18.5kD

Figure 19

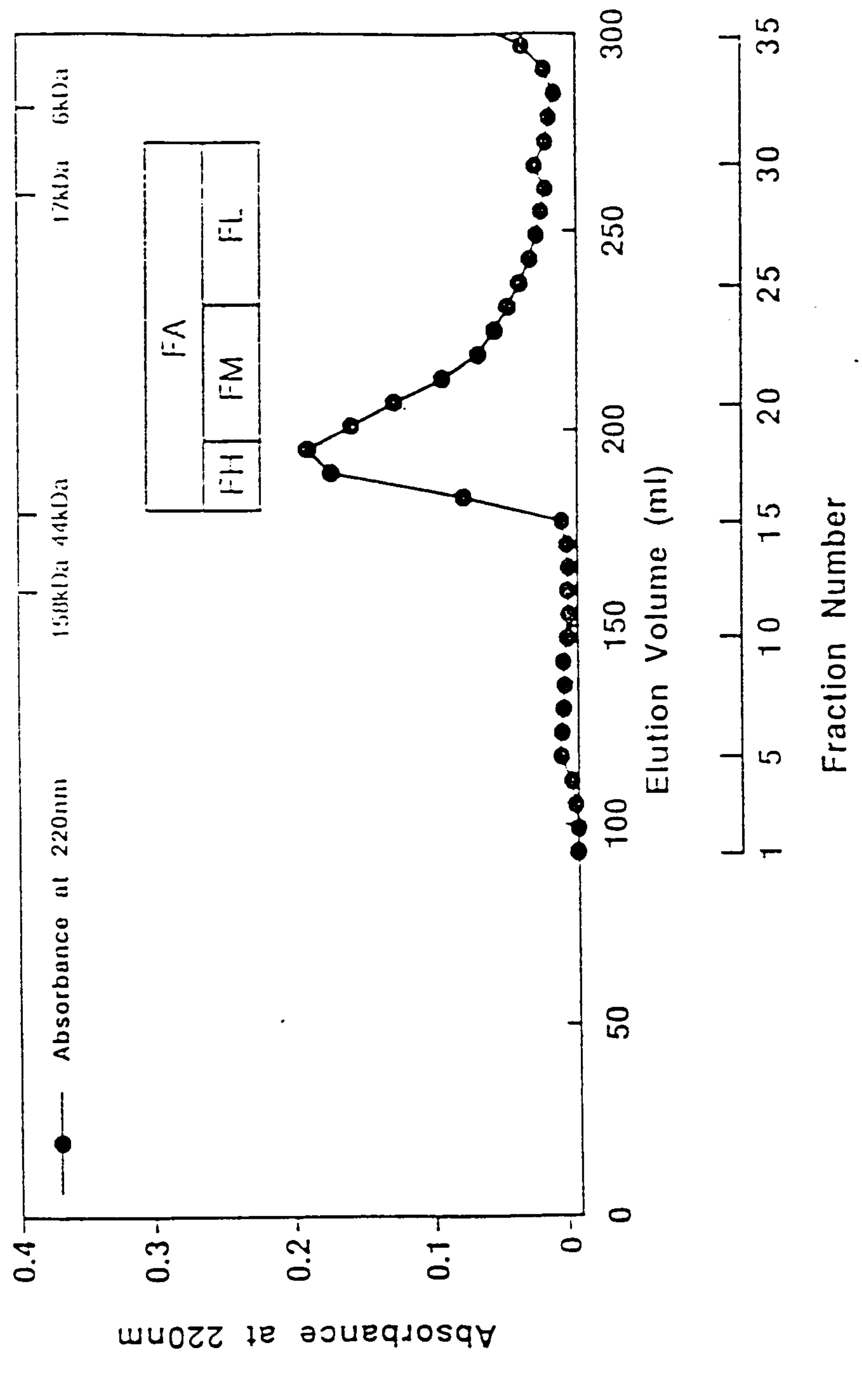
Absorbance at 220nm
158kDa 44kDa
17kDa 6kDa
FA
FH
FM
FL
Absorbance at 220nm
0.4
0.3
0.2
0.1
0
0
50
100
150
200
250
300
Elution Volume (ml)
1
5
10
15
20
25
30
35
Fraction Number

Figure 20

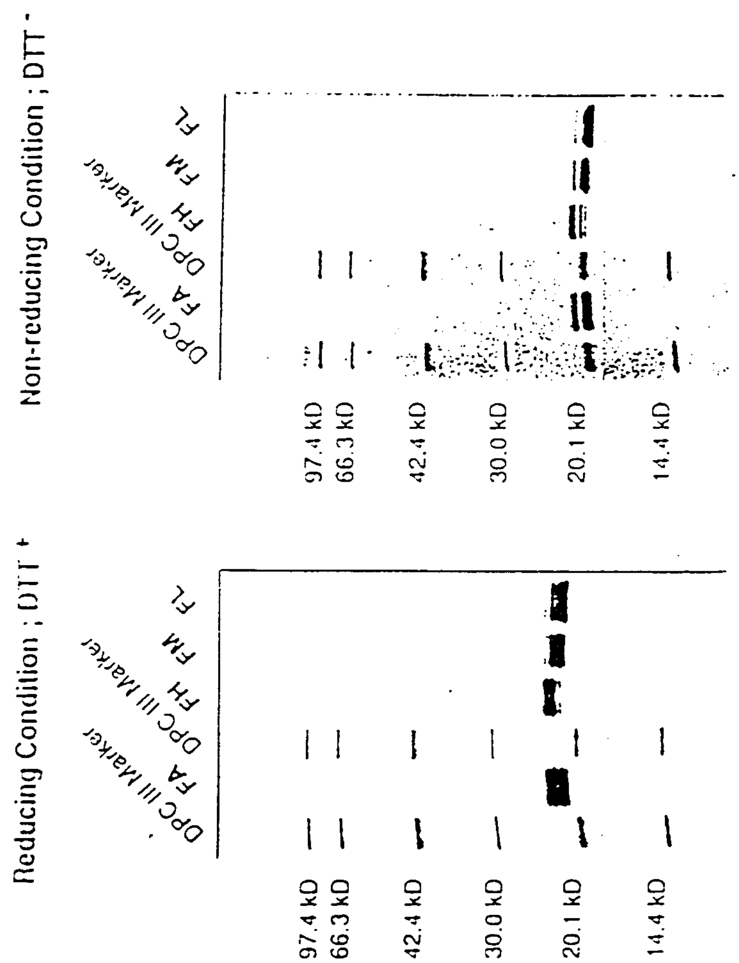
Non-reducing Condition ; DTT -
OPC III Marker
FA
OPC III Marker
FH
FM
FL
97.4 kD
66.3 kD
42.4 kD
30.0 kD
20.1 kD
14.4 kD
Reducing Condition ; DTT +
OPC III Marker
FA
OPC III Marker
FH
FM
FL
97.4 kD
66.3 kD
42.4 kD
30.0 kD
20.1 kD
14.4 kD

Figure 21

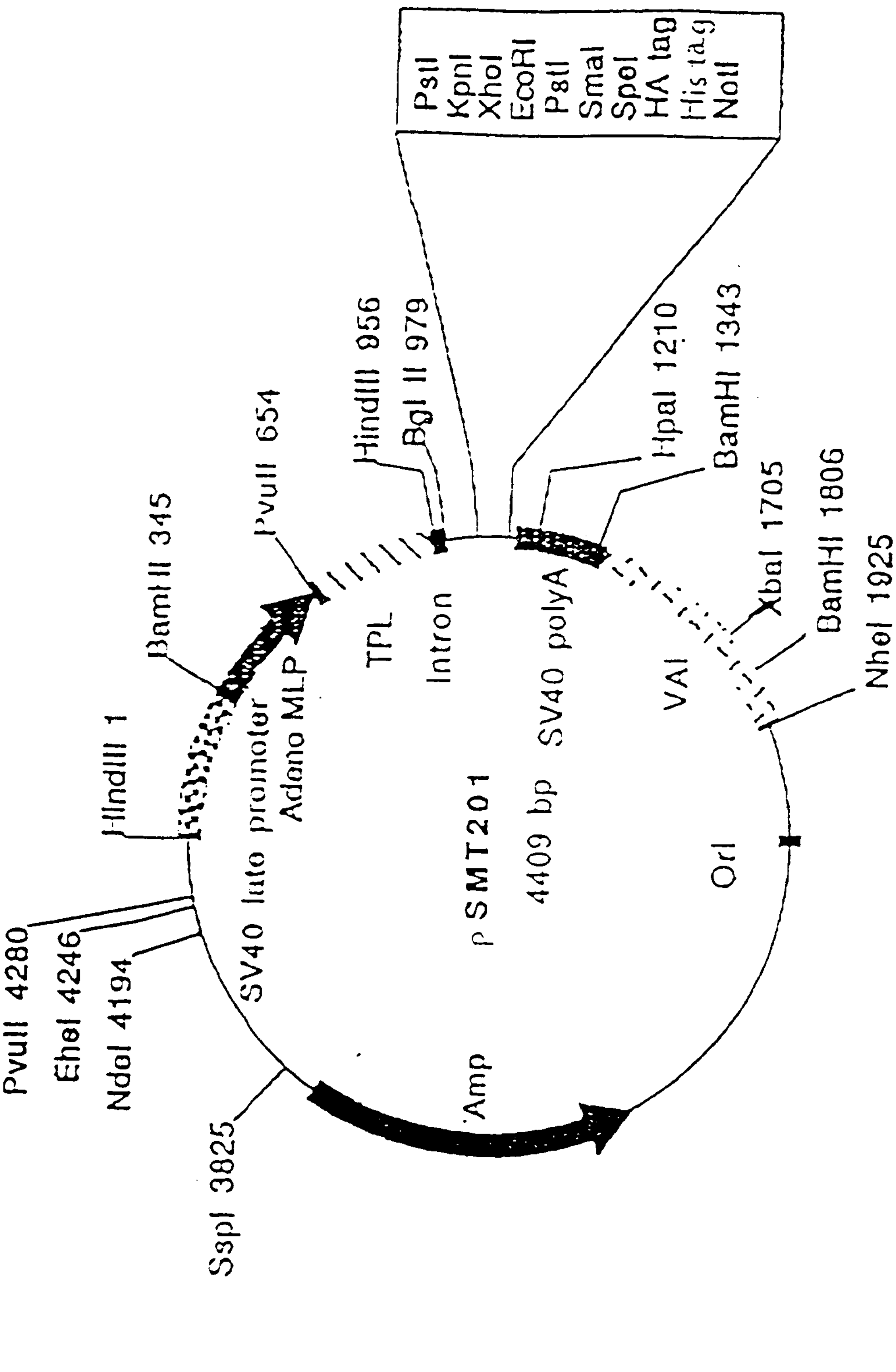
PstI
KpnI
XhoI
EcoRI
PstI
SmaI
SpeI
HA tag
His tag
NotI
HindIII 956
Bgl II 979
HpaI 1210
BamHI 1343
XbaI 1705
BamHI 1806
NheI 1925
PvuII 654
BamHI 345
HindIII 1
PvuII 4280
EheI 4246
NdeI 4194
SspI 3825
TPL
Intron
SV40 polyA
VAI
Adeno MLP
SV40 late promoter
pSMT201
4409 bp
Ori
Amp

Figure 22

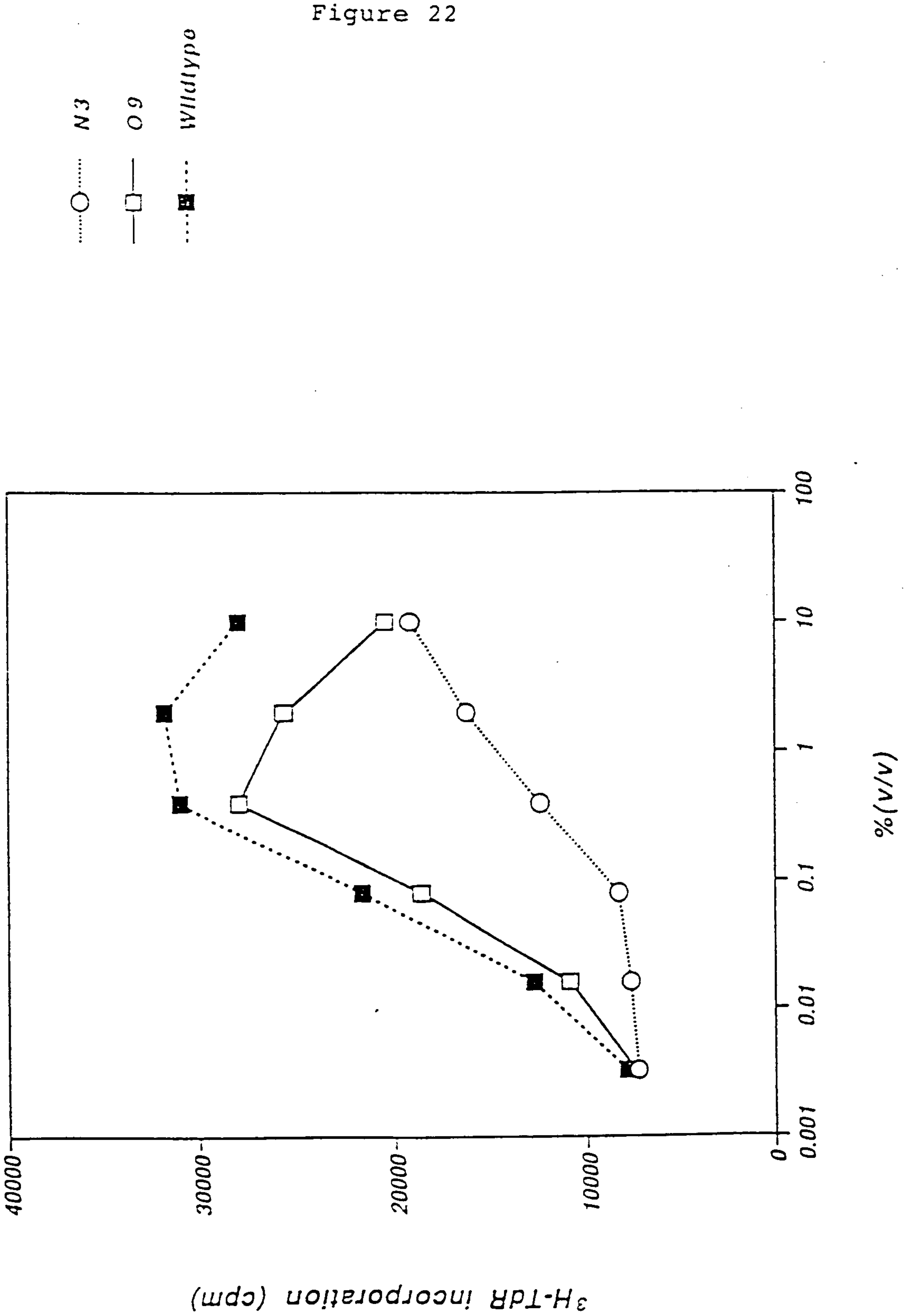
N3
O9
Wildtype
0
10000
20000
30000
40000
0.001
0.01
0.1
1
10
100
%(v/v)
3H-TdR incorporation (cpm)

Figure 23

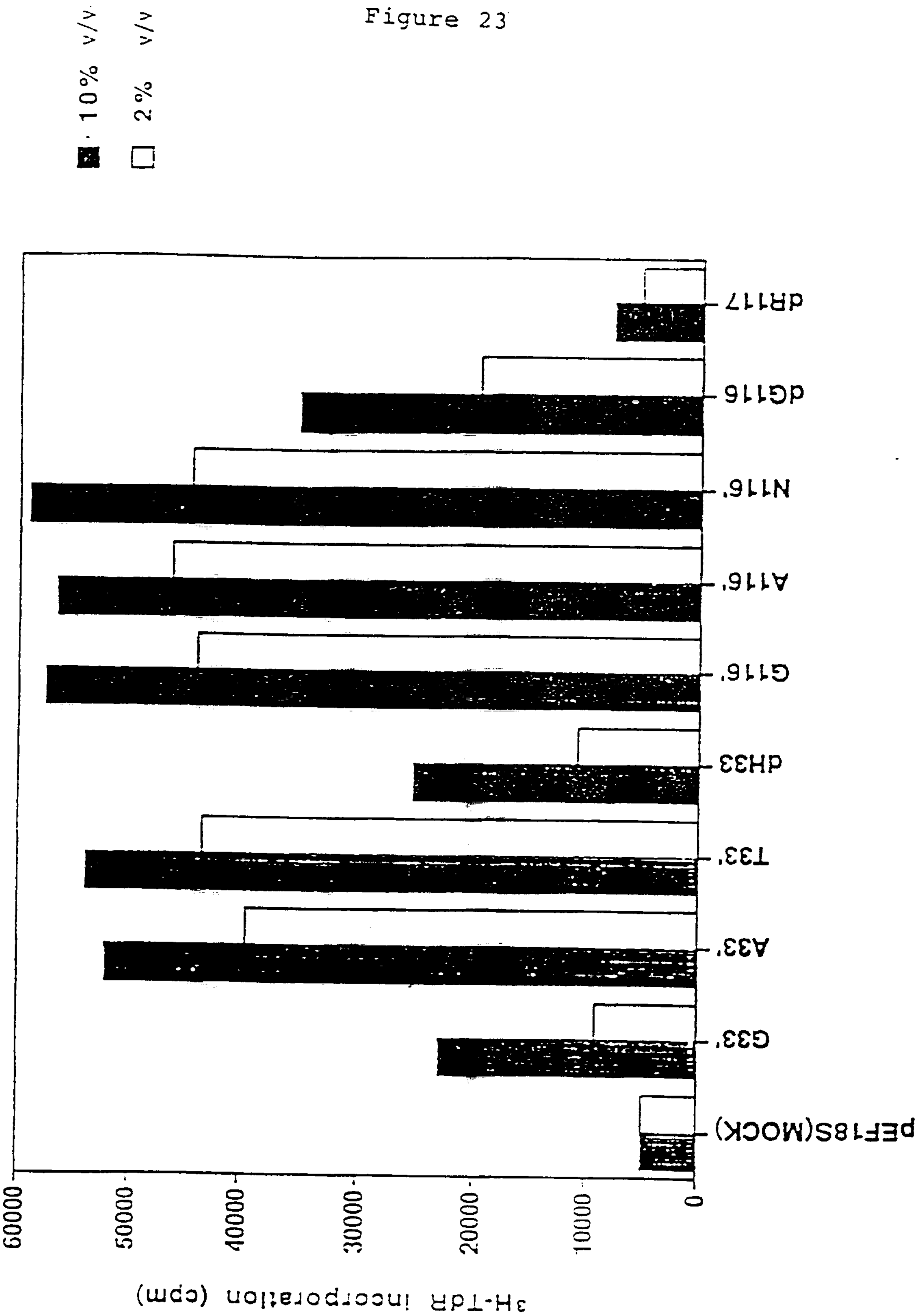
10% v/v
2% v/v
pEF18S(MOCK)
G33'
A33'
T33'
dH33
G116'
A116'
N116'
dG116
dR117
0
10000
20000
30000
40000
50000
60000
3H-TdR incorporation (cpm)

Figure 24

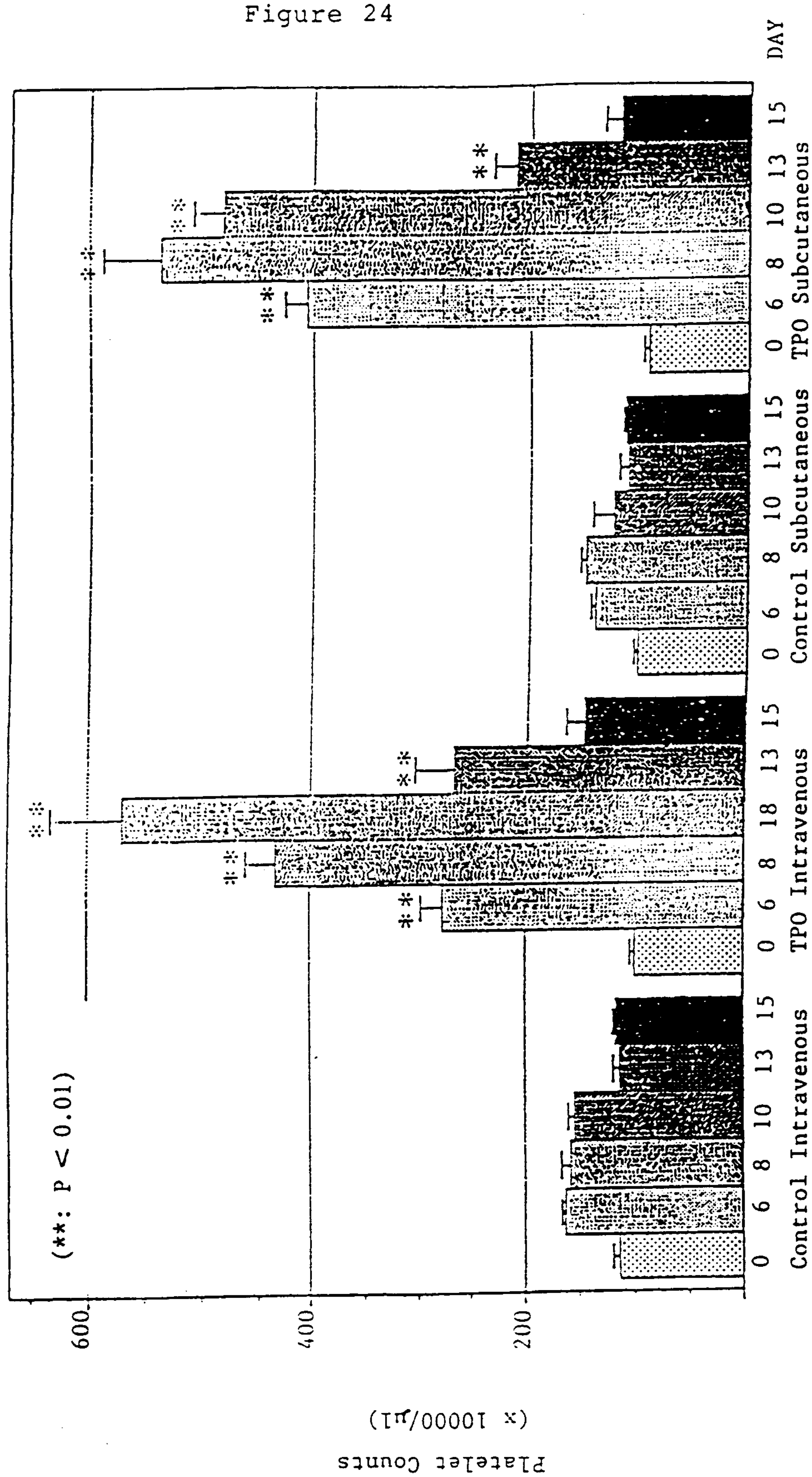
(**: P < 0.01)
Platelet Counts
(x 10000/μl)
600
400
200
DAY
0 6 8 10 13 15
Control Intravenous
0 6 8 18 13 15
TPO Intravenous
0 6 8 10 13 15
Control Subcutaneous
0 6 8 10 13 15
TPO Subcutaneous
**

Figure 25

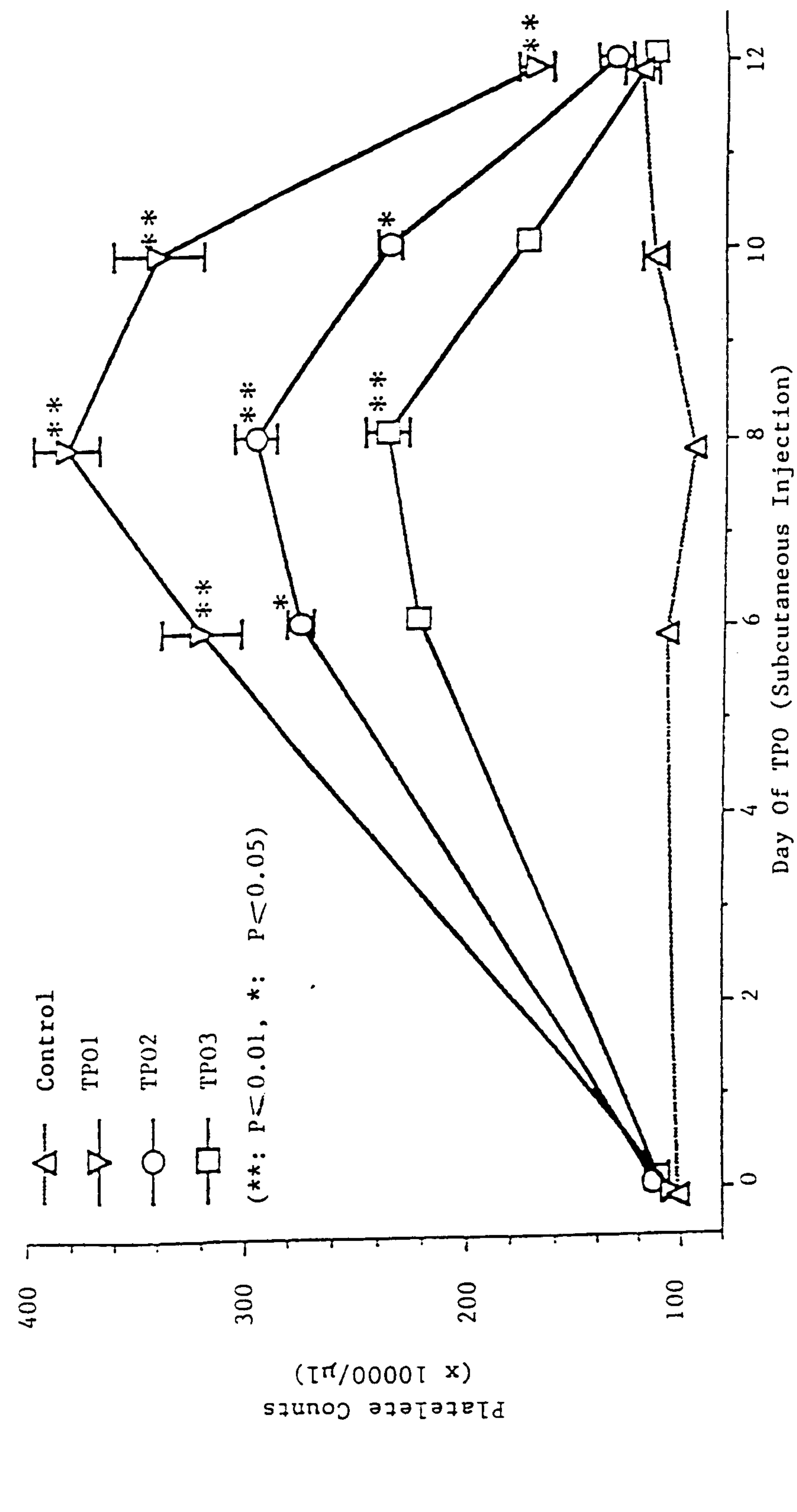

Control
TPO1
TPO2
TPO3
(**: P<0.01, *: P<0.05)
Platelete Counts
(x 10000/μl)
400
300
200
100
0
2
4
6
8
10
12
Day Of TPO (Subcutaneous Injection)

Figure 26

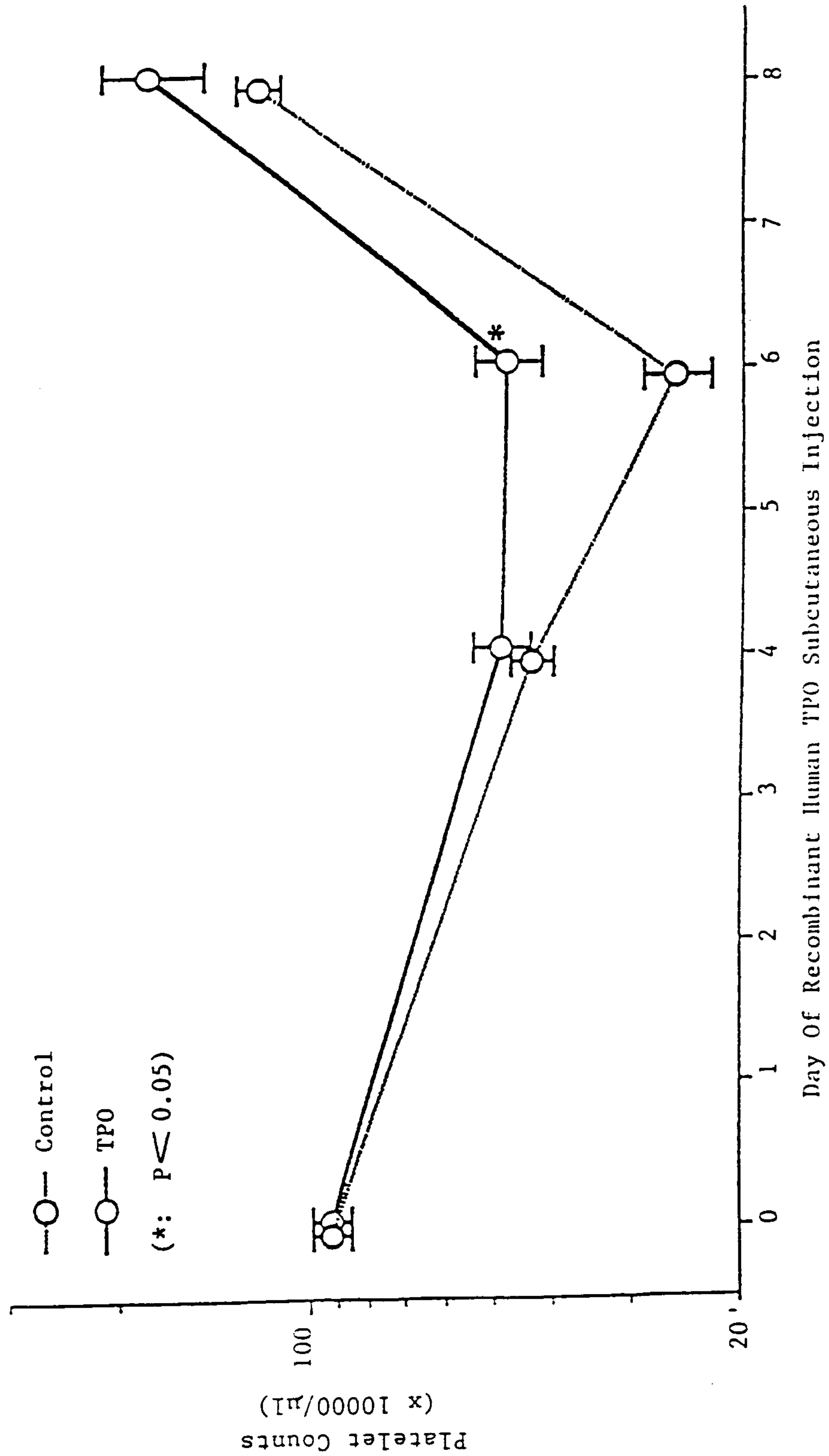
Control
TPO
(*: P< 0.05)
*
Platelet Counts
(x 10000/µl)
100
20
0
1
2
3
4
5
6
7
8
Day Of Recombinant Human TPO Subcutaneous Injection

Figure 27

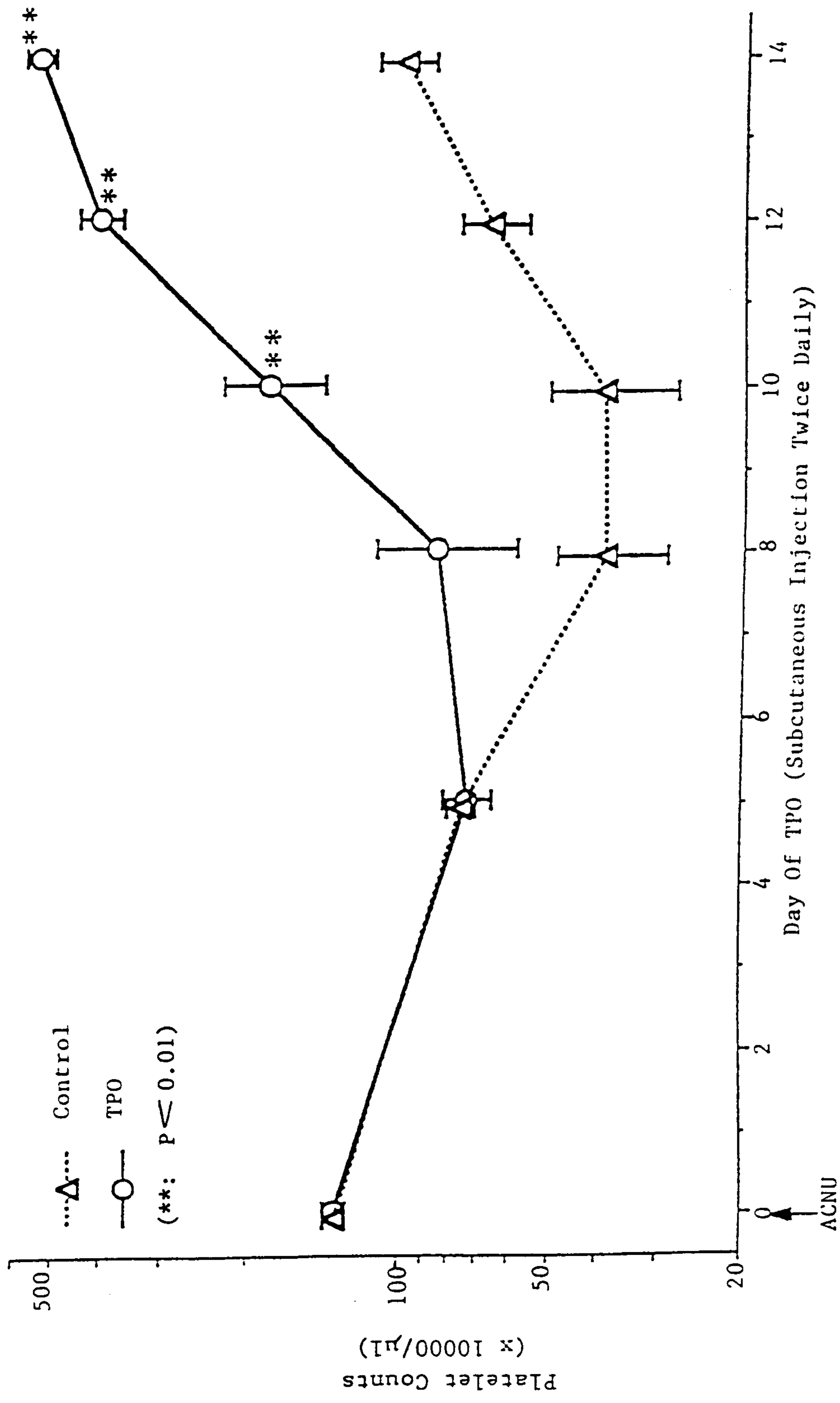

Control
TPO
(**: P < 0.01)
**
Platelet Counts
(x 10000/µl)
500
100
50
20
0
2
4
6
8
10
12
14
Day Of TPO (Subcutaneous Injection Twice Daily)
ACNU

Figure 28

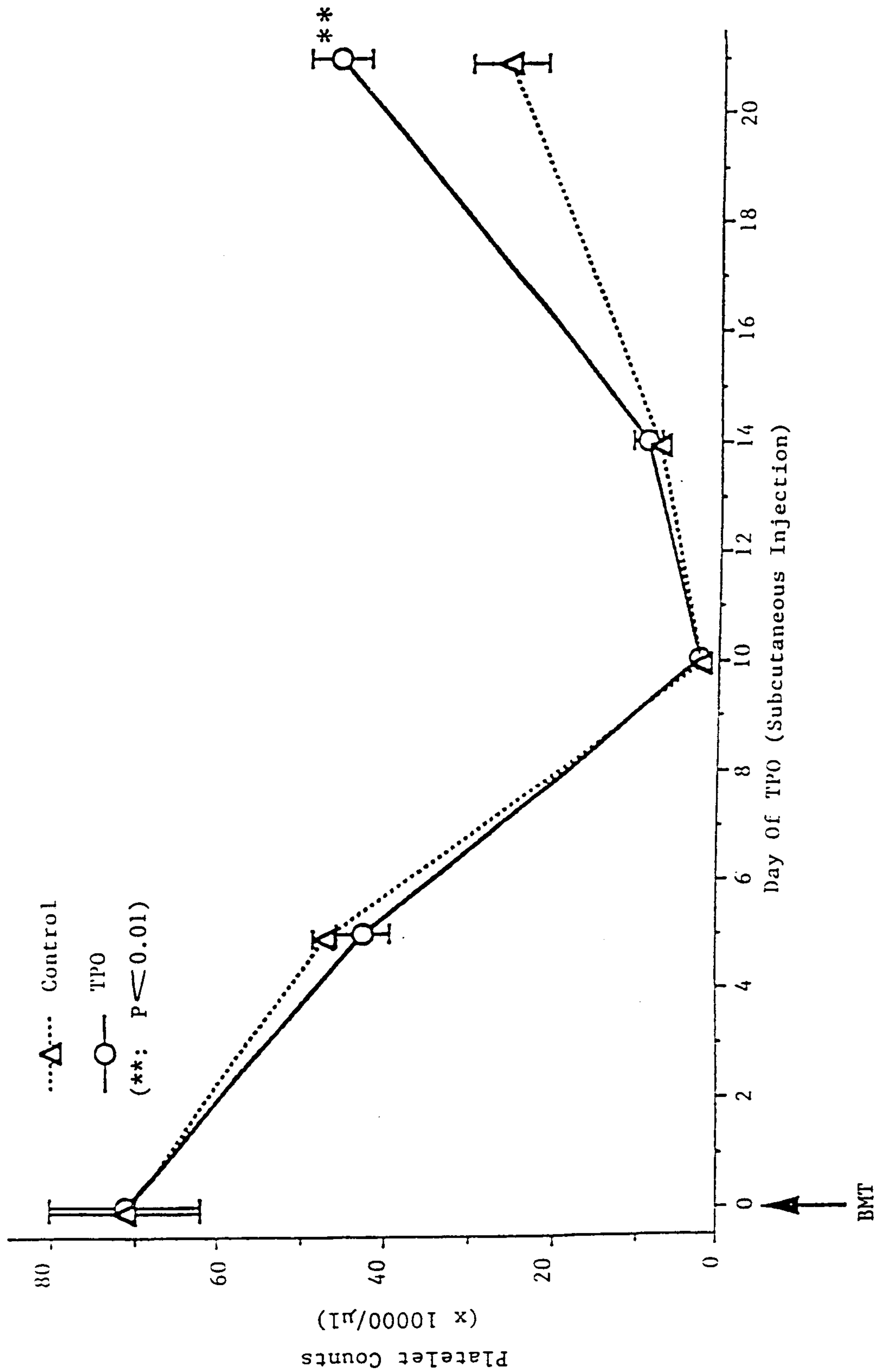
Control
TPO
(**; P<0.01)
**
Platelet Counts
(x 10000/μl)
80
60
40
20
0
0
2
4
6
8
10
12
14
16
18
20
Day Of TPO (Subcutaneous Injection)
BMT

Figure 29

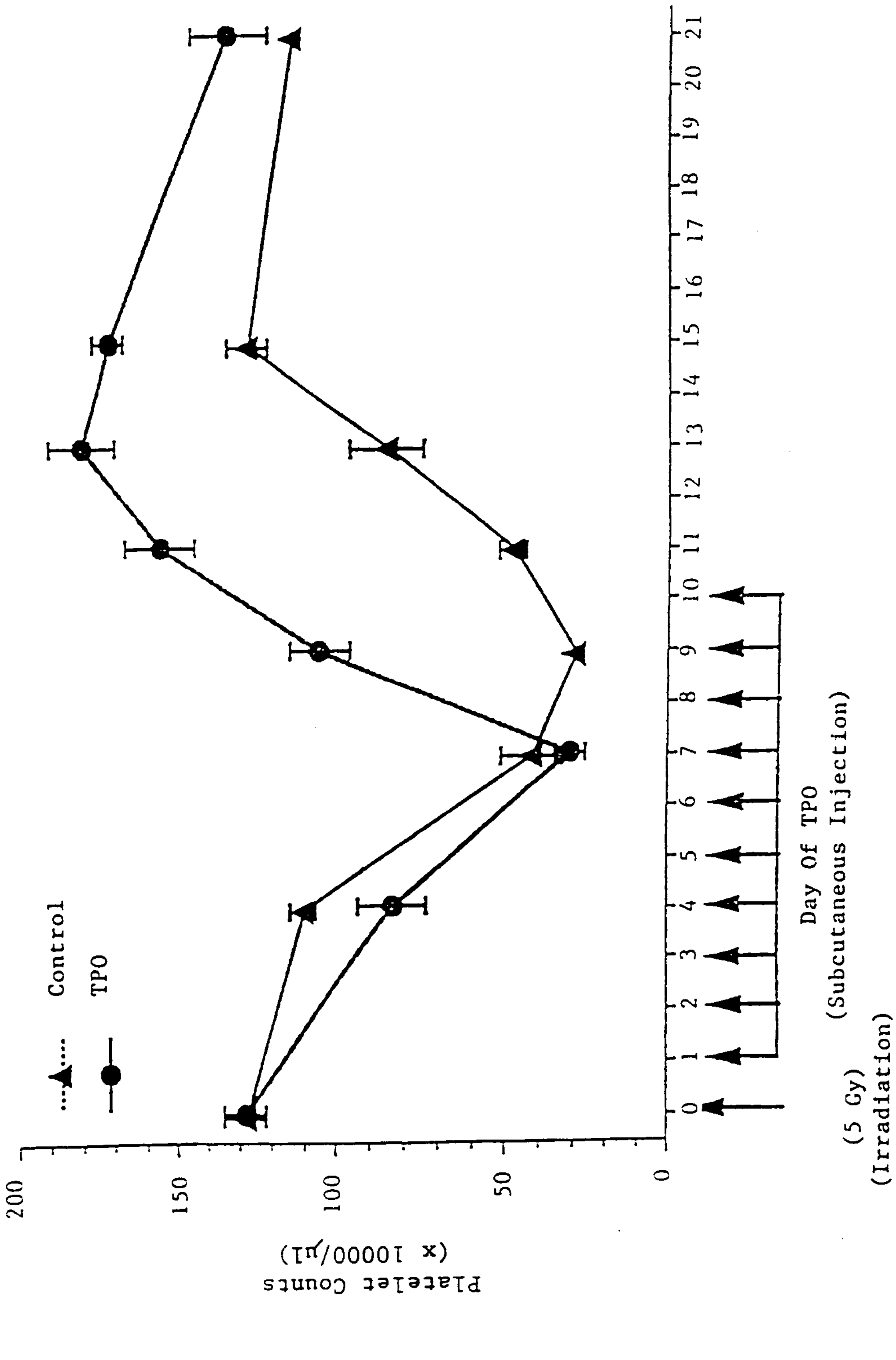
Control
TPO
Platelet Counts
(x 10000/μl)
200
150
100
50
0
0
1
2
3
4
5
6
7
8
9
10
11
12
13
14
15
16
17
18
19
20
21
Day Of TPO
(Subcutaneous Injection)
(5 Gy)
(Irradiation)

Figure 30

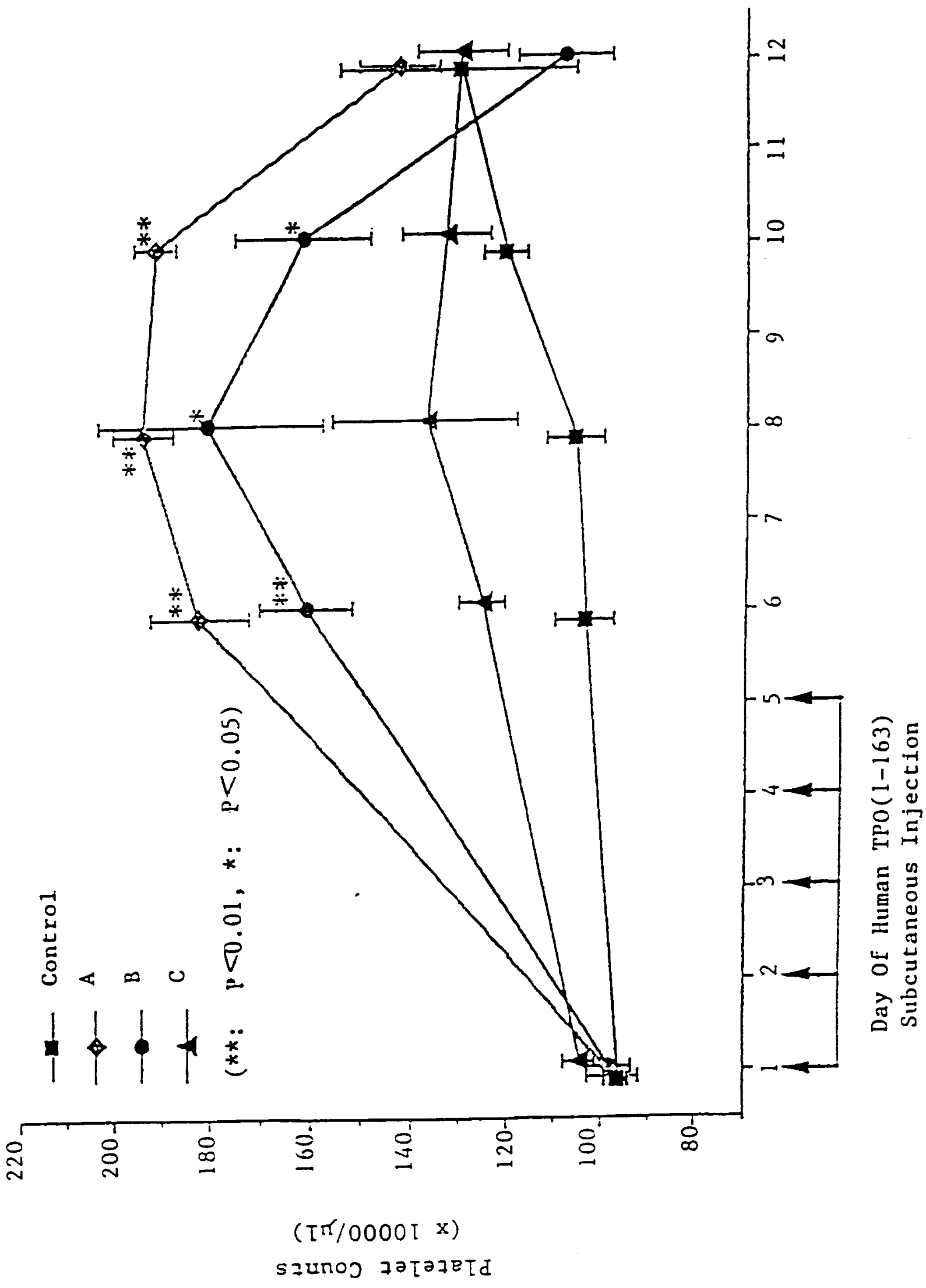

Control
A
B
C
(**: P<0.01, *: P<0.05)
Platelet Counts
(x 10000/μl)
220
200
180
160
140
120
100
80
1
2
3
4
5
6
7
8
9
10
11
12
Day Of Human TPO(1-163)
Subcutaneous Injection

Figure 31

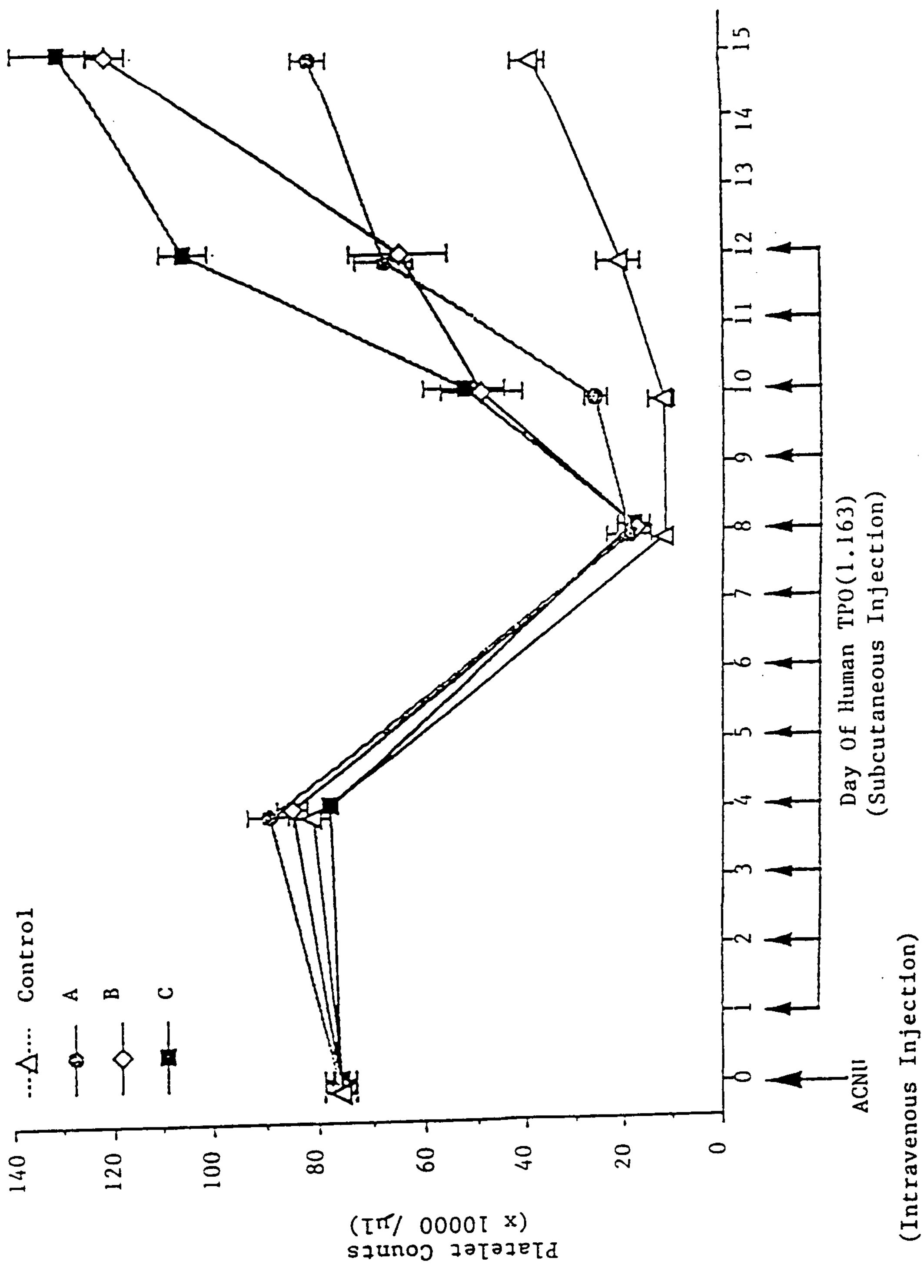
Control
A
B
C
Platelet Counts
(x 10000 /µl)
0
20
40
60
80
100
120
140
0
1
2
3
4
5
6
7
8
9
10
11
12
13
14
15
Day Of Human TPO(1.163)
(Subcutaneous Injection)
ACNU
(Intravenous Injection)

Figure. 32

MPL-X completely blocks the ability of APK9 to induce megakaryocyte development

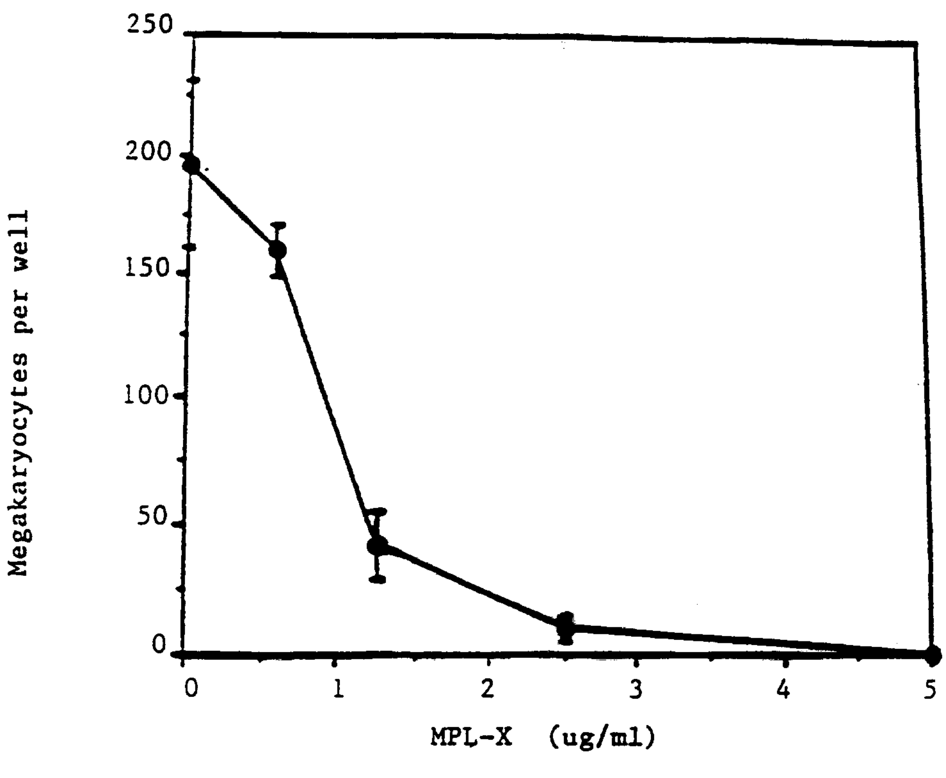

Figure 33

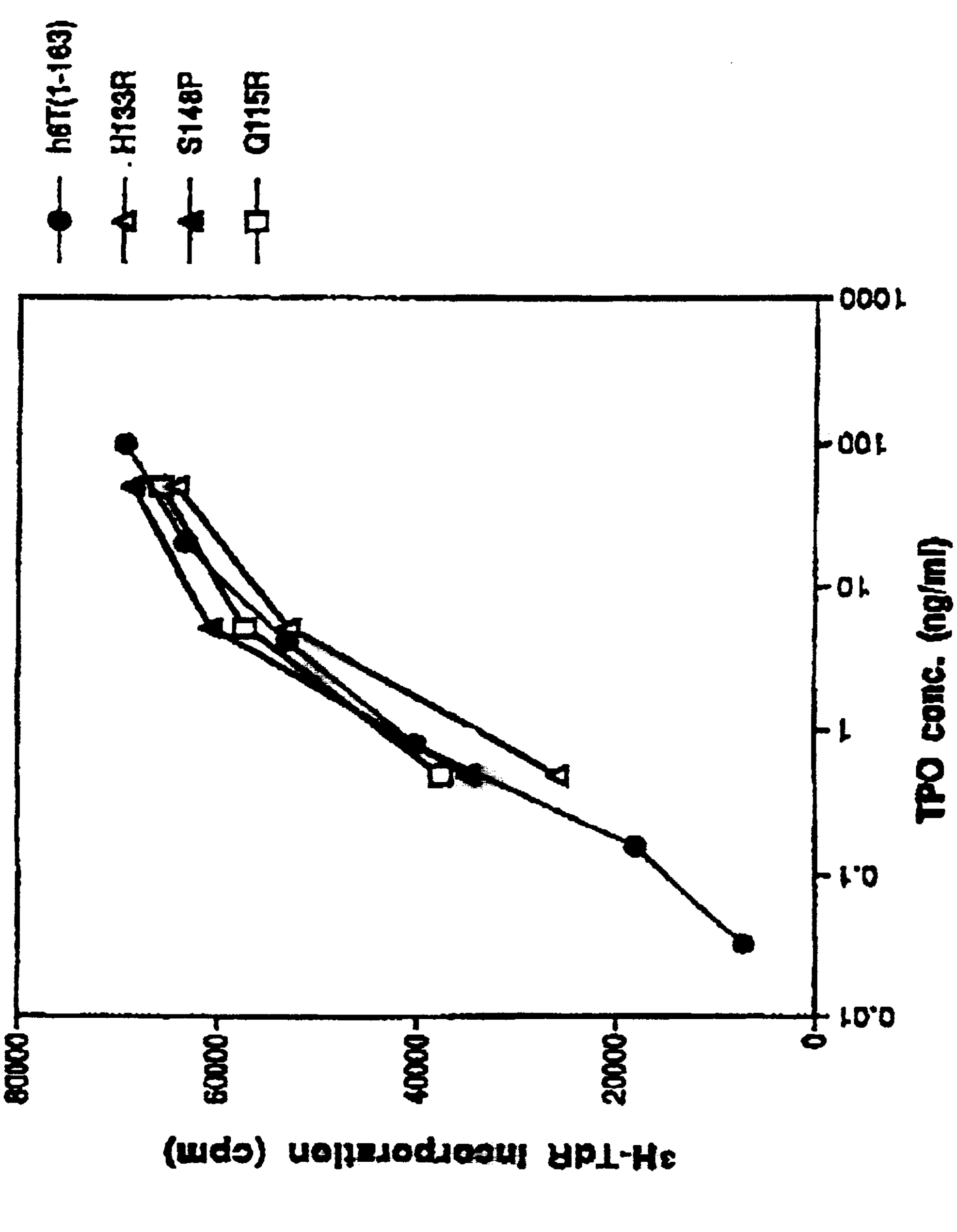
h6T(1-163)
H133R
S148P
Q115R
3H-TdR incorporation (cpm)
TPO conc. (ng/ml)
0
20000
40000
60000
80000
0.01
0.1
1
10
100
1000

Figure 34

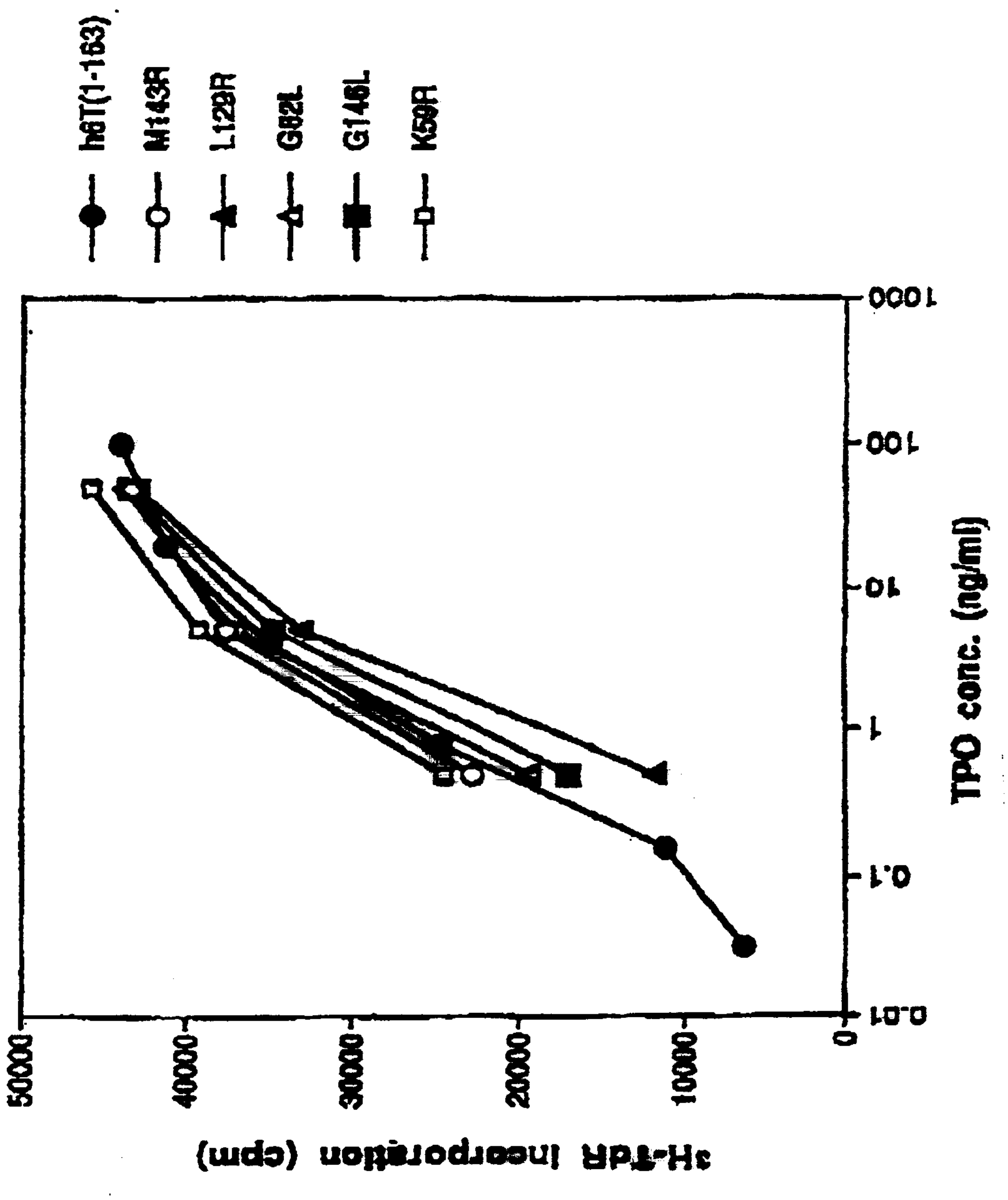

h6T(1-163)
M143R
L129R
G82L
G146L
K59R
0.01
0.1
1
10
100
1000
TPO conc. (ng/ml)
0
10000
20000
30000
40000
50000
3H-TdR incorporation (cpm)

European Patent Office

# EUROPEAN SEARCH REPORT

Application Number
EP 95 20 0385

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EXPERIMENTAL HEMATOLOGY, vol. 16,no. 3, March 1988 pages 201-205, MCDONALD 'THROMBOPOIETIN:ITS BIOLOGY,PURIFICATION,AND CHARACTERIZATION' * the whole document * --- | 1-40 | C12N15/19 C07K14/52 C07K16/24 A61K38/19 |
| P,X | FEBS LETTERS, vol. 353,no. 1, 10 October 1994 pages 57-61, SOHMA ET AL 'MOLECULAR CLONING AND CHROMOSOMAL LOCALIZATION OF THE HUMAN THROMBOPOIETIN GENE' * the whole document * --- | 1-10, 16-22, 29-37 | |
| D,P, X | NATURE, vol. 369, 16 June 1994 pages 533-538, DE SAUVAGE ET AL 'STIMULATION OF MEGAKARYOCYTOPOIESIS AND THROMBOPOIESIS BY THE C-MPL LIGAND' * the whole document * --- | 1-10, 16-22, 29-37 | TECHNICAL FIELDS SEARCHED (Int.Cl.6) |
| D,P, X | CELL, vol. 77, 1 July 1994 pages 1117-1124, BARTLEY ET AL 'IDENTIFICATION AND CLONING OF A MEGAKARYOCYTE GROWTH AND DEVLOPMENT FACTOR THAT IS A LIGAND FOR THE CYTOKINE RECEPTOR MPL' * the whole document * ----- | 1-10, 16-22, 29-40 | C07K |

The present search report has been drawn up for all claims

| Place of search | Date of completion of the search | Examiner |
|---|---|---|
| THE HAGUE | 22 May 1995 | Sitch, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)